# EUROPEAN PATENT SPECIFICATION

(11) **EP 1 947 184 B1**
(45) Date of publication and mention of the grant of the patent: **30.03.2011**
(21) Application number: 08005377.0
(22) Date of filing: 20.02.2004
(51) Int. Cl.: C12N 15/32, C12N 15/82, C12N 5/14, C12N 15/63, A01H 1/00, C07K 14/325, C07K 16/12, A01N 63/00

(54) **Delta-endotoxin genes and methods for their use**
Delta-Endotoxin-Gene und Verfahren zu ihrer Verwendung
Gènes delta-endotoxine et procédés d'utilisation

(30) Priority: 20.02.2003 US 448632 P; 20.02.2003 US 448633 P; 20.02.2003 US 448797 P; 20.02.2003 US 448806 P; 20.02.2003 US 448810 P; 20.02.2003 US 448812 P; 19.02.2004 US 782141; 19.02.2004 US 782096; 19.02.2004 US 781979; 19.02.2004 US 783417; 19.02.2004 US 782020; 19.02.2004 US 782570
(43) Date of publication of application: 23.07.2008
(62) Divisional of application: 04713453.1
(73) Proprietor: Athenix Corporation, Research Triangle Park, NC 27709 (US)
(72) Inventor: Carozzi, Nadine, Raleigh, NC 27615 (US); Hargiss, Tracy, Kernersville, NC 27284 (US); Koziel, Michael G., Raleigh, NC 27613 (US); Duck, Nicholas B., Apex, NC 27502 (US); Carr, Brian, Raleigh, NC 27613 (US)
(74) Representative: Power, David

(56) References cited:
- US-B1- 6 177 615
- SUE KALMAN ET AL.: "Cloning of a novel cryIC-type gene from a strain of Bacillus thurigiensis subsp. galleriae" APPLIED AND ENVIRONMENTAL MICROBIOLOGY, vol. 59, no. 4, April 1993 (1993-04), pages 1131-1137, XP008031966
- DATABASE EMBL [Online] 16 November 2001 (2001-11-16), "Bacillus thuringiensis serovar aizawai cry40A and 40orf2 genes for putative mosquitocidal toxin and Cry40ORF2 protein, partial and complete cds." XP002483175 retrieved from EBI accession no. EMBL:AB074414 Database accession no. AB074414
- ITO T ET AL: "Cloning and expression of novel crystal protein genes cry39A and 39orf2 from Bacillus thuringiensis subsp. aizawai Bun1-14 encoding mosquitocidal proteins" JOURNAL OF INSECT BIOTECHNOLOGY AND SERICOLOGY, JAPANESE SOCIETY OF SERICULTURAL SCIENCE, TSUKUBA, JP, vol. 71, no. 3, 1 October 2002 (2002-10-01), pages 123-128, XP008115282 ISSN: 1346-8073
- MARIE-LAURE ROSSO ET AL.: "Contribution of the 65-Kilodalton protein encoded by the cloned gene cry19A to the mosquitocidal activity of Bacillus thurigiensis subsp. jegathesan" APPLIED AND ENVIRONMENTAL MICROBIOLOGY, vol. 63, no. 11, November 1997 (1997-11), pages 4449-4455,
- R. TRACY ELLIS ET AL.: "Novel Bacillus thurigiensis binary insecticidal crystal proteins active on Western Corn Rootworm, Diabrotica virgifera LeConte" APPLIED AND ENIRONMENTAL MICROBIOLOGY, vol. 68, no. 3, March 2002 (2002-03), pages 1137-1145,
- IAN J. LENANE ET AL.: "A pair of adjacent genes, cry5Ad and orf2-5Ad, encode the typical N- and C-terminal regions of a Cry5Adelta-endotoxin as two separate proteins in Bacillus thurigiensis strain L366" FEMS MICROBIOL. LETT., 2007, pages 1-6,

## Description

### FIELD OF THE INVENTION

This invention relates to the field of molecular biology. Provided are novel genes that encode pesticidal proteins. These proteins and the nucleic acid sequences that encode them are useful in preparing pesticidal formulations and in the production of transgenic pest-resistant plants.

### BACKGROUND OF THE INVENTION

*Bacillus thuringiensis* is a Grram-positive spore forming soil bacterium characterized by its ability to produce crystalline inclusions that are specifically toxic to certain orders and species of insects, but are harmless to plants and other non-targeted organisms. For this reason, compositions including *Bacillus thuringiensis* strains or their insecticidal proteins can be used as environmentally acceptable insecticides to control agricultural insect pests or insect vectors for a variety of human or animal diseases.

Crystal (Cry) proteins (delta-endotoxins) from *Bacillus thuringiensis* have potent insecticidal activity against predominantly Lepidopteran, Dipteran, and Coleopteran larvae. These proteins also have shown activity against Hymenoptera, Homoptera, Phthiraptera, Mallophaga, and Acari pest orders, as well as other invertebrate orders such as Nemathelminthes, Platyhelminthes, and Sarcomastigorphora (Feitelson (1993) The Bacillus Thuringiensis family tree. In Advanced Engineered Pesticides. Marcel Dekker, Inc., New York, N.Y.) These proteins were originally classified as CryI to CryV based primarily on their insecticidal activity. The major classes were Lepidoptera-specific (I), Lepidoptera-and Diptera-specific (II), Coleoptera-specific (III), Diptera-specific (IV), and nematode-specific (V) and (VI). The proteins were further classified into subfamilies; more highly related proteins within each family were assigned divisional letters such as Cry1A, Cry1B, Cry1C, etc. Even more closely related proteins within each division were given names such as Cry1C1, Cry1C2, etc.

Ito et al (2002) Journal of Insect Biotechnology and Sericology, 71:123 to 128, is concerned with cloning and expression of novel crystal protein genes *cry39A* and *39 or ƒ2* from *Bacillus thuringiensis* subsp. *aizawai* Bun1-14 encoding mosquitocidal proteins.

A new nomenclature was recently described for the Cry genes based upon amino acid sequence homology rather than insect target specificity (Crickmore et al. (1998) Microbiol. Mol. Bio/. Rev. 62:807-813). In the new classification, each toxin is assigned a unique name incorporating a primary rank (an Arabic number), a secondary rank (an uppercase letter), a tertiary rank (a lowercase letter), and a quaternary rank (another Arabic number). In the new classification, Roman numerals have been exchanged for Arabic numerals in the primary rank. Proteins with less than 45% sequence identity have different primary ranks, and the criteria for secondary and tertiary ranks are 78% and 95%, respectively.

The crystal protein does not exhibit insecticidal activity until it has been ingested and solubilized in the insect midgut. The ingested protoxin is hydrolyzed by proteases in the insect digestive tract to an active toxic molecule. (Höfte and Whiteley (1989) Microbiol. Rev. 53:242-255). This toxin binds to apical brush border receptors in the midgut of the target larvae and inserts into the apical membrane creating ion channels or pores, resulting in larval death.

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in 'jelly-roll' formation (de Maagd *et al.* (2001) supra). Domains II and in are involved in receptor recognition and binding, and are therefore considered determinants of toxin specificity.

Because of the devastation that insects can confer, there is a continual need to discover new forms of *Bacillus thuringiensis* delta-endotoxins.

### SUMMARY OF INVENTION

Compositions and methods for conferring pesticide resistance to bacteria, plants, plant cells, tissues, and seeds are provided. Compositions include isolated nucleic acid molecules encoding sequences for delta-endotoxin and delta-endotoxin-associated polypeptides, vectors comprising those nucleic acid molecules, and host cells comprising the vectors. Compositions also include isolated or recombinant polypeptide sequences of the endotoxin, compositions comprising these polypeptides, and antibodies to those polypeptides. The nucleotide sequences can be used in DNA constructs or expression cassettes for transformation and expression in organisms, including microorganisms and plants. The nucleotide or amino acid sequences may be synthetic sequences that have been designed for optimum expression in an organism, including, but not limited to, a microorganism or a plant. Compositions also comprise transformed bacteria, plants, plant cells, tissues, and seeds.

In particular, the present invention provides for isolated nucleic acid molecules comprising a nucleotide sequence encoding an amino acid sequence shown in SEQ ID NO: 14, 16 or 18, or a nucleotide sequence set forth in SEQ ID NO: 12, 13 or 15 or 28, as well as variants and fragments thereof. Nucleotide sequences that are complementary to a nucleotide sequence of the invention, or that hybridize to a sequence of the invention, are also encompassed.

Methods are provided for producing the polypeptides of the invention, and for using those polypeptides for controlling or killing a lepidopteran or coleopteran pest

The compositions and methods of the invention are useful for the production of organisms with pesticide resistance, specifically bacteria and plants. These organisms and compositions derived from them are desirable for agricultural purposes. The compositions of the invention are also useful for generating altered or improved delta-endotoxin or delta-endotoxin-associated proteins that have pesticidal activity, or for detecting the presence of delta-endotoxin or delta-endotoxin-associated proteins or nucleic acids in products or organisms.

### DESCRIPTION OF FIGURES

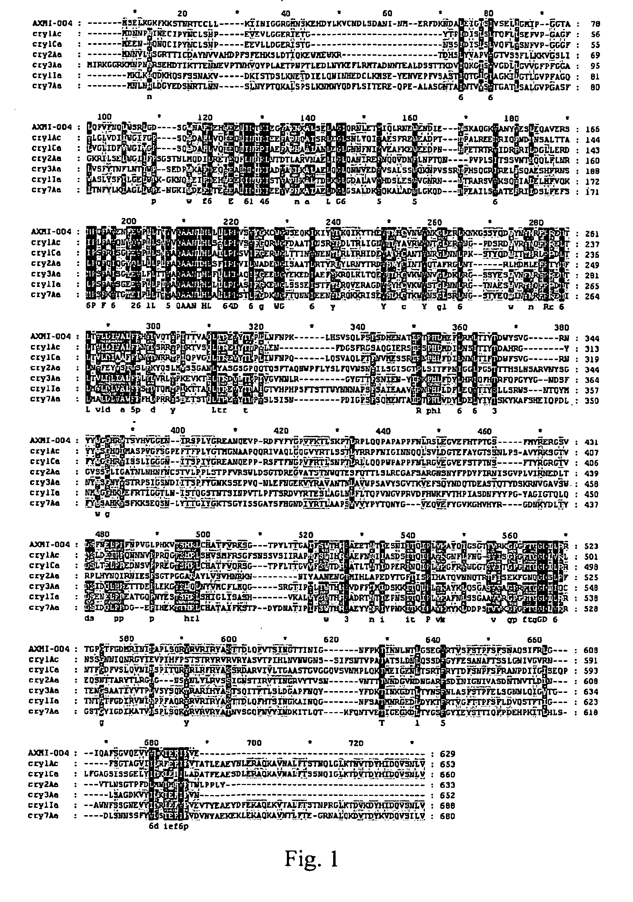

Figure.1 shows an alignment of AXMI-004 (SEQ ID NO:3) with cry1Ac (SEQ ID NO:31), cry1Ca (SEQ ID NO:32), cry2Aa (SEQ ID NO:34), cry3Aa1 (SEQ ID NO:35), cry1Ia (SEQ ID NO:33), and cry7Aa (SEQ ID NO:41). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 174 to about 196 of SEQ ID NO:3. Conserved group 2 is found from about amino acid residue 250 to about 292 of SEQ ID NO:3. Conserved group 3 is found from about amino acid residue 476 to about 521 of SEQ ID NO:3. Conserved group 4 is found from about amino acid residue 542 to about 552 of SEQ ID NO:3. Conserved group 5 is found from about amino acid residue 618 to about 628 of SEQ ID NO:3

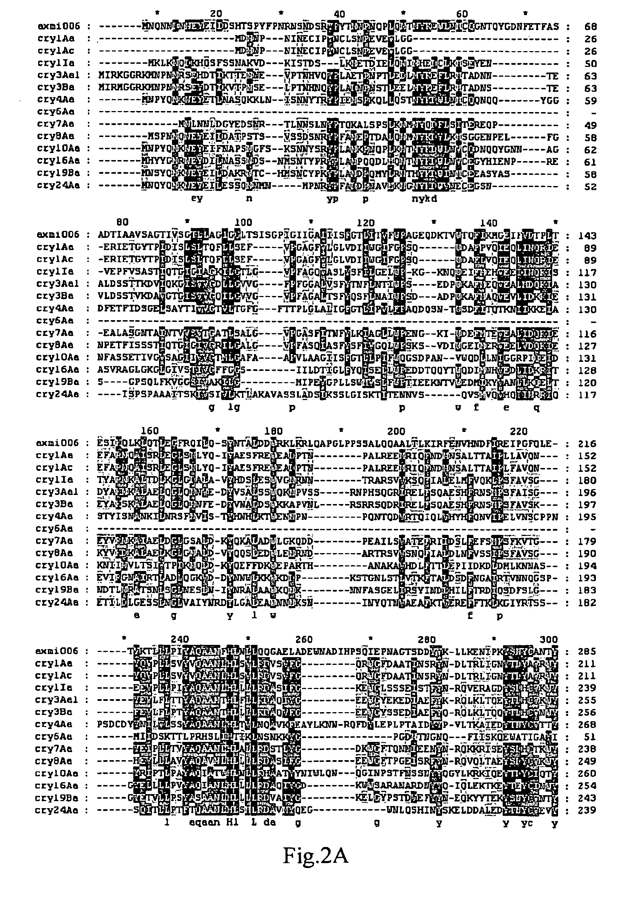

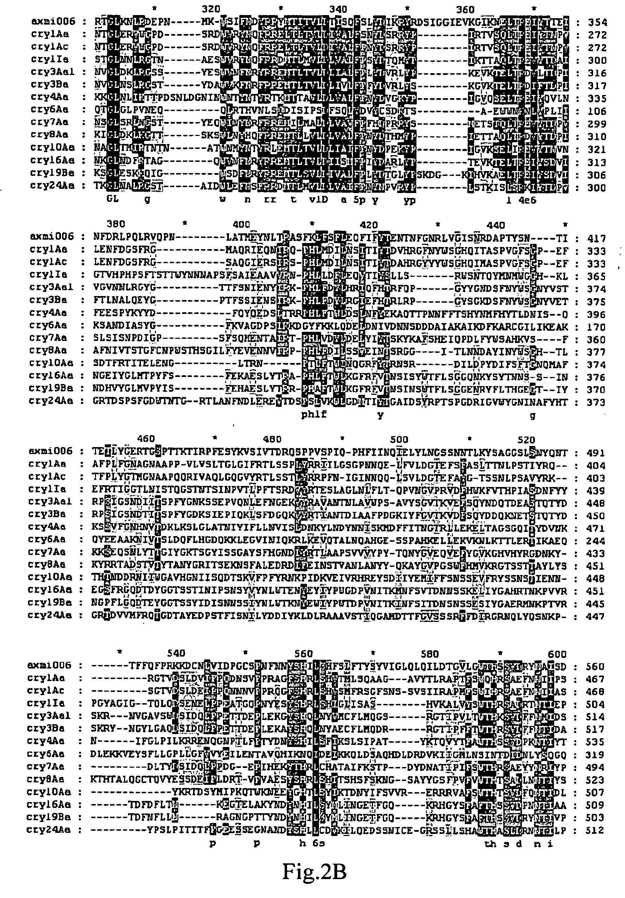

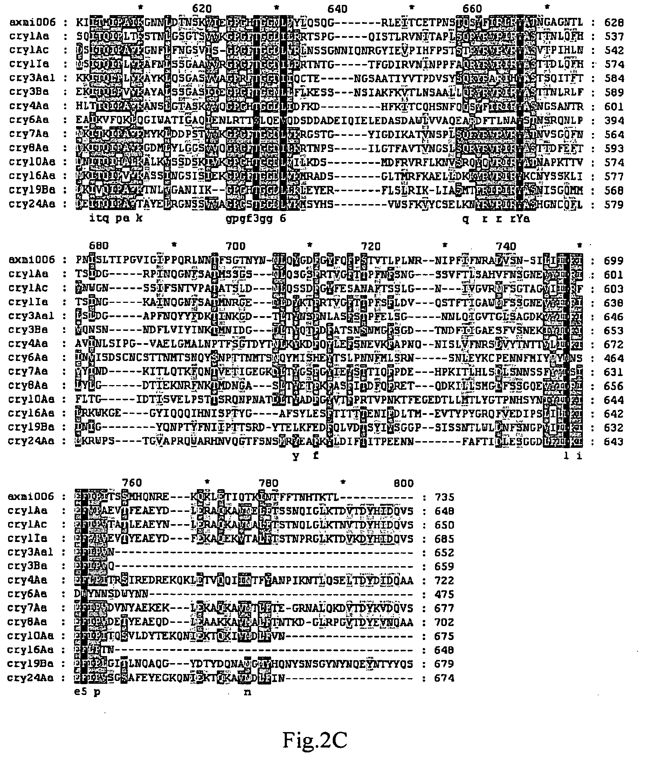

Figures 2A, B, and C show an alignment of AXMI-006 (SEQ ID NO:7) with cry1Aa (SEQ ID NO:30), cry1Ac (SEQ ID NO:31), cry1Ia (SEQ ID NO:33), cry3Aa1 (SEQ ID NO:35), cry3Ba (SEQ ID NO:36), cry 4Aa (SEQ ID NO:38), cry6Aa (SEQ ID NO:40), cry7Aa (SEQ ID NO:41), cry8Aa (SEQ ID NO:42), cry10Aa (SEQ ID NO:43), cry16Aa (SEQ ID NO:44), cry19Ba (SEQ ID NO:45), and cry24Aa (SEQ ID NO:47). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 218 to about 239 of SEQ ID NO:7. Conserved group 2 is found from about amino acid residue 300 to about 350 of SEQ ID NO:7. Conserved group 3 is found from about amino acid residue 547 to about 592 of SEQ ID NO:7. Conserved group 4 is found from about amino acid residue 611 to about 621 of SEQ ID NO:7. Conserved group 5 is found from about amino acid residue 694 to about 704 of SEQ ID NO:7.

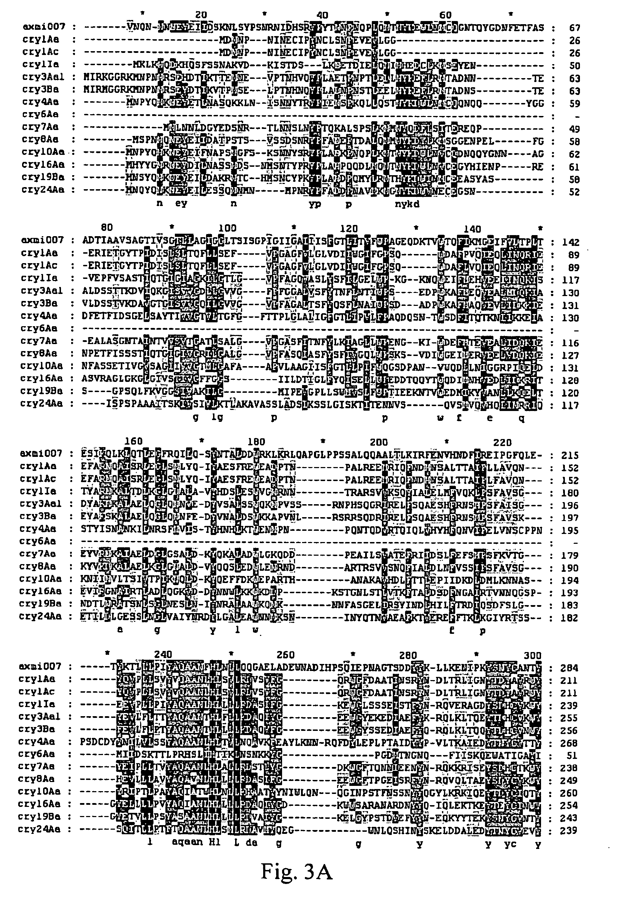

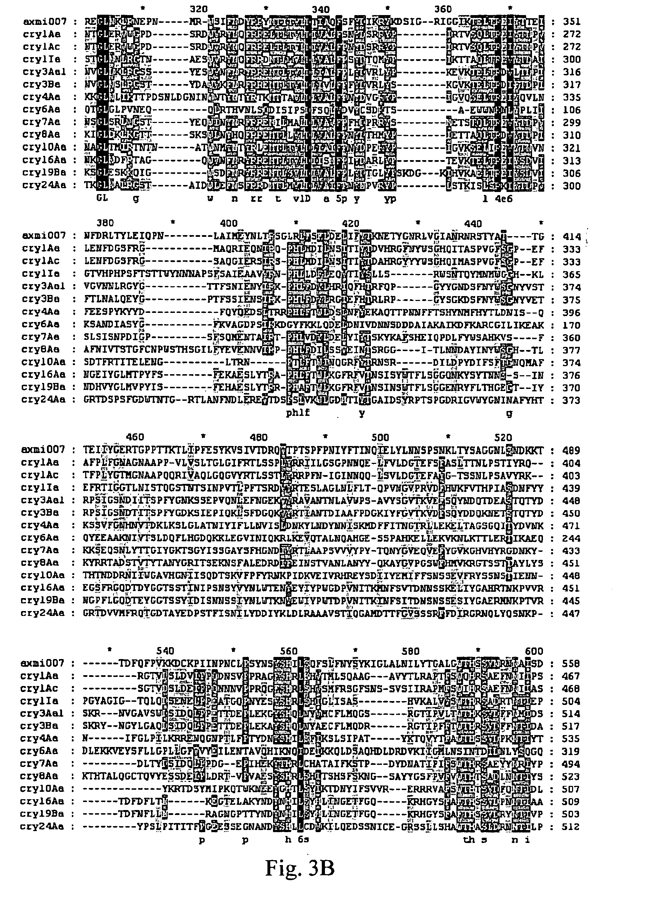

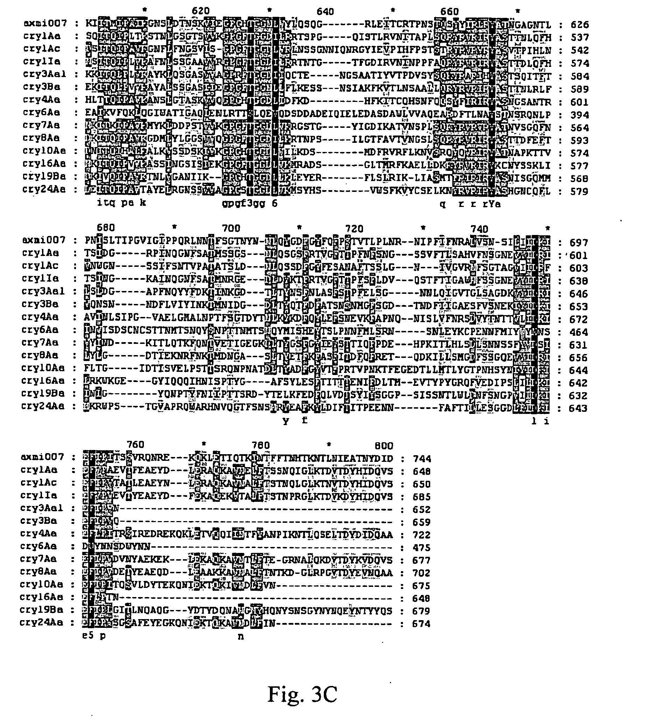

Figures 3A, B, and C show an alignment of AXMI-007 (SEQ ID NO:9) with cry1Aa (SEQ ID NO:30), cry1Ac (SEQ ID NO:31), cry1Ia (SEQ ID NO:33), cry3Aa1 (SEQ ID NO:35), cry3Ba (SEQ ID NO:36), cry 4Aa (SEQ ID NO:38), cry6Aa (SEQ ID NO:40), cry7Aa (SEQ ID NO:41), cry8Aa (SEQ ID NO:42), cry10Aa. (SEQ ID NO:43), cry16Aa (SEQ ID NO:44), cry19Ba (SEQ ID NO:45), and cry24Aa (SEQ ID NO:47). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 217 to about 238 of SEQ ID NO:9. Conserved group 2 is found from about amino acid residue 299 to about 347 of SEQ ID NO:9. Conserved group 3 is found from about amino acid residue 445 to about 590 of SEQ ID NO:9. Conserved group 4 is found from about amino acid residue 609 to about 619 of SEQ ID NO:9. Conserved group 5 is found from about amino acid residue 692 to about 702 of SEQ ID NO:9.

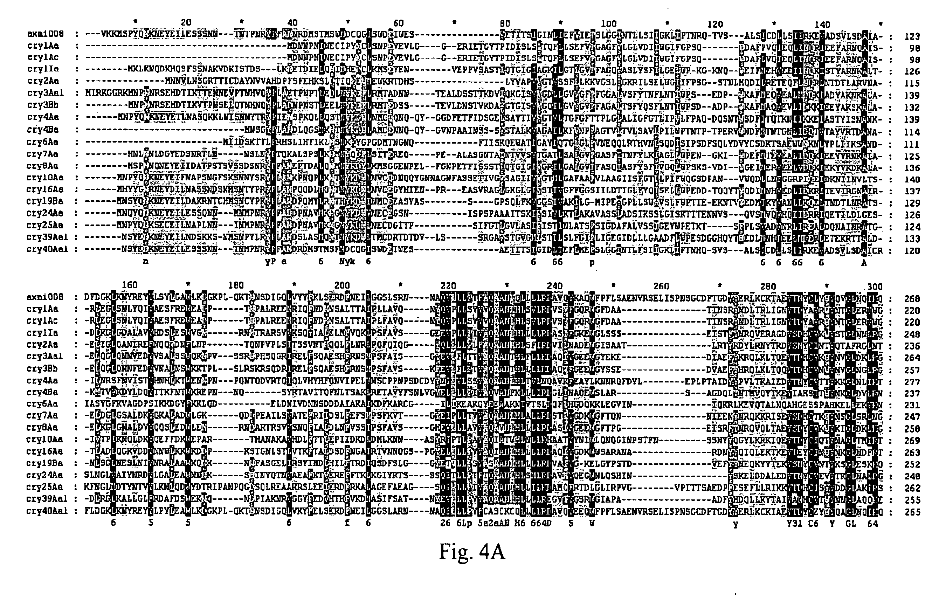

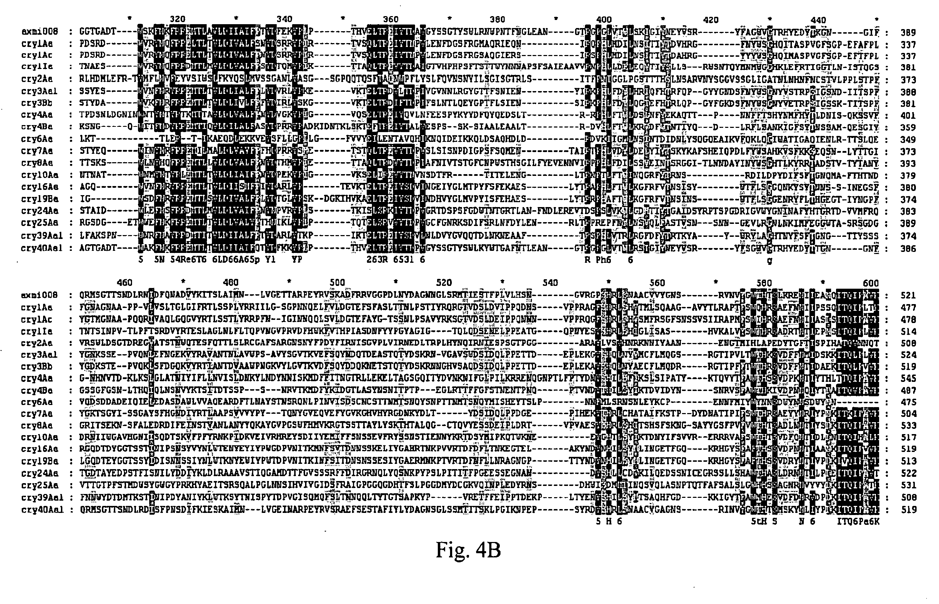

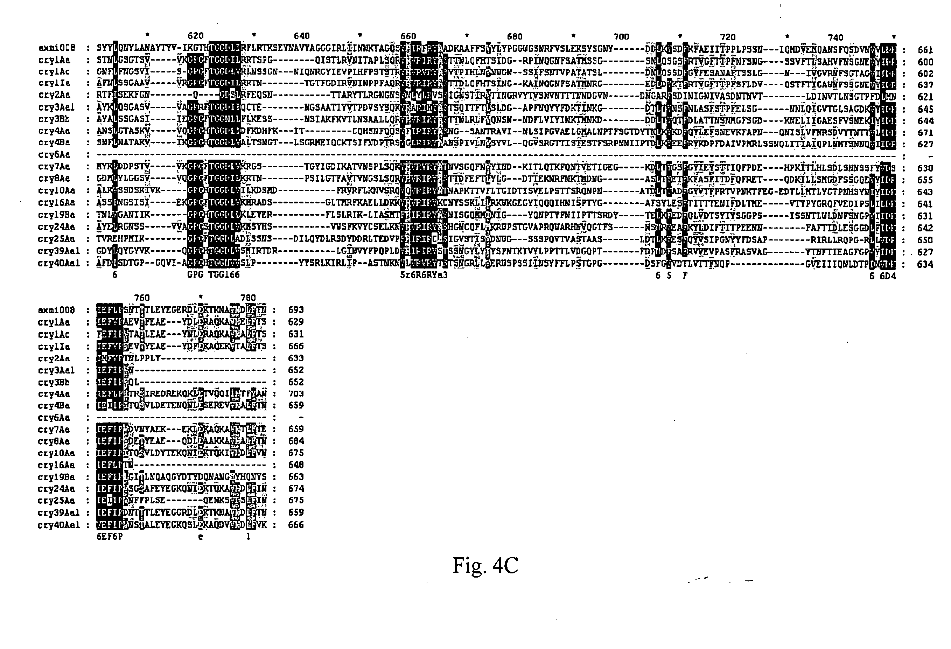

Figures 4A, B, and C show an alignment of AXMI-008 (SEQ ID NO:14) with cry1Aa (SEQ ID NO:30), cry1Ac (SEQ ID NO:31), cry1Ia (SEQ ID NO:33), cry2Aa (SEQ ID NO:34), cry3Aa1 (SEQ ID NO:35), cry3Bb (SEQ ID NO:37), cry4Aa (SEQ ID NO:38), cry4Ba (SEQ ID NO:39), cry6Aa (SEQ ID NO:40), cry7Aa (SEQ ID NO:41), cry8Aa (SEQ ID NO:42), cry10Aa (SEQ ID NO:43), cry16Aa (SEQ ID NO:44), cry19Ba (SEQ ID NO:45), cry24Aa (SEQ ID NO:47), cry25Aa (SEQ ID NO:48), cry39Aa1 (SEQ ID NO:49), and cry40Aa1 (SEQ ID NO:51). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 185 to about 206 of SEQ ID NO:14. Conserved group 2 is found from about amino acid residue 276 to about 318 of SEQ ID NO:14. Conserved group 3 is found from about amino acid residue 497 to about 547 of SEQ ID NO:14. Conserved group 4 is found from about amino acid residue 576 to about 586 of SEQ ID NO:14. Conserved group 5 is found from about amino acid residue 657 to about 667 of SEQ ID NO:14.

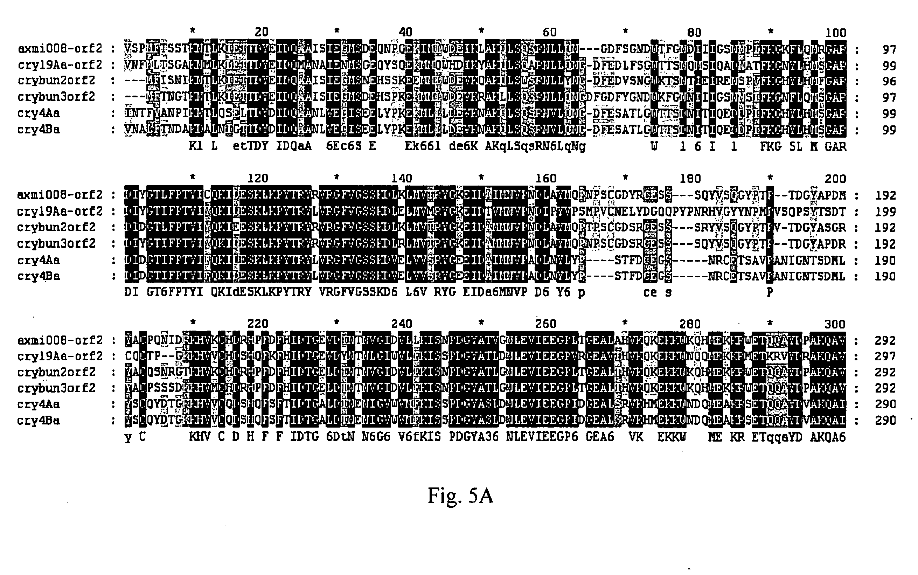

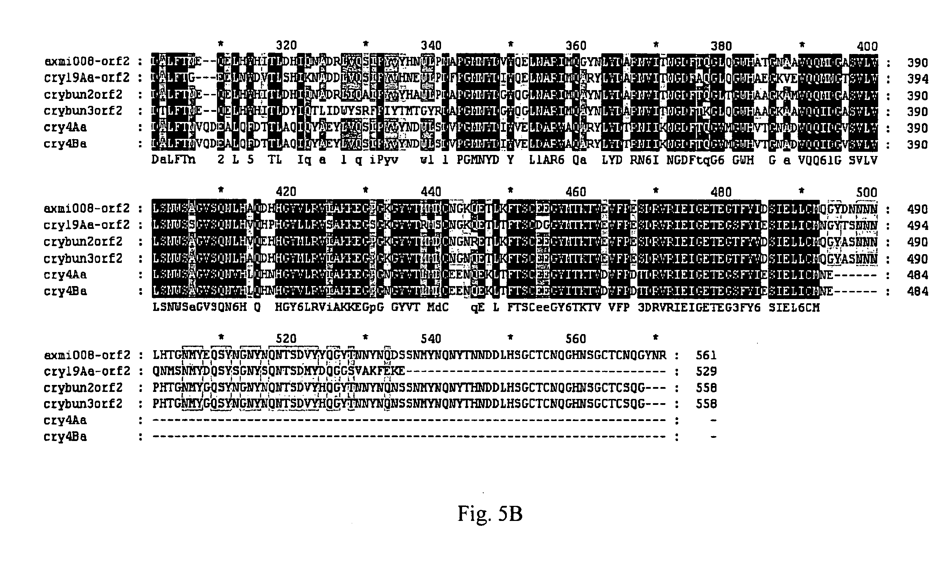

Figures 5A and B show an alignment of AXMI-008orf2 (SEQ ID NO:18) with cry19Aa-orf2 (SEQ ID NO:46), crybun2-orf2 (SEQ ID NO:50), crybun3-orf2 (SEQ ID NO:52), cry4Aa (SEQ ID NO:38), and cry4Ba (SEQ ID NO:39). The alignment shows the most highly conserved amino acid residues highlighted in black, and highly conserved amino acid residues highlighted in gray.

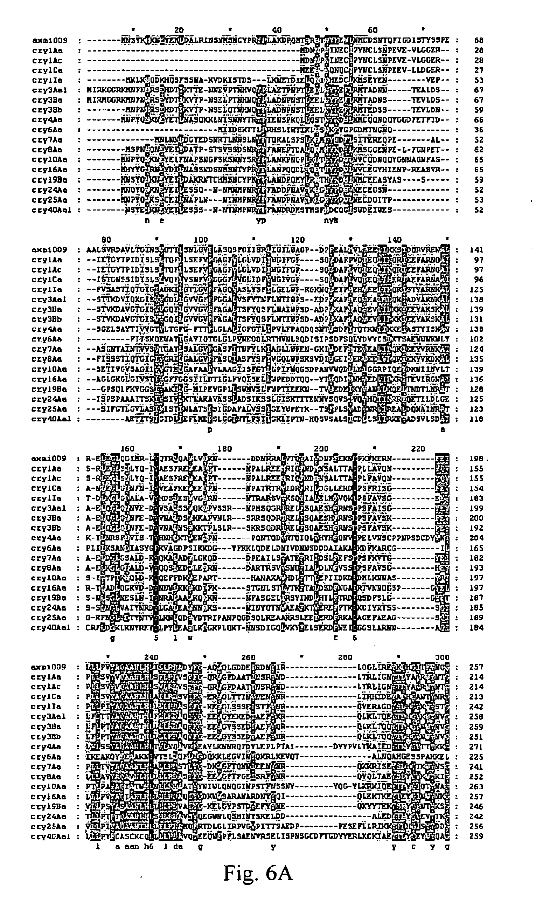

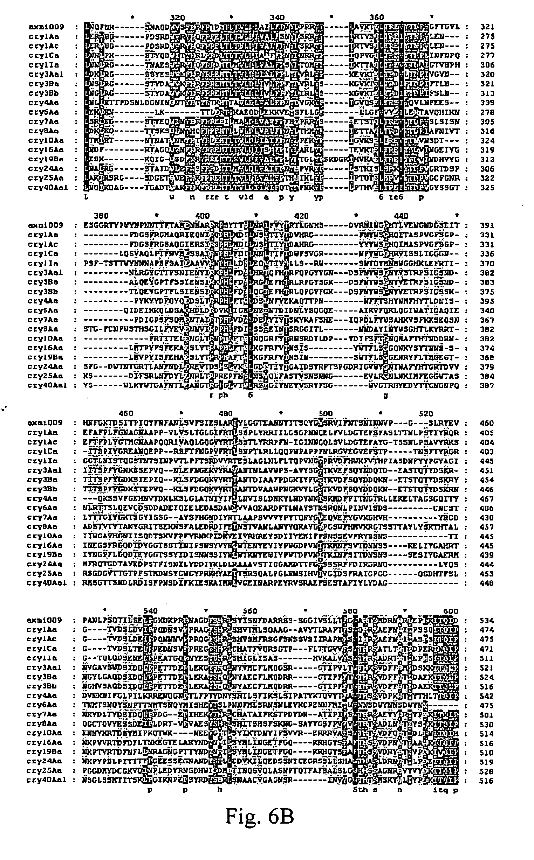

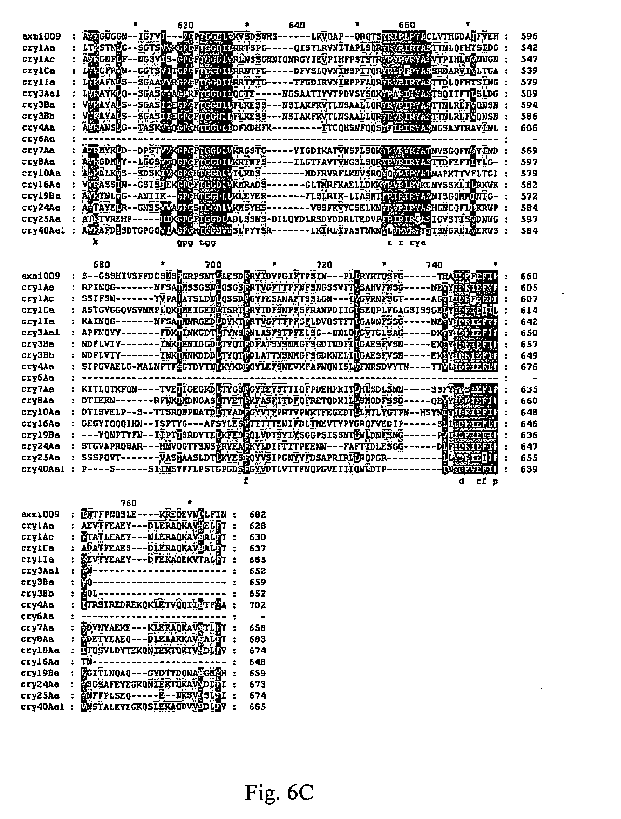

Figures 6A, B, and C show an alignment of AXMI-009 (SEQ ID NO:20) with cry1Aa (SEQ ID NO:30), cry1Ac (SEQ ID NO:31), cry1Ca (SEQ ID NO:32), cry1Ia (SEQ ID NO:33), cry3Aa1 (SEQ ID NO:35), cry3Ba (SEQ ID NO:36), cry3Bb (SEQ ID NO:37), cry4Aa (SEQ ID NO:38), cry6Aa (SEQ ID NO:40), cry7Aa (SEQ ID NO:41), cry8Aa (SEQ ID NO:42), cry10Aa (SEQ ID NO:43), cry16Aa (SEQ ID NO:44), cry19Ba (SEQ ID NO:45), cry24Aa (SEQ ID NO:47), cry25Aa (SEQ ID NO:48), cry40Aa1 (SEQ ID NO:51). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 196 to about 217 of SEQ ID NO:20. Conserved group 2 is found from about amino acid residue 269 to about 311 of SEQ ID NO:20. Conserved group 3 is found from about amino acid residue 514 to about 556 of SEQ ID NO:20. Conserved group 4 is found from about amino acid residue 574 to about 584 of SEQ ID NO:20. Conserved group 5 is found from about amino acid residue 651 to about 661 of SEQ ID NO:20.

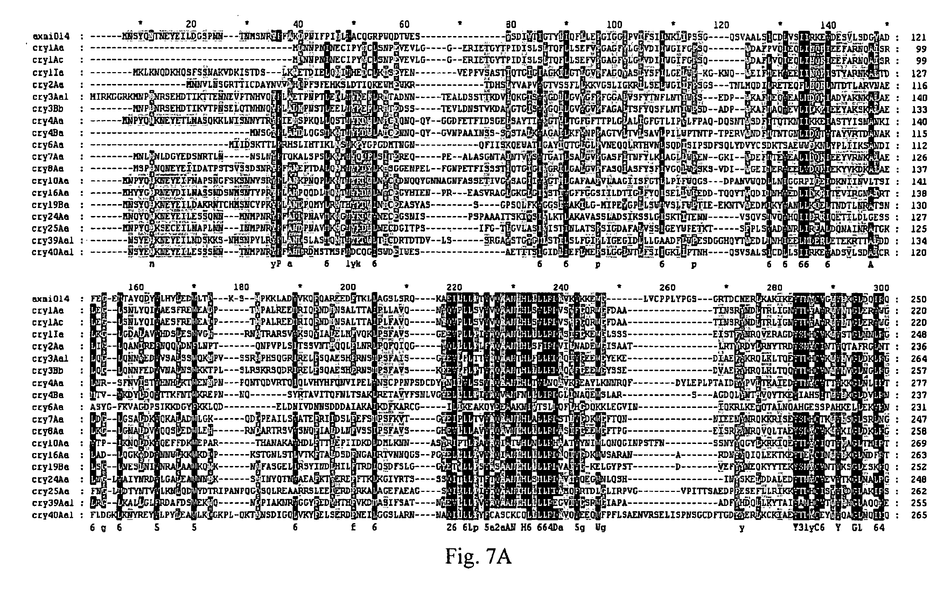

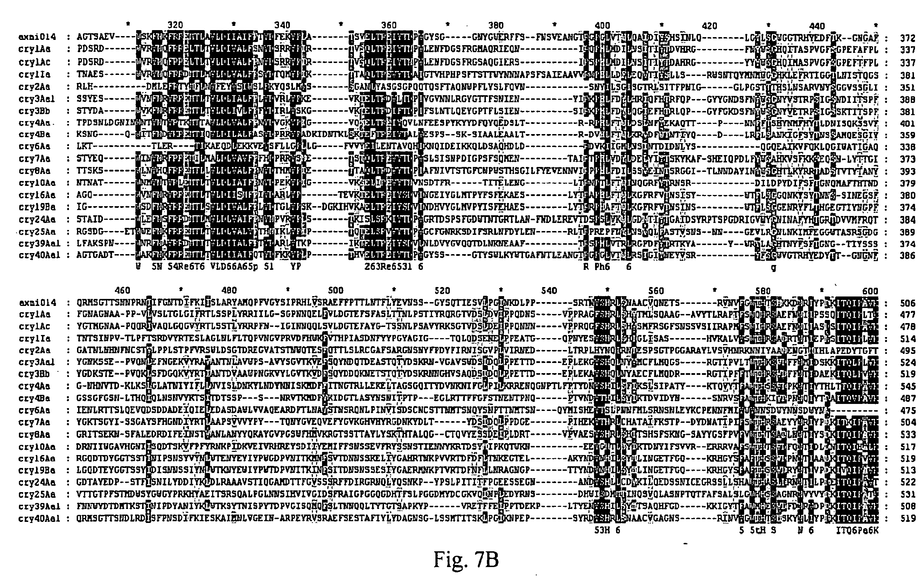

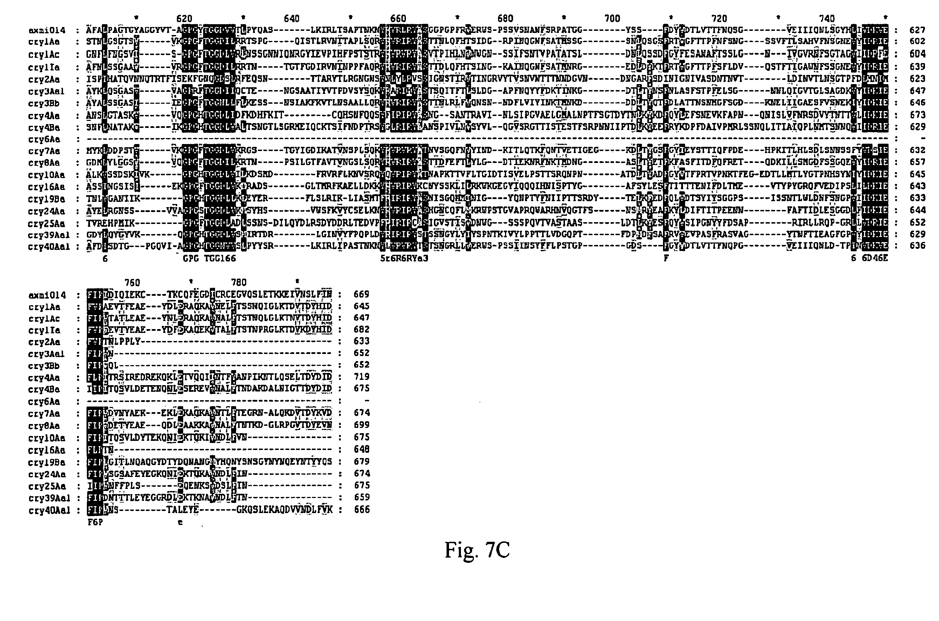

Figures 7A, B, and C show an alignment of AXMI-014 (SEQ ID NO:27) with cry1Aa (SEQ ID NO:30), cry1Ac (SEQ ID NO:31), cry1Ia (SEQ ID NO:33), cry2Aa (SEQ ID NO:34), cry3Aal (SEQ ID NO:35), cry3Bb (SEQ ID NO:37), cry4Aa (SEQ ID NO:38), cry4Ba (SEQ ID NO:39), cry6Aa (SEQ ID NO:40), cry7Aa (SEQ ID NO:41), cry8Aa (SEQ ID NO:42), cry10Aa (SEQ ID NO:43), cry16Aa (SEQ ID NO:44), cry19Ba (SEQ ID NO:45), cry24Aa (SEQ ID NO:47), cry25Aa (SEQ ID NO:48), cry39Aa1 (SEQ ID NO:49), and cry40Aa1 (SEQ ID NO:51). Toxins having C-terminal non-toxic domains were artificially truncated as shown. The alignment shows the most highly conserved amino acid residues highlighted in black, and highly conserved amino acid residues highlighted in gray. Conserved group 1 is found from about amino acid residue 177 to about 188 of SEQ ID NO:27. Conserved group 2 is found from about amino acid residue 251 to about 293 of SEQ ID NO:27. Conserved group 3 is found from about amino acid residue 483 to about 533 of SEQ ID NO:27. Conserved group 4 is found from about amino acid residue 552 to about 562 of SEQ ID NO:27.

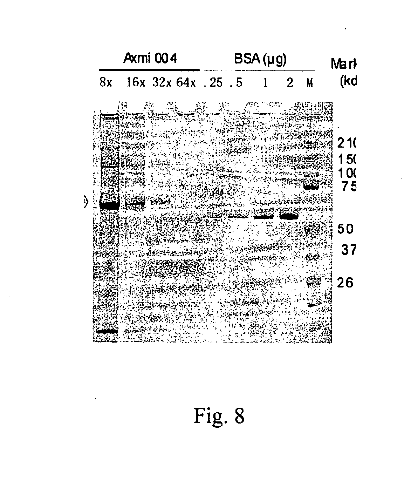

Figure 8 shows a photograph of a 4-20% gradient SDS acrylamide gel. Lanes 1-4 contain various concentrations of sporulated *Bacillus* cell culture expressing 69 kD AXMI-004 protein. Lanes 5-8 contain various concentrations of BSA. Lane 9 contains a size marker. An arrow indicates the 69 kD band.

### DETAILED DESCRIPTION

The present invention is drawn to compositions and methods for regulating pest resistance in organisms, particularly plants or plant cells. The methods involve transforming organisms with a nucleotide sequence encoding a delta-endotoxin or delta-endotoxin-associated protein of the invention. In particular, the nucleotide sequences of the invention are useful for preparing plants and microorganisms that possess pesticidal activity. Thus, transformed bacteria, plants, plant cells, plant tissues and seeds are provided. Compositions are delta-endotoxin or delta-endotoxin-associated nucleic acids and proteins of *Bacillus thuringiensis.* The sequences find use in the construction of expression vectors for subsequent transformation into organisms of interest, as probes for the isolation of other delta-endotoxin or delta-endotoxin-associated genes, and for the generation of altered pesticidal proteins by methods known in the art, such as domain swapping or DNA shuffling. The proteins find use in controlling or killing lepidopteran or coleopteran pest populations and for producing compositions with pesticidal activity.

### Definitions

By "delta-endotoxin" is intended a toxin from *Bacillus thuringiensis* that has toxic activity against one or more pests, including, but not limited to, members of the Lepidoptera, Diptera, and Coleoptera orders. In some cases, delta-endotoxin proteins have been isolated from other organisms, including *Clostridium bifermentans* and *Paenibacillus popilliae.* Delta-endotoxin proteins include amino acid sequences deduced from the full-length nucleotide sequences disclosed herein, and amino acid sequences that are shorter than the full-length sequences, either due to the use of an alternate downstream start site, or due to processing that produces a shorter protein having pesticidal activity. Processing may occur in the organism the protein is expressed in, or in the pest after ingestion of the protein. Delta-endotoxins include proteins identified as cry1 through cry43, cyt1 and cyt2, and Cyt-like toxin. There are currently over 250 known species of delta-endotoxins with a wide range of specificities and toxicities. For an expansive list see Crickmore et al. (1998), Microbiol. Mol. Biol. Rev. 62:807-813, and for regular updates see Crickmore et al. (2003) "Bacillus thuringiensis toxin nomenclature," at www.biols.susx.ac.uk/Home/Neil_Crickmore/Bt/index.

Bacterial genes, such as the AXMI genes quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. Furthermore, it is not often determined *a priori* which of these codons are used naturally in the bacterium. Thus, it is understood that use of one of the alternate methionine codons may also lead to generation of delta-endotoxin proteins that encode pesticidal activity. For example, an alternate start site for an AXMI-004 delta-endotoxin protein is at base pair 385 of SEQ ID NO:1. Translation from this alternate start site results in the amino acid sequence found in SEQ ID NO:5. An alternate start site for an AXMI-007 delta-endotoxin protein may be at base pair 151 of SEQ ID NO:8. Translation from this alternate start site results in the amino acid sequence found in SEQ ID NO:11. An alternate start site for an AXMI-008 delta-endotoxin protein of the invention may be at nucleotide 177 of SEQ ID NO:12. Translation from this alternate start site results in the amino acid sequence found in SEQ ID NO:16. An alternate start site for an AXMI-009 delta-endotoxin protein may be at nucleotide 34 of SEQ ID NO:19. Translation from this alternate start site results in the amino acid sequence found in SEQ ID NO:22. An additional alternate start site for an AXMI-009 delta-endotoxin protein may be at nucleotide 64 of SEQ ID NO:1. Translation from this alternate start site results in the amino acid sequence found in SEQ ID NO:24. An alternate start site for an AXMI-014 delta-endotoxin protein may be at base pair 136 of SEQ ID NO:25. Translation from this alternate start site results in the amino acid sequence found in SEQ ID NO:29. The AXMI-008 delta-endotoxin proteins are encompassed in the present invention and may be used in the methods of the present invention.

In addition, there may be one or more additional open reading frames in the disclosed nucleotide sequences that encode one or more AXMI-008 org2 delta-endotoxin-associated proteins. By such a "delta-enotoxin-associated protein" is intended a protein encoded by a nucleotide sequence disclosed herein using an alternate open reading frame than that used by the delta-endotoxins of the present invention. Proteins such as these are known in the art as helper proteins, stabilizing sequences, or delta-endotoxin-associated proteins. These delta-endotoxin-associated proteins may have pesticidal activity, or may be important in facilitating expression of delta-endotoxin proteins. Methods are known in the art for measuring pesticidal activity and for determining the effects of delta-endotoxin-associated proteins on delta-endotoxin protein expression and crystal formation (see, for example, Park et al. (1999) FEMS Microbiol. Lett. 181:319-327; Ge et al. (1998) FEMS Microbiol. Lett. 165:35-41; Rosso and Delecluse (1997) Appl. Environ. Microbiol. 63:4449-4455). These delta-endotoxin-associated proteins are encompassed by the present invention, and may be used in the methods disclosed herein, either alone or in combination with known delta-endotoxin proteins. In one embodiment, the delta-endotoxin-associated protein has the amino acid sequence found in SEQ ID NO:18 and is encoded by the nucleotide sequence of SEQ ID NO:17.

By "plant cell" is intended all known forms of plant, including undifferentiated tissue (e.g. callus), suspension culture cells, protoplasts, leaf cells, root cells, phloem cells, plant seeds, pollen, propagules, embryos and the like. By "plant expression cassette" is intended a DNA construct that is capable of resulting in the expression of a protein from an open reading frame in a plant cell. Typically these contain a promoter and a coding sequence. Often, such constructs will also contain a 3' untranslated region. Such constructs may contain a 'signal sequence' or 'leader sequence' to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this typically involves secretion into the Golgi apparatus, with some resulting glycosylation. By "leader sequence" is intended any sequence that when translated, results in an amino acid sequence sufficient to trigger cotranslational transport of the peptide chain to a sub-cellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one 'vector' DNA molecule. For example, binary vectors are plant transformation vectors that utilize two non-contiguous DNA vectors to encode all requisite cis- and transacting functions for transformation of plant cells (Hellens and Mullineaux (2000) Trends in Plant Science 5:446-451). "Vector" refers to a nucleic acid construct designed for transfer between different host cells. "Expression vector" refers to a vector that has ability to incorporate, integrate and express heterologous DNA sequences or fragments in a foreign cell.

"Transgenic plants" or "transformed plants" or "stably transformed plants or cells or tissues" refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

"Promoter" refers to a nucleic acid sequence that functions to direct transcription of a downstream coding sequence. The promoter together with other transcriptional and translational regulatory nucleic acid sequences (also termed "control sequences are necessary for the expression of a DNA sequence of interest.

Provided herein are novel isolated nucleotide sequences that confer pesticidal activity. Also provided are the amino acid sequences for the delta-endotoxin and delta-endotoxin-associated proteins. The protein resulting from translation of this gene allows cells to control or kill pests that ingest it.

An "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various embodiments, the isolated delta-endotoxin or delta-endotoxin-associated-encoding nucleic acid molecule can contain less than about 5 kb, 4 kb, 3 kb, 2 kb, 1 kb, 0.5 kb, or 0.1 kb of nucleotide sequence that naturally flanks the nucleic acid molecule in genomic DNA of the cell from which the nucleic acid is derived. A delta-endotoxin or delta-endotoxin-associated protein that is substantially free of cellular material includes preparations of protein having less than about 30%, 20%, 10%, or 5% (by dry weight) of non-delta-endotoxin or non-delta-endotoxin-associated protein (also referred to herein as a "contaminating protein"). Various aspects of the invention are described in further detail in the following subsections.

### Isolated Nucleic Acid Molecules, and Variants and Fragments Thereof

One aspect of the invention pertains to isolated nucleic acid molecules comprising nucleotide sequences encoding delta-endotoxin or delta-endotoxin-associated proteins and polypeptides or biologically active portions thereof, as well as nucleic acid molecules sufficient for use as hybridization probes to identify delta-endotoxin or delta-endotoxin-associated-encoding nucleic acids. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

Nucleotide sequences encoding the proteins of the present invention includes the sequences set forth in SEQ ID NOS: 12, 13, 15 and 17 and complements thereof. By "complement" is intended a nucleotide sequence that is sufficiently complementary to a given nucleotide sequence such that it can hybridize to the given nucleotide sequence to thereby form a stable duplex. The corresponding amino acid sequences for the delta-endotoxin or delta-endotoxin-associated proteins encoded by these nucleotide sequences are set forth in SEQ ID NOS: 14, 16 and 18.

Nucleic acid molecules that are fragments of these delta-endotoxin or delta-endotoxin-associated protein-encoding nucleotide sequences are also encompassed by the present invention. By "fragment" is intended a portion of the nucleotide sequence encoding a delta-endotoxin protein or delta-endotoxin-associated protein. A fragments of a nucleotide sequence may encode a biologically active portion of a delta-endotoxin or delta-endotoxin-associated protein, or it may be a fragment that can be used as a hybridization probe or PCR primer using methods disclosed below. Nucleic acid molecules that are fragments of a delta-endotoxin or a delta-endotoxin-associated nucleotide sequence comprise at least about 15, 20, 50, 75, 100, 200, 300, 350, 400, 450, 500, 550, 600, 650, 700, 750, 800, 850, 900, 950, 1000, 1100,1200, 1300,1400, 1500, 1600, 1700, 1800, 1900, 2000, 2100, 2200, 2300, 2400, 2500, 3000, 3500, 4000, 4500, 5000, 5500 nucleotides, or up to the number of nucleotides present in a full-length delta-endotoxin or delta-endotoxin-associated protein-encoding nucleotide sequence disclosed herein (for example, 2190 nucleotides for SEQ ID NO:1, 1890 for SEQ ID NO:2, etc.), depending upon the intended use.

Fragments of the nucleotide sequences of the present invention will encode protein fragments that retain the biological activity of the delta endotoxin or delta-endotoxin-associated protein and, hence, retain pesticidal activity or delta-endotoxin-associated protein activity, respectively. By "delta-endotoxin activity" is intended pesticidal activity. By "delta-endotoxin-associated protein activity" is intended that the protein have pesticidal activity, or that the protein improves expression of a delta-endotoxin protein. This improvement in protein expression can happen by any mechanism. By "retains activity" is intended that the fragment will have at least about 30%, preferably at least about 50%, more preferably at least about 70%, even more preferably at least about 80% of the activity of the delta-endotoxin or delta-endotoxin-associated protein. Methods are known in the art for determining the effects of delta-endotoxin-associated proteins on delta-endotoxin protein expression and crystal formation (see, for example, Park et al. (1999) FEMS Microbiol. Lett. 181:319-327; Ge et al. (1998) FEMS Microbiol. Lett. 165:35-41; Rosso and Delecluse (1997) Appl. Environ. Microbiol. 63:4449-4455). Methods for measuring pesticidal activity are well known in the art. See, for example, Czapla and Lang (1990) J. Econ. Entomol. 83(6): 2480-2485; Andrews et al. (1988) Biochem. J. 252:199-206; Marrone et al. (1985) J. of Economic Entomology 78:290-293; and U.S. Patent No. 5,743,477.

A fragment of a delta-endotoxin or delta-endotoxin-associated protein-encoding nucleotide sequence that encodes a biologically active portion of a protein of the invention will encode at least about 15, 25, 30, 50, 75, 100, 125,150, 175, 200, 250, 300, 350, 400, 450, 500, 550, 600, or 650 contiguous amino acids, or up to the total number of amino acids present in a full-length delta-endotoxin or delta-endotoxin-associated protein of the invention (for example, 629 amino acids for SEQ ID NO:3, 601 amino acids for SEQ ID NO:5, etc.).

Preferred delta-endotoxin or delta-endotoxin-associated proteins of the present invention are encoded by a nucleotide sequences sufficiently identical to the nucleotide sequences of in SEQ ID NO: 14, 16 or 18. By "sufficiently identical" is intended an amino acid or nucleotide sequence that has at least 95%, 96%, 97%, 98% or 99% sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score =100, wordlength =12, to obtain nucleotide sequences homologous to delta-endotoxin or delta-endotoxin-associated nucleic acid molecules of the invention. BLAST protein searches can be performed with the BLASTX program, score =50, wordlength= 3, to obtain amino acid sequences homologous to delta-endotoxin or delta-endotoxin-associated protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. See, Altschul *et al.* (1997) *supra.* When utilizing BLAST, Gapped BLAST, and PSI-Blast programs, the default parameters of the respective programs (e.g., BLASTX and BLASTN) can be used. See, www.ncbi.nlm.nih.gov. Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the vector NTi Program Suite (Informax, Inc). After alignment of amino acid sequences with ClustalW, the percent amino acid identity can be assessed. A non-limiting example of a software program useful for analysis of ClustalW alignments is GeneDoc™. Genedoc™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package (available from Accelrys, Inc., 9865 Scranton Rd., San Diego, California, USA). When utilizing the ALIGN program for comparing amino acid sequences, a PAM120 weight residue table, a gap length penalty of 12, and a gap penalty of 4 can be used.

The invention also encompasses variant nucleic acid molecules. "Variants" of the delta-endotoxin or delta-endotoxin-associated protein-encoding nucleotide sequences include those sequences that encode the delta-endotoxin or delta-endotoxin-associated proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the delta-endotoxin or delta-endotoxin-associated proteins disclosed in the present invention as discussed below. Variant proteins encompassed by the present invention are biologically active, that is they continue to possess the desired biological activity of the native protein, that is, retaining pesticidal activity. By "retains activity" is intended that the variant will have at least about 30%, preferably at least about 50%, more preferably at least about 70%, even more preferably at least about 80% of the activity of the delta-endotoxin or delta-endotoxin-associated protein. Methods for measuring pesticidal activity are well known in the art. See, for example, Czapla and Lang (1990) J. Econ. Entornol. 83(6): 2480-2485; Andrews et al. (1988) Biochem. J. 252:199-206; Marrone et al. (1985) J. of Economic Entomology 78:290-293; and U.S. Patent No. 5,743,477, all of which are herein incorporated by reference in their entirety.

The invention also encompasses variant nucleic acid molecules. "Variants" of the delta-endotoxin or delta-endotoxin-associated-encoding nucleotide sequences include those sequences that encode the delta-endotoxin or delta-endotoxin-associated proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the delta-endotoxin or delta-endotoxin-associated proteins disclosed in the present invention as discussed below.

The skilled artisan will further appreciate that changes can be introduced by mutation into the nucleotide sequences of the invention thereby leading to changes in the amino acid sequence of the encoded delta-endotoxin or delta-endotoxin-associated proteins, without altering the biological activity of the proteins. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, preferably, conservative amino acid substitutions may be made at one or more predicted, preferably nonessential amino acid residues. A "nonessential" amino acid residue is a residue that can be altered from the wild-type sequence of a delta-endotoxin or delta-endotoxin-associated protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

There are generally five highly conserved regions among the delta-endotoxin proteins, concentrated largely in the center of the domain or at the junction between domains (Rajamohan et al. (1998) Prog. Nucleic Acid Res. Mol. Biol. 60:1-23). The blocks of conserved amino acids for various delta-endotoxins as well as consensus sequences maybe found in Schnepf et al. (1998) Microbio. Mol. Biol. Rev. 62:775-806 and Lereclus et al. (1989) Role, Structure, and Molecular Organization of the Genes Coding for the Parasporal d-endotoxins of Bacillus thuringiensis. In Regulation of Procaryotic Development. Issar Smit, Slepecky, R.A., Setlow, P. American Society for Microbiology, Washington, D.C. 20006. It has been proposed that delta-endotoxins having these conserved regions may share a similar structure, consisting of three domains (Li et al. (1991) Nature 353: 815-821). Domain I has the highest similarity between delta-endotoxins (Bravo (1997) J. Bacteriol. 179:2793-2801).

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in the alignments provided. Examples of residues that are conserved but that may allow conservative amino acid substitutions and still retain activity include, for example, residues that have only conservative substitutions between all proteins contained in the alignments provided. However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for ability to confer delta endotoxin or delta-endotoxin-associated activity to identify mutants that retain activity. Following mutagenesis, the encoded protein can be expressed recombinantly, and the activity of the protein can be determined using standard assay techniques.

Using methods such as PCR, hybridization, and the like corresponding delta-endotoxin or delta-endotoxin-associated sequences can be identified, such sequences having substantial identity to the sequences of the invention. *See,* for example, Sambrook J., and Russell, D.W. (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the delta-endotoxin or delta-endotoxin-associated nucleotide sequence can be used to screen cDNA or genomic libraries. Methods for construction of such cDNA and genomic libraries are generally known in the art and are disclosed in Sambrook and Russell, 2001. The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known delta-endotoxin or delta-endotoxin-associated-encoding nucleotide sequence disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, preferably about 25, more preferably at least about 50, 75, 100, 125,150, 175, 200, 250, 300, 350, or 400 consecutive nucleotides of delta-endotoxin or delta-endotoxin-associated-encoding nucleotide sequence of the invention or a fragment or variant thereof. Preparation of probes for hybridization is generally known in the art and is disclosed in Sambrook and Russell, 2001, herein incorporated by reference.

In hybridization techniques, all or part of a known nucleotide sequence is used as a probe that selectively hybridizes to other corresponding nucleotide sequences present in a population of cloned genomic DNA fragments or cDNA fragments (i.e., genomic or cDNA libraries) from a chosen organism. The hybridization probes may be genomic DNA fragments, cDNA fragments, RNA fragments, or other oligonucleotides, and may be labeled with a detectable group such as ³²P, or any other detectable marker. Thus, for example, probes for hybridization can be made by labeling synthetic oligonucleotides based on the delta-endotoxin or delta-endotoxin-associated sequence of the invention. Methods for preparation of probes for hybridization and for construction of cDNA and genomic libraries are generally known in the art and are disclosed in Sambrook et al. (1989) Molecular Cloning. A Laboratory Manual (2d ed., Cold Spring Harbor Laboratory Press, Plainview, New York).

For example, the entire delta-endotoxin or delta-endotoxin-associated sequence disclosed herein, or one or more portions thereof, may be used as a probe capable of specifically hybridizing to corresponding delta-endotoxin or delta-endotoxin-associated-like sequences and messenger RNAs. To achieve specific hybridization under a variety of conditions, such probes include sequences that are unique and are preferably at least about 10 nucleotides in length, and most preferably at least about 20 nucleotides in length. Such probes may be used to amplify corresponding delta-endotoxin or delta-endotoxin-associated sequences from a chosen organism by PCR This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1M NaCl, 1% SDS at 37°C, and a wash in 0.1X SSC at 60 to 65°C. Optionally, wash buffers may comprise about 0.1 % to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC)-0.61 (% form)-500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Plainview, New York).

### Isolated Proteins and Variants and Fragments Thereof

Delta-endotoxin and delta-endotoxin-associated proteins are also encompassed within the present invention. By "delta-endotoxin protein" is intended a protein having the amino acid sequence set forth in SEQ ID NO: 14, 16 or 20. By "delta-endotoxin-associated protein" is intended a protein having the amino acid sequence set forth in SEQ ID NO:18. Fragments, biologically active portions, and variants thereof are also provided, and may be used to practice the methods of the present invention.

"Fragments" or "biologically active portions" include polypeptide fragments comprising a portion of an amino acid sequence encoding a delta-endotoxin or delta-endotoxin-associated protein as set forth in SEQ ID NO: 14, 16 or 18 , and that retain delta-endotoxin activity or delta-endotoxin-associated activity. A biologically active portion of a delta-endotoxin or delta-endotoxin-associated protein can be a polypeptide that is, for example, 10, 25, 50,100 or more amino acids in length. Such biologically active portions can be prepared by recombinant techniques and evaluated for delta-endotoxin or delta-endotoxin-associated activity. Methods for measuring pesticidal activity are well known in the art. See, for example, Czapla and Lang (1990) J. Econ. Entomol. 83(6): 2480-2485; Andrews et al. (1988) Biochem. J. 252:199-206; Marrone et al. (1985) J. of Economic Entomology 78:290-293; and U.S. Patent No. 5,743,477. As used here, a fragment comprises at least 8 contiguous amino acids SEQ ID NO: 14,16 or 18. The invention encompasses other fragments, however, such as any fragment in the protein greater than about 10, 20, 34, 50, 100, 150, 200, 250, 300, 350, 400, 450, 500, 550, 600, and 650 amino acids.

By "variants" is intended proteins or polypeptides having an amino acid sequence that is at least 96%, 97%, 98% or 99% identical to the amino acid sequence of SEQ ID NO: 14, 16 or 18. Variants also include polypeptides encoded by a nucleic acid molecule that hybridizes to the nucleic acid molecule of SEQ ID NO: 12, 13, 15 or 17, or a complement thereof, under stringent conditions. Such variants generally retain delta-endotoxin or delta-endotoxin-associated activity. Variants include polypeptides that differ in amino acid sequence due to mutagenesis. Variant proteins encompassed by the present invention are biologically active, that is they continue to possess the desired biological activity of the native protein, that is, retaining pesticidal activity. Methods for measuring pesticidal activity are well known in the art. See, for example, Czapla and Lang (1990) J. Econ. Entomol. 83(6): 2480-2485; Andrews et al. (1988) Biochem. J. 252:199-206; Marrone et al. (1985) J. of Economic Entomology 78:290-293; and U.S. Patent No. 5,743,477.

### Altered or Improved Variants

It is recognized that DNA sequences of a delta-endotoxin or delta-endotoxin-associated protein may be altered by various methods, and that these alterations may result in DNA sequences encoding proteins with amino acid sequences different than that encoded by the delta-endotoxin or delta-endotoxin-associated protein of the present invention. This protein may be altered in various ways including amino acid substitutions, deletions, truncations, and insertions. Methods for such manipulations are generally known in the art. For example, amino acid sequence variants of the delta-endotoxin or delta-endotoxin-associated protein can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a delta-endotoxin or delta-endotoxin-associated protein to confer pesticidal activity may be improved by the use of such techniques upon the compositions of this invention. For example, one may express the delta-endotoxin or delta-endotoxin-associated protein in host cells that exhibit high rates of base misincorporation during DNA replication, such as XL-1 Red (Stratagene). After propagation in such strains, one can isolate the delta-endotoxin or delta-endotoxin-associated DNA (for example by preparing plasmid DNA, or by amplifying by PCR and cloning the resulting PCR fragment into a vector), culture the delta-endotoxin or delta-endotoxin-associated mutations in a non-mutagenic strain, and identify mutated delta-endotoxin or delta-endotoxin-associated genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. This can include insertions, deletions, or alterations introduced by modern molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different delta-endotoxin or delta-endotoxin-associated protein coding regions can be used to create a new delta-endotoxin or delta-endotoxin-associated protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the delta-endotoxin or delta-endotoxin-associated gene of the invention and other known delta-endotoxin or delta-endotoxin-associated genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-43 8; Moore et al. (1997) J. Mol. Biol. 272:336-347; Zhang et al. (1997) Proc. Natl: Acad. Sci. USA 94:4504-4509; Crameri et al. (1998) Nature 391:288-291; and U.S. Patent Nos. 5,605,793 and 5,837,458.

Domain swapping or shuffling is another mechanism for generating altered delta-endotoxin or delta-endotoxin-associated proteins. Domains II and III may be swapped between delta-endotoxin proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepf et al. (1990) J. Biol. Chem. 265:20923-20930; Rang et al. 91999) Appl. Environ. Micriobiol. 65:2918-2925).

### Plant Transformation

Transformation of plant cells can be accomplished by one of several techniques known in the art. First, one engineers the delta-endotoxin or delta-endotoxin-associated gene in a way that allows its expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this 'plant expression cassette' will be inserted into a 'plant transformation vector'. This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as 'binary vectors'. Binary vectors as well as vectors with helper plasmids are most often used for *Agrobacterium-mediated* transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a 'gene of interest' (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the gene of interest are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and virmediated DNA transfer, as in understood in the art (Hellens and Mullineaux (2000) Trends in Plant Science, 5:446-451). Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethelene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6: 271-282; Ishida et al. (1996) Nature Biotechnology 14: 745-750). Explants are typically transferred to a fresh supply of the same medium and cultured routinely. A general description of the techniques and methods for generating transgenic plantlets are found in Ayres and Park, 1994 (Critical Reviews in Plant Science 13: 219-239) and Bommineni and Jauhar, 1997 (Maydica 42: 107-120). Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Generation of transgenic plants may be performed by one of several methods, including but not limited to introduction of heterologous DNA by *Agrobacterium* into plant cells (Agrobacterium-mediated transformation), bombardment of plant cells with heterologous foreign DNA adhered to particles, and various other non-particle direct-mediated methods (e.g. Hiei et al. (1994) The Plant Journal 6: 271-282; Ishida et al. (1996) Nature Biotechnology 14: 745-750; Ayres and Park (1994) Critical Reviews in Plant Science 13: 219-239; Bommineni and Jauhar (1997) Maydica 42: 107-120) to transfer DNA.

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway et al. (1986) Biotechniques 4:320-334), electroporation (Riggs et al. (1986) Proc. Natl. Acad. Sci. USA 83:5602-5606, *Agrobacterium-mediated* transformation (U.S. Patent No. 5,563,055; U.S. Patent No. 5,981,840), direct gene transfer (Paszkowski et al. (1984) EMBO J. 3:2717-2722), and ballistic particle acceleration (see, for example, U.S. Patent No. 4,945,050; U.S. Patent No. 5,879,918; U.S. Patent No. 5,886,244; U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); aerosol beam transformation (U.S. Published Application No. 20010026941; U.S. Patent No. 4,945,050; International Publication No. WO 91/00915; U.S. Published Application No. 2002015066); and Lecl transformation (WO 00/28058). Also see Weissinger et al. (1988) Ann. Rev. Genet. 22:421-477; Sanford et al. (1987) Particulate Science and Technology 5:27-37; Christou et al. (1988) Plant Physiol. 87:671-674; McCabe et al. (1988) Bio/Technology 6:923-926; Finer and McMullen (1991) In Vitro Cell Dev. Biol. 27P:175-182; Singh et al. (1998) Theor. Appl. Genet. 96:319-324 (soybean); Datta et al. (1990) Biotechnology 8:736-740; Klein et al. (1988) Proc. Natl. Acad. Sci. USA 85:4305-4309; U.S. Patent No. 5,240,855; U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin); Klein et al. (1988) Plant Physiol. 91:440-444; Hooykaas-Van Slogteren et al. (1984) Nature (London) 311:763-764; U.S. Patent No. 5,736,369; Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209; Kaeppler et al. (1990) Plant Cell Reports 9:415-418 and Kaeppler et al. (1992) Theor. Appl. Genet. 84:560-566; D'Halluin et al. (1992) Plant Cell 4:1495-1505; Li et al. (1993) Plant Cell Reports 12:250-255 and Christou and Ford (1995) Annals of Botany 75:407-413; Osjoda et al. (1996) Nature Biotechnology 14:745-750.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Then molecular and biochemical methods will be used for confirming the presence of the integrated heterologous gene of interest in the genome of transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

The delta-endotoxin or delta-endotoxin-associated sequences of the invention may be provided in expression cassettes for expression in the plant of interest. The cassette will include 5' and 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the delta-endotoxin or delta-endotoxin-associated sequence to be under the transcriptional regulation of the regulatory regions.

The expression cassette will include in the 5'-3' direction of transcription, a transcriptional and translational initiation region (i.e., a promoter), a DNA sequence of the invention, and a transcriptional and translational termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably-linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed host cell. That is, the genes can be synthesized using host cell-preferred codons for improved expression, or may be synthesized using codons at a host-preferred codon usage frequency. See, for example, Campbell and Gowri (1990) Plant Physiol. 92:1-11 for a discussion of host-preferred codon usage. Methods are known in the art for synthesizing plant-preferred genes. See, for example, U.S. Patent Nos. 6,320,100; 6,075,185; 5,380,831; and 5,436,391, U.S. Published Application Nos. 20040005600 and 20010003849, and Murray et al. (1989) Nucleic Acids Res. 17:477-498.

In one embodiment, the nucleic acids of interest are targeted to the chloroplast for expression. In this manner, where the nucleic acid of interest is not directly inserted into the chloroplast, the expression cassette will additionally contain a nucleic acid encoding a transit peptide to direct the gene product of interest to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-bome transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR Analysis: PCR analysis is a rapid method to screen transformed cells, tissue or shoots for the presence of incorporated gene at the earlier stage before transplanting into the soil (Sambrook and Russell, 2001). PCR is carried out using oligonucleotide primers specific to the gene of interest or *Agrobacterium* vector background, etc.

Southern Analysis: Plant transformation is confirmed by Southern blot analysis of genomic DNA (Sambrook and Russell, 2001). In general, total DNA is extracted from the transformant, digested with appropriate restriction enzymes, fractionated in an agarose gel and transferred to a nitrocellulose or nylon membrane. The membrane or "blot" then is probed with, for example, radiolabeled ³²P target DNA fragment to confirm the integration of introduced gene in the plant genome according to standard techniques (Sambrook and Russell, 2001. Molecular Cloning: A Laboratory Manual. Cold Spring Harbor Laboratory Press, Cold Spring Harbor, NY).

Northern Analysis: RNA is isolated from specific tissues of transformant, fractionated in a formaldehyde agarose gel, blotted onto a nylon filter according to standard procedures that are routinely used in the art (Sambrook, J., and Russell, D.W. 2001. Molecular Cloning: A Laboratory Manual, Cold Spring Harbor Laboratory Press, Cold Spring Harbor, NY). Expression of RNA encoded by the delta-endotoxin or delta-endotoxin-associated is then tested by hybridizing the filter to a radioactive probe derived from a delta-endotoxin or delta-endotoxin-associated protein, by methods known in the art (Sambrook and Russell, 2001).

Western blot and Biochemical assays: Western blot and biochemical assays and the like may be carried out on the transgenic plants to confirm the presence of protein encoded by the delta-endotoxin or delta-endotoxin-associated gene by standard procedures (Sambrook, J., and Russell, D.W. 2001. Molecular Cloning: A Laboratory Manual, Cold Spring Harbor Laboratory Press, Cold Spring Harbor, NY) using antibodies that bind to one or more epitopes present on the delta-endotoxin or delta-endotoxin-associated protein.

### Pesticidal activity in plants

In another aspect of the invention, one may generate transgenic plants expressing delta-endotoxin or delta-endotoxin-associated proteins that have pesticidal activity. Methods described above by way of example may be utilized to generate transgenic plants, but the manner in which the transgenic plant cells are generated is not critical to this invention. Methods known or described in the art such as *Agrobacterium-mediated* transformation, aerosol beam, biolistic transformation, and non-particle-mediated methods may be used at the discretion of the experimenter. Plants expressing delta-endotoxin or delta-endotoxin-associated proteins may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene).

Fertile plants expressing a delta-endotoxin or a delta-endotoxin-associated protein may be tested for pesticidal activity, and the plants showing optimal activity selected for further breeding. Methods are available in the art to assay for pest activity. Generally, the protein is mixed and used in feeding assays. See, for example Marrone et al. (1985) J. of Economic Entomology 78:290-293.

### Use in Pesticidal Control

General methods for employing the strains of the invention in pesticide control or in engineering other organisms as pesticidal agents are known in the art. See, for example U.S. Patent No. 5,039,523 and EP 0480762A2.

The *Bacillus* strains of the invention or the microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticide is produced by introducing a heterologous gene into a cellular host. Expression of the heterologous gene results, directly or indirectly, in the intracellular production and maintenance of the pesticide. In one aspect of this invention, these cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing the genes of this invention such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, mollusocides or mixtures of several of these preparations, if desired, together with further agriculturally acceptable cameras, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Preferred methods of applying an active ingredient of the present invention or an agrochemical composition of the present invention which contains at least one of the pesticidal proteins produced by the bacterial strains of the present invention are leaf application, seed coating and soil application. The number of applications and the rate of application depend on the intensity of infestation by the corresponding pest

The composition may be formulated as a powder, dust, pellet, granule, spray, emulsion, colloid, solution, or such like, and may be preparable by such conventional means as desiccation, lyophilization, homogenation, extraction, filtration, centrifugation, sedimentation, or concentration of a culture of cells comprising the polypeptide. In all such compositions that contain at least one such pesticidal polypeptide, the polypeptide may be present in a concentration of from about 1% to about 99% by weight.

Lepidopteran or coleopteran pests may be killed or reduced in numbers in a given area by the methods of the invention, or may be prophylactically applied to an environmental area to prevent infestation by a susceptible pest. Preferably the pest ingests, or is contacted with, a pesticidally-effective amount of the polypeptide. By "pesticidally-effective amount" is intended an amount of the pesticide that is able to bring about death to at least one pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating either the bacterial cell, crystal and/or spore suspension, or isolated protein component with the desired agriculturally-acceptable carrier. The compositions may be formulated prior to administration in an appropriate means such as lyophilized, freeze-dried, desiccated, or in an aqueous carrier; medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No.6,468,523.

"Pest" includes but is not limited to, insects, fungi, bacteria, nematodes, mites, ticks, and the like. Insect pests include insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc., particularly Coleoptera, Lepidoptera, and Diptera.

Insect pests include insects selected from the orders Coleoptera, Diptera, Hymenoptera, Lepidoptera, Mallophaga, Homoptera, Hemiptera, Orthoptera, Thysanoptera, Dermaptera, Isoptera, Anoplura, Siphonaptera, Trichoptera, etc., particularly Coleoptera and Lepidoptera. Insect pests of the invention for the major crops include: Maize: *Ostrinia nubilalis,* European corn borer; *Agrotis ipsilon,* black cutworm; *Helicoverpa zea,* corn earworm; *Spodoplera frugiperda,* fall armyworm; *Diatraea grandiosella,* southwestern corn borer; *Elasmopalpus lignosellus,* lesser cornstalk borer; *Diatraea saccharalis,* surgarcane borer; *Diabrotica virgifera,* western corn rootworm; *Diabrotica longicornis barberi,* northern corn rootworm; *Diabrotica undecimpunctata howardi,* southern corn rootworm; *Melanotus* spp., wireworms; *Cyclocephala borealis,* northern masked chafer (white grub); *Cyclocephala immaculata,* southern masked chafer (white grub); *Popillia japonica,* Japanese beetle; *Chaetocnema pulicaria,* corn flea beetle; *Sphenophorus maidis,* maize billbug; *Rhopalosiphum maidis,* corn leaf aphid; *Anuraphis maidiradicis,* corn root aphid; *Blissus leucopterus leucopterus,* chinch bug; *Melanoplus femurrubrum,* redlegged grasshopper; *Melanoplus sanguinipes,* migratory grasshopper, *Hylemya platura,* seedcorn maggot; *Agromyza parvicornis,* corn blot leafininer, *Anaphothrips obscrurus,* grass thrips; *Solenopsis milesta,* thief ant; *Tetranychus urticae,* twospotted spider mite; Sorghum: *Chilo partellus,* sorghum borer, *Spodoptera frugiperda,* fall armyworm; *Helicoverpa zea,* corn earworm; *Elasmopalpus lignosellus,* lesser cornstalk borer, *Feltia subterranea,* granulate cutworm; *Phyllophaga crinita,* white grub; *Eleodes, Conoderus,* and *Aeolus* spp., wireworms; *Oulema melanopus,* cereal leaf beetle; *Chaetocnema pulicaria,* corn flea beetle; *Sphenophorus maidis,* maize billbug; *Rhopalosiphum maidis;* corn leaf aphid; *Sipha flava,* yellow sugarcane aphid; *Blissus leucopterus leucopterus,* chinch bug; *Contarinia sorghicola,* sorghum midge; *Tetranychus cinnabarinus,* carmine spider mite; *Tetranychus urticae,* twospotted spider mite; Wheat: *Pseudaletia unipunctata,* army worm; *Spodoptera frugiperda,* fall armyworm; *Elasmopalpus lignosellus,* lesser cornstalk borer, *Agrotis orthogonia,* western cutworm; *Elasmopalpus lignosellus,* lesser cornstalk borer; *Oulema melanopus,* cereal leaf beetle; *Hypera punctata,* clover leaf weevil; *Diabrotica undecimpunctata howardi,* southern corn rootworm; Russian wheat aphid; *Schizaphis graminum,* greenbug; *Macrosiphum avenae,* English grain aphid; *Melanoplus femurrubrum,* redlegged grasshopper, *Melanoplus differentialis,* differential grasshopper; *Melanoplus sanguinipes,* migratory grasshopper; *Mayetiola destructor,* Hessian fly; *Sitodiplosis mosellana,* wheat midge; *Meromyza americana,* wheat stem maggot; *Hylemya coarctata,* wheat bulb fly; *Frankliniella fusca,* tobacco thrips; *Cephus cinctus,* wheat stem sawfly; *Aceria tulipae,* wheat curl mite; Sunflower: *Suleima helianthana,* sunflower bud moth; *Homoeosoma electellum,* sunflower moth; *zygogramma exclamationis,* sunflower beetle; *Bothyrus gibbosus,* carrot beetle; *Neolasioptera murtfeldtiana,* sunflower seed midge; Cotton: *Heliothis virescens,* cotton budworm; *Helicoverpa zea,* cotton bollworm; *Spodoptera exigua,* beet armyworm; *Pedinophora gossypiella,* pink bollworm; *Anthonomus grandis,* boll weevil; *Aphis gossypii,* cotton aphid; *Pseudatomoscelis seriatus,* cotton fleahopper; *Trialeurodes abutilonea,* bandedwinged whitefly; *Lygus lineolaris,* tarnished plant bug; *Melanoplus femurrubrum,* redlegged grasshopper; *Melanoplus differentialis,* differential grasshopper, *Thrips tabaci,* onion thrips; *Franklinkiella fusca,* tobacco thrips; *Tetranychus cinnabarinus,* carmine spider mite; *Tetranychus urticae,* twospotted spider mite; Rice: *Diatraea saccharalis,* sugarcane borer; *Spodoptera frugiperda,* fall armyworm; *Helicoverpa zea,* corn earworm; *Colaspis brunnea,* grape colaspis; *Lissorhoptrus oryzophilus,* rice water weevil; *Sitophilus oryzae,* rice weevil; *Nephotettix nigropictus,* rice leafhopper; *Blissus leucopterus leucopterus,* chinch bug; *Acrosternum hilare,* green stink bug; Soybean: *Pseudoplusia includens,* soybean looper; *Anticarsia gemmatalis,* velvetbean caterpillar; *Plathypena scabra,* green cloverworm; *Ostrinia nubilalis,* European corn borer, *Agrotis ipsilon,* black cutworm; *Spodoptera exigua,* beet armyworm; *Heliothis virescens,* cotton budworm; *Helicoverpa zea,* cotton bollworm; *Epilachna varivestis,* Mexican bean beetle; *Myzus persicae,* green peach aphid; *Empoasca fabae,* potato leafhopper; *Acrosternum hilare,* green stink bug; *Melanoplus femurrubrum,* redlegged grasshopper; *Melanoplus differentialis,* differential grasshopper; *Hylemya platura,* seedcorn maggot; *Sericothrips variabilis,* soybean thrips; *Thrips tabaci,* onion thrips; *Tetranychus turkestani,* strawberry spider mite; *Tetranychus urticae,* twospotted spider mite; Barley: *Ostrinia nubilalis,* European corn borer; *Agrotis ipsilon,* black cutworm; *Schizaphis graminum,* greenbug; *Blissus leucopterus leucopterus,* chinch bug; *Acrosternum hilare,* green stink bug; *Euschistus servus,* brown stink bug; *Delia platura,* seedcorn maggot; *Mayetiola destructor,* Hessian fly; *Petrobia latens,* brown wheat mite; Oil Seed Rape: *Brevicoryne brassicae,* cabbage aphid; *Phyllotreta cruciferae,* Flea beetle; *Mamestra configurata,* Bertha armyworm; *Plutella xylostella,* Diamond-back moth; *Delia* ssp., Root maggots.

Nematodes include parasitic nematodes such as root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

The following examples are offered by way of illustration and not by way of limitation.

### EXPERIMENTAL

### Example 1. Extraction of Plasmid DNA

Plasmid DNA from strains ATX 13026 or ATX 13002 were prepared in the following way. A pure culture of strain ATX13026 or strain ATX13002 was grown in large quantities of rich media. The culture was centrifuged to harvest the cell pellet. The cell pellet was then prepared by treatment with SDS by methods known in the art, resulting in breakage of the cell wall and release of DNA. Proteins and large genomic DNA was then precipitated by a high salt concentration. The plasmid DNA was precipitated by standard ethanol precipitation. The plasmid DNA was separated from any remaining chromosomal DNA by high-speed centrifugation through a cesium chloride gradient. The DNA was visualized in the gradient by UV light and the band of lower density (i.e. the lower band) was extracted using a syringe. This band contained the plasmid DNA from the strain (either ATX 13026 or ATX 13002) The quality of the DNA was checked by visualization on an agarose gel by methods known in the art.

### Example 2. Cloning of Genes

The purified plasmid DNA was sheared into 5-10 kb sized fragments and the 5' and 3' single stranded overhangs repaired using T4 DNA polymerase and Klenow fragment in the presence of all four dNTPs, as known in the art. Phosphates were then attached to the 5' ends by treatment with T4 polynucleotide kinase, as known in the art. The repaired DNA fragments were ligated overnight into a standard high copy vector (i.e. pBluescript SK+), suitably prepared to accept the inserts as known in the art (for example by digestion with a restriction enzyme producing blunt ends).

The quality of the library was analyzed by digesting a subset of clones with a restriction enzyme know to have a cleavage site flanking the cloning site. A high percentage of clones were determined to contain inserts, with an average insert size of 5-6 kb.

### Example 3. High Throughput Sequencing of Library Plates

The libraries prepared by the methods above were plated onto rich media containing the appropriate antibiotic to maintain the plasmids clones, and colonies were individually picked into 96-well blocks containing 2 mls of media containing the appropriate antibiotic. These blocks were grown overnight at 37°C at a shaking speed of 350 rpm. The blocks were centrifuged to harvest the cells to the bottom of the block. Plasmid DNA was isolated from these cultures by standard alkaline lysis prep in a high throughput format.

The end sequences of clones from this library were determined for a large number of clones from each block in the following way: The DNA sequence of each clone chosen for analysis was determined using the fluorescent dye terminator sequencing technique (Applied Biosystems) and standard primers flanking each side of the cloning site. Once the reactions had been carried out in the thermocycler, the DNA was precipitated using standard ethanol precipitation. The DNA was resuspended in water and loaded onto a capillary sequencing machine. Each library plate of DNA was sequenced from either end of the cloning site, yielding two reads per plate over each insert.

### Example 4. Assembly and Screening of Sequencing Data

DNA sequences obtained were compiled into an assembly project and aligned together to form contigs. This can be done efficiently using a computer program, such as Vector NTi, or alternatively by using the Pred/Phrap suite of DNA alignment and analysis programs. These contigs, along with any individual read that may not have been added to a contig, were compared to a compiled database of all classes of known pesticidal genes. Contigs or individual reads identified as having identity to a known endotoxin or pesticidal gene were analyzed further. Among the sequences obtained, clones pAX004, pAX006, pAX007, pAX008, pAX009, and pAX014 contained DNA identified as having homology to known endotoxin genes. Therefore, these clones were selected for further sequencing.

### Example 5. Sequencing and Identification of Delta-Endotoxin Genes

Primers were designed to anneal to sequences with homology to endotoxin genes, in a manner such that DNA sequences generated from such primers would overlap existing DNA sequence of the clone(s). This process, known as "oligo walking," is well known in the art. This process was utilized to determine the entire DNA sequence of the region exhibiting homology to a known endotoxin gene. In the case of the clones mentioned above, this process was used to determine the DNA sequence of the entire clone, resulting in a single nucleotide sequence for each gene. The completed DNA sequence was then placed back into the original large assembly for further validation. This allowed incorporation of more DNA sequence reads into the contig, resulting in multiple reads of coverage over the entire region.

Analysis of the DNA sequence of each region with homology to a known endotoxin gene identified an open reading frame with homology to a known delta-endotoxin gene. The open reading frame identified from pAX004 is designated as AXMI-004. The open reading frame identified from pAX006 is designated AXMI-006. The open reading frame identified from pAX007 is designated AXMI-007. The open reading frame identified from pAX008 is designated AXMI-008. The open reading frame identified from pAX009 is designated AXMI-009. The open reading frame identified from pAX014 is designated AXMI-014. The DNA sequence of AXMI-004 is provided as SEQ ID NOS:1 and 2, and the amino acid sequence of the predicted AMXI-004 protein is provided as SEQ ID NO:3. An alternate start site for AXMI-004 at nucleotide 385 of SEQ ID NO: 1 generates the amino acid sequence provided as SEQ ID NO:5. The DNA sequence of AXMI-006 is provided as SEQ ID NO:6, and the amino acid sequence of the predicted AMXI-006 protein is provided in SEQ ID NO:7. The DNA sequence of AXMI-007 is provided as SEQ ID NO:8, and the amino acid sequence of the predicted AMXI-007 protein is provided in SEQ ID NO:9. An alternate start site for AXMI-007 at nucleotide 151 of SEQ ID NO:8 generates the amino acid sequence provided as SEQ ID NO:11. The DNA sequence of AXMI-008 is provided as SEQ ID NOS:12 and 13, and the amino acid sequence of the predicted AMXI-008 protein is provided in SEQ ID NO:14. An alternate start site for AXMI-008 at nucleotide 177 of SEQ ID NO:12 generates the amino acid sequence provided as SEQ ID NO:16. Further analysis identified an open reading frame immediately 3' to the end of the AXMI-008 open reading frame. This predicted amino acid sequence of this orf, referred to herein as AXMI-008orf2, is provided in SEQ ID NO:18. The DNA sequence of AXMI-009 is provided as SEQ ID NO:19, and the amino acid sequence of the predicted AMXI-009 protein is provided in SEQ ID NO:20. An alternate start site for AXMI-009 at nucleotide 34 of SEQ ID NO:19 generates the amino acid sequence provided as SEQ ID NO:22. Another alternate start site for AXMI-009 at nucleotide 64 of SEQ ID NO:19 generates the amino acid sequence provided as SEQ ID NO:24. The DNA sequence of AXMI-014 is provided as SEQ ID NOS:25 and 26, and the amino acid sequence of the predicted AMXI-008 protein is provided as SEQ ID NO:27. An alternate start site for AXMI-014 at nucleotide 136 of SEQ ID NO:25 generates the amino acid sequence provided as SEQ ID NO:29.

### Example 6. Homology of Isolated Genes to Known Endotoxin Genes

Searches of DNA and protein databases with these DNA sequences and amino acid sequences reveal that these sequences are homologous to known endotoxins.

### AXMI-004

Figure 1 shows an alignment of AXMI-004 with several endotoxins. Blast searches identify cry1Ca as having the strongest block of homology, with an overall sequence identity in the toxic domain of 43% (see Table 1).

**Table 1. Amino Acid Identity of AXMI-004 with Exemplary Endotoxin Classes**

| Endotoxin | Percent Amino Acid Identity to AXMI-004 | Percent Amino Acid Identity in Toxic Domains |
|---|---|---|
| cry1Ac* | 17% | 30% |
| cry1Ca* | 24% | 43% |
| cry2Aa | 12% | 12% |
| cry3Aa | 33% | 33% |
| cry1Ia | 35% | 37% |
| cry7Aa | 19% | 31% |

### AXMI-006

Figure 2 shows an alignment of AXMI-006 with several endotoxins. Blast searches identify cry4Aa as having the strongest block of homology, though alignment of AMXI-006 protein (SEQ ID NO:7) to a large set of endotoxin proteins shows that the most homologous protein is cry10Aa. The overall amino acid identity of cry10Aa to AXMI-006 is 25% (see Table 2). Inspection of the amino acid sequence of AXMI-006 suggests that it does not contain a C-terminal non-toxic domain as is present in several endotoxin families. By removing this C-terminal protein of the toxins from the alignment, the alignment reflects the amino acid identity present solely in the toxin domains (see Table 2, column three). This 'trimmed' alignment is shown in Figure 2.

**Table 2. Amino Acid Identity of AXMI-006 with Exemplary Endotoxin Classes**

| Endotoxin | Percent Amino Acid Identity to AXMI-006 | Percent Amino Acid Identity of truncated Toxins to AXMI-006 |
|---|---|---|
| *cry1Aa* | 11% | 17% |
| *cry1Ac* | 12% | 19% |
| *cry1Ia* | 20% | 19% |
| *cry3Aa* | 20% | 19% |
| *cry3Bb* | 22% | 21% |
| *cry4Aa* | 16% | 26% |
| *cry6Aa* | 5% | 4% |
| *cry7Aa* | 12% | 19% |
| *cry8Aa* | 13% | 21% |
| *cry10Aa* | 25% | 25% |
| *cry16Aa* | 23% | 23% |
| *cry19Ba* | 24% | 24% |
| *cry24Aa* | 19% | 20% |

### AXMI-007

Blast searches identify *cry4Aa* as having the strongest block of homology to AXMI-007, though alignment of AMXI-007 protein (SEQ ID NO:9) to a large set of endotoxin proteins shows that the most homologous protein is *cry1 0Aa.* The overall amino acid identity of *cry10Aa* to AXMI-007 is 25% (see Table 3). Inspection of the amino acid sequence of AXMI-007 suggests that it does not contain a C-terminal non-toxic domain as is present in several endotoxin families. By removing this C-terminal protein of the toxins from the alignment, the alignment reflects the amino acid identify present solely in the toxin domains (see Table 3, column three). This 'trimmied' alignment is shown in Figure 3.

**Table 3. Amino Acid Identity of AXMI-007 with Exemplary Endotoxin Classes**

| Endotoxin | Percent Amino Acid Identity to AXMI-007 | Percent Amino Acid Identity of truncated Toxins to AXMI-007 |
|---|---|---|
| *cry1Aa* | 11% | 17% |
| *cry1Ac* | 12% | 20% |
| *cry1Ia* | 19% | 18% |
| *cry3Aa* | 19% | 19% |
| *cry3Bb* | 21% | 21% |
| *cry4Aa* | 17% | 27% |
| *cry6Aa* | 5% | 4% |
| *cry7Aa* | 13% | 19% |
| *cry8Aa* | 13% | 20% |
| *cry10Aa* | 25% | 25% |
| *cry16Aa* | 24% | 24% |
| *cry19Ba* | 25% | 25% |
| *cry24Aa* | 19% | 19% |

### AXMI-008

Blast searches identify *cry40Aa* as having the strongest block of homology to AXMI-008, and alignment of AMXI-008 protein (SEQ ID NO:14) to a large set of endotoxin proteins shows that the most homologous protein is cry40Aa. The overall amino acid identity of *cry40Aa* to AXMI-008 is 66% (see Table 4). Inspection of the amino acid sequence of AXMI-008 suggests that it does not contain a C-terminal non-toxic domain as is present in several endotoxin families. By removing this C-terminal protein of the toxins from the alignment, the alignment reflects the amino acid identify present solely in the toxin domains (see Table 4, column three). This 'trimmed' alignment is shown in Figure 4.

**Table 4. Amino Acid Identity of AXMI-008 with Exemplary Endotoxin Classes**

| Endotoxin | Percent Amino Acid Identity to AXMI-008 | Percent Amino Acid Identity of truncated Toxins to AXMI-008 |
|---|---|---|
| *cry1Aa* | 11% | 20% |
| *cry1Ac* | 11% | 20% |
| *cry1Ia* | 22% | 22% |
| *cry2A* | 10% | 10% |
| *cry3Aa* | 21% | 21% |
| *cry3Bb* | 21% | 21% |
| *cry4Aa* | 13% | 21% |
| *cry4Ba* | 13% | 20% |
| *cry6Aa* | 5% | 5% |
| *cry7Aa* | 12% | 20% |
| *cry8Aa* | 13% | 22% |
| *cry10Aa* | 20% | 20% |
| *cry16Aa* | 22% | 22% |
| *cry19Ba* | 21% | 22% |
| *cry24Aa* | 26% | 26% |
| *cry25Aa* | 23% | 23% |
| *cry39Aa* | 25% | 25% |
| *cry40Aa* | 66% | 66% |

The open reading frame immediately downstream (3') to the AXMI-008 coding region has homology to known endotoxin-related proteins. Blast searches identify *crybun3orf2* (the downstream orf of *cry40Aa*) as having the strongest block of homology. Several other orf-2 like proteins are present in databases, and an alignment of AMXI-008-orf2 protein (SEQ ID NO:18) to a set of these proteins is shown in Figure 5. These proteins also share homology to the C-terminal non-toxic domain of *cry4Aa* and *cry4Ba.* The overall amino acid identity of AXMI-8-orf2 to *cry40Aaorf2* is 86% (see Table 5).

**Table 5. Amino acid identity of AXMI-008-orf2 to related proteins**

| Protein | Percent amino acid identity to AXMI-008-orf2 |
|---|---|
| *crybun3orf2* (*cry40Aa orf2*) | 86% |
| *crybun2orf2* (*cry39Aa orf2*) | 85% |
| *cry19Aorf2* | 62% |
| C-terminus *cry4Aa* | 53% |
| C-terminus *cry4Ba* | 54% |

### AXMI-009

Blast searches identify cryBAa as having the strongest block of homology to AXMI-009, and alignment of AMXI-009 protein (SEQ ID NO:20) to a large set of endotoxin proteins shows that the most homologous proteins are cry3Ba and cry16Aa. The overall amino acid identity of cry3Ba and cry16Aa to AXMI-009 is 26% (see Table 6). Inspection of the amino acid sequence of AXMI-009 suggests that it does not contain a C-terminal non-toxic domain as is present in several endotoxin families. By removing this C-terminal protein of the toxins from the alignment, the alignment reflects the amino acid identify present solely in the toxin domains (see Table 6, column three). This 'trimmed' alignment is shown in Figure 6.

**Table 6. Amino Acid Identity of AXMI-009 with Exemplary Endotoxin Classes**

| Endotoxin | Percent Amino Acid Identity to AXMI-009 | Percent Amino Acid Identity of truncated Toxins to AXMI-009 |
|---|---|---|
| *cry1Aa* | 12% | 20% |
| *cry1Ac* | 13% | 23% |
| *cry1Ca* | 13% | 24 |
| *cry1Ia* | 24% | 26% |
| *cry3Aa* | 24% | 25% |
| *cry3Ba* | 26% | 27% |
| *cry3Bb* | 25% | 27% |
| *cry4Aa* | 13% | 24% |
| *cry6Aa* | 7% | 5% |
| *cry7Aa* | 15% | 25% |
| *cry8Aa* | 17% | 28% |
| *cry10Aa* | 24% | 24% |
| *cry16Aa* | 26% | 26% |
| *cry19Ba* | 24% | 25% |
| *cry24Aa* | 25% | 27% |
| *cry25Aa* | 23% | 23% |
| *cry40Aa* | 19% | 24% |

### AXMI-014

Blast searches identify *cry40Aa* as having the strongest block of homology to AXMI-014, and alignment of AMXI-0014 protein (SEQ ID NO:27) to a large set of endotoxin proteins shows that the most homologous protein is cry40Aa. The overall amino acid identity of *cry40Aa* to AXMI-014 is 55% (see Table 7). Inspection of the amino acid sequence of AXMI-014 suggests that it does not contain a C-terminal non-toxic domain as is present in several endotoxin families. By removing this C-terminal protein of the toxins from the alignment, the alignment reflects the amino acid identify present solely in the toxin domains (see Table 7, column three). This 'trimmed' alignment is shown in Figure 7.

**Table 7. Amino Acid Identity of AXMI-014 with Exemplary Endotoxin Classes**

| Endotoxin | Percent Amino Acid Identity to AXMI-014 | Percent Amino Acid Identity of truncated Toxins to AXMI-014 |
|---|---|---|
| *cry1Aa* | 12% | 21% |
| *cry1Ac* | 12% | 22% |
| *cry1Ia* | 20% | 21% |
| *cry2A* | 12% | 12% |
| *cry3Aa* | 20% | 20% |
| *cry3Bb* | 22% | 22% |
| *cry4Aa* | 12% | 19% |
| *cry4Ba* | 11% | 19% |
| *cry6Aa* | 6% | 6% |
| *cry7Aa* | 13% | 21% |
| *cry8Aa* | 14% | 22% |
| *cry10Aa* | 22% | 22% |
| *cry16Aa* | 24% | 24% |
| *cry19Ba* | 24% | 24% |
| *cry24Aa* | 29% | 29% |
| *cry25Aa* | 23% | 23% |
| *cry39Aa* | 22% | 22% |
| *cry40Aa* | 55% | 55% |

### Example 7. Homology between AXMI-006 and AXMI-007

Comparison of the amino acid sequences of AXMI-007 with AXMI-006 shows that the two toxins share significant amino acid homology. Alignment of the amino acid sequence of AXMI-006 (SEQ ID NO:7) and AXMI-007 (SEQ ID NO:9) show the proteins to be 85 % identical at the amino acid level. Thus AXMI-006 and AXMI-007 constitute a new class of related endotoxins.

### Example 8. Assays for Pesticidal Activity

The ability of a pesticidal protein to act as a pesticide upon a pest is often assessed in a number of ways. One way well known in the art is to perform a feeding assay. In such a feeding assay, one exposes the pest to a sample containing either compounds to be tested, or control samples. Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouthparts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson, J. L. & H. K. Preisler. 1992. Pesticide bioassays with arthropods. CRC, Boca Raton, FL. Alternatively, assays are commonly described in the journals "Arthropod Management Tests" and "Journal of Economic Entomology" or by discussion with members of the Entomological Society of America (ESA).

### Example 9. Cloning of AXMI-006 for Protein Expression

AXMI-006 was cloned into a vector for *E*. *coli* expression as follows. pAX480 contains the kanamycin resistance gene for selection of transformants, and the tac promoter which is inducible by IPTG for regulated protein expression. pAX480 was modified by inserting a DNA segment encoding a 6xHis-tag region immediately upstream of the insert cloning region, such that resulting clones contain a 6xHis-tag at the N-terminus of the expressed protein. Methods for expressing proteins with 6xHis-tag fusions, and their use for purification and analysis of protein expression are well known in the art.

The coding sequence for AXMI-006 was PCR-amplified using *PfuUltra*™ High-Fidelity DNA Polymerase (Stratagene). Oligonucleotide primers were designed such that the resulting PCR product contained desired restriction sites near each end, to facilitate cloning. The resulting PCR product (approximately 2.2 kb) was digested with the appropriate restriction enzyme, and subcloned into the modified pAX480. Insert-containing clones were identified by restriction analysis. The resulting clone, pAX906, contained the AXMI-006 open reading frame fused to the six his tag, such that transcription and translation resulted in production of a 'fusion protein' with a stretch of six histidines. The DNA sequence of pAX906 was confirmed by DNA sequence analysis and subsequently transformed into chemically competent *E*. *coli* BL21, as described by the manufacturer (Stratagene, La Jolla, CA).

A single colony of pAX906 in BL21 was inoculated into LB media supplemented with kanamycin and grown for several hours at 37°C with vigorous agitation. These cultures were grown to an OD₆₀₀ ranging from 0.6-0.8; then protein production was induced by addition of 0.1 mM IPTG. Cultures were grown under inducing conditions for 3 hours, and then the cells were pelleted by centrifugation and resuspended in PBS. Cells were sonicated using a Misonix Sonicator 3000 for a total of 30 seconds using 10-second sonication intervals and incubation on ice for one minute.

### Example 10. Bioassay of AXMI-006 Activity on Heliothis virescens and Spodoptera frugiperda

Bioassays of sonicated pAX906 cultures were performed using artificial diet (Multiple Species Diet, Southland Products, Lake Village, Arkansas). Bioassays were carried out by applying sonicated pAX906 cells, or cells of the *E.coli* strain as a control, to the diet surface and allowing the diet surface to dry. Bioassays were performed in 24-well tissue culture plates. The bioassays were held in the dark at 25° C and 65% relative humidity. Trays were sealed with Breathe Easy Sealing Tape (Diversified Biotech, Boston, MA). Results were recorded at 5 days.

**Table 8. Bioassay of AXMI-006 on Spodoptera frugiperda**

| **Sample** | **Bioassay Result** |
|---|---|
| AXMI-006 | Stunting |
| Negative control | No Stunting |

**Table 9. Bioassay of AXMI-006 on Heliothis virescens**

| **Sample** | **Bioassay Result** |
|---|---|
| AXMI-006 | Stunting |
| Negative Control | No Stunting |

Stunting is defined reduced insect size, and severally reduced larval feeding. Stunted insects may also demonstrate avoidance of the treated diet compared to the untreated diet.

### Example 11. Pesticidal Activity of AXMI-008, AXMI-009, and AXMI-014 on Trichoplusia ni (Cabbage Looper)

*Escherichia coli* strains containing either pAX008, pAX009 or pAX-014, as well as a culture of untransformed *Escherichia coli* were grown in 2 ml of LB Broth (Luria-Bertani Broth, Becton Dickinson & Company, Sparks, Md.) for 24 hours at 37° C with agitation at 250 rpm. Plasmid-containing strains were grown in grown in LB containing the appropriate antibiotic to select for maintenance of the plasmid in *E*. *coli.*

Bioassays were performed using artificial diet (Multiple Species Diet, Southland Products, Lake Village, Arkansas) in 24 well tissue culture plates. Bioassays were carried out by applying the *Escherichia coli* culture containing pAX-014 to the diet surface and allowing the diet surface to dry. The strains were applied as whole cultures to the diet at a concentration of 40 µl of culture per well. The bioassays were held in the dark at 25° C and 65% relative humidity. Trays were sealed with Breathe Easy Sealing Tape (Diversified Biotech, Boston, MA). Results were recorded at 5 days.

**Table 10. Pesticidal Activity on T. ni**

| **Sample** | **# Dead/ Total** | **% Mortality** |
|---|---|---|
| AXMI-014 | 13/13 | 100% |
| AXMI-008 | 6/6 | 100% |
| AXMI-009 | 17/17 | 100% |
| Negative Control | 0/13 | 0% |

### Example 12. Expression of Delta-Endotoxin Genes in Bacillus

AXMI-004, AXMI-006, AXMI-007, AXMI-008, and AXMI-009 were amplified by PCR from the clones from Example 4, and cloned into the *Bacillus* Expression vector pAX916 by methods well known in the art. For AXMI-004 the resulting clone was designated pAX920. For AXMI-006 the resulting clone was designated pAX921. For AXMI-007 the resulting clone was designated pAX919. For AXMI-008 the resulting clone was designated pAX922. For AXMI-009 the resulting clone was designated pAX917. The resulting clones expressed the relevant protein when transformed into cells of a *cry(-) Bacillus thuringiensis* strain. The *Bacillus* strains containing delta-endotoxin genes and expressing the delta-endotoxin proteins may be cultured on a variety of conventional growth media. A *Bacillus* strain containing the desired gene was grown in CYS media (10 g/l Bacto-casitone; 3 g/l yeast extract; 6 g/l KH₂PO₄; 14 g/l K₂HPO₄; 0.5 mM MgSO₄; 0.05 mM MnCl₂; 0.05 mM FeSO₄), until sporulation was evident by microscopic examination. Samples were prepared, and delta-endotoxin proteins were tested for insecticidal activity in bioassays against important insect pests.

### Methods

To prepare CYS media: 10 g/l Bacto-casitone; 3 g/l yeast extract; 6 g/l KH₂PO₄; 14 g/l K₂HPO₄; 0.5 mM MgSO₄; 0.05 mM MnCl₂; 0.05 mM FeSO₄. The CYS mix should be pH 7, if adjustment is necessary. NaOH or HCl are preferred. The media is then autoclaved and 100 ml of 10X filtered glucose is added after autoclaving. If the resultant solution is cloudy it can be stirred at room temperature to clear.

### Example 13. N-terminal Amino Acid Sequence of AXMI-004 Expressed in Bacillus

Analysis of AXMI-004 expressed in *Bacillus* suggested that the protein product detected in these cultures may be reduced in size relative to the full-length AXMI-004 protein. Since many endotoxin proteins are cleaved at the N-terminus after expression in *Bacillus,* we determined the N-terminus of the AXMI-004 protein resulting from *Bacillus* expression. Protein samples from AXMI-004 were separated on PAGE gels, and the protein transferred to PVDF membrane by methods known in the art. The protein band corresponding to AXMI-004 was excised. The N-terminal amino acid sequence of this protein was determined by serial Edman degradation as known in the art. The sequence obtained was as follows:
ERFDKNDALE

Comparison of this amino acid sequence with the sequence of the full length AXMI-004 (SEQ ID NO:3) demonstrates that this amino sequence results from internal cleavage of the AXMI-004 after expression in *Bacillus,* resulting in a protein with an N-terminus corresponding to amino acid 28 of SEQ ID NO:3 (disclosed as SEQ ID NO:5).

### Example 14. Bioassay of AXMI-004 on Insect Pests

Insecticidal activity of AXMI-004 was established utilizing accepted bioassay procedures using a sporulated *Bacillus* cell culture lysate expressing AXMI-004. The *Bacillus* culture was grown in 50 ml CYS media for both standard bioassay and LC₅₀ bioassays. The cultures were then grown for 2 to 3 days at 30°C, 250 rpm until the cells were sporulated. Sporulation was determined by examining microscopically for the presence of spores. AXMI-004 protein samples were prepared by centrifugation of the sporulated cultures at 12,000 x g for 10 min. The pellet was collected and resuspended in 4 ml 20 mM Tris-HCl, pH 8.0. The suspension was sonicated for 20 seconds (at top power using a micro probe) while placing the tube on ice. The protein concentration of the sample was determined by electrophoresis on an SDS 4-20% gradient acrylamide gel along with a known quantity of bovine serum albumin (BSA) (Figure 8). The concentration of AXMI-004 was determined to be 0.4 µg/ul.

AXMI-004 insecticidal activity was tested using a surface treatment bioassay with artificial diet (Multiple Species diet, Southland Products, Lake Village, Arkansas) prepared as known in the art. Bioassays were carried out by applying the *Bacillus* culture expressing AXMI-004 to the diet surface and allowing the surface to air-dry. Standard bioassays utilized five eggs per well and LC₅₀ bioassays utilized ten neonate insect larvae per well. The eggs or larvae were applied using a fine tip paintbrush. Standard surface bioassays were carried out in 24 well tissue culture plates. 40ul of each sample was applied to each well. Since each well has a surface area of 2 cm² (plate source), a 40 µl cell lysate sample contained approximately 0.4 ug/ul AXMI-004. Bioassays where the LC₅₀ was determined were done in 48 well tissue culture plates, each well representing a surface area of 1 cm² (source) using approximately 20ul of 0.4 µg/ul AXMI-004 per well. The final amount of AXMI-004 protein in each bioassay was approximately 8 µg/cm². Bioassay trays were sealed with Breathe Easy Sealing Tape (Diversified Biotech, Boston MA). Control samples included media only samples, and wells that were not treated with samples. Bioassays were then held for five days in the dark at 25° C and 65% relative humidity and results recorded.

**Table 11. Insecticidal Activity of AXMI-004**

| **Insect (Latin Name)** | **Common Name** | **Activity of AXMI-004** |
|---|---|---|
| *Ostrinia nubilalis* | European Corn Borer | 100% mortality |
| *Agrotis ipsilon* | Black Cutworm | Stunted |
| *Heliothis zea* | Corn Earworm | Stunted |
| *Spodoptera frugiperda* | Fall Armyworm | Stunted |
| *Heliothis virescens* | Tobacco Budworm | 100% mortality |
| *Pectinophora gossypiella* | Pink Bollworm | 75% mortality |
| *Manduca sexta* | Tobacco Hornworm | 100% mortality |
| *Trichoplusia ni* | Cabbage Looper | 100% mortality |

AXMI-004 showed strong insecticidal activity (100% mortality) against *Ostrinia nubilalis* and *Heliothis virescens.* AXMI-004 also showed insecticidal activity of 50-75% mortality against *Pectinophora gossypiella.* A concentration of 43 µg/cm² AXMI-004 gave 70% mortality against *Pectinophora gossypiella.* AXMI-004 severely stunted the growth of *Agrotis ipsilon, Heliothis zea,* and *Spodoptera frugiperda.*

### Example 15. Quantitation of AXMI-004 Insecticidal Activity against Heliothis virescens and Ostrinia nubilalis

The LC₅₀ of AXMI-004 protein on *Ostrinia nubilalis and Heliothis virescens* larvae were determined by testing a range of AXMI-004 protein concentrations in insect bioassays, and applying these protein samples to the surface of insect diet. Mortality was recorded at each protein concentration and analyzed using a Probit analysis program. Results were significant at the 95% confidence interval. Since assays were performed by surface contamination, LC₅₀s were determined assuming that the entire protein sample remained at the surface during the assay, with little diffusion below the level ingested by the insects. Thus, the values determined may somewhat underestimate the toxicity of the AXMI-004 protein on the tested insects.

**Table 12. LC₅₀ of AXMI-004 on Ostrinia nubilalis**

| **AXMI-004 (µg/ml)** | **# dead/total** | **% Mortality** |
|---|---|---|
| 1000 | 40/46 | 86.9 |
| 500 | 28/45 | 62.2 |
| 250 | 16/43 | 32.7 |
| 125 | 12/38 | 31.6 |

LC₅₀= 297 ng/cm² ; 95% CI = 218-3 84.

**Table 13. LC₅₀ of AXMI-004 on Heliothis virescens**

| **AXMI-004 (µg/ml)** | **# dead/total** | **% Mortality** |
|---|---|---|
| 8000 | 35/47 | 74.5 |
| 4000 | 26/44 | 59.1 |
| 2000 | 18/42 | 42.9 |
| 1000 | 6/27 | 22.2 |
| 500 | 4/36 | 11.1 |
| 250 | 2/37 | 5.4 |

LC₅₀= 2874 ng/cm²; 95% CI = 2189-3933

### Example 16. Quantitation of AXMI-004, AXMI-006, AXMI-007, and AXMI-009 Insecticidal Activity against Lygus lineolaris

Bacterial lysates were prepared by growing the *Bacillus* in 50 ml of CYS media for 60 hours. The *Bacillus* culture was then centrifuged at 12,000 rpm for ten minutes and the supernatant discarded. The pellet was resuspended in 5 ml of 20 mM Tris HCl at pH 8.

Bioassays were performed by cutting both the tip and the cap off an Eppendorf tube to form a feeding chamber. The insecticidal protein or control was presented to the insect in a solution that was poured into the cap and covered with parafilm (Pechiney Plastic Packaging, Chicago IL) that the insect could pierce upon feeding. The Eppendorf tube was placed back on the cap top down and 1^{st} or 2^{nd} instar *Lygus* nymphs were placed into the Eppendorf chamber with a fine tip brush. The cut Eppendorf tube tip was sealed with parafilm creating an assay chamber. The resultant assay chamber was incubated at ambient temperature cap side down. Insecticidal proteins were tested in a solution of 15% glucose at a concentration of 6.6 ug/ml.

**Table 14. Insecticidal Activity on Lygus lineolaris**

| **Protein** | **No. Dead/Total** | **% Mortality** |
|---|---|---|
| AXMI-004 | 2/4 | 50% |
| AXMI-006 | 1/6 | 16.7% |
| AXMI-007 | 3/6 | 50% |
| AXMI-009 | 2/4 | 50% |
| Control | 0/9 | 0% |

### Example 17. Bioassay of AXMI-008 on Tenebrio molitor

Samples of *Bacillus* cultures expressing AXMI -008 were prepared and tested for pesticidal activity on *Tenebrio molitor.* When pAX 922 is prepared as an insoluble fraction at pH 4.0 it showed activity against commonly called the yellow mealworm.

Samples of AXMI-008 were prepared from a culture of a *Bacillus* strain containing pAX 922. The bioassay sample was prepared by growing a culture in CYS media for 4 days, until sporulation. The sample was centrifuged at 10,000 rpm for 10 minutes and the supernatant discarded. The pellet was washed in 20mM Tris pH 8 and spun at 10,000 rpm for 10 minutes. The supernatant was discarded and the pellet resuspended in 3 mls of 50 mM Sodium Citrate and 25 mM Sodium Chloride with 2mM DTT at pH 4. The sample was incubated at 37° C for 1 hour. After incubation the sample was spun at 13,000 rpm for 10 minutes and the supernatant discarded. The pellet was resuspended in 50 mM Sodium Citrate and 25 mM Sodium Chloride at pH 4.

Bioassays of samples on *Tenebrio molitor* were performed on an artificial diet (Southern Corn Rootworm Diet, Bioserv, Frenchtown, NJ, #F9757B) in 24 well tissue culture plates. The sample was applied as a surface treatment with a concentration of Axmi008 at 8 ug/cm² and allowed to air dry. The insects were applied using a fine tip brush. Bioassay trays were sealed with Breathe Easy Sealing Tape (Diversified Biotech, Boston, MA) and incubated without light at 65% relative humidity, 25° C. for seven days and results recorded.

**Table 15. Pesticidal Activity of AXMI-008 on T. molitor**

| **Sample** | **# Dead/ Total** | **% Mortality** |
|---|---|---|
| AXMI-008 | 3 of 4 | 75%* |

| | | |
|---|---|---|
| * Remaining *Tenebrio molitor* was stunted. Stunting is observed as reduced larval size and growth, and severely reduced or minimal feeding. The insect may also demonstrate avoidance of the treated diet compared to the untreated diet | | |

### Example 18. Bioassay of AXMI-009 Protein on Coleopteran Pests

Bioassays of AXMI-009 protein preparations were performed by pipetting 40 µl of insoluble fraction onto a 2 cm² diet surface for a final total protein concentration of 8µg/cm². *Diabrotica virgifera virgifera* and *Diabrotica undecimpunctata* were tested using Southern Corn Rootworm Diet (Bioserv, Frenchtown, NJ, #F9757B). Bioassays were carried out by applying the *Bacillus* culture expressing AXMI-009 to the diet surface and allowing the diet surface to dry. Bioassays were performed in 24 well tissue culture plates. Standard bioassays utilized 25 eggs per well. The eggs were applied in a solution containing 0.1 % agar and 30 ug/ml nystatin. Trays were sealed with Breathe Easy Sealing Tape (Diversified Biotech, Boston, MA) and the lids placed back on the trays. Bioassays were incubated without light at 65% Relative Humidity (RH), 25 ° C for seven days. Activity was seen with the insoluble fraction for both *Diabrotica virgifera virgifera* and *Diabrotica undecimpunctata.* Controls were a media only, buffer of 1 mM Tris at pH 10.5, and the *Bacillus* expression vector pAX916.

**Table 16. Western Corn Rootworm (Diabrotica virgifera virgifera)**

| | **# Dead/Total** | **% Mortality** |
|---|---|---|
| AXMI-009 insoluble fraction | 38/38 | 100% |
| Media only (CYS) | 1/25 | 4% |
| Buffer | 2/24 | 8.3% |
| Vector (pAX916) | 1/12 | 8.3% |

**Table 17. Southern Corn Rootworn (Diabrotica undecimpunctata)**

| | **# Dead/Total** | **% Mortality** |
|---|---|---|
| AXMI-009 insoluble fraction | 20/20 | 100% |
| Media only (CYS) | 0/20 | 0% |
| Buffer | 0/23 | 0% |
| Vector (pAX916) | 0/19 | 0% |

### Example 19. Vectoring for Plant Expression

The delta-endotoxin coding region DNA is operably connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

The plant expression cassettes described above are combined with an appropriate plant selectable marker to aid in the selections of transformed cells and tissues, and ligated into plant transformation vectors. These may include binary vectors from Agrobacterium-mediated transformation or simple plasmid vectors for aerosol or biolistic transformation.

### Example 20. Transformation of Maize Cells

Maize ears are collected 8-12 days after pollination. Embryos are isolated from the ears, and those embryos 0.8-1.5 mm in size are used for transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casaminoacids; 50 g/L sucrose; 1 mL/L (of 1 mg/mL Stock) 2,4-D), and incubated overnight at 25°C in the dark.

The resulting explants are transferred to mesh squares (30-40 per plate), transferred onto osmotic media for 30-45 minutes, then transferred to a beaming plate (see, for example, PCT Publication No. WO/0138514 and U.S. Patent No. 5,240,842).

DNA constructs designed to express the delta-endotoxin in plant cells are accelerated into plant tissue using an aerosol beam accelerator, using conditions essentially as described in PCT Publication No. WO/0138514. After beaming, embryos are incubated for 30 min on osmotic media, then placed onto incubation media overnight at 25°C in the dark. To avoid unduly damaging beamed explants, they are incubated for at least 24 hours prior to transfer to recovery media. Embryos are then spread onto recovery period media, for 5 days, 25°C in the dark, then transferred to a selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

| **DN62A5S Media** | | |
|---|---|---|
| **Components** | **per liter** | **Source** |
| Chu'S N6 Basal Salt Mixture (Prod. No. C 416) | 3.98 g/L | Phytotechnology Labs |
| Chu's N6 Vitamin Solution (Prod. No. C 149) | 1 mL/L (of 1000x Stock) | Phytotechnology Labs |
| L-Asparagine | 800 mg/L | Phytotecbnology Labs |
| Myo-inositol | 100 mg/L | Sigma |
| L-Proline | 1.4 g/L | Phytotechnology Labs |
| Casaminoacids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

Adjust the pH of the solution to pH to 5.8 with 1N KOH/1N KCl, add Gelrite (Sigma) to 3g/L, and autoclave. After cooling to 50°C, add 2 ml/L of a 5 mg/ml stock solution of Silver Nitrate (Phytotechnology Labs). Recipe yields about 20 plates.

### Example 21. Transformation into Plant Cells by Agrobacterium-Mediated Transformation

Ears are collected 8-12 days after pollination. Embryos are isolated from the ears, and those embryos 0.8-1.5 mm in size are used for transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for 5-10 min, and then plated onto co-cultivation media for 3 days (25°C in the dark). After co-cultivation, explants are transferred to recovery period media for five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art. The resulting shoots are allowed to root on rooting media, and the resulting plants are transferred to nursery pots and propagated as transgenic plants.

### Conclusions

The delta-endotoxin proteins described herein have activity against numerous pests, as shown in the examples above. AXMI-004 has pesticidal activity against pests including *Ostrinia nubilalis, Agrotis ipsilon, Heliothis zea, Spodoptera frugiperda, Heliothis virescens, Pectinophora gossypiella, Manduca Sexta, Trichoplasia ni,* and *Lygus lineolaris.* AXMI-006 has pesticidal activity against pests including *Heliothis virescens, Spodoptera frugiperda,* and *Lygus lineolaris.* AXMI-007 has pesticidal activity against pests including *Lygus lineolaris.* AXMI-008 has pesticidal activity against pests including *Tenebrio molitor* and *Trichoplusia ni.* AXMI-009 has pesticidal activity against pests including *Lygus lineolaris, Trichoplusia ni, Diabrotica virgifera virgifera,* and *Diabrotica undecimpunctata.* AXMI-014 has pesticidal activity against pests including *Trichoplusia ni.*

Although the foregoing invention has been described in some detail by way of illustration and example for purposes of clarity of understanding, it will be obvious that certain changes and modifications may be practiced within the scope of the appended claims.

## Claims

1. A nucleic acid molecule selected from the group consisting of:
a) a nucleic acid molecule comprising the nucleotide sequence of SEQ ID NO: 12, 13, 15, or 17;
b) a nucleic acid molecule comprising a nucleotide sequence having at least 95% sequence identity to the nucleotide sequence of SEQ ID NO:12, 13, 15, or 17, wherein said nucleotide sequence encodes a polypeptide having pesticidal activity;
c) a nucleic acid molecule which encodes a polypeptide comprising the amino acid sequence of SEQ ID NO: 14, 16, or 18;
d) a nucleic acid molecule comprising a nucleotide sequence encoding a polypeptide having at least 95% amino acid sequence identity to the amino acid sequence of SEQ ID NO:14, 16, or 18, wherein said polypeptide has pesticidal activity; and,
e) a complement of any of a)-d).

2. The nucleic acid molecule of claim 1, wherein said nucleotide sequence is a synthetic sequence that has been designed for expression in a plant.

3. The nucleic acid molecule of claim 2, wherein said synthetic sequence has an increased GC content relative to the GC content of SEQ ID NO: 12, 13, 15, or 17.

4. The nucleic acid molecule of claim 1, wherein said nucleotide sequence is operably linked to a promoter that drives expression of said nucleotide sequence in a plant cell.

5. A vector comprising the nucleic acid molecule of claim 1.

6. A host cell that contains the vector of claim 5.

7. The host cell of claim 6 that is a bacterial host cell or a plant host cell.

8. A polypeptide selected from the group consisting of:
a) a polypeptide comprising the amino acid sequence of SEQ ID NO: 14, 16, or 18;
b) a polypeptide encoded by the nucleotide sequence of SEQ ID NO: 12, 13, 15, or 17, wherein said polypeptide has pesticidal activity;
c) a polypeptide comprising an amino acid sequence having at least 95% sequence identity to the amino acid sequence of SEQ ID NO: 14, 16, or 18, wherein said polypeptide has pesticidal activity; and,
d) a polypeptide that is encoded by a nucleotide sequence that is at least 95% identical to the nucleotide sequence of SEQ ID NO:12, 13, 15, or 17, wherein said polypeptide has pesticidal activity.

9. The polypeptide of claim 8, further comprising a heterologous amino acid sequence.

10. An antibody that selectively binds to a polypeptide of claim 8.

11. A composition comprising the polypeptide of claim 8, wherein said composition is optionally selected from the group consisting of a powder, dust, pellet, granule, spray, emulsion, colloid, and solution, wherein said composition is optionally prepared by desiccation, lyophilization, homogenization, extraction, filtration, centrifugation, sedimentation, or concentration of a culture of *Bacillus thuringiensis* cells, and wherein said composition comprises from 1% to 99% by weight of said polypeptide.

12. A method for producing a polypeptide with pesticidal activity, comprising culturing the host cell of claim 6 under conditions in which a nucleic acid molecule encoding the polypeptide is expressed, said polypeptide being selected from the group consisting of:
a) a polypeptide comprising the amino acid of SEQ ID NO: 14, 16, or 18;
b) a polypeptide encoded by the nucleotide sequence of SEQ ID NO: 12, 13, 15, or 17, wherein said polypeptide has pesticidal activity;
c) a polypeptide comprising an amino acid sequence having at least 95% sequence identity to the amino acid sequence of SEQ ID NO:14, 16, or 18, wherein said polypeptide has pesticidal activity; and,
d) a polypeptide that is encoded by a nucleotide sequence that is at least 95% identical to a nucleotide sequence of SEQ ID NO:12, 13, 15, or 17, wherein said polypeptide has pesticidal activity.

13. A method for controlling or killing a lepidopteran or coleopteran pest population comprising contacting said population with a pesticidally-effective amount of a polypeptide of claim 8.

14. A plant or a plant cell having stably incorporated into its genome a DNA construct comprising a nucleotide sequence that encodes a protein having pesticidal activity, wherein said nucleotide sequence is selected from the group consisting of:
a) a nucleotide sequence of SEQ ID NO:12, 13, or 15;
b) a nucleotide sequence having at least 95% sequence identity to a nucleotide sequence of SEQ ID NO:12, 13, or 15, wherein said nucleotide sequence encodes a polypeptide having pesticidal activity;
c) a nucleotide sequence encoding a polypeptide comprising an amino acid sequence of SEQ ID NO:14 or 16; and,
d) a nucleotide sequence encoding a polypeptide having at least 95% amino acid sequence identity to the amino acid sequence of SEQ ID NO:14 or 16, wherein said polypeptide has pesticidal activity;
wherein said nucleotide sequence is operably linked to a promoter that drives expression of a coding sequence in a plant cell.

15. A transgenic seed of the plant or plant cell of claim 14.

16. The plant or plant cell of claim 14 or the transgenic seed of claim 15, wherein said plant, plant cell, or transgenic seed further comprises the nucleotide sequence of SEQ ID NO:17.

17. The transgenic plant or plant cell of claim 14 or the transgenic seed of claim 15, wherein said plant, plant cell, or seed is selected from the group consisting of maize, sorghum, wheat, sunflower, tomato, crucifers, peppers, potato, cotton, rice, soybean, sugarbeet, sugarcane, tobacco, barley, and oilseed rape.

## Patentansprüche

1. Nucleinsäuremolekül, ausgewählt aus der Gruppe, bestehend aus:
a) einem Nucleinsäuremolekül, das die Nucleotidsequenz von SEQ ID NO:12, 13, 15 oder 17 umfasst;
b) einem Nucleinsäuremolekül, das eine Nucleotidsequenz umfasst, die wenigstens 95% Sequenzidentität zu der Nucleotidsequenz von SEQ ID NO:12, 13, 15 oder 17 hat, wobei die Nucleotidsequenz für ein Polypeptid mit pestizider Aktivität codiert;
c) einem Nucleinsäuremolekül, welches für ein Polypeptid codiert, das die Aminosäuresequenz von SEQ ID NO:14, 16 oder 18 umfasst;
d) einem Nucleinsäuremolekül, das eine Nucleotidsequenz umfasst, die für ein Polypeptid codiert, das wenigstens 95% Aminosäuresequenzidentität zu der Aminosäuresequenz von SEQ ID NO:14, 16 oder 18 hat, wobei das Polypeptid pestizide Aktivität hat; und
e) einem Komplement von einem von a) bis d).

2. Nucleinsäuremolekül nach Anspruch 1, wobei die Nucleotidsequenz eine synthetische Sequenz ist, die zur Expression in einer Pflanze entwickelt wurde.

3. Nucleinsäuremolekül nach Anspruch 2, wobei die synthetische Sequenz einen erhöhten GC-Gehalt im Vergleich zu dem GC-Gehalt von SEQ ID NO:12, 13, 15 oder 17 hat.

4. Nucleinsäuremolekül nach Anspruch 1, wobei die Nucleotidsequenz funktionell mit einem Promotor verknüpft ist, der die Expression der Nucleotidsequenz in einer Pflanzenzelle steuert.

5. Vektor, umfassend das Nucleinsäuremolekül nach Anspruch 1.

6. Wirtszelle, die den Vektor nach Anspruch 5 enthält.

7. Wirtszelle nach Anspruch 6, die eine bakterielle Wirtszelle oder eine Pflanzenwirtszelle ist.

8. Polypeptid, ausgewählt aus der Gruppe, bestehend aus:
a) einem Polypeptid, das die Aminosäuresequenz von SEQ ID NO:14, 16 oder 18 umfasst;
b) einem Polypeptid, das durch die Nucleotidsequenz von SEQ ID NO:12, 13, 15 oder 17 codiert wird, wobei das Polypeptid pestizide Aktivität hat;
c) einem Polypeptid, das eine Aminosäuresequenz umfasst, die wenigstens 95% Sequenzidentität zu der Aminosäuresequenz von SEQ ID NO: 14, 16 oder 18 hat, wobei das Polypeptid pestizide Aktivität hat; und
d) einem Polypeptid, das durch eine Nucleotidsequenz codiert wird, die wenigstens 95% identisch zu der Nucleotidsequenz von SEQ ID NO:12, 13, 15 oder 17 ist, wobei das Polypeptid pestizide Aktivität hat.

9. Polypeptid nach Anspruch 8, das außerdem eine heterologe Aminosäuresequenz umfasst.

10. Antikörper, der selektiv an ein Polypeptid nach Anspruch 8 bindet.

11. Zusammensetzung, umfassend das Polypeptid nach Anspruch 8, wobei die Zusammensetzung gegebenenfalls ausgewählt ist aus der Gruppe, bestehend aus Pulver, Stäubepulver, Pellets, Granulat, Spray, Emulsion, Kolloid und Lösung, wobei die Zusammensetzung gegebenenfalls durch Trocknung, Lyophilisierung, Homogenisierung, Extraktion, Filtration, Zentrifugation, Sedimentation oder Konzentration einer Kultur von Bacil*lus thuringiensis-Zellen* hergestellt wird und wobei die Zusammensetzung 1 bis 99 Gew.-% des Polypeptids umfasst.

12. Verfahren zur Herstellung eines Polypeptids mit pestizider Aktivität, umfassend Kultivieren der Wirtszelle nach Anspruch 6 unter Bedingungen, bei denen ein Nucleinsäuremolekül, das für das Polypeptid codiert, exprimiert wird, wobei das Polypeptid ausgewählt ist aus der Gruppe, bestehend aus:
a) einem Polypeptid, das die Aminosäuresequenz von SEQ ID NO:14, 16 oder 18 umfasst;
b) einem Polypeptid, das durch die Nucleotidsequenz von SEQ ID NO:12, 13, 15 oder 17 codiert wird, wobei das Polypeptid pestizide Aktivität hat;
c) einem Polypeptid, das eine Aminosäuresequenz umfasst, die wenigstens 95% Sequenzidentität zu der Aminosäuresequenz von SEQ ID NO: 14, 16 oder 18 hat, wobei das Polypeptid pestizide Aktivität hat; und
d) einem Polypeptid, das durch eine Nucleotidsequenz codiert wird, die wenigstens 95% identisch zu einer Nucleotidsequenz von SEQ ID NO:12, 13, 15 oder 17 ist, wobei das Polypeptid pestizide Aktivität hat.

13. Verfahren zur Bekämpfung oder Abtötung einer Lepidopteren- oder Coleopteren-Schädlingspopulation, umfassend Inkontaktbringen der Population mit einer pestizid wirksamen Menge eines Polypeptids nach Anspruch 8.

14. Pflanze oder Pflanzenzelle, die stabil in ihr Genom ein DNA-Konstrukt eingebaut hat, umfassend eine Nucleotidsequenz, die für ein Protein mit pestizider Aktivität codiert, wobei die Nucleotidsequenz ausgewählt ist aus der Gruppe, bestehend aus:
a) einer Nucleotidsequenz von SEQ ID NO:12, 13 oder 15;
b) einer Nucleotidsequenz, die wenigstens 95% Sequenzidentität zu einer Nucleotidsequenz von SEQ ID NO:12, 13 oder 15 hat, wobei die Nucleotidsequenz für ein Polypeptid mit pestizider Aktivität codiert;
c) einer Nucleotidsequenz, welche für ein Polypeptid codiert, das die Aminosäuresequenz von SEQ ID NO:14 oder 16 umfasst;
d) einer Nucleotidsequenz, die für ein Polypeptid codiert, das wenigstens 95% Aminosäuresequenzidentität zu der Aminosäuresequenz von SEQ ID NO: 14 oder 16 hat, wobei das Polypeptid pestizide Aktivität hat;
wobei die Nucleotidsequenz funktionell mit einem Promotor verknüpft ist, der die Expression einer codierenden Sequenz in einer Pflanzenzelle steuert.

15. Transgener Samen der Pflanze oder Pflanzenzelle nach Anspruch 14.

16. Pflanze oder Pflanzenzelle nach Anspruch 14 oder transgener Samen nach Anspruch 15, wobei die Pflanze, die Pflanzenzelle oder der transgene Samen außerdem die Nucleotidsequenz von SEQ ID NO 17 umfasst.

17. Transgene Pflanze oder Pflanzenzelle nach Anspruch 14 oder transgener Samen nach Anspruch 15, wobei die Pflanze, die Pflanzenzelle oder der Samen ausgewählt ist aus der Gruppe, bestehend aus Mais, Sorghum, Weizen, Sonnenblume, Tomate, Cruciferen, Paprika, Kartoffel, Baumwolle, Reis, Sojabohne, Zuckerrübe, Zuckerrohr, Tabak, Gerste und Ölsamenraps.

## Revendications

1. Molécule d'acide nucléique choisie dans le groupe constitué de :
a) une molécule d'acide nucléique comprenant la séquence nucléotidique de SEQ ID NO:12, 13, 15, ou 17 ;
b) une molécule d'acide nucléique comprenant une séquence nucléotidique ayant au moins 95 % d'identité de séquence avec la séquence nucléotidique de SEQ ID NO:12, 13, 15, ou 17, dans laquelle ladite séquence nucléotidique code pour un polypeptide ayant un activité pesticide ;
c) une molécule d'acide nucléique qui code pour un polypeptide comprenant la séquence d'acides aminés de SEQ ID NO : 14, 16 ou 18 ;
d) une molécule d'acide nucléique comprenant une séquence nucléotidique codant pour un polypeptide ayant au moins 95 % d'identité de séquence d'acides aminés avec la séquence d'acides aminés de SEQ ID NO : 14, 16 ou 18, dans laquelle ledit polypeptide a une activité pesticide ; et,
e) un complément de l'une quelconque de a) à d).

2. Molécule d'acide nucléique de la revendication 1, dans laquelle ladite séquence nucléotidique est une séquence de synthèse qui a été conçue pour l'expression dans une plante.

3. Molécule d'acide nucléique de la revendication 2, dans laquelle ladite séquence de synthèse a un contenu en GC accru par rapport au contenu en GC de SEQ ID NO: 12, 13, 15, ou 17.

4. Molécule d'acide nucléique de la revendication 1, dans laquelle ladite séquence nucléotidique est liée de manière fonctionnelle à un promoteur qui commande l'expression de ladite séquence nucléotidique dans une cellule végétale.

5. Vecteur comprenant la molécule d'acide nucléique de la revendication 1.

6. Cellule hôte qui contient le vecteur de la revendication 5.

7. Cellule hôte de la revendication 6 qui est une cellule hôte bactérienne ou une cellule hôte végétale.

8. Polypeptide choisi dans le groupe constitué de :
a) un polypeptide comprenant la séquence d'acides aminés de SEQ ID NO : 14, 16 ou 18 ;
b) un polypeptide codé par la séquence nucléotidique de SEQ ID NO : 12, 13, 15, ou 17, dans lequel ledit polypeptide a une activité pesticide.
c) un polypeptide comprenant une séquence d'acides aminés ayant au moins 95 % d'identité de séquence avec la séquence d'acides aminés de SEQ ID NO : 14, 16 ou 18 dans lequel ledit polypeptide a une activité pesticide ; et,
d) un polypeptide qui est codé par une séquence nucléotidique qui est au moins identique à 95 % à la séquence nucléotidique de SEQ ID NO : 12, 13, 15 ou 17, dans lequel ledit polypeptide a une activité pesticide.

9. Polypeptide de la revendication 8, comprenant en outre une séquence d'acides aminés hétérologue.

10. Anticorps qui se lie sélectivement à un polypeptide de la revendication 8.

11. Composition comprenant le polypeptide de la revendication 8, dans laquelle ladite composition est éventuellement choisie dans le groupe constitué d'une poudre, poussière, pastille, granule, spray, émulsion, colloïde et solution, dans laquelle ladite composition est éventuellement préparée par dessiccation, lyophilisation, homogénéisation, extraction, filtration, centrifugation, sédimentation ou concentration d'une culture de cellules de *Bacillus thuringiensis,* et dans laquelle ladite. composition comprend de 1 % à 99 % en poids dudit polypeptide.

12. Méthode de production d'un polypeptide présentant une activité pesticide, comprenant la culture de la cellule hôte de la revendication 6 dans des conditions dans lesquelles une molécule d'acide nucléique codant pour le polypeptide est exprimée, ledit polypeptide étant choisi dans le groupe constitué de :
a) un polypeptide comprenant la séquence d'acides aminés de SEQ ID NO : 14, 16 ou 18 ;
b) un polypeptide codé par la séquence nucléotidique de SEQ ID NO : 12, 13, 15, ou 17, dans laquelle ledit polypeptide a une activité pesticide ;
c) un polypeptide comprenant une séquence d'acides aminés ayant au moins 95 % d'identité de séquence avec la séquence d'acides aminés de SEQ ID NO : 14, 16 ou 18, dans laquelle ledit polypeptide a une activité pesticide ; et,
d) un polypeptide qui est codé par une séquence nucléotidique qui est au moins identique à 95 % à la séquence nucléotidique de SEQ ID NO : 12, 13,15 ou 17, dans laquelle ledit polypeptide a une activité pesticide.

13. Méthode pour contrôler ou tuer une population de lépidoptères ou coléoptères ravageurs comprenant la mise en contact de ladite population avec une quantité pesticide efficace d'un polypeptide de la revendication 8.

14. Plante ou cellule végétale ayant incorporé de manière stable dans son génome une construction d'ADN comprenant une séquence nucléotidique qui code pour une protéine ayant une activité pesticide, dans laquelle ladite séquence nucléotidique est choisie dans le groupe constitué de :
a) une séquence nucléotidique de SEQ ID NO : 12, 13 ou 15 ;
b) une séquence nucléotidique ayant au moins 95 % d'identité de séquence avec une séquence nucléotidique de SEQ ID NO : 12, 13 ou 15, dans laquelle ladite séquence nucléotidique code pour un polypeptide ayant une activité pesticide ;
c) une séquence nucléotidique codant pour un polypeptide comprenant une séquence d'acides aminés de SEQ ID NO : 14 ou 16 ; et,
d) une séquence nucléotidique codant pour un polypeptide ayant au moins 95% d'identité de séquence d'acides aminés avec la séquence d'acides aminés de SEQ ID NO : 14 ou 16, dans laquelle ledit polypeptide a une activité pesticide ;
dans laquelle ladite séquence nucléotidique est liée de manière fonctionnelle à un promoteur qui commande l'expression d'une séquence codante dans une cellule végétale.

15. Semence transgénique de la plante ou de la cellule végétale de la revendication 14.

16. Plante ou cellule végétale de la revendication 14 ou semence transgénique de la revendication 15, dans laquelle ladite plante, cellule végétale ou semence transgénique comprend en outre la séquence nucléotidique de SEQ ID NO: 17.

17. Plante transgénique ou cellule végétale de la revendication 14 ou semence transgénique de la revendication 15, dans laquelle ladite plante, cellule végétale ou semence est choisie dans le groupe constitué du maïs, sorgho, blé, tournesol, tomate, crucifères, poivrons, pomme de terre, cotonnier, riz, soja, betterave sucrière, canne à sucre, tabac, orge, et colza oléagineux.


### SEQUENCE LISTING

<110> Carozzi, Nadine
   Hargies, Tracy
   Koziel, Michael G.
   Duck, Nicholas B.
   Carr, Brian
<120> Delta-Endotoxin Genes and Methods for Their Use
<130> 045600/274379
<150> 60/448,632
   <151> 2003-02-20
<150> 60/448,633
   <151> 2003-02-20
<150> 60/448,797
   <151> 2003-02-20
<150> 60/448,806
   <151> 2003-02-20
<150> 60/448,810
   <151> 2003-02-20
<150> 60/448,812
   <151> 2003-02-20
<160> 52
<170> FastSEQ for Windows Version 4.0
<210> 1
   <211> 2190
   <212> DNA
   <213> Bacillus thuringiensis
<400> 1
   tatgatgata actttgacaa cagccgcatc cataacgtac tttttcaata ttaattgtat 60
   ccaagcttat tttgtacaaa ttacacttta ttgtatgcat aatagtttct tattgattga 120
   gcatataaga aaattttttc atatataagt gaaaatattt ttgcttttct tttttttatt 180
   taggataaac tttacttgta aacacaagta aatagtttat tagagataga gcctcatttt 240
   aaaatattta cgaatcctat ctataaaaat ctaattttaa aattattcct tttaaatgta 300
   atgagtgaat tgaaggggaa atttaagaaa agtactaatc gaacttgttg tttgctaaaa 360
   ataataaata taggaggaag aggtatgaat tcaaaggaac atgattatct aaaagtttgt 420
   aatgatttaa gtgacgccaa tattaatatg gaacggtttg ataagaatga tgcactggaa 480
   attggtatgt ccattgtatc tgaacttatt ggtatgattc caggcggaac agctttgcaa 540
   tttgtgttta atcaattgtg gtctcgttta ggtgattctg gatggaatgc gttcatggaa 600
   catgtggagg aattaattga tactaaaata gaagggtatg caaaaaataa agccttatct 660
   gaattagcag gtatacaaag aaaccttgaa acatatatac aattacgtaa tgaatgggaa 720
   aatgatattg aaaactcaaa ggctcaaggt aaggtagcta attactatga aagtcttgag 780
   caggcggttg aaaggagtat gcctcaattt gcagtggaga attttgaagt accactttta 840
   actgtctatg tgcaagctgc taatcttcat ttattattat taagagatgt ttcagtttat 900
   ggaaagtgtt ggggatggtc ggagcagaaa attaaaattt attatgataa acagattaag 960
   tatacccatg aatacacaaa tcattgtgta aattggtata ataaaggact tgagagatta 1020
   aaaaataaag gttcttctta tcaagattgg tacaattata atcgtttccg tagagaaatg 1080
   actcttactg ttttagatat cgttgcttta ttcccgcact atgatgtaca aacttatcca 1140
   ataacaaccg ttgctcagct aacaagggaa gtttatacgg atcctttact taattttaat 1200
   cctaaattac attctgtgtc tcaattacct agttttagtg acatggaaaa tgcaacaatt 1260
   agaactccac atctgatgga atttttaaga atgctaacaa tttatacaga ttggtatagt 1320
   gtgggaagaa actattattg gggaggacat cgcgtgacgt cttaccatgt aggaggagag 1380
   aatataagat cacctctata tggtagagag gcaaatcaag aggttcctag agatttttat 1440
   ttttatggac ccgtttttaa gacgttatca aagccgactc taagaccatt acagcagcct 1500
   gcaccagctc ctccttttaa tttacgtagc ttagagggag tagaattcca cactcctaca 1560
   ggtagtttta tgtatcgtga aagaggatcg gtagattctt ttaatgagtt gccgcctttt 1620
   aatccagttg ggttacctca taaggtatac agtcaccgtt tatgtcatgc aacgtttgtt 1680
   cgtaaatctg ggacccctta tttaacaaca ggtgccatct tttcttggac acatcgtagt 1740
   gctgaagaaa ccaatacaat tgaatcaaat attattacgc aaatcccgtt agtaaaagca 1800
   tatcaaattg ggtcaggcac tactgtaagg aaaggaccag gattcacagg aggggatata 1860
   cttcgaagaa caggtcctgg aacatttgga gatatgagaa taaatattaa tgcaccatta 1920
   tctcaaagat atcgtgtaag gattcgttat gcttctacga cagatttaca atttgtcacg 1980
   agtattaatg ggaccaccat taatattggt aacttcccga aaactattaa taatctaaat 2040
   actttaggtt ctgagggcta tagaacagta tcgtttagta ctccatttag tttctcaaat 2100
   gcacaaagca tatttagatt aggtatacaa gcattttctg gagttcaaga agtttatgtg 2160
   gataaaattg aatttattcc tgttgaatag                                  2190
<210> 2
   <211> 1890
   <212> DNA
   <213> Bacillus thuringiensis
<220>
   <221> CDS
   <222> (1)...(1890)
<400> 2
   atg agt gaa ttg aag ggg aaa ttt aag aaa agt act aat cga act tgt    48
   Met Ser Glu Leu Lys Gly Lys Phe Lys Lys Ser Thr Asn Arg Thr Cys
    1               5                  10                  15
   tgt ttg cta aaa ata ata aat ata gga gga aga ggt atg aat tca aag    96
   Cys Leu Leu Lys Ile Ile Asn Ile Gly Gly Arg Gly Met Asn Ser Lys
               20                  25                  30
   gaa cat gat tat cta aaa gtt tgt aat gat tta agt gac gcc aat att    144
   Glu His Asp Tyr Leu Lys Val Cys Asn Asp Leu Ser Asp Ala Asn Ile
           35                  40                  45
   aat atg gaa cgg ttt gat aag aat gat gca ctg gaa att ggt atg tcc    192
   Asn Met Glu Arg Phe Asp Lys Asn Asp Ala Leu Glu Ile Gly Met Ser
       50                  55                  60
   att gta tct gaa ctt att ggt atg att cca ggc gga aca gct ttg caa    240
   Ile Val Ser Glu Leu Ile Gly Met Ile Pro Gly Gly Thr Ala Leu Gln
   65                  70                  75                  80
   ttt gtg ttt aat caa ttg tgg tct cgt tta ggt gat tct gga tgg aat    288
   Phe Val Phe Asn Gln Leu Trp Ser Arg Leu Gly Asp Ser Gly Trp Asn
                   85                  90                  95
   gcg ttc atg gaa cat gtg gag gaa tta att gat act aaa ata gaa ggg    336
   Ala Phe Met Glu His Val Glu Glu Leu Ile Asp Thr Lys Ile Glu Gly
               100                 105                 110
   tat gca aaa aat aaa gcc tta tct gaa tta gca ggt ata caa aga aac    384
   Tyr Ala Lys Asn Lys Ala Leu Ser Glu Leu Ala Gly Ile Gln Arg Asn
           115                 120                 125
   ctt gaa aca tat ata caa tta cgt aat gaa tgg gaa aat gat att gaa    432
   Leu Glu Thr Tyr Ile Gln Leu Arg Asn Glu Trp Glu Asn Asp Ile Glu
       130                 135                 140
   aac tca aag gct caa ggt aag gta gct aat tac tat gaa agt ctt gag    480
   Asn Ser Lys Ala Gln Gly Lys Val Ala Asn Tyr Tyr Glu Ser Leu Glu
   145                 150                 155                 160
   cag gcg gtt gaa agg agt atg cct caa ttt gca gtg gag aat ttt gaa    528
   Gln Ala Val Glu Arg Ser Met Pro Gln Phe Ala Val Glu Asn Phe Glu
                   165                 170                 175
   gta cca ctt tta act gtc tat gtg caa gct gct aat ctt cat tta tta    576
   Val Pro Leu Leu Thr Val Tyr Val Gln Ala Ala Asn Leu His Leu Leu
               180                 185                 190
   tta tta aga gat gtt tca gtt tat gga aag tgt tgg gga tgg tcg gag      624
   Leu Leu Arg Asp Val Ser Val Tyr Gly Lys Cys Trp Gly Trp Ser Glu
           195                 200                 205
   cag aaa att aaa att tat tat gat aaa cag att aag tat acc cat gaa      672
   Gln Lys Ile Lys Ile Tyr Tyr Asp Lys Gln Ile Lys Tyr Thr His Glu
       210                 215                 220
   tac aca aat cat tgt gta aat tgg tat aat aaa gga ctt gag aga tta      720
   Tyr Thr Asn His Cys Val Asn Trp Tyr Asn Lys Gly Leu Glu Arg Leu
   225                 230                 235                 240
   aaa aat aaa ggt tct tct tat caa gat tgg tac aat tat aat cgt ttc      768
   Lys Asn Lys Gly Ser Ser Tyr Gln Asp Trp Tyr Asn Tyr Asn Arg Phe
                   245                 250                 255
   cgt aga gaa atg act ctt act gtt tta gat atc gtt gct tta ttc ccg      816
   Arg Arg Glu Met Thr Leu Thr Val Leu Asp Ile Val Ala Leu Phe Pro
               260                 265                 270
   cac tat gat gta caa act tat cca ata aca acc gtt gct cag cta aca      864
   His Tyr Asp Val Gln Thr Tyr Pro Ile Thr Thr Val Ala Gln Leu Thr
           275                 280                 285
   agg gaa gtt tat acg gat cct tta ctt aat ttt aat cct aaa tta cat      912
   Arg Glu Val Tyr Thr Asp Pro Leu Leu Asn Phe Asn Pro Lys Leu His
       290                 295                 300
   tct gtg tct caa tta cct agt ttt agt gac atg gaa aat gca aca att      960
   Ser Val Ser Gln Leu Pro Ser Phe Ser Asp Met Glu Asn Ala Thr Ile
   305                 310                 315                 320
   aga act cca cat ctg atg gaa ttt tta aga atg cta aca att tat aca     1008
   Arg Thr Pro His Leu Met Glu Phe Leu Arg Met Leu Thr Ile Tyr Thr
                   325                 330                 335
   gat tgg tat agt gtg gga aga aac tat tat tgg gga gga cat cgc gtg     1056
   Asp Trp Tyr Ser Val Gly Arg Asn Tyr Tyr Trp Gly Gly His Arg Val
               340                 345                 350
   acg tct tac cat gta gga gga gag aat ata aga tca cct cta tat ggt     1104
   Thr Ser Tyr His Val Gly Gly Glu Asn Ile Arg Ser Pro Leu Tyr Gly
           355                 360                 365
   aga gag gca aat caa gag gtt cct aga gat ttt tat ttt tat gga ccc     1152
   Arg Glu Ala Asn Gln Glu Val Pro Arg Asp Phe Tyr Phe Tyr Gly Pro
       370                 375                 380
   gtt ttt aag acg tta tca aag ccg act cta aga cca tta cag cag cct     1200
   Val Phe Lys Thr Leu Ser Lys Pro Thr Leu Arg Pro Leu Gln Gln Pro
   385                 390                 395                 400
   gca cca gct cct cct ttt aat tta cgt agc tta gag gga gta gaa ttc     1248
   Ala Pro Ala Pro Pro Phe Asn Leu Arg Ser Leu Glu Gly Val Glu Phe
                   405                 410                 415
   cac act cct aca ggt agt ttt atg tat cgt gaa aga gga tcg gta gat     1296
   His Thr Pro Thr Gly Ser Phe Met Tyr Arg Glu Arg Gly Ser Val Asp
               420                 425                 430
   tct ttt aat gag ttg ccg cct ttt aat cca gtt ggg tta cct cat aag     1344
   Ser Phe Asn Glu Leu Pro Pro Phe Asn Pro Val Gly Leu Pro His Lys
           435                 440                 445
   gta tac agt cac cgt tta tgt cat gca acg ttt gtt cgt aaa tct ggg     1392
   Val Tyr Ser His Arg Leu Cys His Ala Thr Phe Val Arg Lys Ser Gly
       450                 455                 460
   acc cct tat tta aca aca ggt gcc atc ttt tct tgg aca cat cgt agt    1440
   Thr Pro Tyr Leu Thr Thr Gly Ala Ile Phe Ser Trp Thr His Arg Ser
   465                 470                 475                 480
   gct gaa gaa acc aat aca att gaa tca aat att att acg caa atc ccg    1488
   Ala Glu Glu Thr Asn Thr Ile Glu Ser Asn Ile Ile Thr Gln Ile Pro
                   485                 490                 495
   tta gta aaa gca tat caa att ggg tca ggc act act gta agg aaa gga    1536
   Leu Val Lys Ala Tyr Gln Ile Gly Ser Gly Thr Thr Val Arg Lys Gly
               500                 505                 510
   cca gga ttc aca gga ggg gat ata ctt cga aga aca ggt cct gga aca    1584
   Pro Gly Phe Thr Gly Gly Asp Ile Leu Arg Arg Thr Gly Pro Gly Thr
           515                 520                 525
   ttt gga gat atg aga ata aat att aat gca cca tta tct caa aga tat    1632
   Phe Gly Asp Met Arg Ile Asn Ile Asn Ala Pro Leu Ser Gln Arg Tyr
       530                 535                 540
   cgt gta agg att cgt tat gct tct acg aca gat tta caa ttt gtc acg    1680
   Arg Val Arg Ile Arg Tyr Ala Ser Thr Thr Asp Leu Gln Phe Val Thr
   545                 550                 555                 560
   agt att aat ggg acc acc att aat att ggt aac ttc ccg aaa act att    1728
   Ser Ile Asn Gly Thr Thr Ile Asn Ile Gly Asn Phe Pro Lys Thr Ile
                   565                 570                 575
   aat aat cta aat act tta ggt tct gag ggc tat aga aca gta tcg ttt    1776
   Asn Asn Leu Asn Thr Leu Gly Ser Glu Gly Tyr Arg Thr Val Ser Phe
               580                 585                 590
   agt act cca ttt agt ttc tca aat gca caa agc ata ttt aga tta ggt    1824
   Ser Thr Pro Phe Ser Phe Ser Asn Ala Gln Ser Ile Phe Arg Leu Gly
           595                 600                 605
   ata caa gca ttt tct gga gtt caa gaa gtt tat gtg gat aaa att gaa    1872
   Ile Gln Ala Phe Ser Gly Val Gln Glu Val Tyr Val Asp Lys Ile Glu
       610                 615                 620
   ttt att cct gtt gaa tag                                            1890
   Phe Ile Pro Val Glu  *
   625
<210> 3
   <211> 629
   <212> PRT
   <213> Bacillus thuringiensis
<400> 3
   Met Ser Glu Leu Lys Gly Lys Phe Lys Lys Ser Thr Asn Arg Thr Cys
   1               5                   10                  15
   Cys Leu Leu Lys Ile Ile Asn Ile Gly Gly Arg Gly Met Asn Ser Lys
               20                  25                  30
   Glu His Asp Tyr Leu Lys Val Cys Asn Asp Leu Ser Asp Ala Asn Ile
           35                  40                  45
   Asn Met Glu Arg Phe Asp Lys Asn Asp Ala Leu Glu Ile Gly Met Ser
       50                  55                  60
   Ile Val Ser Glu Leu Ile Gly Met Ile Pro Gly Gly Thr Ala Leu Gln
   65                  70                  75                  80
   Phe Val Phe Asn Gln Leu Trp Ser Arg Leu Gly Asp Ser Gly Trp Asn
                   85                  90                  95
   Ala Phe Met Glu His Val Glu Glu Leu Ile Asp Thr Lys Ile Glu Gly
               100                 105                 110
   Tyr Ala Lys Asn Lys Ala Leu Ser Glu Leu Ala Gly Ile Gln Arg Asn
           115                 120                 125
   Leu Glu Thr Tyr Ile Gln Leu Arg Asn Glu Trp Glu Asn Asp Ile Glu
       130                 135                 140
   Asn Ser Lys Ala Gln Gly Lys Val Ala Asn Tyr Tyr Glu Ser Leu Glu
   145                 150                 155                 160
   Gln Ala Val Glu Arg Ser Met Pro Gln Phe Ala Val Glu Asn Phe Glu
                   165                 170                 175
   Val Pro Leu Leu Thr Val Tyr Val Gln Ala Ala Asn Leu His Leu Leu
               180                 185                 190
   Leu Leu Arg Asp Val Ser Val Tyr Gly Lys Cys Trp Gly Trp Ser Glu
           195                 200                 205
   Gln Lys Ile Lys Ile Tyr Tyr Asp Lys Gln Ile Lys Tyr Thr His Glu
       210                 215                 220
   Tyr Thr Asn His Cys Val Asn Trp Tyr Asn Lys Gly Leu Glu Arg Leu
   225                 230                 235                 240
   Lys Asn Lys Gly Ser Ser Tyr Gln Asp Trp Tyr Asn Tyr Asn Arg Phe
                   245                 250                 255
   Arg Arg Glu Met Thr Leu Thr Val Leu Asp Ile Val Ala Leu Phe Pro
               260                 265                 270
   His Tyr Asp Val Gln Thr Tyr Pro Ile Thr Thr Val Ala Gln Leu Thr
           275                 280                 285
   Arg Glu Val Tyr Thr Asp Pro Leu Leu Asn Phe Asn Pro Lys Leu His
       290                 295                 300
   Ser Val Ser Gln Leu Pro Ser Phe Ser Asp Met Glu Asn Ala Thr Ile
   305                 310                 315                 320
   Arg Thr Pro His Leu Met Glu Phe Leu Arg Met Leu Thr Ile Tyr Thr
                   325                 330                 335
   Asp Trp Tyr Ser Val Gly Arg Asn Tyr Tyr Trp Gly Gly His Arg Val
               340                 345                 350
   Thr Ser Tyr His Val Gly Gly Glu Asn Ile Arg Ser Pro Leu Tyr Gly
           355                 360                 365
   Arg Glu Ala Asn Gln Glu Val Pro Arg Asp Phe Tyr Phe Tyr Gly Pro
       370                 375                 380
   Val Phe Lys Thr Leu Ser Lys Pro Thr Leu Arg Pro Leu Gln Gln Pro
   385                 390                 395                 400
   Ala Pro Ala Pro Pro Phe Asn Leu Arg Ser Leu Glu Gly Val Glu Phe
                   405                 410                 415
   His Thr Pro Thr Gly Ser Phe Met Tyr Arg Glu Arg Gly Ser Val Asp
               420                 425                 430
   Ser Phe Asn Glu Leu Pro Pro Phe Asn Pro Val Gly Leu Pro His Lys
           435                 440                 445
   Val Tyr Ser His Arg Leu Cys His Ala Thr Phe Val Arg Lys Ser Gly
       450                 455                 460
   Thr Pro Tyr Leu Thr Thr Gly Ala Ile Phe Ser Trp Thr His Arg Ser
   465                 470                 475                 480
   Ala Glu Glu Thr Asn Thr Ile Glu Ser Asn Ile Ile Thr Gln Ile Pro
                   485                 490                 495
   Leu Val Lys Ala Tyr Gln Ile Gly Ser Gly Thr Thr Val Arg Lys Gly
               500                 505                 510
   Pro Gly Phe Thr Gly Gly Asp Ile Leu Arg Arg Thr Gly Pro Gly Thr
           515                 520                 525
   Phe Gly Asp Met Arg Ile Asn Ile Asn Ala Pro Leu Ser Gln Arg Tyr
       530                 535                 540
   Arg Val Arg Ile Arg Tyr Ala Ser Thr Thr Asp Leu Gln Phe Val Thr
   545                 550                 555                 560
   Ser Ile Asn Gly Thr Thr Ile Asn Ile Gly Asn Phe Pro Lys Thr Ile
                   565                 570                 575
   Asn Asn Leu Asn Thr Leu Gly Ser Glu Gly Tyr Arg Thr Val Ser Phe
               580                 585                 590
   Ser Thr Pro Phe Ser Phe Ser Asn Ala Gln Ser Ile Phe Arg Leu Gly
           595                 600                 605
   Ile Gln Ala Phe Ser Gly Val Gln Glu Val Tyr Val Asp Lys Ile Glu
       610                 615                 620
   Phe Ile Pro Val Glu
   625
<210> 4
   <211> 1806
   <212> DNA
   <213> Bacillus thuringiensis
<220>
   <221> CDS
   <222> (1)...(1806)
<400> 4
   atg aat tca aag gaa cat gat tat cta aaa gtt tgt aat gat tta agt     48
   Met Asn Ser Lys Glu His Asp Tyr Leu Lys Val Cys Asn Asp Leu Ser
    1               5                  10                  15
   gac gcc aat att aat atg gaa cgg ttt gat aag aat gat gca ctg gaa     96
   Asp Ala Asn Ile Asn Met Glu Arg Phe Asp Lys Asn Asp Ala Leu Glu
               20                  25                  30
   att ggt atg tcc att gta tct gaa ctt att ggt atg att cca ggc gga     144
   Ile Gly Met Ser Ile Val Ser Glu Leu Ile Gly Met Ile Pro Gly Gly
           35                  40                  45
   aca gct ttg caa ttt gtg ttt aat caa ttg tgg tct cgt tta ggt gat     192
   Thr Ala Leu Gln Phe Val Phe Asn Gln Leu Trp Ser Arg Leu Gly Asp
       50                  55                  60
   tct gga tgg aat gcg ttc atg gaa cat gtg gag gaa tta att gat act     240
   Ser Gly Trp Asn Ala Phe Met Glu His Val Glu Glu Leu Ile Asp Thr
   65                  70                  75                  80
   aaa ata gaa ggg tat gca aaa aat aaa gcc tta tct gaa tta gca ggt     288
   Lys Ile Glu Gly Tyr Ala Lys Asn Lys Ala Leu Ser Glu Leu Ala Gly
                   85                  90                  95
   ata caa aga aac ctt gaa aca tat ata caa tta cgt aat gaa tgg gaa     336
   Ile Gln Arg Asn Leu Glu Thr Tyr Ile Gln Leu Arg Asn Glu Trp Glu
               100                 105                 110
   aat gat att gaa aac tca aag gct caa ggt aag gta gct aat tac tat     384
   Asn Asp Ile Glu Asn Ser Lys Ala Gln Gly Lys Val Ala Asn Tyr Tyr
           115                 120                 125
   gaa agt ctt gag cag gcg gtt gaa agg agt atg cct caa ttt gca gtg     432
   Glu Ser Leu Glu Gln Ala Val Glu Arg Ser Met Pro Gln Phe Ala Val
       130                 135                 140
   gag aat ttt gaa gta cca ctt tta act gtc tat gtg caa gct gct aat     480
   Glu Asn Phe Glu Val Pro Leu Leu Thr Val Tyr Val Gln Ala Ala Asn
   145                 150                 155                 160
   ctt cat tta tta tta tta aga gat gtt tca gtt tat gga aag tgt tgg     528
   Leu His Leu Leu Leu Leu Arg Asp Val Ser Val Tyr Gly Lys Cys Trp
                   165                 170                 175
   gga tgg tcg gag cag aaa att aaa att tat tat gat aaa cag att aag     576
   Gly Trp Ser Glu Gln Lys Ile Lys Ile Tyr Tyr Asp Lys Gln Ile Lys
               180                 185                 190
   tat acc cat gaa tac aca aat cat tgt gta aat tgg tat aat aaa gga     624
   Tyr Thr His Glu Tyr Thr Asn His Cys Val Asn Trp Tyr Asn Lys Gly
           195                 200                 205
   ctt gag aga tta aaa aat aaa ggt tct tct tat caa gat tgg tac aat     672
   Leu Glu Arg Leu Lys Asn Lys Gly Ser Ser Tyr Gln Asp Trp Tyr Asn
       210                 215                 220
   tat aat cgt ttc cgt aga gaa atg act ctt act gtt tta gat atc gtt     720
   Tyr Asn Arg Phe Arg Arg Glu Met Thr Leu Thr Val Leu Asp Ile Val
   225                 230                 235                 240
   gct tta ttc ccg cac tat gat gta caa act tat cca ata aca acc gtt      768
   Ala Leu Phe Pro His Tyr Asp Val Gln Thr Tyr Pro Ile Thr Thr Val
                   245                 250                 255
   gct cag cta aca agg gaa gtt tat acg gat cct tta ctt aat ttt aat      816
   Ala Gln Leu Thr Arg Glu Val Tyr Thr Asp Pro Leu Leu Asn Phe Asn
               260                 265                 270
   cct aaa tta cat tct gtg tct caa tta cct agt ttt agt gac atg gaa      864
   Pro Lys Leu His Ser Val Ser Gln Leu Pro Ser Phe Ser Asp Met Glu
           275                 280                 285
   aat gca aca att aga act cca cat ctg atg gaa ttt tta aga atg cta      912
   Asn Ala Thr Ile Arg Thr Pro His Leu Met Glu Phe Leu Arg Met Leu
       290                 295                 300
   aca att tat aca gat tgg tat agt gtg gga aga aac tat tat tgg gga      960
   Thr Ile Tyr Thr Asp Trp Tyr Ser Val Gly Arg Asn Tyr Tyr Trp Gly
   305                 310                 315                 320
   gga cat cgc gtg acg tct tac cat gta gga gga gag aat ata aga tca     1008
   Gly His Arg Val Thr Ser Tyr His Val Gly Gly Glu Asn Ile Arg Ser
                   325                 330                 335
   cct cta tat ggt aga gag gca aat caa gag gtt cct aga gat ttt tat     1056
   Pro Leu Tyr Gly Arg Glu Ala Asn Gln Glu Val Pro Arg Asp Phe Tyr
               340                 345                 350
   ttt tat gga ccc gtt ttt aag acg tta tca aag ccg act cta aga cca     1104
   Phe Tyr Gly Pro Val Phe Lys Thr Leu Ser Lys Pro Thr Leu Arg Pro
           355                 360                 365
   tta cag cag cct gca cca gct cct cct ttt aat tta cgt agc tta gag     1152
   Leu Gln Gln Pro Ala Pro Ala Pro Pro Phe Asn Leu Arg Ser Leu Glu
       370                 375                 380
   gga gta gaa ttc cac act cct aca ggt agt ttt atg tat cgt gaa aga     1200
   Gly Val Glu Phe His Thr Pro Thr Gly Ser Phe Met Tyr Arg Glu Arg
   385                 390                 395                 400
   gga tcg gta gat tct ttt aat gag ttg ccg cct ttt aat cca gtt ggg     1248
   Gly Ser Val Asp Ser Phe Asn Glu Leu Pro Pro Phe Asn Pro Val Gly
                   405                 410                 415
   tta cct cat aag gta tac agt cac cgt tta tgt cat gca acg ttt gtt     1296
   Leu Pro His Lys Val Tyr Ser His Arg Leu Cys His Ala Thr Phe Val
               420                 425                 430
   cgt aaa tct ggg acc cct tat tta aca aca ggt gcc atc ttt tct tgg     1344
   Arg Lys Ser Gly Thr Pro Tyr Leu Thr Thr Gly Ala Ile Phe Ser Trp
           435                 440                 445
   aca cat cgt agt gct gaa gaa acc aat aca att gaa tca aat att att     1392
   Thr His Arg Ser Ala Glu Glu Thr Asn Thr Ile Glu Ser Asn Ile Ile
       450                 455                 460
   acg caa atc ccg tta gta aaa gca tat caa att ggg tca ggc act act     1440
   Thr Gln Ile Pro Leu Val Lys Ala Tyr Gln Ile Gly Ser Gly Thr Thr
   465                 470                 475                 480
   gta agg aaa gga cca gga ttc aca gga ggg gat ata ctt cga aga aca     1488
   Val Arg Lys Gly Pro Gly Phe Thr Gly Gly Asp Ile Leu Arg Arg Thr
                   485                 490                 495
   ggt cct gga aca ttt gga gat atg aga ata aat att aat gca cca tta     1536
   Gly Pro Gly Thr Phe Gly Asp Met Arg Ile Asn Ile Asn Ala Pro Leu
                   500                 505                 510
   tct caa aga tat cgt gta agg att cgt tat gct tct acg aca gat tta     1584
   Ser Gln Arg Tyr Arg Val Arg Ile Arg Tyr Ala Ser Thr Thr Asp Leu
               515                 520                 525
   caa ttt gtc acg agt att aat ggg acc acc att aat att ggt aac ttc     1632
   Gln Phe Val Thr Ser Ile Asn Gly Thr Thr Ile Asn Ile Gly Asn Phe
       530                 535                 540
   ccg aaa act att aat aat cta aat act tta ggt tct gag ggc tat aga     1680
   Pro Lys Thr Ile Asn Asn Leu Asn Thr Leu Gly Ser Glu Gly Tyr Arg
   545                 550                 555                 560
   aca gta tcg ttt agt act cca ttt agt ttc tca aat gca caa agc ata     1728
   Thr Val Ser Phe Ser Thr Pro Phe Ser Phe Ser Asn Ala Gln Ser Ile
                   565                 570                 575
   ttt aga tta ggt ata caa gca ttt tct gga gtt caa gaa gtt tat gtg     1776
   Phe Arg Leu Gly Ile Gln Ala Phe Ser Gly Val Gln Glu Val Tyr Val
                   580                 585                 590
   gat aaa att gaa ttt att cct gtt gaa tag                             1806
   Asp Lys Ile Glu Phe Ile Pro Val Glu  *
               595                 600
<210> 5
   <211> 601
   <212> PRT
   <213> Bacillus thuringiensis
<400> 5
   Met Asn Ser Lys Glu His Asp Tyr Leu Lys Val Cys Asn Asp Leu Ser
   1               5                   10                  15
   Asp Ala Asn Ile Asn Met Glu Arg Phe Asp Lys Asn Asp Ala Leu Glu
               20                  25                  30
   Ile Gly Met Ser Ile Val Ser Glu Leu Ile Gly Met Ile Pro Gly Gly
           35                  40                  45
   Thr Ala Leu Gln Phe Val Phe Asn Gln Leu Trp Ser Arg Leu Gly Asp
       50                  55                  60
   Ser Gly Trp Asn Ala Phe Met Glu His Val Glu Glu Leu Ile Asp Thr
   65                  70                  75                  80
   Lys Ile Glu Gly Tyr Ala Lys Asn Lys Ala Leu Ser Glu Leu Ala Gly
                   85                  90                  95
   Ile Gln Arg Asn Leu Glu Thr Tyr Ile Gln Leu Arg Asn Glu Trp Glu
               100                 105                 110
   Asn Asp Ile Glu Asn Ser Lys Ala Gln Gly Lys Val Ala Asn Tyr Tyr
           115                 120                 125
   Glu Ser Leu Glu Gln Ala Val Glu Arg Ser Met Pro Gln Phe Ala Val
       130                 135                 140
   Glu Asn Phe Glu Val Pro Leu Leu Thr Val Tyr Val Gln Ala Ala Asn
   145                 150                 155                 160
   Leu His Leu Leu Leu Leu Arg Asp Val Ser Val Tyr Gly Lys Cys Trp
                   165                 170                 175
   Gly Trp Ser Glu Gln Lys Ile Lys Ile Tyr Tyr Asp Lys Gln Ile Lys
               180                 185                 190
   Tyr Thr His Glu Tyr Thr Asn His Cys Val Asn Trp Tyr Asn Lys Gly
           195                 200                 205
   Leu Glu Arg Leu Lys Asn Lys Gly Ser Ser Tyr Gln Asp Trp Tyr Asn
       210                 215                 220
   Tyr Asn Arg Phe Arg Arg Glu Met Thr Leu Thr Val Leu Asp Ile Val
   225                 230                 235                 240
   Ala Leu Phe Pro His Tyr Asp Val Gln Thr Tyr Pro Ile Thr Thr Val
                   245                 250                 255
   Ala Gln Leu Thr Arg Glu Val Tyr Thr Asp Pro Leu Leu Asn Phe Asn
               260                 265                 270
   Pro Lys Leu His Ser Val Ser Gln Leu Pro Ser Phe Ser Asp Met Glu
               275                 280                 285
   Asn Ala Thr Ile Arg Thr Pro His Leu Met Glu Phe Leu Arg Met Leu
           290                 295                 300
   Thr Ile Tyr Thr Asp Trp Tyr Ser Val Gly Arg Asn Tyr Tyr Trp Gly
   305                 310                 315                 320
   Gly His Arg Val Thr Ser Tyr His Val Gly Gly Glu Asn Ile Arg Ser
                   325                 330                 335
   Pro Leu Tyr Gly Arg Glu Ala Asn Gln Glu Val Pro Arg Asp Phe Tyr
               340                 345                 350
   Phe Tyr Gly Pro Val Phe Lys Thr Leu Ser Lys Pro Thr Leu Arg Pro
           355                 360                 365
   Leu Gln Gln Pro Ala Pro Ala Pro Pro Phe Asn Leu Arg Ser Leu Glu
       370                 375                 380
   Gly Val Glu Phe His Thr Pro Thr Gly Ser Phe Met Tyr Arg Glu Arg
   385                 390                 395                 400
   Gly Ser Val Asp Ser Phe Asn Glu Leu Pro Pro Phe Asn Pro Val Gly
                   405                 410                 415
   Leu Pro His Lys Val Tyr Ser His Arg Leu Cys His Ala Thr Phe Val
               420                 425                 430
   Arg Lys Ser Gly Thr Pro Tyr Leu Thr Thr Gly Ala Ile Phe Ser Trp
           435                 440                 445
   Thr His Arg Ser Ala Glu Glu Thr Asn Thr Ile Glu Ser Asn Ile Ile
       450                 455                 460
   Thr Gln Ile Pro Leu Val Lys Ala Tyr Gln Ile Gly Ser Gly Thr Thr
   465                 470                 475                 480
   Val Arg Lys Gly Pro Gly Phe Thr Gly Gly Asp Ile Leu Arg Arg Thr
                   485                 490                 495
   Gly Pro Gly Thr Phe Gly Asp Met Arg Ile Asn Ile Asn Ala Pro Leu
               500                 505                 510
   Ser Gln Arg Tyr Arg Val Arg Ile Arg Tyr Ala Ser Thr Thr Asp Leu
           515                 520                 525
   Gln Phe Val Thr Ser Ile Asn Gly Thr Thr Ile Asn Ile Gly Asn Phe
       530                 535                 540
   Pro Lys Thr Ile Asn Asn Leu Asn Thr Leu Gly Ser Glu Gly Tyr Arg
   545                 550                 555                 560
   Thr Val Ser Phe Ser Thr Pro Phe Ser Phe Ser Asn Ala Gln Ser Ile
                   565                 570                 575
   Phe Arg Leu Gly Ile Gln Ala Phe Ser Gly Val Gln Glu Val Tyr Val
               580                 585                 590
   Asp Lys Ile Glu Phe Ile Pro Val Glu
           595                 600
<210> 6
   <211> 2208
   <212> DNA
   <213> Bacillus thuringiensis
<220>
   <221> CDS
   <222> (1)...(2208)
<400> 6
   atg aat caa aat aac gat aat aac gaa tat gaa att att gat tcg cat     48
   Met Asn Gln Asn Asn Asp Asn Asn Glu Tyr Glu Ile Ile Asp Ser His
    1               5                  10                  15
   acc tca cct tat ttt ccg aac aga aac agt aat gat tct aga tac cct     96
   Thr Ser Pro Tyr Phe Pro Asn Arg Asn Ser Asn Asp Ser Arg Tyr Pro
               20                  25                  30
   tac aca aat aat cca aat caa cca tta caa aac aca aat tac aaa gag    144
   Tyr Thr Asn Asn Pro Asn Gln Pro Leu Gln Asn Thr Asn Tyr Lys Glu
           35                  40                  45
   tgg ctc aat atg tgt caa ggg aat aca caa tat ggt gat aat ttc gag    192
   Trp Leu Asn Met Cys Gln Gly Asn Thr Gln Tyr Gly Asp Asn Phe Glu
       50                  55                  60
   aca ttt gct agt gct gat aca att gct gca gtt agt gca ggt act att      240
   Thr Phe Ala Ser Ala Asp Thr Ile Ala Ala Val Ser Ala Gly Thr Ile
    65                 70                  75                  80
   gta tcc ggt act ctg tta gcc ggt ata ggt ggg ctc act tct ata tcc      288
   Val Ser Gly Thr Leu Leu Ala Gly Ile Gly Gly Leu Thr Ser Ile Ser
                   85                  90                  95
   gga ccg ata gga ata ata ggt gct ata ata ata tct ttt ggt acc cta      336
   Gly Pro Ile Gly Ile Ile Gly Ala Ile Ile Ile Ser Phe Gly Thr Leu
               100                 105                 110
   atc act gtc ttt tgg ccc gcg gga gaa caa gac aaa aca gta tgg aca      384
   Ile Thr Val Phe Trp Pro Ala Gly Glu Gln Asp Lys Thr Val Trp Thr
           115                 120                 125
   caa ttt att aaa atg gga gaa att ttt gtt gat aca ccg tta aca gaa      432
   Gln Phe Ile Lys Met Gly Glu Ile Phe Val Asp Thr Pro Leu Thr Glu
       130                 135                 140
   agc ata aaa cag cta aag tta caa act tta gaa gga ttt aga caa ata      480
   Ser Ile Lys Gln Leu Lys Leu Gln Thr Leu Glu Gly Phe Arg Gln Ile
   145                 150                 155                 160
   tta caa agc tat aat aca gca tta gat gat tgg aga aaa tta aaa aga      528
   Leu Gln Ser Tyr Asn Thr Ala Leu Asp Asp Trp Arg Lys Leu Lys Arg
                   165                 170                 175
   cta caa gct cct gga tta cca cca tca tca gca tta caa caa gct gcc      576
   Leu Gln Ala Pro Gly Leu Pro Pro Ser Ser Ala Leu Gln Gln Ala Ala
               180                 185                 190
   ttg act ctt aaa ata cga ttt gag aat gtt cac aat gat ttt att cga      624
   Leu Thr Leu Lys Ile Arg Phe Glu Asn Val His Asn Asp Phe Ile Arg
           195                 200                 205
   gaa ata cct ggt ttc caa ctt gaa act tat aaa acg cta tta cta cct      672
   Glu Ile Pro Gly Phe Gln Leu Glu Thr Tyr Lys Thr Leu Leu Leu Pro
       210                 215                 220
   att tat gcg caa gct gct aat ttt cat tta aat tta tta caa caa ggt      720
   Ile Tyr Ala Gln Ala Ala Asn Phe His Leu Asn Leu Leu Gln Gln Gly
   225                 230                 235                 240
   gct gaa ttg gct gat gaa tgg aat gca gat ata cat cct tca caa att      768
   Ala Glu Leu Ala Asp Glu Trp Asn Ala Asp Ile His Pro Ser Gln Ile
                   245                 250                 255
   gaa cct aat gct gga aca tca gat gac tat tat aaa ctt tta aaa gaa      816
   Glu Pro Asn Ala Gly Thr Ser Asp Asp Tyr Tyr Lys Leu Leu Lys Glu
               260                 265                 270
   aat ata cct aaa tat agt aac tat tgt gca aat acc tat aga aca gga      864
   Asn Ile Pro Lys Tyr Ser Asn Tyr Cys Ala Asn Thr Tyr Arg Thr Gly
           275                 280                 285
   cta aaa aat ctt aga gac gaa cca aat atg aaa tgg agt ata ttt aat      912
   Leu Lys Asn Leu Arg Asp Glu Pro Asn Met Lys Trp Ser Ile Phe Asn
       290                 295                 300
   gac tat cga aga tat atg acc att act gta tta gat acc atc tct caa      960
   Asp Tyr Arg Arg Tyr Met Thr Ile Thr Val Leu Asp Thr Ile Ser Gln
   305                 310                 315                 320
   ttt tct tta tat gat ata aaa aga tat aga gat tca ata gga gga ata      1008
   Phe Ser Leu Tyr Asp Ile Lys Arg Tyr Arg Asp Ser Ile Gly Gly Ile
                   325                 330                 335
   gaa gta aaa ggc att aag aat gaa ctc aca aga gaa att tat aca act   1056
   Glu Val Lys Gly Ile Lys Asn Glu Leu Thr Arg Glu Ile Tyr Thr Thr
               340                 345                 350
   gaa ata aat ttt gat cgt ctt cct caa ctt aga gtt caa ccc aat cta   1104
   Glu Ile Asn Phe Asp Arg Leu Pro Gln Leu Arg Val Gln Pro Asn Leu
           355                 360                 365
   gct acg atg gaa tat aat tta aca cgt gca agt ttt aaa tta ttt tca   1152
   Ala Thr Met Glu Tyr Asn Leu Thr Arg Ala Ser Phe Lys Leu Phe Ser
       370                 375                 380
   ttt tta gaa caa ttt att ttt tat aca gaa aat aca aat ttc ggg aat   1200
   Phe Leu Glu Gln Phe Ile Phe Tyr Thr Glu Asn Thr Asn Phe Gly Asn
   385                 390                 395                 400
   cgt tta gtt ggt att tct aat cgt gat gca cct act tat agc aat act   1248
   Arg Leu Val Gly Ile Ser Asn Arg Asp Ala Pro Thr Tyr Ser Asn Thr
                   405                 410                 415
   ata act gaa act tta tat gga gaa aga aca ggt tca ccc aca aca aaa   1296
   Ile Thr Glu Thr Leu Tyr Gly Glu Arg Thr Gly Ser Pro Thr Thr Lys
               420                 425                 430
   aca ata aga cca ttt gaa tct tat aaa gtt tca att gta act gat aga   1344
   Thr Ile Arg Pro Phe Glu Ser Tyr Lys Val Ser Ile Val Thr Asp Arg
           435                 440                 445
   caa tca cct cct gtt tcc cct att caa cca cac ttt ata att aat caa   1392
   Gln Ser Pro Pro Val Ser Pro Ile Gln Pro His Phe Ile Ile Asn Gln
       450                 455                 460
   att gaa ctt tat tta aat ggc tca tct aac aac aca ctc aaa tat tca   1440
   Ile Glu Leu Tyr Leu Asn Gly Ser Ser Asn Asn Thr Leu Lys Tyr Ser
   465                 470                 475                 480
   gca gga ggg tct tta tct aat tat caa aac aca act ttt ttt caa ttt   1488
   Ala Gly Gly Ser Leu Ser Asn Tyr Gln Asn Thr Thr Phe Phe Gln Phe
                   485                 490                 495
   cct aga aaa aaa gac tgc aat cta gtt att gat cca ggt tgt tca cca   1536
   Pro Arg Lys Lys Asp Cys Asn Leu Val Ile Asp Pro Gly Cys Ser Pro
               500                 505                 510
   aac ttt aat aac tat agt cat att tta tcc cat ttt tca tta ttt act   1584
   Asn Phe Asn Asn Tyr Ser His Ile Leu Ser His Phe Ser Leu Phe Thr
           515                 520                 525
   tat tcc tat gtg att gga tta cag cta caa ata tta gat aca ggt gta   1632
   Tyr Ser Tyr Val Ile Gly Leu Gln Leu Gln Ile Leu Asp Thr Gly Val
       530                 535                 540
   tta gga tgg aca cac agt agt gtt gat aga tat aat gca ata tca gat   1680
   Leu Gly Trp Thr His Ser Ser Val Asp Arg Tyr Asn Ala Ile Ser Asp
   545                 550                 555                 560
   aaa ata att aca atg atc cca gca atc aaa ggt aac aat ctt gat aca   1728
   Lys Ile Ile Thr Met Ile Pro Ala Ile Lys Gly Asn Asn Leu Asp Thr
                   565                 570                 575
   aac tct aag gta att gaa gga cct ggt cat aca gga gga aac ttg gtt   1776
   Asn Ser Lys Val Ile Glu Gly Pro Gly His Thr Gly Gly Asn Leu Val
               580                 585                 590
   tat tta caa agt caa ggg cgt tta gaa att aca tgt gaa act cct aat   1824
   Tyr Leu Gln Ser Gln Gly Arg Leu Glu Ile Thr Cys Glu Thr Pro Asn
           595                 600                 605
   tct aca caa tct tat ttc att aga ctt cga tat gct aca aat ggt gct     1872
   Ser Thr Gln Ser Tyr Phe Ile Arg Leu Arg Tyr Ala Thr Asn Gly Ala
       610                 615                 620
   gga aat act ctt cct aat ata tct ctt aca ata cca gga gta ata gga     1920
   Gly Asn Thr Leu Pro Asn Ile Ser Leu Thr Ile Pro Gly Val Ile Gly
   625                 630                 635                 640
   ata cca cct caa cga ctc aac aac act ttt tct ggt aca aat tat aat     1968
   Ile Pro Pro Gln Arg Leu Asn Asn Thr Phe Ser Gly Thr Asn Tyr Asn
                   645                 650                 655
   aat tta caa tac gga gat ttt ggg tat ttc caa ttt cca agt aca gta     2016
   Asn Leu Gln Tyr Gly Asp Phe Gly Tyr Phe Gln Phe Pro Ser Thr Val
               660                 665                 670
   aca tta cct tta aat cga aac ata cca ttt ata ttt aat cgt gca gat     2064
   Thr Leu Pro Leu Asn Arg Asn Ile Pro Phe Ile Phe Asn Arg Ala Asp
           675                 680                 685
   gta tca aat tca att tta atc att gat aaa att gaa ttt ata cca att     2112
   Val Ser Asn Ser Ile Leu Ile Ile Asp Lys Ile Glu Phe Ile Pro Ile
       690                 695                 700
   act tcc tct atg cac caa aat aga gaa aaa caa aaa tta gaa act atc     2160
   Thr Ser Ser Met His Gln Asn Arg Glu Lys Gln Lys Leu Glu Thr Ile
   705                 710                 715                 720
   caa aca aaa ata aat aca ttt ttc aca aat cat aca aaa aca ctt tga     2208
   Gln Thr Lys Ile Asn Thr Phe Phe Thr Asn His Thr Lys Thr Leu  *
                   725                 730                 735
<210> 7
   <211> 735
   <212> PRT
   <213> Bacillus thuringiensis
<400> 7
   Met Asn Gln Asn Asn Asp Asn Asn Glu Tyr Glu Ile Ile Asp Ser His
   1               5                   10                  15
   Thr Ser Pro Tyr Phe Pro Asn Arg Asn Ser Asn Asp Ser Arg Tyr Pro
               20                  25                  30
   Tyr Thr Asn Asn Pro Asn Gln Pro Leu Gln Asn Thr Asn Tyr Lys Glu
           35                  40                  45
   Trp Leu Asn Met Cys Gln Gly Asn Thr Gln Tyr Gly Asp Asn Phe Glu
       50                  55                  60
   Thr Phe Ala Ser Ala Asp Thr Ile Ala Ala Val Ser Ala Gly Thr Ile
   65                  70                  75                  80
   Val Ser Gly Thr Leu Leu Ala Gly Ile Gly Gly Leu Thr Ser Ile Ser
                   85                  90                  95
   Gly Pro Ile Gly Ile Ile Gly Ala Ile Ile Ile Ser Phe Gly Thr Leu
               100                 105                 110
   Ile Thr Val Phe Trp Pro Ala Gly Glu Gln Asp Lys Thr Val Trp Thr
           115                 120                 125
   Gln Phe Ile Lys Met Gly Glu Ile Phe Val Asp Thr Pro Leu Thr Glu
       130                 135                 140
   Ser Ile Lys Gln Leu Lys Leu Gln Thr Leu Glu Gly Phe Arg Gln Ile
   145                 150                 155                 160
   Leu Gln Ser Tyr Asn Thr Ala Leu Asp Asp Trp Arg Lys Leu Lys Arg
                   165                 170                 175
   Leu Gln Ala Pro Gly Leu Pro Pro Ser Ser Ala Leu Gln Gln Ala Ala
               180                 185                 190
   Leu Thr Leu Lys Ile Arg Phe Glu Asn Val His Asn Asp Phe Ile Arg
           195                 200                 205
   Glu Ile Pro Gly Phe Gln Leu Glu Thr Tyr Lys Thr Leu Leu Leu Pro
           210                 215                 220
   Ile Tyr Ala Gln Ala Ala Asn Phe His Leu Asn Leu Leu Gln Gln Gly
   225                 230                 235                 240
   Ala Glu Leu Ala Asp Glu Trp Asn Ala Asp Ile His Pro Ser Gln Ile
                   245                 250                 255
   Glu Pro Asn Ala Gly Thr Ser Asp Asp Tyr Tyr Lys Leu Leu Lys Glu
               260                 265                 270
   Asn Ile Pro Lys Tyr Ser Asn Tyr Cys Ala Asn Thr Tyr Arg Thr Gly
           275                 280                 285
   Leu Lys Asn Leu Arg Asp Glu Pro Asn Met Lys Trp Ser Ile Phe Asn
       290                 295                 300
   Asp Tyr Arg Arg Tyr Met Thr Ile Thr Val Leu Asp Thr Ile Ser Gln
   305                 310                 315                 320
   Phe Ser Leu Tyr Asp Ile Lys Arg Tyr Arg Asp Ser Ile Gly Gly Ile
                   325                 330                 335
   Glu Val Lys Gly Ile Lys Asn Glu Leu Thr Arg Glu Ile Tyr Thr Thr
               340                 345                 350
   Glu Ile Asn Phe Asp Arg Leu Pro Gln Leu Arg Val Gln Pro Asn Leu
           355                 360                 365
   Ala Thr Met Glu Tyr Asn Leu Thr Arg Ala Ser Phe Lys Leu Phe Ser
       370                 375                 380
   Phe Leu Glu Gln Phe Ile Phe Tyr Thr Glu Asn Thr Asn Phe Gly Asn
   385                 390                 395                 400
   Arg Leu Val Gly Ile Ser Asn Arg Asp Ala Pro Thr Tyr Ser Asn Thr
                   405                 410                 415
   Ile Thr Glu Thr Leu Tyr Gly Glu Arg Thr Gly Ser Pro Thr Thr Lys
               420                 425                 430
   Thr Ile Arg Pro Phe Glu Ser Tyr Lys Val Ser Ile Val Thr Asp Arg
           435                 440                 445
   Gln Ser Pro Pro Val Ser Pro Ile Gln Pro His Phe Ile Ile Asn Gln
       450                 455                 460
   Ile Glu Leu Tyr Leu Asn Gly Ser Ser Asn Asn Thr Leu Lys Tyr Ser
   465                 470                 475                 480
   Ala Gly Gly Ser Leu Ser Asn Tyr Gln Asn Thr Thr Phe Phe Gln Phe
                   485                 490                 495
   Pro Arg Lys Lys Asp Cys Asn Leu Val Ile Asp Pro Gly Cys Ser Pro
               500                 505                 510
   Asn Phe Asn Asn Tyr Ser His Ile Leu Ser His Phe Ser Leu Phe Thr
           515                 520                 525
   Tyr Ser Tyr Val Ile Gly Leu Gln Leu Gln Ile Leu Asp Thr Gly Val
       530                 535                 540
   Leu Gly Trp Thr His Ser Ser Val Asp Arg Tyr Asn Ala Ile Ser Asp
   545                 550                 555                 560
   Lys Ile Ile Thr Met Ile Pro Ala Ile Lys Gly Asn Asn Leu Asp Thr
                   565                 570                 575
   Asn Ser Lys Val Ile Glu Gly Pro Gly His Thr Gly Gly Asn Leu Val
               580                 585                 590
   Tyr Leu Gln Ser Gln Gly Arg Leu Glu Ile Thr Cys Glu Thr Pro Asn
           595                 600                 605
   Ser Thr Gln Ser Tyr Phe Ile Arg Leu Arg Tyr Ala Thr Asn Gly Ala
       610                 615                 620
   Gly Asn Thr Leu Pro Asn Ile Ser Leu Thr Ile Pro Gly Val Ile Gly
   625                 630                 635                 640
   Ile Pro Pro Gln Arg Leu Asn Asn Thr Phe Ser Gly Thr Asn Tyr Asn
                   645                 650                 655
   Asn Leu Gln Tyr Gly Asp Phe Gly Tyr Phe Gln Phe Pro Ser Thr Val
               660                 665                 670
   Thr Leu Pro Leu Asn Arg Asn Ile Pro Phe Ile Phe Asn Arg Ala Asp
           675                 680                 685
   Val Ser Asn Ser Ile Leu Ile Ile Asp Lys Ile Glu Phe Ile Pro Ile
       690                 695                 700
   Thr Ser Ser Met His Gln Asn Arg Glu Lys Gln Lys Leu Glu Thr Ile
   705                 710                 715                 720
   Gln Thr Lys Ile Asn Thr Phe Phe Thr Asn His Thr Lys Thr Leu
                   725                 730                 735
<210> 8
   <211> 2235
   <212> DNA
   <213> Bacillus thuringiensis
<220>
   <221> CDS
   <222> (1)...(2235)
<400> 8
   gtg aat caa aat aat aat aat gaa tat gag att atc gat tca aag aat      48
   Met Asn Gln Asn Asn Asn Asn Glu Tyr Glu Ile Ile Asp Ser Lys Asn
    1               5                  10                  15
   tta tct tat cct tct aac aga aat att gat cat tct aga tac cct tac      96
   Leu Ser Tyr Pro Ser Asn Arg Asn Ile Asp His Ser Arg Tyr Pro Tyr
               20                  25                  30
   aca aat aat cca aat caa cca tta caa aac aca aat tac aaa gag tgg      144
   Thr Asn Asn Pro Asn Gln Pro Leu Gln Asn Thr Asn Tyr Lys Glu Trp
           35                  40                  45
   ctc aat atg tgt caa ggg aat aca caa tat ggt gat aat ttc gag aca      192
   Leu Asn Met Cys Gln Gly Asn Thr Gln Tyr Gly Asp Asn Phe Glu Thr
       50                  55                  60
   ttt gct agt gct gat aca att gct gca gtt agt gca ggt act att gta      240
   Phe Ala Ser Ala Asp Thr Ile Ala Ala Val Ser Ala Gly Thr Ile Val
    65                  70                  75                  80
   tcc ggt act ctg tta gcc ggt ata ggt ggg ctc act tct ata tcc gga      288
   Ser Gly Thr Leu Leu Ala Gly Ile Gly Gly Leu Thr Ser Ile Ser Gly
                   85                  90                  95
   ccg ata gga ata ata ggt gct ata ata ata tct ttt ggt acc cta atc      336
   Pro Ile Gly Ile Ile Gly Ala Ile Ile Ile Ser Phe Gly Thr Leu Ile
               100                 105                 110
   act gtc ttt tgg ccc gcg gga gaa caa gac aaa aca gta tgg aca caa      384
   Thr Val Phe Trp Pro Ala Gly Glu Gln Asp Lys Thr Val Trp Thr Gln
           115                 120                 125
   ttt att aaa atg gga gaa att ttt gtt gat aca ccg tta aca gaa agc      432
   Phe Ile Lys Met Gly Glu Ile Phe Val Asp Thr Pro Leu Thr Glu Ser
       130                 135                 140
   ata aaa cag cta aag tta caa act tta gaa gga ttt aga caa ata tta      480
   Ile Lys Gln Leu Lys Leu Gln Thr Leu Glu Gly Phe Arg Gln Ile Leu
   145                 150                 155                 160
   caa agc tat aat aca gca tta gat gat tgg aga aaa tta aaa aga cta      528
   Gln Ser Tyr Asn Thr Ala Leu Asp Asp Trp Arg Lys Leu Lys Arg Leu
                   165                 170                 175
   caa gct cct gga tta cca cca tca tca gca tta caa caa gct gcc ttg      576
   Gln Ala Pro Gly Leu Pro Pro Ser Ser Ala Leu Gln Gln Ala Ala Leu
               180                 185                 190
   act ctt aaa ata cga ttt gag aat gtt cac aat gat ttt att cga gaa      624
   Thr Leu Lys Ile Arg Phe Glu Asn Val His Asn Asp Phe Ile Arg Glu
           195                 200                 205
   ata cct ggt ttc caa ctt gaa act tat aaa acg cta tta cta cct att      672
   Ile Pro Gly Phe Gln Leu Glu Thr Tyr Lys Thr Leu Leu Leu Pro Ile
       210                 215                 220
   tat gcg caa gct gct aat ttt cat tta aat tta tta caa caa ggt gct      720
   Tyr Ala Gln Ala Ala Asn Phe His Leu Asn Leu Leu Gln Gln Gly Ala
   225                 230                 235                 240
   gaa ttg gct gat gaa tgg aat gca gat ata cat cct tca caa att gaa    768
   Glu Leu Ala Asp Glu Trp Asn Ala Asp Ile His Pro Ser Gln Ile Glu
                   245                 250                 255
   cct aat gct gga aca tca gat gac tat tat aaa ctt tta aaa gaa aat    816
   Pro Asn Ala Gly Thr Ser Asp Asp Tyr Tyr Lys Leu Leu Lys Glu Asn
               260                 265                 270
   ata cct aaa tat agt aac tat tgt gca aat acc tat aga gaa gga cta    864
   Ile Pro Lys Tyr Ser Asn Tyr Cys Ala Asn Thr Tyr Arg Glu Gly Leu
           275                 280                 285
   aat aaa ctt cga aac gaa cct aat atg aga tgg agt ata ttt aat gat    912
   Asn Lys Leu Arg Asn Glu Pro Asn Met Arg Trp Ser Ile Phe Asn Asp
       290                 295                 300
   tat cga aga tat atg act att act gta tta gat act atc gct caa ttt    960
   Tyr Arg Arg Tyr Met Thr Ile Thr Val Leu Asp Thr Ile Ala Gln Phe
   305                 310                 315                 320
   tct ttt tat gat ata aag aga tac aaa gat tca ata gga aga ata ggt    1008
   Ser Phe Tyr Asp Ile Lys Arg Tyr Lys Asp Ser Ile Gly Arg Ile Gly
                   325                 330                 335
   ggc att aaa act gaa ctt aca aga gaa att tat aca act gaa ata aat    1056
   Gly Ile Lys Thr Glu Leu Thr Arg Glu Ile Tyr Thr Thr Glu Ile Asn
               340                 345                 350
   ttt gac cgt ctt act tac ctt gaa att caa ccc aat ctc gct ata atg    1104
   Phe Asp Arg Leu Thr Tyr Leu Glu Ile Gln Pro Asn Leu Ala Ile Met
           355                 360                 365
   gaa tat aat tta aca cgt tca ggg ctt aga tta ttt tca ttt tta gat    1152
   Glu Tyr Asn Leu Thr Arg Ser Gly Leu Arg Leu Phe Ser Phe Leu Asp
       370                 375                 380
   gaa ctt ata ttt tat aca aaa aat gaa acg tac ggg aat cgt tta gtt    1200
   Glu Leu Ile Phe Tyr Thr Lys Asn Glu Thr Tyr Gly Asn Arg Leu Val
   385                 390                 395                 400
   ggt att gcg aat cgt aat aga tct act tat gct acg aca gga act gaa    1248
   Gly Ile Ala Asn Arg Asn Arg Ser Thr Tyr Ala Thr Thr Gly Thr Glu
                   405                 410                 415
   att ata tat gga gaa aga aca ggt cca ccc aca aca aaa act tta ata    1296
   Ile Ile Tyr Gly Glu Arg Thr Gly Pro Pro Thr Thr Lys Thr Leu Ile
               420                 425                 430
   cca ttt gaa tcc tat aaa gtt tca att gta act gat aga caa gta act    1344
   Pro Phe Glu Ser Tyr Lys Val Ser Ile Val Thr Asp Arg Gln Val Thr
           435                 440                 445
   cct act tcc cct ttt cct aac ata tac ttt aca att aat caa att gaa    1392
   Pro Thr Ser Pro Phe Pro Asn Ile Tyr Phe Thr Ile Asn Gln Ile Glu
       450                 455                 460
   ctt tat tta aat aat tca cct agt aat aaa tta aca tat tca gct ggg    1440
   Leu Tyr Leu Asn Asn Ser Pro Ser Asn Lys Leu Thr Tyr Ser Ala Gly
   465                 470                 475                 480
   ggg aat tta tct aat gat aaa aaa aca act gat ttt caa ttt cct gta    1488
   Gly Asn Leu Ser Asn Asp Lys Lys Thr Thr Asp Phe Gln Phe Pro Val
                   485                 490                 495
   aaa aaa gac tgt aaa cca att att aat cca aat tgt tta cca agc tat    1536
   Lys Lys Asp Cys Lys Pro Ile Ile Asn Pro Asn Cys Leu Pro Ser Tyr
               500                 505                 510
   aat agt tat agt cat att tta tcc cag ttt tct tta ttt aat tat tcc     1584
   Asn Ser Tyr Ser His Ile Leu Ser Gln Phe Ser Leu Phe Asn Tyr Ser
           515                 520                 525
   tat aaa att gga tta gcg cta aat ata tta tat aca ggt gca tta gga     1632
   Tyr Lys Ile Gly Leu Ala Leu Asn Ile Leu Tyr Thr Gly Ala Leu Gly
       530                 535                 540
   tgg aca cac agt agt gtt aat aga aat aat gca ata tca gat aaa ata     1680
   Trp Thr His Ser Ser Val Asn Arg Asn Asn Ala Ile Ser Asp Lys Ile
   545                 550                 555                 560
   att aca atg atc cca gca atc aaa ggt aac agt ctt gat aca aac tct     1728
   Ile Thr Met Ile Pro Ala Ile Lys Gly Asn Ser Leu Asp Thr Asn Ser
                   565                 570                 575
   aag gta att gaa gga cct ggt cat aca gga gga aac ttg gtt tat tta     1776
   Lys Val Ile Glu Gly Pro Gly His Thr Gly Gly Asn Leu Val Tyr Leu
               580                 585                 590
   caa agt caa ggg cgt tta gag att aca tgt aga act cct aat tct aca     1824
   Gln Ser Gln Gly Arg Leu Glu Ile Thr Cys Arg Thr Pro Asn Ser Thr
           595                 600                 605
   caa tct tat tac att aga ctt cga tac gct aca aat ggt gct gga aat     1872
   Gln Ser Tyr Tyr Ile Arg Leu Arg Tyr Ala Thr Asn Gly Ala Gly Asn
       610                 615                 620
   act ctt cct aat ata tct ctt aca ata cca gga gta ata gga ata cca     1920
   Thr Leu Pro Asn Ile Ser Leu Thr Ile Pro Gly Val Ile Gly Ile Pro
   625                 630                 635                 640
   cct caa cga ctc aac aac act ttt tct ggt aca aat tat aat aat tta     1968
   Pro Gln Arg Leu Asn Asn Thr Phe Ser Gly Thr Asn Tyr Asn Asn Leu
                   645                 650                 655
   caa tac gga gat ttt ggg tat ttc caa ttt cca agt aca gta aca tta     2016
   Gln Tyr Gly Asp Phe Gly Tyr Phe Gln Phe Pro Ser Thr Val Thr Leu
               660                 665                 670
   cct tta aat cga aac ata cca ttt ata ttt aat cgt gca gat gta tca     2064
   Pro Leu Asn Arg Asn Ile Pro Phe Ile Phe Asn Arg Ala Asp Val Ser
           675                 680                 685
   aat tca att tta atc att gat aaa att gaa ttt ata cca att act tcc     2112
   Asn Ser Ile Leu Ile Ile Asp Lys Ile Glu Phe Ile Pro Ile Thr Ser
       690                 695                 700
   tct gta cgc caa aat aga gaa aaa caa aaa tta gaa act atc caa aca     2160
   Ser Val Arg Gln Asn Arg Glu Lys Gln Lys Leu Glu Thr Ile Gln Thr
   705                 710                 715                 720
   aaa ata aat aca ttt ttc aca aat cat aca aaa aat act tta aat ata     2208
   Lys Ile Asn Thr Phe Phe Thr Asn His Thr Lys Asn Thr Leu Asn Ile
                   725                 730                 735
   gaa gcc aca aac tat gat att gat taa                                 2235
   Glu Ala Thr Asn Tyr Asp Ile Asp  *
               740
<210> 9
   <211> 744
   <212> PRT
   <213> Bacillus thuringiensis
<400> 9
   Met Asn Gln Asn Asn Asn Asn Glu Tyr Glu Ile Ile Asp Ser Lys Asn
   1               5                   10                  15
   Leu Ser Tyr Pro Ser Asn Arg Asn Ile Asp His Ser Arg Tyr Pro Tyr
               20                  25                  30
   Thr Asn Asn Pro Asn Gln Pro Leu Gln Asn Thr Asn Tyr Lys Glu Trp
           35                  40                  45
   Leu Asn Met Cys Gln Gly Asn Thr Gln Tyr Gly Asp Asn Phe Glu Thr
       50                  55                  60
   Phe Ala Ser Ala Asp Thr Ile Ala Ala Val Ser Ala Gly Thr Ile Val
   65                  70                  75                  80
   Ser Gly Thr Leu Leu Ala Gly Ile Gly Gly Leu Thr Ser Ile Ser Gly
                   85                  90                  95
   Pro Ile Gly Ile Ile Gly Ala Ile Ile Ile Ser Phe Gly Thr Leu Ile
               100                 105                 110
   Thr Val Phe Trp Pro Ala Gly Glu Gln Asp Lys Thr Val Trp Thr Gln
           115                 120                 125
   Phe Ile Lys Met Gly Glu Ile Phe Val Asp Thr Pro Leu Thr Glu Ser
       130                 135                 140
   Ile Lys Gln Leu Lys Leu Gln Thr Leu Glu Gly Phe Arg Gln Ile Leu
   145                 150                 155                 160
   Gln Ser Tyr Asn Thr Ala Leu Asp Asp Trp Arg Lys Leu Lys Arg Leu
                   165                 170                 175
   Gln Ala Pro Gly Leu Pro Pro Ser Ser Ala Leu Gln Gln Ala Ala Leu
               180                 185                 190
   Thr Leu Lys Ile Arg Phe Glu Asn Val His Asn Asp Phe Ile Arg Glu
           195                 200                 205
   Ile Pro Gly Phe Gln Leu Glu Thr Tyr Lys Thr Leu Leu Leu Pro Ile
       210                 215                 220
   Tyr Ala Gln Ala Ala Asn Phe His Leu Asn Leu Leu Gln Gln Gly Ala
   225                 230                 235                 240
   Glu Leu Ala Asp Glu Trp Asn Ala Asp Ile His Pro Ser Gln Ile Glu
                   245                 250                 255
   Pro Asn Ala Gly Thr Ser Asp Asp Tyr Tyr Lys Leu Leu Lys Glu Asn
               260                 265                 270
   Ile Pro Lys Tyr Ser Asn Tyr Cys Ala Asn Thr Tyr Arg Glu Gly Leu
           275                 280                 285
   Asn Lys Leu Arg Asn Glu Pro Asn Met Arg Trp Ser Ile Phe Asn Asp
       290                 295                 300
   Tyr Arg Arg Tyr Met Thr Ile Thr Val Leu Asp Thr Ile Ala Gln Phe
   305                 310                 315                 320
   Ser Phe Tyr Asp Ile Lys Arg Tyr Lys Asp Ser Ile Gly Arg Ile Gly
                   325                 330                 335
   Gly Ile Lys Thr Glu Leu Thr Arg Glu Ile Tyr Thr Thr Glu Ile Asn
               340                 345                 350
   Phe Asp Arg Leu Thr Tyr Leu Glu Ile Gln Pro Asn Leu Ala Ile Met
           355                 360                 365
   Glu Tyr Asn Leu Thr Arg Ser Gly Leu Arg Leu Phe Ser Phe Leu Asp
       370                 375                 380
   Glu Leu Ile Phe Tyr Thr Lys Asn Glu Thr Tyr Gly Asn Arg Leu Val
   385                 390                 395                 400
   Gly Ile Ala Asn Arg Asn Arg Ser Thr Tyr Ala Thr Thr Gly Thr Glu
                   405                 410                 415
   Ile Ile Tyr Gly Glu Arg Thr Gly Pro Pro Thr Thr Lys Thr Leu Ile
               420                 425                 430
   Pro Phe Glu Ser Tyr Lys Val Ser Ile Val Thr Asp Arg Gln Val Thr
           435                 440                 445
   Pro Thr Ser Pro Phe Pro Asn Ile Tyr Phe Thr Ile Asn Gln Ile Glu
       450                 455                 460
   Leu Tyr Leu Asn Asn Ser Pro Ser Asn Lys Leu Thr Tyr Ser Ala Gly
   465                 470                 475                 480
   Gly Asn Leu Ser Asn Asp Lys Lys Thr Thr Asp Phe Gln Phe Pro Val
                   485                 490                 495
   Lys Lys Asp Cys Lys Pro Ile Ile Asn Pro Asn Cys Leu Pro Ser Tyr
               500                 505                 510
   Asn Ser Tyr Ser His Ile Leu Ser Gln Phe Ser Leu Phe Asn Tyr Ser
           515                 520                 525
   Tyr Lys Ile Gly Leu Ala Leu Asn Ile Leu Tyr Thr Gly Ala Leu Gly
       530                 535                 540
   Trp Thr His Ser Ser Val Asn Arg Asn Asn Ala Ile Ser Asp Lys Ile
   545 550 555 560
   Ile Thr Met Ile Pro Ala Ile Lys Gly Asn Ser Leu Asp Thr Asn Ser
   565 570 575
   Lys Val Ile Glu Gly Pro Gly His Thr Gly Gly Asn Leu Val Tyr Leu
   580 585 590
   Gln Ser Gln Gly Arg Leu Glu Ile Thr Cys Arg Thr Pro Asn Ser Thr
   595 600 605
   Gln Ser Tyr Tyr Ile Arg Leu Arg Tyr Ala Thr Asn Gly Ala Gly Asn
   610 615 620
   Thr Leu Pro Asn Ile Ser Leu Thr Ile Pro Gly Val Ile Gly Ile Pro
   625 630 635 640
   Pro Gln Arg Leu Asn Asn Thr Phe Ser Gly Thr Asn Tyr Asn Asn Leu
   645 650 655
   Gln Tyr Gly Asp Phe Gly Tyr Phe Gln Phe Pro Ser Thr Val Thr Leu
   660 665 670
   Pro Leu Asn Arg Asn Ile Pro Phe Ile Phe Asn Arg Ala Asp Val Ser
   675 680 685
   Asn Ser Ile Leu Ile Ile Asp Lys Ile Glu Phe Ile Pro Ile Thr Ser
   690 695 700
   Ser Val Arg Gln Asn Arg Glu Lys Gln Lys Leu Glu Thr Ile Gln Thr
   705 710 715 720
   Lys Ile Asn Thr Phe Phe Thr Asn His Thr Lys Asn Thr Leu Asn Ile
   725 730 735
   Glu Ala Thr Asn Tyr Asp Ile Asp
   740
<210> 10
   <211> 2085
   <212> DNA
   <213> Bacillus thuringiensis
<220>
   <221> CDS
   <222> (1)...(2085)
<400> 10
   atg tgt caa ggg aat aca caa tat ggt gat aat ttc gag aca ttt gct      48
   Met Cys Gln Gly Asn Thr Gln Tyr Gly Asp Asn Phe Glu Thr Phe Ala
    1               5                  10                  15
   agt gct gat aca att gct gca gtt agt gca ggt act att gta tcc ggt      96
   Ser Ala Asp Thr Ile Ala Ala Val Ser Ala Gly Thr Ile Val Ser Gly
               20                  25                  30
   act ctg tta gcc ggt ata ggt ggg ctc act tct ata tcc gga ccg ata     144
   Thr Leu Leu Ala Gly Ile Gly Gly Leu Thr Ser Ile Ser Gly Pro Ile
           35                  40                  45
   gga ata ata ggt gct ata ata ata tct ttt ggt acc cta atc act gtc     192
   Gly Ile Ile Gly Ala Ile Ile Ile Ser Phe Gly Thr Leu Ile Thr Val
       50                  55                  60
   ttt tgg ccc gcg gga gaa caa gac aaa aca gta tgg aca caa ttt att     240
   Phe Trp Pro Ala Gly Glu Gln Asp Lys Thr Val Trp Thr Gln Phe Ile
   65                  70                  75                  80
   aaa atg gga gaa att ttt gtt gat aca ccg tta aca gaa agc ata aaa     288
   Lys Met Gly Glu Ile Phe Val Asp Thr Pro Leu Thr Glu Ser Ile Lys
                   85                  90                  95
   cag cta aag tta caa act tta gaa gga ttt aga caa ata tta caa agc     336
   Gln Leu Lys Leu Gln Thr Leu Glu Gly Phe Arg Gln Ile Leu Gln Ser
               100                 105                 110
   tat aat aca gca tta gat gat tgg aga aaa tta aaa aga cta caa gct     384
   Tyr Asn Thr Ala Leu Asp Asp Trp Arg Lys Leu Lys Arg Leu Gln Ala
           115                 120                 125
   cct gga tta cca cca tca tca gca tta caa caa gct gcc ttg act ctt    432
   Pro Gly Leu Pro Pro Ser Ser Ala Leu Gln Gln Ala Ala Leu Thr Leu
       130                 135                 140
   aaa ata cga ttt gag aat gtt cac aat gat ttt att cga gaa ata cct    480
   Lys Ile Arg Phe Glu Asn Val His Asn Asp Phe Ile Arg Glu Ile Pro
   145                 150                 155                 160
   ggt ttc caa ctt gaa act tat aaa acg cta tta cta cct att tat gcg    528
   Gly Phe Gln Leu Glu Thr Tyr Lys Thr Leu Leu Leu Pro Ile Tyr Ala
                   165                 170                 175
   caa gct gct aat ttt cat tta aat tta tta caa caa ggt gct gaa ttg    576
   Gln Ala Ala Asn Phe His Leu Asn Leu Leu Gln Gln Gly Ala Glu Leu
               180                 185                 190
   gct gat gaa tgg aat gca gat ata cat cct tca caa att gaa cct aat    624
   Ala Asp Glu Trp Asn Ala Asp Ile His Pro Ser Gln Ile Glu Pro Asn
           195                 200                 205
   gct gga aca tca gat gac tat tat aaa ctt tta aaa gaa aat ata cct    672
   Ala Gly Thr Ser Asp Asp Tyr Tyr Lys Leu Leu Lys Glu Asn Ile Pro
       210                 215                 220
   aaa tat agt aac tat tgt gca aat acc tat aga gaa gga cta aat aaa    720
   Lys Tyr Ser Asn Tyr Cys Ala Asn Thr Tyr Arg Glu Gly Leu Asn Lys
   225                 230                 235                 240
   ctt cga aac gaa cct aat atg aga tgg agt ata ttt aat gat tat cga    768
   Leu Arg Asn Glu Pro Asn Met Arg Trp Ser Ile Phe Asn Asp Tyr Arg
                   245                 250                 255
   aga tat atg act att act gta tta gat act atc gct caa ttt tct ttt    816
   Arg Tyr Met Thr Ile Thr Val Leu Asp Thr Ile Ala Gln Phe Ser Phe
               260                 265                 270
   tat gat ata aag aga tac aaa gat tca ata gga aga ata ggt ggc att    864
   Tyr Asp Ile Lys Arg Tyr Lys Asp Ser Ile Gly Arg Ile Gly Gly Ile
           275                 280                 285
   aaa act gaa ctt aca aga gaa att tat aca act gaa ata aat ttt gac    912
   Lys Thr Glu Leu Thr Arg Glu Ile Tyr Thr Thr Glu Ile Asn Phe Asp
       290                 295                 300
   cgt ctt act tac ctt gaa att caa ccc aat ctc gct ata atg gaa tat    960
   Arg Leu Thr Tyr Leu Glu Ile Gln Pro Asn Leu Ala Ile Met Glu Tyr
   305                 310                 315                 320
   aat tta aca cgt tca ggg ctt aga tta ttt tca ttt tta gat gaa ctt    1008
   Asn Leu Thr Arg Ser Gly Leu Arg Leu Phe Ser Phe Leu Asp Glu Leu
                   325                 330                 335
   ata ttt tat aca aaa aat gaa acg tac ggg aat cgt tta gtt ggt att    1056
   Ile Phe Tyr Thr Lys Asn Glu Thr Tyr Gly Asn Arg Leu Val Gly Ile
               340                 345                 350
   gcg aat cgt aat aga tct act tat gct acg aca gga act gaa att ata    1104
   Ala Asn Arg Asn Arg Ser Thr Tyr Ala Thr Thr Gly Thr Glu Ile Ile
           355                 360                 365
   tat gga gaa aga aca ggt cca ccc aca aca aaa act tta ata cca ttt    1152
   Tyr Gly Glu Arg Thr Gly Pro Pro Thr Thr Lys Thr Leu Ile Pro Phe
       370                 375                 380
   gaa tcc tat aaa gtt tca att gta act gat aga caa gta act cct act    1200
   Glu Ser Tyr Lys Val Ser Ile Val Thr Asp Arg Gln Val Thr Pro Thr
   385 390 395 400
   tcc cct ttt cct aac ata tac ttt aca att aat caa att gaa ctt tat 1248
   Ser Pro Phe Pro Asn Ile Tyr Phe Thr Ile Asn Gln Ile Glu Leu Tyr
   405 410 415
   tta aat aat tca cct agt aat aaa tta aca tat tca gct ggg ggg aat 1296
   Leu Asn Asn Ser Pro Ser Asn Lys Leu Thr Tyr Ser Ala Gly Gly Asn
   420 425 430
   tta tct aat gat aaa aaa aca act gat ttt caa ttt cct gta aaa aaa 1344
   Leu Ser Asn Asp Lys Lys Thr Thr Asp Phe Gln Phe Pro Val Lys Lys
   435 440 445
   gac tgt aaa cca att att aat cca aat tgt tta cca agc tat aat agt 1392
   Asp Cys Lys Pro Ile Ile Asn Pro Asn Cys Leu Pro Ser Tyr Asn Ser
   450 455 460
   tat agt cat att tta tcc cag ttt tct tta ttt aat tat tcc tat aaa 1440
   Tyr Ser His Ile Leu Ser Gln Phe Ser Leu Phe Asn Tyr Ser Tyr Lys
   465 470 475 480
   att gga tta gcg cta aat ata tta tat aca ggt gca tta gga tgg aca 1488
   Ile Gly Leu Ala Leu Asn Ile Leu Tyr Thr Gly Ala Leu Gly Trp Thr
   485 490 495
   cac agt agt gtt aat aga aat aat gca ata tca gat aaa ata att aca 1536
   His Ser Ser Val Asn Arg Asn Asn Ala Ile Ser Asp Lys Ile Ile Thr
   500 505 510
   atg atc cca gca atc aaa ggt aac agt ctt gat aca aac tct aag gta 1584
   Met Ile Pro Ala Ile Lys Gly Asn Ser Leu Asp Thr Asn Ser Lys Val
   515 520 525
   att gaa gga cct ggt cat aca gga gga aac ttg gtt tat tta caa agt 1632
   Ile Glu Gly Pro Gly His Thr Gly Gly Asn Leu Val Tyr Leu Gln Ser
   530 535 540
   caa ggg cgt tta gag att aca tgt aga act cct aat tct aca caa tct 1680
   Gln Gly Arg Leu Glu Ile Thr Cys Arg Thr Pro Asn Ser Thr Gln Ser
   545 550 555 560
   tat tac att aga ctt cga tac gct aca aat ggt gct gga aat act ctt 1728
   Tyr Tyr Ile Arg Leu Arg Tyr Ala Thr Asn Gly Ala Gly Asn Thr Leu
   565 570 575
   cct aat ata tct ctt aca ata cca gga gta ata gga ata cca cct caa 1776
   Pro Asn Ile Ser Leu Thr Ile Pro Gly Val Ile Gly Ile Pro Pro Gln
   580 585 590
   cga ctc aac aac act ttt tct ggt aca aat tat aat aat tta caa tac 1824
   Arg Leu Asn Asn Thr Phe Ser Gly Thr Asn Tyr Asn Asn Leu Gln Tyr
   595 600 605
   gga gat ttt ggg tat ttc caa ttt cca agt aca gta aca tta cct tta 1872
   Gly Asp Phe Gly Tyr Phe Gln Phe Pro Ser Thr Val Thr Leu Pro Leu
   610 615 620
   aat cga aac ata cca ttt ata ttt aat cgt gca gat gta tca aat tca 1920
   Asn Arg Asn Ile Pro Phe Ile Phe Asn Arg Ala Asp Val Ser Asn Ser
   625 630 635 640
   att tta atc att gat aaa att gaa ttt ata cca att act tcc tct gta 1968
   Ile Leu Ile Ile Asp Lys Ile Glu Phe Ile Pro Ile Thr Ser Ser Val
   645 650 655
   cgc caa aat aga gaa aaa caa aaa tta gaa act atc caa aca aaa ata 2016
   Arg Gln Asn Arg Glu Lys Gln Lys Leu Glu Thr Ile Gln Thr Lys Ile
                   660                 665                 670
   aat aca ttt ttc aca aat cat aca aaa aat act tta aat ata gaa gcc    2064
   Asn Thr Phe Phe Thr Asn His Thr Lys Asn Thr Leu Asn Ile Glu Ala
           675                 680                 685
   aca aac tat gat att gat taa                                        2085
   Thr Asn Tyr Asp Ile Asp  *
       690
<210> 11
   <211> 694
   <212> PRT
   <213> Bacillus thuringiensis
<400> 11
   Met Cys Gln Gly Asn Thr Gln Tyr Gly Asp Asn Phe Glu Thr Phe Ala
    1               5                  10                  15
   Ser Ala Asp Thr Ile Ala Ala Val Ser Ala Gly Thr Ile Val Ser Gly
               20                  25                  30
   Thr Leu Leu Ala Gly Ile Gly Gly Leu Thr Ser Ile Ser Gly Pro Ile
           35                  40                  45
   Gly Ile Ile Gly Ala Ile Ile Ile Ser Phe Gly Thr Leu Ile Thr Val
       50                  55                  60
   Phe Trp Pro Ala Gly Glu Gln Asp Lys Thr Val Trp Thr Gln Phe Ile
   65                  70                  75                  80
   Lys Met Gly Glu Ile Phe Val Asp Thr Pro Leu Thr Glu Ser Ile Lys
                   85                  90                  95
   Gln Leu Lys Leu Gln Thr Leu Glu Gly Phe Arg Gln Ile Leu Gln Ser
               100                 105                 110
   Tyr Asn Thr Ala Leu Asp Asp Trp Arg Lys Leu Lys Arg Leu Gln Ala
           115                 120                 125
   Pro Gly Leu Pro Pro Ser Ser Ala Leu Gln Gln Ala Ala Leu Thr Leu
       130                 135                 140
   Lys Ile Arg Phe Glu Asn Val His Asn Asp Phe Ile Arg Glu Ile Pro
   145                 150                 155                 160
   Gly Phe Gln Leu Glu Thr Tyr Lys Thr Leu Leu Leu Pro Ile Tyr Ala
                   165                 170                 175
   Gln Ala Ala Asn Phe His Leu Asn Leu Leu Gln Gln Gly Ala Glu Leu
               180                 185                 190
   Ala Asp Glu Trp Asn Ala Asp Ile His Pro Ser Gln Ile Glu Pro Asn
           195                 200                 205
   Ala Gly Thr Ser Asp Asp Tyr Tyr Lys Leu Leu Lys Glu Asn Ile Pro
       210                 215                 220
   Lys Tyr Ser Asn Tyr Cys Ala Asn Thr Tyr Arg Glu Gly Leu Asn Lys
   225                 230                 235                 240
   Leu Arg Asn Glu Pro Asn Met Arg Trp Ser Ile Phe Asn Asp Tyr Arg
                   245                 250                 255
   Arg Tyr Met Thr Ile Thr Val Leu Asp Thr Ile Ala Gln Phe Ser Phe
               260                 265                 270
   Tyr Asp Ile Lys Arg Tyr Lys Asp Ser Ile Gly Arg Ile Gly Gly Ile
           275                 280                 285
   Lys Thr Glu Leu Thr Arg Glu Ile Tyr Thr Thr Glu Ile Asn Phe Asp
       290                 295                 300
   Arg Leu Thr Tyr Leu Glu Ile Gln Pro Asn Leu Ala Ile Met Glu Tyr
   305                 310                 315                 320
   Asn Leu Thr Arg Ser Gly Leu Arg Leu Phe Ser Phe Leu Asp Glu Leu
                   325                 330                 335
   Ile Phe Tyr Thr Lys Asn Glu Thr Tyr Gly Asn Arg Leu Val Gly Ile
               340                 345                 350
   Ala Asn Arg Asn Arg Ser Thr Tyr Ala Thr Thr Gly Thr Glu Ile Ile
           355                 360                 365
   Tyr Gly Glu Arg Thr Gly Pro Pro Thr Thr Lys Thr Leu Ile Pro Phe
       370                 375                 380
   Glu Ser Tyr Lys Val Ser Ile Val Thr Asp Arg Gln Val Thr Pro Thr
   385                 390                 395                 400
   Ser Pro Phe Pro Asn Ile Tyr Phe Thr Ile Asn Gln Ile Glu Leu Tyr
               405                 410                 415
   Leu Asn Asn Ser Pro Ser Asn Lys Leu Thr Tyr Ser Ala Gly Gly Asn
           420                 425                 430
   Leu Ser Asn Asp Lys Lys Thr Thr Asp Phe Gln Phe Pro Val Lys Lys
       435                 440                 445
   Asp Cys Lys Pro Ile Ile Asn Pro Asn Cys Leu Pro Ser Tyr Asn Ser
       450                 455                 460
   Tyr Ser His Ile Leu Ser Gln Phe Ser Leu Phe Asn Tyr Ser Tyr Lys
   465                 470                 475                 480
   Ile Gly Leu Ala Leu Asn Ile Leu Tyr Thr Gly Ala Leu Gly Trp Thr
                   485                 490                 495
   His Ser Ser Val Asn Arg Asn Asn Ala Ile Ser Asp Lys Ile Ile Thr
               500                 505                 510
   Met Ile Pro Ala Ile Lys Gly Asn Ser Leu Asp Thr Asn Ser Lys Val
           515                 520                 525
   Ile Glu Gly Pro Gly His Thr Gly Gly Asn Leu Val Tyr Leu Gln Ser
       530                 535                 540
   Gln Gly Arg Leu Glu Ile Thr Cys Arg Thr Pro Asn Ser Thr Gln Ser
   545                 550                 555                 560
   Tyr Tyr Ile Arg Leu Arg Tyr Ala Thr Asn Gly Ala Gly Asn Thr Leu
                   565                 570                 575
   Pro Asn Ile Ser Leu Thr Ile Pro Gly Val Ile Gly Ile Pro Pro Gln
               580                 585                 590
   Arg Leu Asn Asn Thr Phe Ser Gly Thr Asn Tyr Asn Asn Leu Gln Tyr
           595                 600                 605
   Gly Asp Phe Gly Tyr Phe Gln Phe Pro Ser Thr Val Thr Leu Pro Leu
       610                 615                 620
   Asn Arg Asn Ile Pro Phe Ile Phe Asn Arg Ala Asp Val Ser Asn Ser
   625                 630                 635                 640
   Ile Leu Ile Ile Asp Lys Ile Glu Phe Ile Pro Ile Thr Ser Ser Val
                   645                 650                 655
   Arg Gln Asn Arg Glu Lys Gln Lys Leu Glu Thr Ile Gln Thr Lys Ile
               660                 665                 670
   Asn Thr Phe Phe Thr Asn His Thr Lys Asn Thr Leu Asn Ile Glu Ala
           675                 680                 685
   Thr Asn Tyr Asp Ile Asp
       690
<210> 12
   <211> 5980
   <212> DNA
   <213> Bacillus thruingiensis
<400> 12
   tacatgcaat acataaagag aaggtttaaa aaatcaatac ctcaccaaaa ataatgggtt 60
   tatttgtaga aacattgtta caggaataca ttggggtact acgaatatat agaaagacac 120
   ctaacatata tttattaggt gtcttaaaaa taaggactat ataaggagtg aaaaagatga 180
   gtccatatca aaataaaaat gaatatgaaa tattggaatc ctcatcgaat aacacaaata 240
   cgccaaacag atatcctttt gcaaataatc gggatatgtc tactatgtct tggaatgatt 300
   gtcagggaat ctcatgggat gaaatttggg aatcagtcga aacgataaca agtattggga 360
   taaatcttat agagtttgtg atagaaccta gtttgggtgg aattaataca ctattatcaa 420
   taataggaaa actaattccg actaatcgtc aaactgtgtc agcactttct atatgtgatt 480
   tattatctat aattcgtaaa gaggtagccg atagtgtttt aagtgatgcg attgcagatt 540
   ttgacggtaa attgaaaaat tatagagagt attatctttc ttatcttggg gcttggctta 600
   aagacggtaa accacttcaa aagacaaata attctgatat cggacaatta gtttattatt 660
   ttaaactttc agaaagagat ttcaatgaaa ttctaggagg gtcattgtca agaaacaatg 720
   ctcaagtatt gttattacct acttttgcac aagctgcaaa tgtgcagtta ttactattaa 780
   gggatgcagt tcaatataaa gcacaatggt tcccattttt gagtgcagag aatgtaagat 840
   cggaattaat atcacctaac agtggttgtg attttaccgg tgattactat gagcgattaa 900
   aatgcaaaac ggcagagtat accaattatt gtttatattg gtatcaggta ggtttaaatc 960
   agataaaaca gggggggaca ggtgctgaca cttggtcgaa atttaataaa tttcgtagag 1020
   aaatgacgtt ggcggtattg gatattatcg ctatatttcc aacttatgat tttgagaaat 1080
   atccattgcc aacacatgta gagttgacta gggaaattta tacagatgca gtgggatatt 1140
   catcgggaac ttatagttgg ttacggaatt ggcctaatac ttttaatggg ttagaggcta 1200
   atggaacacg gggacctggt ttagttactt ggcttagcaa aataggtata tataatgagt 1260
   atgtttcgag atattttgcc ggctgggtag gaactcgtca ttatgaagac tacacaaagg 1320
   gtaacggtat ttttcaacgt atgtctggaa ctacgagtaa tgatctacgt aatattgatt 1380
   ttcagaatgc cgatgtatat aaaattactt cattagctat catgaaccta gtaggagaga 1440
   ctaccgctag accagagtat cgtgtttcaa aggcagattt tcgtagggta gggggacctg 1500
   atttaaatta tgatgcaggt aataatgggc taagcaggat gacaattgaa tctacgttcc 1560
   cacttgtatt gcactctaat ggtgttagag gaccctctca tagattatca aatgcggcat 1620
   gtgttgtata tggaaactcc agagttaacg tatatggttg gacacataca agtttaaaac 1680
   gtgaaaatat aattgaagcc aatcaaatta cacaaatacc ggcggtgaag agttattacc 1740
   ttcaaaatta tcttgctaat gcctatacct atgtaataaa aggcactcat acaggtgggg 1800
   atttaatccg ttttttaaga acaaaatcag agtataacgc agtttatgca ggtggcggaa 1860
   ttagattgat tattaataac aaaactgcag gacaaagtta ccgtattcgt tttcgttatg 1920
   ctgcagataa agctgctttc tttagtgtat atctttatcc aggaggttgg ggttcaaatc 1980
   gttttgtatc gcttgaaaaa tcttactctg gaaattatga cgatttaaaa tatagtgatt 2040
   ttaaattcgc tgaaattatc acacctccat tacctagttc aaacattcag atggatgtgg 2100
   agatgcaagc gaatagtttt caatcagatg taaacgtggt tctcgacaaa attgaattcc 2160
   tcccaagtaa tacaacaact ttagaatatg agggagaacg ggacctagaa aaaacaaaga 2220
   acgcggtgaa cgatctgttt accaattaaa acaaaataat ttactagaat aggtggtatt 2280
   gctgtttaac aaataagcga aaaaggttgt gagtcctatg tttacaagta gtacgaaaaa 2340
   tacgttaaaa atagaaacga cagattatga aatagatcaa gcggccattt ctatagaatg 2400
   tatgtcagat gaacaaaatc ctcaggaaaa aataatgtta tgggatgaaa taaaactggc 2460
   aaaacaactt agtcagtctc gtaatctact ccaaaatgga gacttttctg ggaatgattg 2520
   gacattcggt aatgatatta tcataggatc caataatcct atttttaaag gaaaatttct 2580
   acagatgcgt ggagcacgag acatatatgg aactctattt ccaacctata tctgtcaaaa 2640
   aatagatgag tctaaattaa aaccatatac acgttatcga gtaagagggt ttgtgggaag 2700
   tagtaaagat ttgaaattaa tggtaacacg ttacgggaaa gaaattgatg ctatcatgaa 2760
   tgttccaaat gatttggcct atatgcagcc taatccttca tgtggagatt atcgctgtga 2820
   atcatcgtct cagtatgtga gccaagggta tcctacacca acagatggat atgctcccga 2880
   tatgtatgca tgcccgcaaa atatagatag aaagcatgtg aagtgtcacg atcgtcatcc 2940
   atttgatttt catattgaca ccggagaagt agatacaaat acaaatgtag gtattgatgt 3000
   cttattaaaa atttctaatc cagatggata cgctacagta gggaatctag aagtcattga 3060
   agaaggacca ctaacaggtg aagcattggc acatgtgaaa caaaaggaaa agaaatggaa 3120
   acaacacatg gagaaaaaac gttgggaaac acaacaagcc tatgatccag caaaacaggc 3180
   tgtagatgca ttatttacaa atgaacaaga gttacactat catattactt tagatcatat 3240
   tcaaaacgct gatcgactgg tacagtcgat tccctatgta taccataatt ggttaccgaa 3300
   tgctccaggt atgaactatg atgtatatca agagttaaac gcacgtatca tgcaaggtta 3360
   taatttatat gatgcacgaa atgtcataac aaatggtgac tttacacaag gattacaggg 3420
   atggcacgca acaggaaatg ccgcggtaca acaaatggat ggagcttcag tattagttct 3480
   atcaaattgg agcgcggggg tatctcaaaa cttgcatgct caagatcatc atggatatgt 3540
   gttacgtgtg attgccaaaa aagaaggacc tggaaaaggg tatgtaacga tgatggattg 3600
   taatggaaag caggaaacac ttaagttcac ttcttgcgaa gaaggatata tgacaaaaac 3660
   agtagaggta ttcccagaaa gtgatcgtgt acggattgaa ataggagaaa ccgaaggtac 3720
   attttatata gatagcatcg agttgctttg tatgcaagga tatgataaca ataataacct 3780
   gcacacgggt aatatgtatg agcaaagtta taatggaaat tataatcaaa atactagcga 3840
   tgtgtattac caagggtata caaacaacta taaccaagac tctagtaata tgtataatca 3900
   aaattatact aacaatgatg acctgcattc cggttgcaca tgtaaccaag ggcataactc 3960
   tggctgtaca tgtaatcaag gatataaccg ttaacgattc taaataagaa tcaacatcat 4020
   tgcgaaaaat aaaaacctac tcacaaaatc tattgcatat cataacataa gctttacaaa 4080
   taacggacat attctagaag aggtctcctt aattctaaaa taaggagatt tttttcgttt 4140
   ccccaatatt gattaatgaa aatactcctt tacagaaaga tttaggctga ttggtgtttt 4200
   gtgcaagcag caattcctga aaaatgcaga aaaaattcca gaggcatgca gctacttttt 4260
   taaatgttaa ttttggtatt tccagaagat aaagcatttc gtaaacgata actttcacct 4320
   gtgaatgcaa tgatatgagc atgatggacc aaacggtcca ccaatgcaga agtaagccgg 4380
   ttatcaccaa atatacgatt ccattgacca aattctaagt ttgaagtaac tatgacactt 4440
   ttttgttcgt aacagtcggc aataatatga aataggagct ccgctccttg cttttgaaat 4500
   ggtacataac ctagttcatc taaaattaat aaatcacagg cttcaatttg cttcttgatg 4560
   cgggttaaat tcccttgttt taaagcctct tctaatagac ctactaaatc tgctactcgg 4620
   aaaaacttca cttgatatcc ctgttcacaa gccttcaaac ctaatgctgt agctaaatgt 4680
   gttttcccag ttccaggaga tccaagtaat aaaagatttt gtttcccttc aaaaaatcgc 4740
   aattcacaaa tgtgttcttg attcgttgta gatggcagat gaatttgttc actccattca 4800
   taattcttga gccattttaa ctctcgaaat ttcgctttct tgattaagtt tgctactttt 4860
   ttcgtttgac gagattcaat ctctatattg aaaatatcac gcagaaattg ttctttggtt 4920
   tcaaaaggta tttcatcata atggtctata atatagctta aatgcagtga tttacaggcg 4980
   tcttgcaggt ttcgttgtat cataatgaga atgaacccct ttctaaagtt ggacaaagct 5040
   ttcgatcata ttgctgaaga tctgtttcgt aatctattaa aacaggtggc gtatggtttt 5100
   cttgccattt ttgtggatac gtcacttgtt gagtctgcaa tatgatatcg agttcgtgag 5160
   gagctttttt caaccattct tcattctcta acagtaagtg tagctgctgt aatgagtatt 5220
   tttgaattaa atatttgaat cctgttaaac aagcttttct ttcatcctta tgaaagagca 5280
   gataatcttg aacccgctga ggtaaatatt tgaaaaatct agaataacga acaacgcgtg 5340
   gtttcttttc ccaatcttca aaaatctctt cccatagaat cggcctactc gtgtgcatat 5400
   acggtctgta ttctgtaaaa atgttctcgc cttctgcggt ataacatgta aattgatccc 5460
   attctttttt aatgataatc acttgtttta cgtaaaacaa aacgttcttg atccactgtg 5520
   atttctccat atttatttac cgttgttgtg tccattgaga agaccgttag gtcttccaaa 5580
   ggtaatggtt tcaatgcagc cttatcatca ttccaaaggt cttcaatcat gacttccttc 5640
   tcataatgta ggcgtttctg atcttctatt gcttgtactt ctaaccattg tgcgagttga 5700
   gaaaaactct ccataatcgg agcggttgta aaccaattgt ttcgtgtata actgactttc 5760
   cgttcaacgt tccccttttc atgcccgcta taaggattac aaggttgcac ttcaaagtta 5820
   taatgcattt gaaattgtaa aaatgcatcc gtataagttc gattatcacc ttttcctact 5880
   gtgactacag cggcagataa gttatcaatt cgaagatgcg ttggtacacc accagcttga 5940
   tgaaataatc gttttaagcc ttctaaaaag ccttctgtat                       5980
<210> 13
   <211> 2082
   <212> DNA
   <213> Bacillus thuringiensis
<220>
   <221> CDS
   <222> (1)...(2082)
<400> 13
   gtg aaa aag atg agt cca tat caa aat aaa aat gaa tat gaa ata ttg    48
   Met Lys Lys Met Ser Pro Tyr Gln Asn Lys Asn Glu Tyr Glu Ile Leu
    1               5                  10                  15
   gaa tcc tca tcg aat aac aca aat acg cca aac aga tat cct ttt gca    96
   Glu Ser Ser Ser Asn Asn Thr Asn Thr Pro Asn Arg Tyr Pro Phe Ala
               20                  25                  30
   aat aat cgg gat atg tct act atg tct tgg aat gat tgt cag gga atc    144
   Asn Asn Arg Asp Met Ser Thr Met Ser Trp Asn Asp Cys Gln Gly Ile
           35                  40                  45
   tca tgg gat gaa att tgg gaa tca gtc gaa acg ata aca agt att ggg    192
   Ser Trp Asp Glu Ile Trp Glu Ser Val Glu Thr Ile Thr Ser Ile Gly
       50                  55                  60
   ata aat ctt ata gag ttt gtg ata gaa cct agt ttg ggt gga att aat    240
   Ile Asn Leu Ile Glu Phe Val Ile Glu Pro Ser Leu Gly Gly Ile Asn
   65                  70                  75                  80
   aca cta tta tca ata ata gga aaa cta att ccg act aat cgt caa act    288
   Thr Leu Leu Ser Ile Ile Gly Lys Leu Ile Pro Thr Asn Arg Gln Thr
                   85                  90                  95
   gtg tca gca ctt tct ata tgt gat tta tta tct ata att cgt aaa gag    336
   Val Ser Ala Leu Ser Ile Cys Asp Leu Leu Ser Ile Ile Arg Lys Glu
               100                 105                 110
   gta gcc gat agt gtt tta agt gat gcg att gca gat ttt gac ggt aaa    384
   Val Ala Asp Ser Val Leu Ser Asp Ala Ile Ala Asp Phe Asp Gly Lys
           115                 120                 125
   ttg aaa aat tat aga gag tat tat ctt tct tat ctt ggg gct tgg ctt    432
   Leu Lys Asn Tyr Arg Glu Tyr Tyr Leu Ser Tyr Leu Gly Ala Trp Leu
       130                 135                 140
   aaa gac ggt aaa cca ctt caa aag aca aat aat tct gat atc gga caa    480
   Lys Asp Gly Lys Pro Leu Gln Lys Thr Asn Asn Ser Asp Ile Gly Gln
   145                 150                 155                 160
   tta gtt tat tat ttt aaa ctt tca gaa aga gat ttc aat gaa att cta    528
   Leu Val Tyr Tyr Phe Lys Leu Ser Glu Arg Asp Phe Asn Glu Ile Leu
                   165                 170                 175
   gga ggg tca ttg tca aga aac aat gct caa gta ttg tta tta cct act    576
   Gly Gly Ser Leu Ser Arg Asn Asn Ala Gln Val Leu Leu Leu Pro Thr
               180                 185                 190
   ttt gca caa gct gca aat gtg cag tta tta cta tta agg gat gca gtt    624
   Phe Ala Gln Ala Ala Asn Val Gln Leu Leu Leu Leu Arg Asp Ala Val
           195                 200                 205
   caa tat aaa gca caa tgg ttc cca ttt ttg agt gca gag aat gta aga    672
   Gln Tyr Lys Ala Gln Trp Phe Pro Phe Leu Ser Ala Glu Asn Val Arg
       210                 215                 220
   tcg gaa tta ata tca cct aac agt ggt tgt gat ttt acc ggt gat tac    720
   Ser Glu Leu Ile Ser Pro Asn Ser Gly Cys Asp Phe Thr Gly Asp Tyr
   225                 230                 235                 240
   tat gag cga tta aaa tgc aaa acg gca gag tat acc aat tat tgt tta    768
   Tyr Glu Arg Leu Lys Cys Lys Thr Ala Glu Tyr Thr Asn Tyr Cys Leu
                   245                 250                 255
   tat tgg tat cag gta ggt tta aat cag ata aaa cag ggg ggg aca ggt    816
   Tyr Trp Tyr Gln Val Gly Leu Asn Gln Ile Lys Gln Gly Gly Thr Gly
               260                 265                 270
   gct gac act tgg tcg aaa ttt aat aaa ttt cgt aga gaa atg acg ttg    864
   Ala Asp Thr Trp Ser Lys Phe Asn Lys Phe Arg Arg Glu Met Thr Leu
           275                 280                 285
   gcg gta ttg gat att atc gct ata ttt cca act tat gat ttt gag aaa    912
   Ala Val Leu Asp Ile Ile Ala Ile Phe Pro Thr Tyr Asp Phe Glu Lys
       290                 295                 300
   tat cca ttg cca aca cat gta gag ttg act agg gaa att tat aca gat    960
   Tyr Pro Leu Pro Thr His Val Glu Leu Thr Arg Glu Ile Tyr Thr Asp
   305                 310                 315                 320
   gca gtg gga tat tca tcg gga act tat agt tgg tta cgg aat tgg cct    1008
   Ala Val Gly Tyr Ser Ser Gly Thr Tyr Ser Trp Leu Arg Asn Trp Pro
                   325                 330                 335
   aat act ttt aat ggg tta gag gct aat gga aca cgg gga cct ggt tta    1056
   Asn Thr Phe Asn Gly Leu Glu Ala Asn Gly Thr Arg Gly Pro Gly Leu
               340                 345                 350
   gtt act tgg ctt agc aaa ata ggt ata tat aat gag tat gtt tcg aga    1104
   Val Thr Trp Leu Ser Lys Ile Gly Ile Tyr Asn Glu Tyr Val Ser Arg
           355                 360                 365
   tat ttt gcc ggc tgg gta gga act cgt cat tat gaa gac tac aca aag    1152
   Tyr Phe Ala Gly Trp Val Gly Thr Arg His Tyr Glu Asp Tyr Thr Lys
       370                 375                 380
   ggt aac ggt att ttt caa cgt atg tct gga act acg agt aat gat cta    1200
   Gly Asn Gly Ile Phe Gln Arg Met Ser Gly Thr Thr Ser Asn Asp Leu
   385                 390                 395                 400
   cgt aat att gat ttt cag aat gcc gat gta tat aaa att act tca tta    1248
   Arg Asn Ile Asp Phe Gln Asn Ala Asp Val Tyr Lys Ile Thr Ser Leu
                   405                 410                 415
   gct atc atg aac cta gta gga gag act acc gct aga cca gag tat cgt    1296
   Ala Ile Met Asn Leu Val Gly Glu Thr Thr Ala Arg Pro Glu Tyr Arg
               420                 425                 430
   gtt tca aag gca gat ttt cgt agg gta ggg gga cct gat tta aat tat    1344
   Val Ser Lys Ala Asp Phe Arg Arg Val Gly Gly Pro Asp Leu Asn Tyr
           435                 440                 445
   gat gca ggt aat aat ggg cta agc agg atg aca att gaa tct acg ttc    1392
   Asp Ala Gly Asn Asn Gly Leu Ser Arg Met Thr Ile Glu Ser Thr Phe
   450 455 460
   cca ctt gta ttg cac tct aat ggt gtt aga gga ccc tct cat aga tta 1440
   Pro Leu Val Leu His Ser Asn Gly Val Arg Gly Pro Ser His Arg Leu
   465 470 475 480
   tca aat gcg gca tgt gtt gta tat gga aac tcc aga gtt aac gta tat 1488
   Ser Asn Ala Ala Cys Val Val Tyr Gly Asn Ser Arg Val Asn Val Tyr
   485 490 495
   ggt tgg aca cat aca agt tta aaa cgt gaa aat ata att gaa gcc aat 1536
   Gly Trp Thr His Thr Ser Leu Lys Arg Glu Asn Ile Ile Glu Ala Asn
   500 505 510
   caa att aca caa ata ccg gcg gtg aag agt tat tac ctt caa aat tat 1584
   Gln Ile Thr Gln Ile Pro Ala Val Lys Ser Tyr Tyr Leu Gln Asn Tyr
   515 520 525
   ctt gct aat gcc tat acc tat gta ata aaa ggc act cat aca ggt ggg 1632
   Leu Ala Asn Ala Tyr Thr Tyr Val Ile Lys Gly Thr His Thr Gly Gly
   530 535 540
   gat tta atc cgt ttt tta aga aca aaa tca gag tat aac gca gtt tat 1680
   Asp Leu Ile Arg Phe Leu Arg Thr Lys Ser Glu Tyr Asn Ala Val Tyr
   545 550 555 560
   gca ggt ggc gga att aga ttg att att aat aac aaa act gca gga caa 1728
   Ala Gly Gly Gly Ile Arg Leu Ile Ile Asn Asn Lys Thr Ala Gly Gln
   565 570 575
   agt tac cgt att cgt ttt cgt tat gct gca gat aaa gct gct ttc ttt 1776
   Ser Tyr Arg Ile Arg Phe Arg Tyr Ala Ala Asp Lys Ala Ala Phe Phe
   580 585 590
   agt gta tat ctt tat cca gga ggt tgg ggt tca aat cgt ttt gta tcg 1824
   Ser Val Tyr Leu Tyr Pro Gly Gly Trp Gly Ser Asn Arg Phe Val Ser
   595 600 605
   ctt gaa aaa tct tac tct gga aat tat gac gat tta aaa tat agt gat 1872
   Leu Glu Lys Ser Tyr Ser Gly Asn Tyr Asp Asp Leu Lys Tyr Ser Asp
   610 615 620
   ttt aaa ttc gct gaa att atc aca cct cca tta cct agt tca aac att 1920
   Phe Lys Phe Ala Glu Ile Ile Thr Pro Pro Leu Pro Ser Ser Asn Ile
   625 630 635 640
   cag atg gat gtg gag atg caa gcg aat agt ttt caa tca gat gta aac 1968
   Gln Met Asp Val Glu Met Gln Ala Asn Ser Phe Gln Ser Asp Val Asn
   645 650 655
   gtg gtt ctc gac aaa att gaa ttc ctc cca agt aat aca aca act tta 2016
   Val Val Leu Asp Lys Ile Glu Phe Leu Pro Ser Asn Thr Thr Thr Leu
   660 665 670
   gaa tat gag gga gaa cgg gac cta gaa aaa aca aag aac gcg gtg aac 2064
   Glu Tyr Glu Gly Glu Arg Asp Leu Glu Lys Thr Lys Asn Ala Val Asn
   675 680 685
   gat ctg ttt acc aat taa 2082
   Asp Leu Phe Thr Asn *
   690
<210> 14
   <211> 693
   <212> PRT
   <213> Bacillus thuringiensis
<400> 14
   Met Lys Lys Met Ser Pro Tyr Gln Asn Lys Asn Glu Tyr Glu Ile Leu
   1               5                   10                  15
   Glu Ser Ser Ser Asn Asn Thr Asn Thr Pro Asn Arg Tyr Pro Phe Ala
               20                  25                  30
   Asn Asn Arg Asp Met Ser Thr Met Ser Trp Asn Asp Cys Gln Gly Ile
           35                  40                  45
   Ser Trp Asp Glu Ile Trp Glu Ser Val Glu Thr Ile Thr Ser Ile Gly
       50                  55                  60
   Ile Asn Leu Ile Glu Phe Val Ile Glu Pro Ser Leu Gly Gly Ile Asn
   65                  70                  75                  80
   Thr Leu Leu Ser Ile Ile Gly Lys Leu Ile Pro Thr Asn Arg Gln Thr
                   85                  90                  95
   Val Ser Ala Leu Ser Ile Cys Asp Leu Leu Ser Ile Ile Arg Lys Glu
               100                 105                 110
   Val Ala Asp Ser Val Leu Ser Asp Ala Ile Ala Asp Phe Asp Gly Lys
           115                 120                 125
   Leu Lys Asn Tyr Arg Glu Tyr Tyr Leu Ser Tyr Leu Gly Ala Trp Leu
       130                 135                 140
   Lys Asp Gly Lys Pro Leu Gln Lys Thr Asn Asn Ser Asp Ile Gly Gln
   145                 150                 155                 160
   Leu Val Tyr Tyr Phe Lys Leu Ser Glu Arg Asp Phe Asn Glu Ile Leu
                   165                 170                 175
   Gly Gly Ser Leu Ser Arg Asn Asn Ala Gln Val Leu Leu Leu Pro Thr
               180                 185                 190
   Phe Ala Gln Ala Ala Asn Val Gln Leu Leu Leu Leu Arg Asp Ala Val
           195                 200                 205
   Gln Tyr Lys Ala Gln Trp Phe Pro Phe Leu Ser Ala Glu Asn Val Arg
       210                 215                 220
   Ser Glu Leu Ile Ser Pro Asn Ser Gly Cys Asp Phe Thr Gly Asp Tyr
   225                 230                 235                 240
   Tyr Glu Arg Leu Lys Cys Lys Thr Ala Glu Tyr Thr Asn Tyr Cys Leu
                   245                 250                 255
   Tyr Trp Tyr Gln Val Gly Leu Asn Gln Ile Lys Gln Gly Gly Thr Gly
               260                 265                 270
   Ala Asp Thr Trp Ser Lys Phe Asn Lys Phe Arg Arg Glu Met Thr Leu
           275                 280                 285
   Ala Val Leu Asp Ile Ile Ala Ile Phe Pro Thr Tyr Asp Phe Glu Lys
       290                 295                 300
   Tyr Pro Leu Pro Thr His Val Glu Leu Thr Arg Glu Ile Tyr Thr Asp
   305                 310                 315                 320
   Ala Val Gly Tyr Ser Ser Gly Thr Tyr Ser Trp Leu Arg Asn Trp Pro
                   325                 330                 335
   Asn Thr Phe Asn Gly Leu Glu Ala Asn Gly Thr Arg Gly Pro Gly Leu
               340                 345                 350
   Val Thr Trp Leu Ser Lys Ile Gly Ile Tyr Asn Glu Tyr Val Ser Arg
           355                 360                 365
   Tyr Phe Ala Gly Trp Val Gly Thr Arg His Tyr Glu Asp Tyr Thr Lys
       370                 375                 380
   Gly Asn Gly Ile Phe Gln Arg Met Ser Gly Thr Thr Ser Asn Asp Leu
   385                 390                 395                 400
   Arg Asn Ile Asp Phe Gln Asn Ala Asp Val Tyr Lys Ile Thr Ser Leu
                   405                 410                 415
   Ala Ile Met Asn Leu Val Gly Glu Thr Thr Ala Arg Pro Glu Tyr Arg
               420                 425                 430
   Val Ser Lys Ala Asp Phe Arg Arg Val Gly Gly Pro Asp Leu Asn Tyr
           435                 440                 445
   Asp Ala Gly Asn Asn Gly Leu Ser Arg Met Thr Ile Glu Ser Thr Phe
       450                 455                 460
   Pro Leu Val Leu His Ser Asn Gly Val Arg Gly Pro Ser His Arg Leu
   465                 470                 475                 480
   Ser Asn Ala Ala Cys Val Val Tyr Gly Asn Ser Arg Val Asn Val Tyr
                   485                 490                 495
   Gly Trp Thr His Thr Ser Leu Lys Arg Glu Asn Ile Ile Glu Ala Asn
               500                 505                 510
   Gln Ile Thr Gln Ile Pro Ala Val Lys Ser Tyr Tyr Leu Gln Asn Tyr
           515                 520                 525
   Leu Ala Asn Ala Tyr Thr Tyr Val Ile Lys Gly Thr His Thr Gly Gly
           530                     535                     540
   Asp Leu Ile Arg Phe Leu Arg Thr Lys Ser Glu Tyr Asn Ala Val Tyr
   545                     550                     555                     560
   Ala Gly Gly Gly Ile Arg Leu Ile Ile Asn Asn Lys Thr Ala Gly Gln
                   565                     570                     575
   Ser Tyr Arg Ile Arg Phe Arg Tyr Ala Ala Asp Lys Ala Ala Phe Phe
               580                     585                     590
   Ser Val Tyr Leu Tyr Pro Gly Gly Trp Gly Ser Asn Arg Phe Val Ser
           595                     600                     605
   Leu Glu Lys Ser Tyr Ser Gly Asn Tyr Asp Asp Leu Lys Tyr Ser Asp
       610                     615                     620
   Phe Lys Phe Ala Glu Ile Ile Thr Pro Pro Leu Pro Ser Ser Asn Ile
   625                     630                     635                     640
   Gln Met Asp Val Glu Met Gln Ala Asn Ser Phe Gln Ser Asp Val Asn
                   645                     650                     655
   Val Val Leu Asp Lys Ile Glu Phe Leu Pro Ser Asn Thr Thr Thr Leu
               660                     665                     670
   Glu Tyr Glu Gly Glu Arg Asp Leu Glu Lys Thr Lys Asn Ala Val Asn
           675                     680                     685
   Asp Leu Phe Thr Asn
       690
<210> 15
   <211> 2073
   <212> DNA
   <213> Bacillus thuringiensis
<220>
   <221> CDS
   <222> (1)...(2073)
<400> 15 acc aat taa 2073 Thr Asn * 690
<210> 16
   <211> 690
   <212> PRT
   <213> Bacillus thuringiensis
<400> 16
   Met Ser Pro Tyr Gln Asn Lys Asn Glu Tyr Glu Ile Leu Glu Ser Ser
   1               5                   10                  15
   Ser Asn Asn Thr Asn Thr Pro Asn Arg Tyr Pro Phe Ala Asn Asn Arg
               20                  25                  30
   Asp Met Ser Thr Met Ser Trp Asn Asp Cys Gln Gly Ile Ser Trp Asp
           35                  40                  45
   Glu Ile Trp Glu Ser Val Glu Thr Ile Thr Ser Ile Gly Ile Asn Leu
       50                  55                  60
   Ile Glu Phe Val Ile Glu Pro Ser Leu Gly Gly Ile Asn Thr Leu Leu
   65                  70                  75                  80
   Ser Ile Ile Gly Lys Leu Ile Pro Thr Asn Arg Gln Thr Val Ser Ala
                   85                  90                  95
   Leu Ser Ile Cys Asp Leu Leu Ser Ile Ile Arg Lys Glu Val Ala Asp
               100                 105                 110
   Ser Val Leu Ser Asp Ala Ile Ala Asp Phe Asp Gly Lys Leu Lys Asn
           115                 120                 125
   Tyr Arg Glu Tyr Tyr Leu Ser Tyr Leu Gly Ala Trp Leu Lys Asp Gly
       130                 135                 140
   Lys Pro Leu Gln Lys Thr Asn Asn Ser Asp Ile Gly Gln Leu Val Tyr
   145                 150                 155                 160
   Tyr Phe Lys Leu Ser Glu Arg Asp Phe Asn Glu Ile Leu Gly Gly Ser
                   165                 170                 175
   Leu Ser Arg Asn Asn Ala Gln Val Leu Leu Leu Pro Thr Phe Ala Gln
               180                 185                 190
   Ala Ala Asn Val Gln Leu Leu Leu Leu Arg Asp Ala Val Gln Tyr Lys
           195                 200                 205
   Ala Gln Trp Phe Pro Phe Leu Ser Ala Glu Asn Val Arg Ser Glu Leu
       210                 215                 220
   Ile Ser Pro Asn Ser Gly Cys Asp Phe Thr Gly Asp Tyr Tyr Glu Arg
   225                 230                 235                 240
   Leu Lys Cys Lys Thr Ala Glu Tyr Thr Asn Tyr Cys Leu Tyr Trp Tyr
                   245                 250                 255
   Gln Val Gly Leu Asn Gln Ile Lys Gln Gly Gly Thr Gly Ala Asp Thr
               260                 265                 270
   Trp Ser Lys Phe Asn Lys Phe Arg Arg Glu Met Thr Leu Ala Val Leu
           275                 280                 285
   Asp Ile Ile Ala Ile Phe Pro Thr Tyr Asp Phe Glu Lys Tyr Pro Leu
       290                 295                 300
   Pro Thr His Val Glu Leu Thr Arg Glu Ile Tyr Thr Asp Ala Val Gly
   305                 310                 315                 320
   Tyr Ser Ser Gly Thr Tyr Ser Trp Leu Arg Asn Trp Pro Asn Thr Phe
                   325                 330                 335
   Asn Gly Leu Glu Ala Asn Gly Thr Arg Gly Pro Gly Leu Val Thr Trp
               340                 345                 350
   Leu Ser Lys Ile Gly Ile Tyr Asn Glu Tyr Val Ser Arg Tyr Phe Ala
           355                 360                 365
   Gly Trp Val Gly Thr Arg His Tyr Glu Asp Tyr Thr Lys Gly Asn Gly
       370                 375                 380
   Ile Phe Gln Arg Met Ser Gly Thr Thr Ser Asn Asp Leu Arg Asn Ile
   385                 390                 395                 400
   Asp Phe Gln Asn Ala Asp Val Tyr Lys Ile Thr Ser Leu Ala Ile Met
                   405                 410                 415
   Asn Leu Val Gly Glu Thr Thr Ala Arg Pro Glu Tyr Arg Val Ser Lys
               420                 425                 430
   Ala Asp Phe Arg Arg Val Gly Gly Pro Asp Leu Asn Tyr Asp Ala Gly
           435                 440                 445
   Asn Asn Gly Leu Ser Arg Met Thr Ile Glu Ser Thr Phe Pro Leu Val
       450                 455                 460
   Leu His Ser Asn Gly Val Arg Gly Pro Ser His Arg Leu Ser Asn Ala
   465                 470                 475                 480
   Ala Cys Val Val Tyr Gly Asn Ser Arg Val Asn Val Tyr Gly Trp Thr
                   485                 490                 495
   His Thr Ser Leu Lys Arg Glu Asn Ile Ile Glu Ala Asn Gln Ile Thr
               500                 505                 510
   Gln Ile Pro Ala Val Lys Ser Tyr Tyr Leu Gln Asn Tyr Leu Ala Asn
           515                 520                 525
   Ala Tyr Thr Tyr Val Ile Lys Gly Thr His Thr Gly Gly Asp Leu Ile
       530                 535                 540
   Arg Phe Leu Arg Thr Lys Ser Glu Tyr Asn Ala Val Tyr Ala Gly Gly
   545                 550                 555                 560
   Gly Ile Arg Leu Ile Ile Asn Asn Lys Thr Ala Gly Gln Ser Tyr Arg
                   565                 570                 575
   Ile Arg Phe Arg Tyr Ala Ala Asp Lys Ala Ala Phe Phe Ser Val Tyr
               580                 585                 590
   Leu Tyr Pro Gly Gly Trp Gly Ser Asn Arg Phe Val Ser Leu Glu Lys
           595                 600                 605
   Ser Tyr Ser Gly Asn Tyr Asp Asp Leu Lys Tyr Ser Asp Phe Lys Phe
       610                 615                 620
   Ala Glu Ile Ile Thr Pro Pro Leu Pro Ser Ser Asn Ile Gln Met Asp
   625                 630                 635                 640
   Val Glu Met Gln Ala Asn Ser Phe Gln Ser Asp Val Asn Val Val Leu
                   645                 650                 655
   Asp Lys Ile Glu Phe Leu Pro Ser Asn Thr Thr Thr Leu Glu Tyr Glu
               660                 665                 670
   Gly Glu Arg Asp Leu Glu Lys Thr Lys Asn Ala Val Asn Asp Leu Phe
           675                 680                 685
   Thr Asn
       690
<210> 17
   <211> 1686
   <212> DNA
   <213> Bacillus thuringiensis
<220>
   <221> CDS
   <222> (1)...(1686)
<400> 17
   gtg agt cct atg ttt aca agt agt acg aaa aat acg tta aaa ata gaa    48
   Met Ser Pro Met Phe Thr Ser Ser Thr Lys Asn Thr Leu Lys Ile Glu
    1               5                  10                  15
   acg aca gat tat gaa ata gat caa gcg gcc att tct ata gaa tgt atg    96
   Thr Thr Asp Tyr Glu Ile Asp Gln Ala Ala Ile Ser Ile Glu Cys Met
               20                  25                  30
   tca gat gaa caa aat cct cag gaa aaa ata atg tta tgg gat gaa ata   144
   Ser Asp Glu Gln Asn Pro Gln Glu Lys Ile Met Leu Trp Asp Glu Ile
           35                  40                  45
   aaa ctg gca aaa caa ctt agt cag tct cgt aat cta ctc caa aat gga   192
   Lys Leu Ala Lys Gln Leu Ser Gln Ser Arg Asn Leu Leu Gln Asn Gly
       50                  55                  60
   gac ttt tct ggg aat gat tgg aca ttc ggt aat gat att atc ata gga   240
   Asp Phe Ser Gly Asn Asp Trp Thr Phe Gly Asn Asp Ile Ile Ile Gly
   65                  70                  75                  80
   tcc aat aat cct att ttt aaa gga aaa ttt cta cag atg cgt gga gca   288
   Ser Asn Asn Pro Ile Phe Lys Gly Lys Phe Leu Gln Met Arg Gly Ala
                   85                  90                  95
   cga gac ata tat gga act cta ttt cca acc tat atc tgt caa aaa ata     336
   Arg Asp Ile Tyr Gly Thr Leu Phe Pro Thr Tyr Ile Cys Gln Lys Ile
               100                 105                 110
   gat gag tct aaa tta aaa cca tat aca cgt tat cga gta aga ggg ttt     384
   Asp Glu Ser Lys Leu Lys Pro Tyr Thr Arg Tyr Arg Val Arg Gly Phe
           115                 120                 125
   gtg gga agt agt aaa gat ttg aaa tta atg gta aca cgt tac ggg aaa     432
   Val Gly Ser Ser Lys Asp Leu Lys Leu Met Val Thr Arg Tyr Gly Lys
       130                 135                 140
   gaa att gat gct atc atg aat gtt cca aat gat ttg gcc tat atg cag     480
   Glu Ile Asp Ala Ile Met Asn Val Pro Asn Asp Leu Ala Tyr Met Gln
   145                 150                 155                 160
   cct aat cct tca tgt gga gat tat cgc tgt gaa tca tcg tct cag tat     528
   Pro Asn Pro Ser Cys Gly Asp Tyr Arg Cys Glu Ser Ser Ser Gln Tyr
                   165                 170                 175
   gtg agc caa ggg tat cct aca cca aca gat gga tat gct ccc gat atg     576
   Val Ser Gln Gly Tyr Pro Thr Pro Thr Asp Gly Tyr Ala Pro Asp Met
               180                 185                 190
   tat gca tgc ccg caa aat ata gat aga aag cat gtg aag tgt cac gat     624
   Tyr Ala Cys Pro Gln Asn Ile Asp Arg Lys His Val Lys Cys His Asp
           195                 200                 205
   cgt cat cca ttt gat ttt cat att gac acc gga gaa gta gat aca aat     672
   Arg His Pro Phe Asp Phe His Ile Asp Thr Gly Glu Val Asp Thr Asn
       210                 215                 220
   aca aat gta ggt att gat gtc tta tta aaa att tct aat cca gat gga     720
   Thr Asn Val Gly Ile Asp Val Leu Leu Lys Ile Ser Asn Pro Asp Gly
   225                 230                 235                 240
   tac gct aca gta ggg aat cta gaa gtc att gaa gaa gga cca cta aca     768
   Tyr Ala Thr Val Gly Asn Leu Glu Val Ile Glu Glu Gly Pro Leu Thr
                   245                 250                 255
   ggt gaa gca ttg gca cat gtg aaa caa aag gaa aag aaa tgg aaa caa     816
   Gly Glu Ala Leu Ala His Val Lys Gln Lys Glu Lys Lys Trp Lys Gln
               260                 265                 270
   cac atg gag aaa aaa cgt tgg gaa aca caa caa gcc tat gat cca gca     864
   His Met Glu Lys Lys Arg Trp Glu Thr Gln Gln Ala Tyr Asp Pro Ala
           275                 280                 285
   aaa cag gct gta gat gca tta ttt aca aat gaa caa gag tta cac tat     912
   Lys Gln Ala Val Asp Ala Leu Phe Thr Asn Glu Gln Glu Leu His Tyr
       290                 295                 300
   cat att act tta gat cat att caa aac gct gat cga ctg gta cag tcg     960
   His Ile Thr Leu Asp His Ile Gln Asn Ala Asp Arg Leu Val Gln Ser
   305                 310                 315                 320
   att ccc tat gta tac cat aat tgg tta ccg aat gct cca ggt atg aac    1008
   Ile Pro Tyr Val Tyr His Asn Trp Leu Pro Asn Ala Pro Gly Met Asn
                   325                 330                 335
   tat gat gta tat caa gag tta aac gca cgt atc atg caa ggt tat aat    1056
   Tyr Asp Val Tyr Gln Glu Leu Asn Ala Arg Ile Met Gln Gly Tyr Asn
               340                 345                 350
   tta tat gat gca cga aat gtc ata aca aat ggt gac ttt aca caa gga    1104
   Leu Tyr Asp Ala Arg Asn Val Ile Thr Asn Gly Asp Phe Thr Gln Gly
           355                 360                 365
   tta cag gga tgg cac gca aca gga aat gcc gcg gta caa caa atg gat   1152
   Leu Gln Gly Trp His Ala Thr Gly Asn Ala Ala Val Gln Gln Met Asp
       370                 375                 380
   gga gct tca gta tta gtt cta tca aat tgg agc gcg ggg gta tct caa   1200
   Gly Ala Ser Val Leu Val Leu Ser Asn Trp Ser Ala Gly Val Ser Gln
   385                 390                 395                 400
   aac ttg cat gct caa gat cat cat gga tat gtg tta cgt gtg att gcc   1248
   Asn Leu His Ala Gln Asp His His Gly Tyr Val Leu Arg Val Ile Ala
                   405                 410                 415
   aaa aaa gaa gga cct gga aaa ggg tat gta acg atg atg gat tgt aat   1296
   Lys Lys Glu Gly Pro Gly Lys Gly Tyr Val Thr Met Met Asp Cys Asn
               420                 425                 430
   gga aag cag gaa aca ctt aag ttc act tct tgc gaa gaa gga tat atg   1344
   Gly Lys Gln Glu Thr Leu Lys Phe Thr Ser Cys Glu Glu Gly Tyr Met
           435                 440                 445
   aca aaa aca gta gag gta ttc cca gaa agt gat cgt gta cgg att gaa   1392
   Thr Lys Thr Val Glu Val Phe Pro Glu Ser Asp Arg Val Arg Ile Glu
       450                 455                 460
   ata gga gaa acc gaa ggt aca ttt tat ata gat agc atc gag ttg ctt   1440
   Ile Gly Glu Thr Glu Gly Thr Phe Tyr Ile Asp Ser Ile Glu Leu Leu
   465                 470                 475                 480
   tgt atg caa gga tat gat aac aat aat aac ctg cac acg ggt aat atg   1488
   Cys Met Gln Gly Tyr Asp Asn Asn Asn Asn Leu His Thr Gly Asn Met
                   485                 490                 495
   tat gag caa agt tat aat gga aat tat aat caa aat act agc gat gtg   1536
   Tyr Glu Gln Ser Tyr Asn Gly Asn Tyr Asn Gln Asn Thr Ser Asp Val
               500                 505                 510
   tat tac caa ggg tat aca aac aac tat aac caa gac tct agt aat atg   1584
   Tyr Tyr Gln Gly Tyr Thr Asn Asn Tyr Asn Gln Asp Ser Ser Asn Met
           515                 520                 525
   tat aat caa aat tat act aac aat gat gac ctg cat tcc ggt tgc aca   1632
   Tyr Asn Gln Asn Tyr Thr Asn Asn Asp Asp Leu His Ser Gly Cys Thr
       530                 535                 540
   tgt aac caa ggg cat aac tct ggc tgt aca tgt aat caa gga tat aac   1680
   Cys Asn Gln Gly His Asn Ser Gly Cys Thr Cys Asn Gln Gly Tyr Asn
   545                 550                 555                 560
   cgt taa                                                           1686
   Arg  *
<210> 18
   <211> 561
   <212> PRT
   <213> Bacillus thuringiensis
<400> 18
   Met Ser Pro Met Phe Thr Ser Ser Thr Lys Asn Thr Leu Lys Ile Glu
    1               5                  10                  15
   Thr Thr Asp Tyr Glu Ile Asp Gln Ala Ala Ile Ser Ile Glu Cys Met
               20                  25                  30
   Ser Asp Glu Gln Asn Pro Gln Glu Lys Ile Met Leu Trp Asp Glu Ile
           35                  40                  45
   Lys Leu Ala Lys Gln Leu Ser Gln Ser Arg Asn Leu Leu Gln Asn Gly
       50                  55                  60
   Asp Phe Ser Gly Asn Asp Trp Thr Phe Gly Asn Asp Ile Ile Ile Gly
   65                  70                  75                  80
   Ser Asn Asn Pro Ile Phe Lys Gly Lys Phe Leu Gln Met Arg Gly Ala
                   85                  90                  95
   Arg Asp Ile Tyr Gly Thr Leu Phe Pro Thr Tyr Ile Cys Gln Lys Ile
               100                 105                 110
   Asp Glu Ser Lys Leu Lys Pro Tyr Thr Arg Tyr Arg Val Arg Gly Phe
           115                 120                 125
   Val Gly Ser Ser Lys Asp Leu Lys Leu Met Val Thr Arg Tyr Gly Lys
       130                 135                 140
   Glu Ile Asp Ala Ile Met Asn Val Pro Asn Asp Leu Ala Tyr Met Gln
   145                 150                 155                 160
   Pro Asn Pro Ser Cys Gly Asp Tyr Arg Cys Glu Ser Ser Ser Gln Tyr
                   165                 170                 175
   Val Ser Gln Gly Tyr Pro Thr Pro Thr Asp Gly Tyr Ala Pro Asp Met
               180                 185                 190
   Tyr Ala Cys Pro Gln Asn Ile Asp Arg Lys His Val Lys Cys His Asp
           195                 200                 205
   Arg His Pro Phe Asp Phe His Ile Asp Thr Gly Glu Val Asp Thr Asn
       210                 215                 220
   Thr Asn Val Gly Ile Asp Val Leu Leu Lys Ile Ser Asn Pro Asp Gly
   225                 230                 235                 240
   Tyr Ala Thr Val Gly Asn Leu Glu Val Ile Glu Glu Gly Pro Leu Thr
                   245                 250                 255
   Gly Glu Ala Leu Ala His Val Lys Gln Lys Glu Lys Lys Trp Lys Gln
               260                 265                 270
   His Met Glu Lys Lys Arg Trp Glu Thr Gln Gln Ala Tyr Asp Pro Ala
           275                 280                 285
   Lys Gln Ala Val Asp Ala Leu Phe Thr Asn Glu Gln Glu Leu His Tyr
       290                 295                 300
   His Ile Thr Leu Asp His Ile Gln Asn Ala Asp Arg Leu Val Gln Ser
   305                 310                 315                 320
   Ile Pro Tyr Val Tyr His Asn Trp Leu Pro Asn Ala Pro Gly Met Asn
                   325                 330                 335
   Tyr Asp Val Tyr Gln Glu Leu Asn Ala Arg Ile Met Gln Gly Tyr Asn
               340                 345                 350
   Leu Tyr Asp Ala Arg Asn Val Ile Thr Asn Gly Asp Phe Thr Gln Gly
           355                 360                 365
   Leu Gln Gly Trp His Ala Thr Gly Asn Ala Ala Val Gln Gln Met Asp
       370                 375                 380
   Gly Ala Ser Val Leu Val Leu Ser Asn Trp Ser Ala Gly Val Ser Gln
   385                 390                 395                 400
   Asn Leu His Ala Gln Asp His His Gly Tyr Val Leu Arg Val Ile Ala
                   405                 410                 415
   Lys Lys Glu Gly Pro Gly Lys Gly Tyr Val Thr Met Met Asp Cys Asn
               420                 425                 430
   Gly Lys Gln Glu Thr Leu Lys Phe Thr Ser Cys Glu Glu Gly Tyr Met
           435                 440                 445
   Thr Lys Thr Val Glu Val Phe Pro Glu Ser Asp Arg Val Arg Ile Glu
       450                 455                 460
   Ile Gly Glu Thr Glu Gly Thr Phe Tyr Ile Asp Ser Ile Glu Leu Leu
   465                 470                 475                 480
   Cys Met Gln Gly Tyr Asp Asn Asn Asn Asn Leu His Thr Gly Asn Met
                   485                 490                 495
   Tyr Glu Gln Ser Tyr Asn Gly Asn Tyr Asn Gln Asn Thr Ser Asp Val
               500                 505                 510
   Tyr Tyr Gln Gly Tyr Thr Asn Asn Tyr Asn Gln Asp Ser Ser Asn Met
           515                 520                 525
   Tyr Asn Gln Asn Tyr Thr Asn Asn Asp Asp Leu His Ser Gly Cys Thr
       530                 535                 540
   Cys Asn Gln Gly His Asn Ser Gly Cys Thr Cys Asn Gln Gly Tyr Asn
   545                 550                 555                 560
   Arg
<210> 19
   <211> 2049
   <212> DNA
   <213> Bacillus thuringiensis
<220>
   <221> CDS
   <222> (1)...(2049)
<400> 19
   atg aat tca tat aaa aat aaa aat gaa tat gaa atg ttg gat gct tta     48
   Met Asn Ser Tyr Lys Asn Lys Asn Glu Tyr Glu Met Leu Asp Ala Leu
    1               5                  10                  15
   cga atc aac tct aat atg tct aat tgt tat cca agg tat cca cta gca     96
   Arg Ile Asn Ser Asn Met Ser Asn Cys Tyr Pro Arg Tyr Pro Leu Ala
               20                  25                  30
   aaa gat cca caa atg act atg cga aac acg aac tat aaa gaa tgg cta    144
   Lys Asp Pro Gln Met Thr Met Arg Asn Thr Asn Tyr Lys Glu Trp Leu
           35                  40                  45
   aat atg tgt gat tca aat aca caa ttt att ggt gat ata agc acg tat    192
   Asn Met Cys Asp Ser Asn Thr Gln Phe Ile Gly Asp Ile Ser Thr Tyr
       50                  55                  60
   tct agc cct gaa gct gct tta agt gta cga gat gct gtt tta acg ggt    240
   Ser Ser Pro Glu Ala Ala Leu Ser Val Arg Asp Ala Val Leu Thr Gly
   65                  70                  75                  80
   att aac agt gta ggg act ata ctt tcg aat tta ggg gtc cct ttg gca    288
   Ile Asn Ser Val Gly Thr Ile Leu Ser Asn Leu Gly Val Pro Leu Ala
                   85                  90                  95
   agt caa tca ttt gga ata att agt agg cta ata ggt att tta tgg gca    336
   Ser Gln Ser Phe Gly Ile Ile Ser Arg Leu Ile Gly Ile Leu Trp Ala
               100                 105                 110
   ggg cct gat cca ttt gaa gca ctt atg gtt ctt gtt gaa gag ctt att    384
   Gly Pro Asp Pro Phe Glu Ala Leu Met Val Leu Val Glu Glu Leu Ile
           115                 120                 125
   aag aaa agt ata gat cag cgt gta aga gaa aat gct ctt aga gag cta    432
   Lys Lys Ser Ile Asp Gln Arg Val Arg Glu Asn Ala Leu Arg Glu Leu
       130                 135                 140
   gaa ggt tta cag gga att atg aga cta tat caa act aga ctg caa gca    480
   Glu Gly Leu Gln Gly Ile Met Arg Leu Tyr Gln Thr Arg Leu Gln Ala
   145                 150                 155                 160
   tgg cta gtt aac aag aat gat gac aat cgg agg gca cta gta acg cag    528
   Trp Leu Val Asn Lys Asn Asp Asp Asn Arg Arg Ala Leu Val Thr Gln
                   165                 170                 175
   tat gca att gtt gat aac ttt ttc gaa aag aat atg cca aaa ttc aag    576
   Tyr Ala Ile Val Asp Asn Phe Phe Glu Lys Asn Met Pro Lys Phe Lys
               180                 185                 190
   gaa aga aac ttt gaa att tta ttg tta cca gta tat gca caa gcc gcg    624
   Glu Arg Asn Phe Glu Ile Leu Leu Leu Pro Val Tyr Ala Gln Ala Ala
           195                 200                 205
   aat ttg cat tta att tta tta aga gat gct gat tat ttt gga gca cag    672
   Asn Leu His Leu Ile Leu Leu Arg Asp Ala Asp Tyr Phe Gly Ala Gln
       210                 215                 220
   tgg caa tta ggt gat gat gaa att cgt gat aat tat atc aga cta caa    720
   Trp Gln Leu Gly Asp Asp Glu Ile Arg Asp Asn Tyr Ile Arg Leu Gln
   225                 230                 235                 240
   gga ctg att aga gaa tat aaa gat cat tgt ata aca ttc tat aac cag    768
   Gly Leu Ile Arg Glu Tyr Lys Asp His Cys Ile Thr Phe Tyr Asn Gln
   245 250 255
   ggt tta aat caa ttt aat cgc tca aat gct caa gat tgg gtg agc ttt 816
   Gly Leu Asn Gln Phe Asn Arg Ser Asn Ala Gln Asp Trp Val Ser Phe
   260 265 270
   aat agg ttt cgt aca gat atg aca tta aca gta tta gat ctc gca ata 864
   Asn Arg Phe Arg Thr Asp Met Thr Leu Thr Val Leu Asp Leu Ala Ile
   275 280 285
   tta ttt cca aac tat gat cca cgt agg tat cca tta gca gta aaa acg 912
   Leu Phe Pro Asn Tyr Asp Pro Arg Arg Tyr Pro Leu Ala Val Lys Thr
   290 295 300
   gaa ttg act agg gaa gtt tat aca gat cca gta ggg ttt act ggg gta 960
   Glu Leu Thr Arg Glu Val Tyr Thr Asp Pro Val Gly Phe Thr Gly Val
   305 310 315 320
   tta gaa agt gga ggt agg act tac cct tgg tat aat cct aat aat aca 1008
   Leu Glu Ser Gly Gly Arg Thr Tyr Pro Trp Tyr Asn Pro Asn Asn Thr
   325 330 335
   acc ttt act gct atg gaa aat aac gca aga cga cgt cct tct tat acc 1056
   Thr Phe Thr Ala Met Glu Asn Asn Ala Arg Arg Arg Pro Ser Tyr Thr
   340 345 350
   act tgg ctt aat cgt att ttt gta tat aca agg act cta ggt aat atg 1104
   Thr Trp Leu Asn Arg Ile Phe Val Tyr Thr Arg Thr Leu Gly Asn Met
   355 360 365
   tct gat gtg aga aat att tgg gga ggg cat aca tta gtt gaa aat gga 1152
   Ser Asp Val Arg Asn Ile Trp Gly Gly His Thr Leu Val Glu Asn Gly
   370 375 380
   aat gat ggt tct gaa ata acc cat aac ttt ggt aaa act gat tct att 1200
   Asn Asp Gly Ser Glu Ile Thr His Asn Phe Gly Lys Thr Asp Ser Ile
   385 390 395 400
   act cct att caa tat ttt aat ttc gcg aac ctt tct gtt ttc agt att 1248
   Thr Pro Ile Gln Tyr Phe Asn Phe Ala Asn Leu Ser Val Phe Ser Ile
   405 410 415
   gag tca ctt gct cgt ata tat tta gga gga aca gag gct aat aat tat 1296
   Glu Ser Leu Ala Arg Ile Tyr Leu Gly Gly Thr Glu Ala Asn Asn Tyr
   420 425 430
   att act agt cag tat gga gtc tcg aga gtt att ttt aat aca tca aat 1344
   Ile Thr Ser Gln Tyr Gly Val Ser Arg Val Ile Phe Asn Thr Ser Asn
   435 440 445
   ata aat aat gta cct gga tct tta aga tac gaa gtg cct gct aat ctt 1392
   Ile Asn Asn Val Pro Gly Ser Leu Arg Tyr Glu Val Pro Ala Asn Leu
   450 455 460
   cca tcc caa act ata tta tca gaa tta cca gga aag gat aag cca aga 1440
   Pro Ser Gln Thr Ile Leu Ser Glu Leu Pro Gly Lys Asp Lys Pro Arg
   465 470 475 480
   cca aac gca gga gat ttc agc cat aga tta tct tat ata tca aat ttt 1488
   Pro Asn Ala Gly Asp Phe Ser His Arg Leu Ser Tyr Ile Ser Asn Phe
   485 490 495
   gat gca cgg cga agt agt tca ggc ggt att gtt agt ctt tta acg ttt 1536
   Asp Ala Arg Arg Ser Ser Ser Gly Gly Ile Val Ser Leu Leu Thr Phe
   500 505 510
   ggt tgg gca cat acc agt atg gat cgt aat aat cgt ctt gaa cca gat 1584
   Gly Trp Ala His Thr Ser Met Asp Arg Asn Asn Arg Leu Glu Pro Asp
           515                 520                 525
   aaa att act caa ata gat gca gtt aaa ggt tgg ggg ggg aat atc ggg    1632
   Lys Ile Thr Gln Ile Asp Ala Val Lys Gly Trp Gly Gly Asn Ile Gly
       530                 535                 540
   ttt gtc atc cca gga cct act ggg ggg aat ttg gta aaa gtc agt gat    1680
   Phe Val Ile Pro Gly Pro Thr Gly Gly Asn Leu Val Lys Val Ser Asp
   545                 550                 555                 560
   agt tgg cat tca ctt aaa gtt caa gca cca caa aga caa aca agt tat    1728
   Ser Trp His Ser Leu Lys Val Gln Ala Pro Gln Arg Gln Thr Ser Tyr
                   565                 570                 575
   cgt att cgt ttg cgt tat gct tgt tta gtt acc cat ggg gat gct att    1776
   Arg Ile Arg Leu Arg Tyr Ala Cys Leu Val Thr His Gly Asp Ala Ile
               580                 585                 590
   ttt gta gaa cac agc ggc agt agt cat ata gtt tca ttt ttt gat tgc    1824
   Phe Val Glu His Ser Gly Ser Ser His Ile Val Ser Phe Phe Asp Cys
           595                 600                 605
   tca aat tca tca ggt cgt cca tca aac act ctt cta gag agt gat ttt    1872
   Ser Asn Ser Ser Gly Arg Pro Ser Asn Thr Leu Leu Glu Ser Asp Phe
       610                 615                 620
   cgc tat att gat gtt cca ggt att ttt aca cca tca ata aat ccc tta    1920
   Arg Tyr Ile Asp Val Pro Gly Ile Phe Thr Pro Ser Ile Asn Pro Leu
   625                 630                 635                 640
   ata aga tat aga aca caa agc ttt ggt acc cac gcg ata gac aaa ttt    1968
   Ile Arg Tyr Arg Thr Gln Ser Phe Gly Thr His Ala Ile Asp Lys Phe
                   645                 650                 655
   gaa ttt att cca ctt aac act ttt ccg aat caa tca tta gaa aaa aga    2016
   Glu Phe Ile Pro Leu Asn Thr Phe Pro Asn Gln Ser Leu Glu Lys Arg
               660                 665                 670
   gaa cag gaa gta aat gat cta ttt atc aat taa                        2049
   Glu Gln Glu Val Asn Asp Leu Phe Ile Asn  *
           675                 680
<210> 20
   <211> 682
   <212> PRT
   <213> Bacillus thuringiensis
<400> 20
   Met Asn Ser Tyr Lys Asn Lys Asn Glu Tyr Glu Met Leu Asp Ala Leu
    1              5                   10                  15
   Arg Ile Asn Ser Asn Met Ser Asn Cys Tyr Pro Arg Tyr Pro Leu Ala
               20                  25                  30
   Lys Asp Pro Gln Met Thr Met Arg Asn Thr Asn Tyr Lys Glu Trp Leu
           35                  40                  45
   Asn Met Cys Asp Ser Asn Thr Gln Phe Ile Gly Asp Ile Ser Thr Tyr
       50                  55                  60
   Ser Ser Pro Glu Ala Ala Leu Ser Val Arg Asp Ala Val Leu Thr Gly
   65                  70                  75                  80
   Ile Asn Ser Val Gly Thr Ile Leu Ser Asn Leu Gly Val Pro Leu Ala
                   85                  90                  95
   Ser Gln Ser Phe Gly Ile Ile Ser Arg Leu Ile Gly Ile Leu Trp Ala
               100                 105                 110
   Gly Pro Asp Pro Phe Glu Ala Leu Met Val Leu Val Glu Glu Leu Ile
           115                 120                 125
   Lys Lys Ser Ile Asp Gln Arg Val Arg Glu Asn Ala Leu Arg Glu Leu
       130                 135                 140
   Glu Gly Leu Gln Gly Ile Met Arg Leu Tyr Gln Thr Arg Leu Gln Ala
   145                 150                 155                 160
   Trp Leu Val Asn Lys Asn Asp Asp Asn Arg Arg Ala Leu Val Thr Gln
                   165                 170                 175
   Tyr Ala Ile Val Asp Asn Phe Phe Glu Lys Asn Met Pro Lys Phe Lys
               180                 185                 190
   Glu Arg Asn Phe Glu Ile Leu Leu Leu Pro Val Tyr Ala Gln Ala Ala
           195                 200                 205
   Asn Leu His Leu Ile Leu Leu Arg Asp Ala Asp Tyr Phe Gly Ala Gln
       210                 215                 220
   Trp Gln Leu Gly Asp Asp Glu Ile Arg Asp Asn Tyr Ile Arg Leu Gln
   225                 230                 235                 240
   Gly Leu Ile Arg Glu Tyr Lys Asp His Cys Ile Thr Phe Tyr Asn Gln
                   245                 250                 255
   Gly Leu Asn Gln Phe Asn Arg Ser Asn Ala Gln Asp Trp Val Ser Phe
               260                 265                 270
   Asn Arg Phe Arg Thr Asp Met Thr Leu Thr Val Leu Asp Leu Ala Ile
           275                 280                 285
   Leu Phe Pro Asn Tyr Asp Pro Arg Arg Tyr Pro Leu Ala Val Lys Thr
       290                 295                 300
   Glu Leu Thr Arg Glu Val Tyr Thr Asp Pro Val Gly Phe Thr Gly Val
   305                 310                 315                 320
   Leu Glu Ser Gly Gly Arg Thr Tyr Pro Trp Tyr Asn Pro Asn Asn Thr
                   325                 330                 335
   Thr Phe Thr Ala Met Glu Asn Asn Ala Arg Arg Arg Pro Ser Tyr Thr
               340                 345                 350
   Thr Trp Leu Asn Arg Ile Phe Val Tyr Thr Arg Thr Leu Gly Asn Met
           355                 360                 365
   Ser Asp Val Arg Asn Ile Trp Gly Gly His Thr Leu Val Glu Asn Gly
       370                 375                 380
   Asn Asp Gly Ser Glu Ile Thr His Asn Phe Gly Lys Thr Asp Ser Ile
   385                 390                 395                 400
   Thr Pro Ile Gln Tyr Phe Asn Phe Ala Asn Leu Ser Val Phe Ser Ile
                   405                 410                 415
   Glu Ser Leu Ala Arg Ile Tyr Leu Gly Gly Thr Glu Ala Asn Asn Tyr
               420                 425                 430
   Ile Thr Ser Gln Tyr Gly Val Ser Arg Val Ile Phe Asn Thr Ser Asn
           435                 440                 445
   Ile Asn Asn Val Pro Gly Ser Leu Arg Tyr Glu Val Pro Ala Asn Leu
       450                 455                 460
   Pro Ser Gln Thr Ile Leu Ser Glu Leu Pro Gly Lys Asp Lys Pro Arg
   465                 470                 475                 480
   Pro Asn Ala Gly Asp Phe Ser His Arg Leu Ser Tyr Ile Ser Asn Phe
                   485                 490                 495
   Asp Ala Arg Arg Ser Ser Ser Gly Gly Ile Val Ser Leu Leu Thr Phe
               500                 505                 510
   Gly Trp Ala His Thr Ser Met Asp Arg Asn Asn Arg Leu Glu Pro Asp
           515                 520                 525
   Lys Ile Thr Gln Ile Asp Ala Val Lys Gly Trp Gly Gly Asn Ile Gly
       530                 535                 540
   Phe Val Ile Pro Gly Pro Thr Gly Gly Asn Leu Val Lys Val Ser Asp
   545                 550                 555                 560
   Ser Trp His Ser Leu Lys Val Gln Ala Pro Gln Arg Gln Thr Ser Tyr
                   565                 570                 575
   Arg Ile Arg Leu Arg Tyr Ala Cys Leu Val Thr His Gly Asp Ala Ile
               580                 585                 590
   Phe Val Glu His Ser Gly Ser Ser His Ile Val Ser Phe Phe Asp Cys
           595                 600                 605
   Ser Asn Ser Ser Gly Arg Pro Ser Asn Thr Leu Leu Glu Ser Asp Phe
       610                 615                 620
   Arg Tyr Ile Asp Val Pro Gly Ile Phe Thr Pro Ser Ile Asn Pro Leu
   625                 630                 635                 640
   Ile Arg Tyr Arg Thr Gln Ser Phe Gly Thr His Ala Ile Asp Lys Phe
                   645                 650                 655
   Glu Phe Ile Pro Leu Asn Thr Phe Pro Asn Gln Ser Leu Glu Lys Arg
               660                 665                 670
   Glu Gln Glu Val Asn Asp Leu Phe Ile Asn
           675                 680
<210> 21
   <211> 2016
   <212> DNA
   <213> Bacillus thuringiensis
<220>
   <221> CDS
   <222> (1)...(2016)
<400> 21
   atg ttg gat gct tta cga atc aac tct aat atg tct aat tgt tat cca    48
   Met Leu Asp Ala Leu Arg Ile Asn Ser Asn Met Ser Asn Cys Tyr Pro
    1               5                  10                  15
   agg tat cca cta gca aaa gat cca caa atg act atg cga aac acg aac    96
   Arg Tyr Pro Leu Ala Lys Asp Pro Gln Met Thr Met Arg Asn Thr Asn
               20                  25                  30
   tat aaa gaa tgg cta aat atg tgt gat tca aat aca caa ttt att ggt    144
   Tyr Lys Glu Trp Leu Asn Met Cys Asp Ser Asn Thr Gln Phe Ile Gly
           35                  40                  45
   gat ata agc acg tat tct agc cct gaa gct gct tta agt gta cga gat    192
   Asp Ile Ser Thr Tyr Ser Ser Pro Glu Ala Ala Leu Ser Val Arg Asp
       50                  55                  60
   gct gtt tta acg ggt att aac agt gta ggg act ata ctt tcg aat tta    240
   Ala Val Leu Thr Gly Ile Asn Ser Val Gly Thr Ile Leu Ser Asn Leu
    65                  70                  75                  80
   ggg gtc cct ttg gca agt caa tca ttt gga ata att agt agg cta ata    288
   Gly Val Pro Leu Ala Ser Gln Ser Phe Gly Ile Ile Ser Arg Leu Ile
                   85                  90                  95
   ggt att tta tgg gca ggg cct gat cca ttt gaa gca ctt atg gtt ctt    336
   Gly Ile Leu Trp Ala Gly Pro Asp Pro Phe Glu Ala Leu Met Val Leu
               100                 105                 110
   gtt gaa gag ctt att aag aaa agt ata gat cag cgt gta aga gaa aat    384
   Val Glu Glu Leu Ile Lys Lys Ser Ile Asp Gln Arg Val Arg Glu Asn
           115                 120                 125
   gct ctt aga gag cta gaa ggt tta cag gga att atg aga cta tat caa    432
   Ala Leu Arg Glu Leu Glu Gly Leu Gln Gly Ile Met Arg Leu Tyr Gln
       130                 135                 140
   act aga ctg caa gca tgg cta gtt aac aag aat gat gac aat cgg agg    480
   Thr Arg Leu Gln Ala Trp Leu Val Asn Lys Asn Asp Asp Asn Arg Arg
   145                 150                 155                 160
   gca cta gta acg cag tat gca att gtt gat aac ttt ttc gaa aag aat    528
   Ala Leu Val Thr Gln Tyr Ala Ile Val Asp Asn Phe Phe Glu Lys Asn
                   165                 170                 175
   atg cca aaa ttc aag gaa aga aac ttt gaa att tta ttg tta cca gta    576
   Met Pro Lys Phe Lys Glu Arg Asn Phe Glu Ile Leu Leu Leu Pro Val
               180                 185                 190
   tat gca caa gcc gcg aat ttg cat tta att tta tta aga gat gct gat    624
   Tyr Ala Gln Ala Ala Asn Leu His Leu Ile Leu Leu Arg Asp Ala Asp
           195                 200                 205
   tat ttt gga gca cag tgg caa tta ggt gat gat gaa att cgt gat aat    672
   Tyr Phe Gly Ala Gln Trp Gln Leu Gly Asp Asp Glu Ile Arg Asp Asn
       210                 215                 220
   tat atc aga cta caa gga ctg att aga gaa tat aaa gat cat tgt ata      720
   Tyr Ile Arg Leu Gln Gly Leu Ile Arg Glu Tyr Lys Asp His Cys Ile
   225                 230                 235                 240
   aca ttc tat aac cag ggt tta aat caa ttt aat cgc tca aat gct caa      768
   Thr Phe Tyr Asn Gln Gly Leu Asn Gln Phe Asn Arg Ser Asn Ala Gln
                   245                 250                 255
   gat tgg gtg agc ttt aat agg ttt cgt aca gat atg aca tta aca gta      816
   Asp Trp Val Ser Phe Asn Arg Phe Arg Thr Asp Met Thr Leu Thr Val
               260                 265                 270
   tta gat ctc gca ata tta ttt cca aac tat gat cca cgt agg tat cca      864
   Leu Asp Leu Ala Ile Leu Phe Pro Asn Tyr Asp Pro Arg Arg Tyr Pro
           275                 280                 285
   tta gca gta aaa acg gaa ttg act agg gaa gtt tat aca gat cca gta      912
   Leu Ala Val Lys Thr Glu Leu Thr Arg Glu Val Tyr Thr Asp Pro Val
       290                 295                 300
   ggg ttt act ggg gta tta gaa agt gga ggt agg act tac cct tgg tat      960
   Gly Phe Thr Gly Val Leu Glu Ser Gly Gly Arg Thr Tyr Pro Trp Tyr
   305                 310                 315                 320
   aat cct aat aat aca acc ttt act gct atg gaa aat aac gca aga cga      1008
   Asn Pro Asn Asn Thr Thr Phe Thr Ala Met Glu Asn Asn Ala Arg Arg
                   325                 330                 335
   cgt cct tct tat acc act tgg ctt aat cgt att ttt gta tat aca agg      1056
   Arg Pro Ser Tyr Thr Thr Trp Leu Asn Arg Ile Phe Val Tyr Thr Arg
               340                 345                 350
   act cta ggt aat atg tct gat gtg aga aat att tgg gga ggg cat aca      1104
   Thr Leu Gly Asn Met Ser Asp Val Arg Asn Ile Trp Gly Gly His Thr
           355                 360                 365
   tta gtt gaa aat gga aat gat ggt tct gaa ata acc cat aac ttt ggt      1152
   Leu Val Glu Asn Gly Asn Asp Gly Ser Glu Ile Thr His Asn Phe Gly
       370                 375                 380
   aaa act gat tct att act cct att caa tat ttt aat ttc gcg aac ctt      1200
   Lys Thr Asp Ser Ile Thr Pro Ile Gln Tyr Phe Asn Phe Ala Asn Leu
   385                 390                 395                 400
   tct gtt ttc agt att gag tca ctt gct cgt ata tat tta gga gga aca      1248
   Ser Val Phe Ser Ile Glu Ser Leu Ala Arg Ile Tyr Leu Gly Gly Thr
                   405                 410                 415
   gag gct aat aat tat att act agt cag tat gga gtc tcg aga gtt att      1296
   Glu Ala Asn Asn Tyr Ile Thr Ser Gln Tyr Gly Val Ser Arg Val Ile
               420                 425                 430
   ttt aat aca tca aat ata aat aat gta cct gga tct tta aga tac gaa      1344
   Phe Asn Thr Ser Asn Ile Asn Asn Val Pro Gly Ser Leu Arg Tyr Glu
           435                 440                 445
   gtg cct gct aat ctt cca tcc caa act ata tta tca gaa tta cca gga      1392
   Val Pro Ala Asn Leu Pro Ser Gln Thr Ile Leu Ser Glu Leu Pro Gly
       450                 455                 460
   aag gat aag cca aga cca aac gca gga gat ttc agc cat aga tta tct      1440
   Lys Asp Lys Pro Arg Pro Asn Ala Gly Asp Phe Ser His Arg Leu Ser
   465                 470                 475                 480
   tat ata tca aat ttt gat gca cgg cga agt agt tca ggc ggt att gtt      1488
   Tyr Ile Ser Asn Phe Asp Ala Arg Arg Ser Ser Ser Gly Gly Ile Val
                   485                 490                 495
   agt ctt tta acg ttt ggt tgg gca cat acc agt atg gat cgt aat aat      1536
   Ser Leu Leu Thr Phe Gly Trp Ala His Thr Ser Met Asp Arg Asn Asn
               500                 505                 510
   cgt ctt gaa cca gat aaa att act caa ata gat gca gtt aaa ggt tgg      1584
   Arg Leu Glu Pro Asp Lys Ile Thr Gln Ile Asp Ala Val Lys Gly Trp
           515                 520                 525
   ggg ggg aat atc ggg ttt gtc atc cca gga cct act ggg ggg aat ttg      1632
   Gly Gly Asn Ile Gly Phe Val Ile Pro Gly Pro Thr Gly Gly Asn Leu
       530                 535                 540
   gta aaa gtc agt gat agt tgg cat tca ctt aaa gtt caa gca cca caa      1680
   Val Lys Val Ser Asp Ser Trp His Ser Leu Lys Val Gln Ala Pro Gln
   545                 550                 555                 560
   aga caa aca agt tat cgt att cgt ttg cgt tat gct tgt tta gtt acc      1728
   Arg Gln Thr Ser Tyr Arg Ile Arg Leu Arg Tyr Ala Cys Leu Val Thr
                   565                 570                 575
   cat ggg gat gct att ttt gta gaa cac agc ggc agt agt cat ata gtt      1776
   His Gly Asp Ala Ile Phe Val Glu His Ser Gly Ser Ser His Ile Val
               580                 585                 590
   tca ttt ttt gat tgc tca aat tca tca ggt cgt cca tca aac act ctt      1824
   Ser Phe Phe Asp Cys Ser Asn Ser Ser Gly Arg Pro Ser Asn Thr Leu
           595                 600                 605
   cta gag agt gat ttt cgc tat att gat gtt cca ggt att ttt aca cca      1872
   Leu Glu Ser Asp Phe Arg Tyr Ile Asp Val Pro Gly Ile Phe Thr Pro
       610                 615                 620
   tca ata aat ccc tta ata aga tat aga aca caa agc ttt ggt acc cac      1920
   Ser Ile Asn Pro Leu Ile Arg Tyr Arg Thr Gln Ser Phe Gly Thr His
   625                 630                 635                 640
   gcg ata gac aaa ttt gaa ttt att cca ctt aac act ttt ccg aat caa      1968
   Ala Ile Asp Lys Phe Glu Phe Ile Pro Leu Asn Thr Phe Pro Asn Gln
                   645                 650                 655
   tca tta gaa aaa aga gaa cag gaa gta aat gat cta ttt atc aat taa      2016
   Ser Leu Glu Lys Arg Glu Gln Glu Val Asn Asp Leu Phe Ile Asn  *
               660                 665                 670
<210> 22
   <211> 671
   <212> PRT
   <213> Bacillus thuringiensis
<400> 22
   Met Leu Asp Ala Leu Arg Ile Asn Ser Asn Met Ser Asn Cys Tyr Pro
   1               5                   10                  15
   Arg Tyr Pro Leu Ala Lys Asp Pro Gln Met Thr Met Arg Asn Thr Asn
               20                  25                  30
   Tyr Lys Glu Trp Leu Asn Met Cys Asp Ser Asn Thr Gln Phe Ile Gly
           35                  40                  45
   Asp Ile Ser Thr Tyr Ser Ser Pro Glu Ala Ala Leu Ser Val Arg Asp
       50                  55                  60
   Ala Val Leu Thr Gly Ile Asn Ser Val Gly Thr Ile Leu Ser Asn Leu
   65                  70                  75                  80
   Gly Val Pro Leu Ala Ser Gln Ser Phe Gly Ile Ile Ser Arg Leu Ile
                   85                  90                  95
   Gly Ile Leu Trp Ala Gly Pro Asp Pro Phe Glu Ala Leu Met Val Leu
               100                 105                 110
   Val Glu Glu Leu Ile Lys Lys Ser Ile Asp Gln Arg Val Arg Glu Asn
           115                 120                 125
   Ala Leu Arg Glu Leu Glu Gly Leu Gln Gly Ile Met Arg Leu Tyr Gln
       130                 135                 140
   Thr Arg Leu Gln Ala Trp Leu Val Asn Lys Asn Asp Asp Asn Arg Arg
   145                 150                 155                 160
   Ala Leu Val Thr Gln Tyr Ala Ile Val Asp Asn Phe Phe Glu Lys Asn
                   165                 170                 175
   Met Pro Lys Phe Lys Glu Arg Asn Phe Glu Ile Leu Leu Leu Pro Val
               180                 185                 190
   Tyr Ala Gln Ala Ala Asn Leu His Leu Ile Leu Leu Arg Asp Ala Asp
           195                 200                 205
   Tyr Phe Gly Ala Gln Trp Gln Leu Gly Asp Asp Glu Ile Arg Asp Asn
       210                 215                 220
   Tyr Ile Arg Leu Gln Gly Leu Ile Arg Glu Tyr Lys Asp His Cys Ile
   225                 230                 235                 240
   Thr Phe Tyr Asn Gln Gly Leu Asn Gln Phe Asn Arg Ser Asn Ala Gln
                   245                 250                 255
   Asp Trp Val Ser Phe Asn Arg Phe Arg Thr Asp Met Thr Leu Thr Val
               260                 265                 270
   Leu Asp Leu Ala Ile Leu Phe Pro Asn Tyr Asp Pro Arg Arg Tyr Pro
           275                 280                 285
   Leu Ala Val Lys Thr Glu Leu Thr Arg Glu Val Tyr Thr Asp Pro Val
       290                 295                 300
   Gly Phe Thr Gly Val Leu Glu Ser Gly Gly Arg Thr Tyr Pro Trp Tyr
   305                 310                 315                 320
   Asn Pro Asn Asn Thr Thr Phe Thr Ala Met Glu Asn Asn Ala Arg Arg
                   325                 330                 335
   Arg Pro Ser Tyr Thr Thr Trp Leu Asn Arg Ile Phe Val Tyr Thr Arg
               340                 345                 350
   Thr Leu Gly Asn Met Ser Asp Val Arg Asn Ile Trp Gly Gly His Thr
           355                 360                 365
   Leu Val Glu Asn Gly Asn Asp Gly Ser Glu Ile Thr His Asn Phe Gly
       370                 375                 380
   Lys Thr Asp Ser Ile Thr Pro Ile Gln Tyr Phe Asn Phe Ala Asn Leu
   385                 390                 395                 400
   Ser Val Phe Ser Ile Glu Ser Leu Ala Arg Ile Tyr Leu Gly Gly Thr
                   405                 410                 415
   Glu Ala Asn Asn Tyr Ile Thr Ser Gln Tyr Gly Val Ser Arg Val Ile
               420                 425                 430
   Phe Asn Thr Ser Asn Ile Asn Asn Val Pro Gly Ser Leu Arg Tyr Glu
           435                 440                 445
   Val Pro Ala Asn Leu Pro Ser Gln Thr Ile Leu Ser Glu Leu Pro Gly
       450                 455                 460
   Lys Asp Lys Pro Arg Pro Asn Ala Gly Asp Phe Ser His Arg Leu Ser
   465                 470                 475                 480
   Tyr Ile Ser Asn Phe Asp Ala Arg Arg Ser Ser Ser Gly Gly Ile Val
                   485                 490                 495
   Ser Leu Leu Thr Phe Gly Trp Ala His Thr Ser Met Asp Arg Asn Asn
               500                 505                 510
   Arg Leu Glu Pro Asp Lys Ile Thr Gln Ile Asp Ala Val Lys Gly Trp
           515                 520                 525
   Gly Gly Asn Ile Gly Phe Val Ile Pro Gly Pro Thr Gly Gly Asn Leu
       530                 535                 540
   Val Lys Val Ser Asp Ser Trp His Ser Leu Lys Val Gln Ala Pro Gln
   545                 550                 555                 560
   Arg Gln Thr Ser Tyr Arg Ile Arg Leu Arg Tyr Ala Cys Leu Val Thr
                   565                 570                 575
   His Gly Asp Ala Ile Phe Val Glu His Ser Gly Ser Ser His Ile Val
               580                 585                 590
   Ser Phe Phe Asp Cys Ser Asn Ser Ser Gly Arg Pro Ser Asn Thr Leu
           595                 600                 605
   Leu Glu Ser Asp Phe Arg Tyr Ile Asp Val Pro Gly Ile Phe Thr Pro
       610                 615                 620
   Ser Ile Asn Pro Leu Ile Arg Tyr Arg Thr Gln Ser Phe Gly Thr His
   625                 630                 635                 640
   Ala Ile Asp Lys Phe Glu Phe Ile Pro Leu Asn Thr Phe Pro Asn Gln
                   645                 650                 655
   Ser Leu Glu Lys Arg Glu Gln Glu Val Asn Asp Leu Phe Ile Asn
               660                 665                 670
<210> 23
   <211> 1986
   <212> DNA
   <213> Bacillus thuringiensis
<220>
   <221> CDS
   <222> (1)...(1986)
<400> 23
   atg tct aat tgt tat cca agg tat cca cta gca aaa gat cca caa atg      48
   Met Ser Asn Cys Tyr Pro Arg Tyr Pro Leu Ala Lys Asp Pro Gln Met
    1               5                  10                  15
   act atg cga aac acg aac tat aaa gaa tgg cta aat atg tgt gat tca      96
   Thr Met Arg Asn Thr Asn Tyr Lys Glu Trp Leu Asn Met Cys Asp Ser
               20                  25                  30
   aat aca caa ttt att ggt gat ata agc acg tat tct agc cct gaa gct     144
   Asn Thr Gln Phe Ile Gly Asp Ile Ser Thr Tyr Ser Ser Pro Glu Ala
           35                  40                  45
   gct tta agt gta cga gat gct gtt tta acg ggt att aac agt gta ggg     192
   Ala Leu Ser Val Arg Asp Ala Val Leu Thr Gly Ile Asn Ser Val Gly
       50                  55                  60
   act ata ctt tcg aat tta ggg gtc cct ttg gca agt caa tca ttt gga     240
   Thr Ile Leu Ser Asn Leu Gly Val Pro Leu Ala Ser Gln Ser Phe Gly
   65                  70                  75                  80
   ata att agt agg cta ata ggt att tta tgg gca ggg cct gat cca ttt     288
   Ile Ile Ser Arg Leu Ile Gly Ile Leu Trp Ala Gly Pro Asp Pro Phe
                   85                  90                  95
   gaa gca ctt atg gtt ctt gtt gaa gag ctt att aag aaa agt ata gat     336
   Glu Ala Leu Met Val Leu Val Glu Glu Leu Ile Lys Lys Ser Ile Asp
               100                 105                 110
   cag cgt gta aga gaa aat gct ctt aga gag cta gaa ggt tta cag gga     384
   Gln Arg Val Arg Glu Asn Ala Leu Arg Glu Leu Glu Gly Leu Gln Gly
           115                 120                 125
   att atg aga cta tat caa act aga ctg caa gca tgg cta gtt aac aag     432
   Ile Met Arg Leu Tyr Gln Thr Arg Leu Gln Ala Trp Leu Val Asn Lys
       130                 135                 140
   aat gat gac aat cgg agg gca cta gta acg cag tat gca att gtt gat     480
   Asn Asp Asp Asn Arg Arg Ala Leu Val Thr Gln Tyr Ala Ile Val Asp
   145                 150                 155                 160
   aac ttt ttc gaa aag aat atg cca aaa ttc aag gaa aga aac ttt gaa     528
   Asn Phe Phe Glu Lys Asn Met Pro Lys Phe Lys Glu Arg Asn Phe Glu
                   165                 170                 175
   att tta ttg tta cca gta tat gca caa gcc gcg aat ttg cat tta att     576
   Ile Leu Leu Leu Pro Val Tyr Ala Gln Ala Ala Asn Leu His Leu Ile
               180                 185                 190
   tta tta aga gat gct gat tat ttt gga gca cag tgg caa tta ggt gat     624
   Leu Leu Arg Asp Ala Asp Tyr Phe Gly Ala Gln Trp Gln Leu Gly Asp
           195                 200                 205
   gat gaa att cgt gat aat tat atc aga cta caa gga ctg att aga gaa     672
   Asp Glu Ile Arg Asp Asn Tyr Ile Arg Leu Gln Gly Leu Ile Arg Glu
       210                 215                 220
   tat aaa gat cat tgt ata aca ttc tat aac cag ggt tta aat caa ttt     720
   Tyr Lys Asp His Cys Ile Thr Phe Tyr Asn Gln Gly Leu Asn Gln Phe
   225                 230                 235                 240
   aat cgc tca aat gct caa gat tgg gtg agc ttt aat agg ttt cgt aca     768
   Asn Arg Ser Asn Ala Gln Asp Trp Val Ser Phe Asn Arg Phe Arg Thr
                   245                 250                 255
   gat atg aca tta aca gta tta gat ctc gca ata tta ttt cca aac tat     816
   Asp Met Thr Leu Thr Val Leu Asp Leu Ala Ile Leu Phe Pro Asn Tyr
               260                 265                 270
   gat cca cgt agg tat cca tta gca gta aaa acg gaa ttg act agg gaa     864
   Asp Pro Arg Arg Tyr Pro Leu Ala Val Lys Thr Glu Leu Thr Arg Glu
           275                 280                 285
   gtt tat aca gat cca gta ggg ttt act ggg gta tta gaa agt gga ggt     912
   Val Tyr Thr Asp Pro Val Gly Phe Thr Gly Val Leu Glu Ser Gly Gly
       290                 295                 300
   agg act tac cct tgg tat aat cct aat aat aca acc ttt act gct atg     960
   Arg Thr Tyr Pro Trp Tyr Asn Pro Asn Asn Thr Thr Phe Thr Ala Met
   305                 310                 315                 320
   gaa aat aac gca aga cga cgt cct tct tat acc act tgg ctt aat cgt     1008
   Glu Asn Asn Ala Arg Arg Arg Pro Ser Tyr Thr Thr Trp Leu Asn Arg
                   325                 330                 335
   att ttt gta tat aca agg act cta ggt aat atg tct gat gtg aga aat     1056
   Ile Phe Val Tyr Thr Arg Thr Leu Gly Asn Met Ser Asp Val Arg Asn
               340                 345                 350
   att tgg gga ggg cat aca tta gtt gaa aat gga aat gat ggt tct gaa     1104
   Ile Trp Gly Gly His Thr Leu Val Glu Asn Gly Asn Asp Gly Ser Glu
           355                 360                 365
   ata acc cat aac ttt ggt aaa act gat tct att act cct att caa tat     1152
   Ile Thr His Asn Phe Gly Lys Thr Asp Ser Ile Thr Pro Ile Gln Tyr
       370                 375                 380
   ttt aat ttc gcg aac ctt tct gtt ttc agt att gag tca ctt gct cgt     1200
   Phe Asn Phe Ala Asn Leu Ser Val Phe Ser Ile Glu Ser Leu Ala Arg
   385                 390                 395                 400
   ata tat tta gga gga aca gag gct aat aat tat att act agt cag tat     1248
   Ile Tyr Leu Gly Gly Thr Glu Ala Asn Asn Tyr Ile Thr Ser Gln Tyr
                   405                 410                 415
   gga gtc tcg aga gtt att ttt aat aca tca aat ata aat aat gta cct     1296
   Gly Val Ser Arg Val Ile Phe Asn Thr Ser Asn Ile Asn Asn Val Pro
               420                 425                 430
   gga tct tta aga tac gaa gtg cct gct aat ctt cca tcc caa act ata     1344
   Gly Ser Leu Arg Tyr Glu Val Pro Ala Asn Leu Pro Ser Gln Thr Ile
           435                 440                 445
   tta tca gaa tta cca gga aag gat aag cca aga cca aac gca gga gat     1392
   Leu Ser Glu Leu Pro Gly Lys Asp Lys Pro Arg Pro Asn Ala Gly Asp
       450                 455                 460
   ttc agc cat aga tta tct tat ata tca aat ttt gat gca cgg cga agt     1440
   Phe Ser His Arg Leu Ser Tyr Ile Ser Asn Phe Asp Ala Arg Arg Ser
   465                 470                 475                 480
   agt tca ggc ggt att gtt agt ctt tta acg ttt ggt tgg gca cat acc     1488
   Ser Ser Gly Gly Ile Val Ser Leu Leu Thr Phe Gly Trp Ala His Thr
                   485                 490                 495
   agt atg gat cgt aat aat cgt ctt gaa cca gat aaa att act caa ata     1536
   Ser Met Asp Arg Asn Asn Arg Leu Glu Pro Asp Lys Ile Thr Gln Ile
               500                 505                 510
   gat gca gtt aaa ggt tgg ggg ggg aat atc ggg ttt gtc atc cca gga     1584
   Asp Ala Val Lys Gly Trp Gly Gly Asn Ile Gly Phe Val Ile Pro Gly
           515                 520                 525
   cct act ggg ggg aat ttg gta aaa gtc agt gat agt tgg cat tca ctt     1632
   Pro Thr Gly Gly Asn Leu Val Lys Val Ser Asp Ser Trp His Ser Leu
       530                 535                 540
   aaa gtt caa gca cca caa aga caa aca agt tat cgt att cgt ttg cgt     1680
   Lys Val Gln Ala Pro Gln Arg Gln Thr Ser Tyr Arg Ile Arg Leu Arg
   545                 550                 555                 560
   tat gct tgt tta gtt acc cat ggg gat gct att ttt gta gaa cac agc     1728
   Tyr Ala Cys Leu Val Thr His Gly Asp Ala Ile Phe Val Glu His Ser
                   565                 570                 575
   ggc agt agt cat ata gtt tca ttt ttt gat tgc tca aat tca tca ggt     1776
   Gly Ser Ser His Ile Val Ser Phe Phe Asp Cys Ser Asn Ser Ser Gly
               580                 585                 590
   cgt cca tca aac act ctt cta gag agt gat ttt cgc tat att gat gtt     1824
   Arg Pro Ser Asn Thr Leu Leu Glu Ser Asp Phe Arg Tyr Ile Asp Val
           595                 600                 605
   cca ggt att ttt aca cca tca ata aat ccc tta ata aga tat aga aca     1872
   Pro Gly Ile Phe Thr Pro Ser Ile Asn Pro Leu Ile Arg Tyr Arg Thr
       610                 615                 620
   caa agc ttt ggt acc cac gcg ata gac aaa ttt gaa ttt att cca ctt     1920
   Gln Ser Phe Gly Thr His Ala Ile Asp Lys Phe Glu Phe Ile Pro Leu
   625                 630                 635                 640
   aac act ttt ccg aat caa tca tta gaa aaa aga gaa cag gaa gta aat     1968
   Asn Thr Phe Pro Asn Gln Ser Leu Glu Lys Arg Glu Gln Glu Val Asn
                   645                 650                 655
   gat cta ttt atc aat taa                                             1986
   Asp Leu Phe Ile Asn  *
               660
<210> 24
   <211> 661
   <212> PRT
   <213> Bacillus thuringiensis
<400> 24
   Met Ser Asn Cys Tyr Pro Arg Tyr Pro Leu Ala Lys Asp Pro Gln Met
    1               5                  10                  15
   Thr Met Arg Asn Thr Asn Tyr Lys Glu Trp Leu Asn Met Cys Asp Ser
               20                  25                  30
   Asn Thr Gln Phe Ile Gly Asp Ile Ser Thr Tyr Ser Ser Pro Glu Ala
           35                  40                  45
   Ala Leu Ser Val Arg Asp Ala Val Leu Thr Gly Ile Asn Ser Val Gly
       50                  55                  60
   Thr Ile Leu Ser Asn Leu Gly Val Pro Leu Ala Ser Gln Ser Phe Gly
   65                  70                  75                  80
   Ile Ile Ser Arg Leu Ile Gly Ile Leu Trp Ala Gly Pro Asp Pro Phe
                   85                  90                  95
   Glu Ala Leu Met Val Leu Val Glu Glu Leu Ile Lys Lys Ser Ile Asp
               100                 105                 110
   Gln Arg Val Arg Glu Asn Ala Leu Arg Glu Leu Glu Gly Leu Gln Gly
           115                 120                 125
   Ile Met Arg Leu Tyr Gln Thr Arg Leu Gln Ala Trp Leu Val Asn Lys
       130                 135                 140
   Asn Asp Asp Asn Arg Arg Ala Leu Val Thr Gln Tyr Ala Ile Val Asp
   145                 150                 155                 160
   Asn Phe Phe Glu Lys Asn Met Pro Lys Phe Lys Glu Arg Asn Phe Glu
                   165                 170                 175
   Ile Leu Leu Leu Pro Val Tyr Ala Gln Ala Ala Asn Leu His Leu Ile
               180                 185                 190
   Leu Leu Arg Asp Ala Asp Tyr Phe Gly Ala Gln Trp Gln Leu Gly Asp
           195                 200                 205
   Asp Glu Ile Arg Asp Asn Tyr Ile Arg Leu Gln Gly Leu Ile Arg Glu
       210                 215                 220
   Tyr Lys Asp His Cys Ile Thr Phe Tyr Asn Gln Gly Leu Asn Gln Phe
   225                 230                 235                 240
   Asn Arg Ser Asn Ala Gln Asp Trp Val Ser Phe Asn Arg Phe Arg Thr
                   245                 250                 255
   Asp Met Thr Leu Thr Val Leu Asp Leu Ala Ile Leu Phe Pro Asn Tyr
               260                 265                 270
   Asp Pro Arg Arg Tyr Pro Leu Ala Val Lys Thr Glu Leu Thr Arg Glu
           275                 280                 285
   Val Tyr Thr Asp Pro Val Gly Phe Thr Gly Val Leu Glu Ser Gly Gly
       290                 295                 300
   Arg Thr Tyr Pro Trp Tyr Asn Pro Asn Asn Thr Thr Phe Thr Ala Met
   305                 310                 315                 320
   Glu Asn Asn Ala Arg Arg Arg Pro Ser Tyr Thr Thr Trp Leu Asn Arg
                   325                 330                 335
   Ile Phe Val Tyr Thr Arg Thr Leu Gly Asn Met Ser Asp Val Arg Asn
               340                 345                 350
   Ile Trp Gly Gly His Thr Leu Val Glu Asn Gly Asn Asp Gly Ser Glu
           355                 360                 365
   Ile Thr His Asn Phe Gly Lys Thr Asp Ser Ile Thr Pro Ile Gln Tyr
       370                 375                 380
   Phe Asn Phe Ala Asn Leu Ser Val Phe Ser Ile Glu Ser Leu Ala Arg
   385                 390                 395                 400
   Ile Tyr Leu Gly Gly Thr Glu Ala Asn Asn Tyr Ile Thr Ser Gln Tyr
                   405                 410                 415
   Gly Val Ser Arg Val Ile Phe Asn Thr Ser Asn Ile Asn Asn Val Pro
               420                 425                 430
   Gly Ser Leu Arg Tyr Glu Val Pro Ala Asn Leu Pro Ser Gln Thr Ile
           435                 440                 445
   Leu Ser Glu Leu Pro Gly Lys Asp Lys Pro Arg Pro Asn Ala Gly Asp
       450                 455                 460
   Phe Ser His Arg Leu Ser Tyr Ile Ser Asn Phe Asp Ala Arg Arg Ser
   465                 470                 475                 480
   Ser Ser Gly Gly Ile Val Ser Leu Leu Thr Phe Gly Trp Ala His Thr
                   485                 490                 495
   Ser Met Asp Arg Asn Asn Arg Leu Glu Pro Asp Lys Ile Thr Gln Ile
               500                 505                 510
   Asp Ala Val Lys Gly Trp Gly Gly Asn Ile Gly Phe Val Ile Pro Gly
           515                 520                 525
   Pro Thr Gly Gly Asn Leu Val Lys Val Ser Asp Ser Trp His Ser Leu
       530                 535                 540
   Lys Val Gln Ala Pro Gln Arg Gln Thr Ser Tyr Arg Ile Arg Leu Arg
   545                 550                 555                 560
   Tyr Ala Cys Leu Val Thr His Gly Asp Ala Ile Phe Val Glu His Ser
                   565                 570                 575
   Gly Ser Ser His Ile Val Ser Phe Phe Asp Cys Ser Asn Ser Ser Gly
               580                 585                 590
   Arg Pro Ser Asn Thr Leu Leu Glu Ser Asp Phe Arg Tyr Ile Asp Val
           595                 600                 605
   Pro Gly Ile Phe Thr Pro Ser Ile Asn Pro Leu Ile Arg Tyr Arg Thr
       610                 615                 620
   Gln Ser Phe Gly Thr His Ala Ile Asp Lys Phe Glu Phe Ile Pro Leu
   625                 630                 635                 640
   Asn Thr Phe Pro Asn Gln Ser Leu Glu Lys Arg Glu Gln Glu Val Asn
                   645                 650                 655
   Asp Leu Phe Ile Asn
               660
<210> 25
   <211> 2145
   <212> DNA
   <213> Bacillus thuringiensis
<400> 25
   tttcaccaaa tataatgttt ttttgtagaa acattgttac aggaatacat tggggtacta 60
   cgaatatata gaaagacacc tggcatatat ttattaggtg tcttaaaaat aaggactata 120
   taaggagtga aaaatatgaa ttcttatcaa aatacaaatg aatatgaaat tctggatggt 180
   tccccgaata acacaaatat gtcaaacaga tatccttttg caaaggatcc aaatatattt 240
   cctattaacc tggacgcttg tcagggaagg ccatggcaag atacgtggga atcagtctcg 300
   gatatagtaa ctattgggac ataccttata caattcttgc tagaacccgg tataggtgga 360
   attcctgtaa tattttcaat aataaacaaa ctcattccgt cttctggtca atctgtggca 420
   gcactttcta tatgtgattt agtatctata attcgtaaag aggtagacga gagcgtgtta 480
   agtgacgggg ttgcagattt tgagggtgaa atgactgctt atcaagatta ttatcttcat 540
   tatcttgagg attggcttac agataaatca aatcctaaaa aacttgctga cgtagttaaa 600
   cagttccaag cacgggaaga agatttcact aaacttttag cagggtcatt atcaagacag 660
   aaagctgaaa tattattatt gcctacgtat gtgcaagctg caaatgtgca tttattacta 720
   ttaagggacg cagttaaata taaaaaagaa tggggactag tgtgtccacc gttgtatcca 780
   gggtcaggga gaactgattg taacgagcgg ttaaaagcga aaataaaaga gtatactaat 840
   tattgtgtag ggtggtataa caagggttta gatcagataa gacaggcggg tacaagtgct 900
   gaagtttggt cgaaatttaa taaatttcgt agagaaatga cgttggcggt attggatatt 960
   attgctatat ttccaactta tgattttgaa aaatatccat tagcaacaag tgtagagtta 1020
   actagggaaa tttatacaga tccagtggga tattcagggg gaaattatgg ttgggaacgg 1080
   ttttttagct ttaattcggt agaagcaaat ggaacacggg gacctggttt agttacttgg 1140
   cttcaagcta tagatatata tagtcattct attaatcttc agcttggtta tcttagtggc 1200
   tgggggggaa ctcgtcatta tgaagacttc acaaagggta acggtgcttt tcaacgtatg 1260
   tctggaacta cgagtaataa tccacgtaat attatttttg gcaataccga tatatttaaa 1320
   attatttcat tagctagata tgcaatgcaa ccgtttgttg ggtattcaat cccacggcat 1380
   cttgtttcac gtgcagaatt ttttccgaca acactaaata ctttcctgta tgaggtaaac 1440
   agttctgggt actcacagac aattgaatct gtgttaccag gtattaataa ggatctacca 1500
   cctagtcgta caaattactc tcatagatta tcaaatgcgg catgtgttca aaatgaaacc 1560
   tccagagtta acgtatttgg ttggacacat acaagtatga aaaaagataa tcgaatttat 1620
   ccagataaaa ttacgcaaat tcctgcagta aaagcttttg ccctaccagc aggtacagga 1680
   tatgcaggag gttacgtcac agctgggcct ggttatacag gaggagatgt agtaacgtta 1740
   ccttatcaag caagtttaaa aatacgttta acttctgcac ccacgaataa aaattaccgt 1800
   gttagacttc gctacgcgag tggaggacct ggtccgttcc gagtagaaag atggtcgcca 1860
   agttctgttt caaatgctaa tttttctcgt ccagctacag gtggctatag ttcatttgat 1920
   tatgtggaca ccttagttac tacatttaat caatcaggtg ttgaaataat tatacaaaat 1980
   ctatctggtt accaccttat tgttgacaaa gtcgaattta tcccaattga catccaaatt 2040
   gaaaaatgta cgaaatgtca attcgaagga gacatatgta gatgtgaagg agtacaatcc 2100
   ttggaaacaa aaaaagagat tgtaaatagt ttatttatca attaa                 2145
<210> 26
   <211> 2019
   <212> DNA
   <213> Bacillus thuringiensis
<220>
   <221> CDS
   <222> (1)...(2019)
<400> 26
   gtg aaa aat atg aat tct tat caa aat aca aat gaa tat gaa att ctg    48
   Met Lys Asn Met Asn Ser Tyr Gln Asn Thr Asn Glu Tyr Glu Ile Leu
    1               5                  10                  15
   gat ggt tcc ccg aat aac aca aat atg tca aac aga tat cct ttt gca    96
   Asp Gly Ser Pro Asn Asn Thr Asn Met Ser Asn Arg Tyr Pro Phe Ala
               20                  25                  30
   aag gat cca aat ata ttt cct att aac ctg gac gct tgt cag gga agg    144
   Lys Asp Pro Asn Ile Phe Pro Ile Asn Leu Asp Ala Cys Gln Gly Arg
           35                  40                  45
   cca tgg caa gat acg tgg gaa tca gtc tcg gat ata gta act att ggg    192
   Pro Trp Gln Asp Thr Trp Glu Ser Val Ser Asp Ile Val Thr Ile Gly
               50                  55                  60
   aca tac ctt ata caa ttc ttg cta gaa ccc ggt ata ggt gga att cct    240
   Thr Tyr Leu Ile Gln Phe Leu Leu Glu Pro Gly Ile Gly Gly Ile Pro
    65                  70                  75                  80
   gta ata ttt tca ata ata aac aaa ctc att ccg tct tct ggt caa tct    288
   Val Ile Phe Ser Ile Ile Asn Lys Leu Ile Pro Ser Ser Gly Gln Ser
                    85                  90                  95
   gtg gca gca ctt tct ata tgt gat tta gta tct ata att cgt aaa gag    336
   Val Ala Ala Leu Ser Ile Cys Asp Leu Val Ser Ile Ile Arg Lys Glu
               100                 105                 110
   gta gac gag agc gtg tta agt gac ggg gtt gca gat ttt gag ggt gaa    384
   Val Asp Glu Ser Val Leu Ser Asp Gly Val Ala Asp Phe Glu Gly Glu
           115                 120                 125
   atg act gct tat caa gat tat tat ctt cat tat ctt gag gat tgg ctt    432
   Met Thr Ala Tyr Gln Asp Tyr Tyr Leu His Tyr Leu Glu Asp Trp Leu
       130                 135                 140
   aca gat aaa tca aat cct aaa aaa ctt gct gac gta gtt aaa cag ttc    480
   Thr Asp Lys Ser Asn Pro Lys Lys Leu Ala Asp Val Val Lys Gln Phe
   145                 150                 155                 160
   caa gca cgg gaa gaa gat ttc act aaa ctt tta gca ggg tca tta tca    528
   Gln Ala Arg Glu Glu Asp Phe Thr Lys Leu Leu Ala Gly Ser Leu Ser
                   165                 170                 175
   aga cag aaa gct gaa ata tta tta ttg cct acg tat gtg caa gct gca    576
   Arg Gln Lys Ala Glu Ile Leu Leu Leu Pro Thr Tyr Val Gln Ala Ala
               180                 185                 190
   aat gtg cat tta tta cta tta agg gac gca gtt aaa tat aaa aaa gaa    624
   Asn Val His Leu Leu Leu Leu Arg Asp Ala Val Lys Tyr Lys Lys Glu
           195                 200                 205
   tgg gga cta gtg tgt cca ccg ttg tat cca ggg tca ggg aga act gat    672
   Trp Gly Leu Val Cys Pro Pro Leu Tyr Pro Gly Ser Gly Arg Thr Asp
       210                 215                 220
   tgt aac gag cgg tta aaa gcg aaa ata aaa gag tat act aat tat tgt    720
   Cys Asn Glu Arg Leu Lys Ala Lys Ile Lys Glu Tyr Thr Asn Tyr Cys
   225                 230                 235                 240
   gta ggg tgg tat aac aag ggt tta gat cag ata aga cag gcg ggt aca    768
   Val Gly Trp Tyr Asn Lys Gly Leu Asp Gln Ile Arg Gln Ala Gly Thr
                   245                 250                 255
   agt gct gaa gtt tgg tcg aaa ttt aat aaa ttt cgt aga gaa atg acg    816
   Ser Ala Glu Val Trp Ser Lys Phe Asn Lys Phe Arg Arg Glu Met Thr
               260                 265                 270
   ttg gcg gta ttg gat att att gct ata ttt cca act tat gat ttt gaa    864
   Leu Ala Val Leu Asp Ile Ile Ala Ile Phe Pro Thr Tyr Asp Phe Glu
           275                 280                 285
   aaa tat cca tta gca aca agt gta gag tta act agg gaa att tat aca    912
   Lys Tyr Pro Leu Ala Thr Ser Val Glu Leu Thr Arg Glu Ile Tyr Thr
       290                 295                 300
   gat cca gtg gga tat tca ggg gga aat tat ggt tgg gaa cgg ttt ttt    960
   Asp Pro Val Gly Tyr Ser Gly Gly Asn Tyr Gly Trp Glu Arg Phe Phe
   305                 310                 315                 320
   agc ttt aat tcg gta gaa gca aat gga aca cgg gga cct ggt tta gtt   1008
   Ser Phe Asn Ser Val Glu Ala Asn Gly Thr Arg Gly Pro Gly Leu Val
   325 330 335
   act tgg ctt caa gct ata gat ata tat agt cat tct att aat ctt cag 1056
   Thr Trp Leu Gln Ala Ile Asp Ile Tyr Ser His Ser Ile Asn Leu Gln
   340 345 350
   ctt ggt tat ctt agt ggc tgg ggg gga act cgt cat tat gaa gac ttc 1104
   Leu Gly Tyr Leu Ser Gly Trp Gly Gly Thr Arg His Tyr Glu Asp Phe
   355 360 365
   aca aag ggt aac ggt gct ttt caa cgt atg tct gga act acg agt aat 1152
   Thr Lys Gly Asn Gly Ala Phe Gln Arg Met Ser Gly Thr Thr Ser Asn
   370 375 380
   aat cca cgt aat att att ttt ggc aat acc gat ata ttt aaa att att 1200
   Asn Pro Arg Asn Ile Ile Phe Gly Asn Thr Asp Ile Phe Lys Ile Ile
   385 390 395 400
   tca tta gct aga tat gca atg caa ccg ttt gtt ggg tat tca atc cca 1248
   Ser Leu Ala Arg Tyr Ala Met Gln Pro Phe Val Gly Tyr Ser Ile Pro
   405 410 415
   cgg cat ctt gtt tca cgt gca gaa ttt ttt ccg aca aca cta aat act 1296
   Arg His Leu Val Ser Arg Ala Glu Phe Phe Pro Thr Thr Leu Asn Thr
   420 425 430
   ttc ctg tat gag gta aac agt tct ggg tac tca cag aca att gaa tct 1344
   Phe Leu Tyr Glu Val Asn Ser Ser Gly Tyr Ser Gln Thr Ile Glu Ser
   435 440 445
   gtg tta cca ggt att aat aag gat cta cca cct agt cgt aca aat tac 1392
   Val Leu Pro Gly Ile Asn Lys Asp Leu Pro Pro Ser Arg Thr Asn Tyr
   450 455 460
   tct cat aga tta tca aat gcg gca tgt gtt caa aat gaa acc tcc aga 1440
   Ser His Arg Leu Ser Asn Ala Ala Cys Val Gln Asn Glu Thr Ser Arg
   465 470 475 480
   gtt aac gta ttt ggt tgg aca cat aca agt atg aaa aaa gat aat cga 1488
   Val Asn Val Phe Gly Trp Thr His Thr Ser Met Lys Lys Asp Asn Arg
   485 490 495
   att tat cca gat aaa att acg caa att cct gca gta aaa gct ttt gcc 1536
   Ile Tyr Pro Asp Lys Ile Thr Gln Ile Pro Ala Val Lys Ala Phe Ala
   500 505 510
   cta cca gca ggt aca gga tat gca gga ggt tac gtc aca gct ggg cct 1584
   Leu Pro Ala Gly Thr Gly Tyr Ala Gly Gly Tyr Val Thr Ala Gly Pro
   515 520 525
   ggt tat aca gga gga gat gta gta acg tta cct tat caa gca agt tta 1632
   Gly Tyr Thr Gly Gly Asp Val Val Thr Leu Pro Tyr Gln Ala Ser Leu
   530 535 540
   aaa ata cgt tta act tct gca ccc acg aat aaa aat tac cgt gtt aga 1680
   Lys Ile Arg Leu Thr Ser Ala Pro Thr Asn Lys Asn Tyr Arg Val Arg
   545 550 555 560
   ctt cgc tac gcg agt gga gga cct ggt ccg ttc cga gta gaa aga tgg 1728
   Leu Arg Tyr Ala Ser Gly Gly Pro Gly Pro Phe Arg Val Glu Arg Trp
   565 570 575
   tcg cca agt tct gtt tca aat gct aat ttt tct cgt cca gct aca ggt 1776
   Ser Pro Ser Ser Val Ser Asn Ala Asn Phe Ser Arg Pro Ala Thr Gly
   580 585 590
   ggc tat agt tca ttt gat tat gtg gac acc tta gtt act aca ttt aat 1824
   Gly Tyr Ser Ser Phe Asp Tyr Val Asp Thr Leu Val Thr Thr Phe Asn
                   595                 600                 605
   caa tca ggt gtt gaa ata att ata caa aat cta tct ggt tac cac ctt    1872
   Gln Ser Gly Val Glu Ile Ile Ile Gln Asn Leu Ser Gly Tyr His Leu
       610                 615                 620
   att gtt gac aaa gtc gaa ttt atc cca att gac atc caa att gaa aaa    1920
   Ile Val Asp Lys Val Glu Phe Ile Pro Ile Asp Ile Gln Ile Glu Lys
   625                 630                 635                 640
   tgt acg aaa tgt caa ttc gaa gga gac ata tgt aga tgt gaa gga gta    1968
   Cys Thr Lys Cys Gln Phe Glu Gly Asp Ile Cys Arg Cys Glu Gly Val
                   645                 650                 655
   caa tcc ttg gaa aca aaa aaa gag att gta aat agt tta ttt atc aat    2016
   Gln Ser Leu Glu Thr Lys Lys Glu Ile Val Asn Ser Leu Phe Ile Asn
               660                 665                 670
   taa                                                                2019
    *
<210> 27
   <211> 672
   <212> PRT
   <213> Bacillus thuringiensis
<400> 27
   Met Lys Asn Met Asn Ser Tyr Gln Asn Thr Asn Glu Tyr Glu Ile Leu
   1               5                   10                  15
   Asp Gly Ser Pro Asn Asn Thr Asn Met Ser Asn Arg Tyr Pro Phe Ala
               20                  25                  30
   Lys Asp Pro Asn Ile Phe Pro Ile Asn Leu Asp Ala Cys Gln Gly Arg
           35                  40                  45
   Pro Trp Gln Asp Thr Trp Glu Ser Val Ser Asp Ile Val Thr Ile Gly
       50                  55                  60
   Thr Tyr Leu Ile Gln Phe Leu Leu Glu Pro Gly Ile Gly Gly Ile Pro
   65                  70                  75                  80
   Val Ile Phe Ser Ile Ile Asn Lys Leu Ile Pro Ser Ser Gly Gln Ser
                   85                  90                  95
   Val Ala Ala Leu Ser Ile Cys Asp Leu Val Ser Ile Ile Arg Lys Glu
               100                 105                 110
   Val Asp Glu Ser Val Leu Ser Asp Gly Val Ala Asp Phe Glu Gly Glu
           115                 120                 125
   Met Thr Ala Tyr Gln Asp Tyr Tyr Leu His Tyr Leu Glu Asp Trp Leu
       130                 135                 140
   Thr Asp Lys Ser Asn Pro Lys Lys Leu Ala Asp Val Val Lys Gln Phe
   145                 150                 155                 160
   Gln Ala Arg Glu Glu Asp Phe Thr Lys Leu Leu Ala Gly Ser Leu Ser
                   165                 170                 175
   Arg Gln Lys Ala Glu Ile Leu Leu Leu Pro Thr Tyr Val Gln Ala Ala
               180                 185                 190
   Asn Val His Leu Leu Leu Leu Arg Asp Ala Val Lys Tyr Lys Lys Glu
           195                 200                 205
   Trp Gly Leu Val Cys Pro Pro Leu Tyr Pro Gly Ser Gly Arg Thr Asp
       210                 215                 220
   Cys Asn Glu Arg Leu Lys Ala Lys Ile Lys Glu Tyr Thr Asn Tyr Cys
   225                 230                 235                 240
   Val Gly Trp Tyr Asn Lys Gly Leu Asp Gln Ile Arg Gln Ala Gly Thr
                   245                 250                 255
   Ser Ala Glu Val Trp Ser Lys Phe Asn Lys Phe Arg Arg Glu Met Thr
               260                 265                 270
   Leu Ala Val Leu Asp Ile Ile Ala Ile Phe Pro Thr Tyr Asp Phe Glu
           275                 280                 285
   Lys Tyr Pro Leu Ala Thr Ser Val Glu Leu Thr Arg Glu Ile Tyr Thr
       290                 295                 300
   Asp Pro Val Gly Tyr Ser Gly Gly Asn Tyr Gly Trp Glu Arg Phe Phe
   305 310 315 320
   Ser Phe Asn Ser Val Glu Ala Asn Gly Thr Arg Gly Pro Gly Leu Val
   325 330 335
   Thr Trp Leu Gln Ala Ile Asp Ile Tyr Ser His Ser Ile Asn Leu Gln
   340 345 350
   Leu Gly Tyr Leu Ser Gly Trp Gly Gly Thr Arg His Tyr Glu Asp Phe
   355 360 365
   Thr Lys Gly Asn Gly Ala Phe Gln Arg Met Ser Gly Thr Thr Ser Asn
   370 375 380
   Asn Pro Arg Asn Ile Ile Phe Gly Asn Thr Asp Ile Phe Lys Ile Ile
   385 390 395 400
   Ser Leu Ala Arg Tyr Ala Met Gln Pro Phe Val Gly Tyr Ser Ile Pro
   405 410 415
   Arg His Leu Val Ser Arg Ala Glu Phe Phe Pro Thr Thr Leu Asn Thr
   420 425 430
   Phe Leu Tyr Glu Val Asn Ser Ser Gly Tyr Ser Gln Thr Ile Glu Ser
   435 440 445
   Val Leu Pro Gly Ile Asn Lys Asp Leu Pro Pro Ser Arg Thr Asn Tyr
   450 455 460
   Ser His Arg Leu Ser Asn Ala Ala Cys Val Gln Asn Glu Thr Ser Arg
   465 470 475 480
   Val Asn Val Phe Gly Trp Thr His Thr Ser Met Lys Lys Asp Asn Arg
   485 490 495
   Ile Tyr Pro Asp Lys Ile Thr Gln Ile Pro Ala Val Lys Ala Phe Ala
   500 505 510
   Leu Pro Ala Gly Thr Gly Tyr Ala Gly Gly Tyr Val Thr Ala Gly Pro
   515 520 525
   Gly Tyr Thr Gly Gly Asp Val Val Thr Leu Pro Tyr Gln Ala Ser Leu
   530 535 540
   Lys Ile Arg Leu Thr Ser Ala Pro Thr Asn Lys Asn Tyr Arg Val Arg
   545 550 555 560
   Leu Arg Tyr Ala Ser Gly Gly Pro Gly Pro Phe Arg Val Glu Arg Trp
   565 570 575
   Ser Pro Ser Ser Val Ser Asn Ala Asn Phe Ser Arg Pro Ala Thr Gly
   580 585 590
   Gly Tyr Ser Ser Phe Asp Tyr Val Asp Thr Leu Val Thr Thr Phe Asn
   595 600 605
   Gln Ser Gly Val Glu Ile Ile Ile Gln Asn Leu Ser Gly Tyr His Leu
   610 615 620
   Ile Val Asp Lys Val Glu Phe Ile Pro Ile Asp Ile Gln Ile Glu Lys
   625 630 635 640
   Cys Thr Lys Cys Gln Phe Glu Gly Asp Ile Cys Arg Cys Glu Gly Val
   645 650 655
   Gln Ser Leu Glu Thr Lys Lys Glu Ile Val Asn Ser Leu Phe Ile Asn
   660 665 670
<210> 28
   <211> 2010
   <212> DNA
   <213> Bacillus thuringiensis
<220>
   <221> CDS
   <222> (1)...(2010)
<400> 28
   atg aat tct tat caa aat aca aat gaa tat gaa att ctg gat ggt tcc     48
   Met Asn Ser Tyr Gln Asn Thr Asn Glu Tyr Glu Ile Leu Asp Gly Ser
    1               5                  10                  15
   ccg aat aac aca aat atg tca aac aga tat cct ttt gca aag gat cca     96
   Pro Asn Asn Thr Asn Met Ser Asn Arg Tyr Pro Phe Ala Lys Asp Pro
               20                  25                  30
   aat ata ttt cct att aac ctg gac gct tgt cag gga agg cca tgg caa     144
   Asn Ile Phe Pro Ile Asn Leu Asp Ala Cys Gln Gly Arg Pro Trp Gln
           35                  40                  45
   gat acg tgg gaa tca gtc tcg gat ata gta act att ggg aca tac ctt      192
   Asp Thr Trp Glu Ser Val Ser Asp Ile Val Thr Ile Gly Thr Tyr Leu
        50                  55                  60
   ata caa ttc ttg cta gaa ccc ggt ata ggt gga att cct gta ata ttt      240
   Ile Gln Phe Leu Leu Glu Pro Gly Ile Gly Gly Ile Pro Val Ile Phe
    65                  70                  75                  80
   tca ata ata aac aaa ctc att ccg tct tct ggt caa tct gtg gca gca      288
   Ser Ile Ile Asn Lys Leu Ile Pro Ser Ser Gly Gln Ser Val Ala Ala
                    85                  90                  95
   ctt tct ata tgt gat tta gta tct ata att cgt aaa gag gta gac gag      336
   Leu Ser Ile Cys Asp Leu Val Ser Ile Ile Arg Lys Glu Val Asp Glu
               100                 105                 110
   agc gtg tta agt gac ggg gtt gca gat ttt gag ggt gaa atg act gct      384
   Ser Val Leu Ser Asp Gly Val Ala Asp Phe Glu Gly Glu Met Thr Ala
           115                 120                 125
   tat caa gat tat tat ctt cat tat ctt gag gat tgg ctt aca gat aaa      432
   Tyr Gln Asp Tyr Tyr Leu His Tyr Leu Glu Asp Trp Leu Thr Asp Lys
       130                 135                 140
   tca aat cct aaa aaa ctt gct gac gta gtt aaa cag ttc caa gca cgg      480
   Ser Asn Pro Lys Lys Leu Ala Asp Val Val Lys Gln Phe Gln Ala Arg
   145                 150                 155                 160
   gaa gaa gat ttc act aaa ctt tta gca ggg tca tta tca aga cag aaa      528
   Glu Glu Asp Phe Thr Lys Leu Leu Ala Gly Ser Leu Ser Arg Gln Lys
                   165                 170                 175
   gct gaa ata tta tta ttg cct acg tat gtg caa gct gca aat gtg cat      576
   Ala Glu Ile Leu Leu Leu Pro Thr Tyr Val Gln Ala Ala Asn Val His
               180                 185                 190
   tta tta cta tta agg gac gca gtt aaa tat aaa aaa gaa tgg gga cta      624
   Leu Leu Leu Leu Arg Asp Ala Val Lys Tyr Lys Lys Glu Trp Gly Leu
           195                 200                 205
   gtg tgt cca ccg ttg tat cca ggg tca ggg aga act gat tgt aac gag      672
   Val Cys Pro Pro Leu Tyr Pro Gly Ser Gly Arg Thr Asp Cys Asn Glu
       210                 215                 220
   cgg tta aaa gcg aaa ata aaa gag tat act aat tat tgt gta ggg tgg      720
   Arg Leu Lys Ala Lys Ile Lys Glu Tyr Thr Asn Tyr Cys Val Gly Trp
   225                 230                 235                 240
   tat aac aag ggt tta gat cag ata aga cag gcg ggt aca agt gct gaa      768
   Tyr Asn Lys Gly Leu Asp Gln Ile Arg Gln Ala Gly Thr Ser Ala Glu
                   245                 250                 255
   gtt tgg tcg aaa ttt aat aaa ttt cgt aga gaa atg acg ttg gcg gta      816
   Val Trp Ser Lys Phe Asn Lys Phe Arg Arg Glu Met Thr Leu Ala Val
               260                 265                 270
   ttg gat att att gct ata ttt cca act tat gat ttt gaa aaa tat cca      864
   Leu Asp Ile Ile Ala Ile Phe Pro Thr Tyr Asp Phe Glu Lys Tyr Pro
           275                 280                 285
   tta gca aca agt gta gag tta act agg gaa att tat aca gat cca gtg      912
   Leu Ala Thr Ser Val Glu Leu Thr Arg Glu Ile Tyr Thr Asp Pro Val
       290                 295                 300
   gga tat tca ggg gga aat tat ggt tgg gaa cgg ttt ttt agc ttt aat      960
   Gly Tyr Ser Gly Gly Asn Tyr Gly Trp Glu Arg Phe Phe Ser Phe Asn
   305                 310                 315                 320
   tcg gta gaa gca aat gga aca cgg gga cct ggt tta gtt act tgg ctt    1008
   Ser Val Glu Ala Asn Gly Thr Arg Gly Pro Gly Leu Val Thr Trp Leu
                   325                 330                 335
   caa gct ata gat ata tat agt cat tct att aat ctt cag ctt ggt tat    1056
   Gln Ala Ile Asp Ile Tyr Ser His Ser Ile Asn Leu Gln Leu Gly Tyr
               340                 345                 350
   ctt agt ggc tgg ggg gga act cgt cat tat gaa gac ttc aca aag ggt    1104
   Leu Ser Gly Trp Gly Gly Thr Arg His Tyr Glu Asp Phe Thr Lys Gly
           355                 360                 365
   aac ggt gct ttt caa cgt atg tct gga act acg agt aat aat cca cgt    1152
   Asn Gly Ala Phe Gln Arg Met Ser Gly Thr Thr Ser Asn Asn Pro Arg
       370                 375                 380
   aat att att ttt ggc aat acc gat ata ttt aaa att att tca tta gct    1200
   Asn Ile Ile Phe Gly Asn Thr Asp Ile Phe Lys Ile Ile Ser Leu Ala
   385                 390                 395                 400
   aga tat gca atg caa ccg ttt gtt ggg tat tca atc cca cgg cat ctt    1248
   Arg Tyr Ala Met Gln Pro Phe Val Gly Tyr Ser Ile Pro Arg His Leu
                   405                 410                 415
   gtt tca cgt gca gaa ttt ttt ccg aca aca cta aat act ttc ctg tat    1296
   Val Ser Arg Ala Glu Phe Phe Pro Thr Thr Leu Asn Thr Phe Leu Tyr
               420                 425                 430
   gag gta aac agt tct ggg tac tca cag aca att gaa tct gtg tta cca    1344
   Glu Val Asn Ser Ser Gly Tyr Ser Gln Thr Ile Glu Ser Val Leu Pro
           435                 440                 445
   ggt att aat aag gat cta cca cct agt cgt aca aat tac tct cat aga    1392
   Gly Ile Asn Lys Asp Leu Pro Pro Ser Arg Thr Asn Tyr Ser His Arg
       450                 455                 460
   tta tca aat gcg gca tgt gtt caa aat gaa acc tcc aga gtt aac gta    1440
   Leu Ser Asn Ala Ala Cys Val Gln Asn Glu Thr Ser Arg Val Asn Val
   465                 470                 475                 480
   ttt ggt tgg aca cat aca agt atg aaa aaa gat aat cga att tat cca    1488
   Phe Gly Trp Thr His Thr Ser Met Lys Lys Asp Asn Arg Ile Tyr Pro
                   485                 490                 495
   gat aaa att acg caa att cct gca gta aaa gct ttt gcc cta cca gca    1536
   Asp Lys Ile Thr Gln Ile Pro Ala Val Lys Ala Phe Ala Leu Pro Ala
               500                 505                 510
   ggt aca gga tat gca gga ggt tac gtc aca gct ggg cct ggt tat aca    1584
   Gly Thr Gly Tyr Ala Gly Gly Tyr Val Thr Ala Gly Pro Gly Tyr Thr
           515                 520                 525
   gga gga gat gta gta acg tta cct tat caa gca agt tta aaa ata cgt    1632
   Gly Gly Asp Val Val Thr Leu Pro Tyr Gln Ala Ser Leu Lys Ile Arg
       530                 535                 540
   tta act tct gca ccc acg aat aaa aat tac cgt gtt aga ctt cgc tac    1680
   Leu Thr Ser Ala Pro Thr Asn Lys Asn Tyr Arg Val Arg Leu Arg Tyr
   545                 550                 555                 560
   gcg agt gga gga cct ggt ccg ttc cga gta gaa aga tgg tcg cca agt    1728
   Ala Ser Gly Gly Pro Gly Pro Phe Arg Val Glu Arg Trp Ser Pro Ser
                   565                 570                 575
   tct gtt tca aat gct aat ttt tct cgt cca gct aca ggt ggc tat agt    1776
   Ser Val Ser Asn Ala Asn Phe Ser Arg Pro Ala Thr Gly Gly Tyr Ser
               580                 585                 590
   tca ttt gat tat gtg gac acc tta gtt act aca ttt aat caa tca ggt    1824
   Ser Phe Asp Tyr Val Asp Thr Leu Val Thr Thr Phe Asn Gln Ser Gly
           595                 600                 605
   gtt gaa ata att ata caa aat cta tct ggt tac cac ctt att gtt gac    1872
   Val Glu Ile Ile Ile Gln Asn Leu Ser Gly Tyr His Leu Ile Val Asp
       610                 615                 620
   aaa gtc gaa ttt atc cca att gac atc caa att gaa aaa tgt acg aaa    1920
   Lys Val Glu Phe Ile Pro Ile Asp Ile Gln Ile Glu Lys Cys Thr Lys
   625                 630                 635                 640
   tgt caa ttc gaa gga gac ata tgt aga tgt gaa gga gta caa tcc ttg    1968
   Cys Gln Phe Glu Gly Asp Ile Cys Arg Cys Glu Gly Val Gln Ser Leu
                   645                 650                 655
   gaa aca aaa aaa gag att gta aat agt tta ttt atc aat taa            2010
   Glu Thr Lys Lys Glu Ile Val Asn Ser Leu Phe Ile Asn  *
               660                 665
<210> 29
   <211> 669
   <212> PRT
   <213> Bacillus thuringiensis
<400> 29
   Met Asn Ser Tyr Gln Asn Thr Asn Glu Tyr Glu Ile Leu Asp Gly Ser
    1               5                  10                  15
   Pro Asn Asn Thr Asn Met Ser Asn Arg Tyr Pro Phe Ala Lys Asp Pro
               20                  25                  30
   Asn Ile Phe Pro Ile Asn Leu Asp Ala Cys Gln Gly Arg Pro Trp Gln
           35                  40                  45
   Asp Thr Trp Glu Ser Val Ser Asp Ile Val Thr Ile Gly Thr Tyr Leu
       50                  55                  60
   Ile Gln Phe Leu Leu Glu Pro Gly Ile Gly Gly Ile Pro Val Ile Phe
   65                  70                  75                  80
   Ser Ile Ile Asn Lys Leu Ile Pro Ser Ser Gly Gln Ser Val Ala Ala
                   85                  90                  95
   Leu Ser Ile Cys Asp Leu Val Ser Ile Ile Arg Lys Glu Val Asp Glu
               100                 105                 110
   Ser Val Leu Ser Asp Gly Val Ala Asp Phe Glu Gly Glu Met Thr Ala
           115                 120                 125
   Tyr Gln Asp Tyr Tyr Leu His Tyr Leu Glu Asp Trp Leu Thr Asp Lys
       130                 135                 140
   Ser Asn Pro Lys Lys Leu Ala Asp Val Val Lys Gln Phe Gln Ala Arg
   145                 150                 155                 160
   Glu Glu Asp Phe Thr Lys Leu Leu Ala Gly Ser Leu Ser Arg Gln Lys
                   165                 170                 175
   Ala Glu Ile Leu Leu Leu Pro Thr Tyr Val Gln Ala Ala Asn Val His
               180                 185                 190
   Leu Leu Leu Leu Arg Asp Ala Val Lys Tyr Lys Lys Glu Trp Gly Leu
           195                 200                 205
   Val Cys Pro Pro Leu Tyr Pro Gly Ser Gly Arg Thr Asp Cys Asn Glu
       210                 215                 220
   Arg Leu Lys Ala Lys Ile Lys Glu Tyr Thr Asn Tyr Cys Val Gly Trp
   225                 230                 235                 240
   Tyr Asn Lys Gly Leu Asp Gln Ile Arg Gln Ala Gly Thr Ser Ala Glu
                   245                 250                 255
   Val Trp Ser Lys Phe Asn Lys Phe Arg Arg Glu Met Thr Leu Ala Val
               260                 265                 270
   Leu Asp Ile Ile Ala Ile Phe Pro Thr Tyr Asp Phe Glu Lys Tyr Pro
           275                 280                 285
   Leu Ala Thr Ser Val Glu Leu Thr Arg Glu Ile Tyr Thr Asp Pro Val
       290                 295                 300
   Gly Tyr Ser Gly Gly Asn Tyr Gly Trp Glu Arg Phe Phe Ser Phe Asn
   305                 310                 315                 320
   Ser Val Glu Ala Asn Gly Thr Arg Gly Pro Gly Leu Val Thr Trp Leu
                   325                 330                 335
   Gln Ala Ile Asp Ile Tyr Ser His Ser Ile Asn Leu Gln Leu Gly Tyr
               340                 345                 350
   Leu Ser Gly Trp Gly Gly Thr Arg His Tyr Glu Asp Phe Thr Lys Gly
           355                 360                 365
   Asn Gly Ala Phe Gln Arg Met Ser Gly Thr Thr Ser Asn Asn Pro Arg
       370                 375                 380
   Asn Ile Ile Phe Gly Asn Thr Asp Ile Phe Lys Ile Ile Ser Leu Ala
   385                 390                 395                 400
   Arg Tyr Ala Met Gln Pro Phe Val Gly Tyr Ser Ile Pro Arg His Leu
                   405                 410                 415
   Val Ser Arg Ala Glu Phe Phe Pro Thr Thr Leu Asn Thr Phe Leu Tyr
               420                 425                 430
   Glu Val Asn Ser Ser Gly Tyr Ser Gln Thr Ile Glu Ser Val Leu Pro
           435                 440                 445
   Gly Ile Asn Lys Asp Leu Pro Pro Ser Arg Thr Asn Tyr Ser His Arg
       450                 455                 460
   Leu Ser Asn Ala Ala Cys Val Gln Asn Glu Thr Ser Arg Val Asn Val
   465                 470                 475                 480
   Phe Gly Trp Thr His Thr Ser Met Lys Lys Asp Asn Arg Ile Tyr Pro
                   485                 490                 495
   Asp Lys Ile Thr Gln Ile Pro Ala Val Lys Ala Phe Ala Leu Pro Ala
               500                 505                 510
   Gly Thr Gly Tyr Ala Gly Gly Tyr Val Thr Ala Gly Pro Gly Tyr Thr
           515                 520                 525
   Gly Gly Asp Val Val Thr Leu Pro Tyr Gln Ala Ser Leu Lys Ile Arg
       530                 535                 540
   Leu Thr Ser Ala Pro Thr Asn Lys Asn Tyr Arg Val Arg Leu Arg Tyr
   545                 550                 555                 560
   Ala Ser Gly Gly Pro Gly Pro Phe Arg Val Glu Arg Trp Ser Pro Ser
                   565                 570                 575
   Ser Val Ser Asn Ala Asn Phe Ser Arg Pro Ala Thr Gly Gly Tyr Ser
               580                 585                 590
   Ser Phe Asp Tyr Val Asp Thr Leu Val Thr Thr Phe Asn Gln Ser Gly
           595                 600                 605
   Val Glu Ile Ile Ile Gln Asn Leu Ser Gly Tyr His Leu Ile Val Asp
       610                 615                 620
   Lys Val Glu Phe Ile Pro Ile Asp Ile Gln Ile Glu Lys Cys Thr Lys
   625                 630                 635                 640
   Cys Gln Phe Glu Gly Asp Ile Cys Arg Cys Glu Gly Val Gln Ser Leu
                   645                 650                 655
   Glu Thr Lys Lys Glu Ile Val Asn Ser Leu Phe Ile Asn
               660                 665
<210> 30
   <211> 1176
   <212> PRT
   <213> Bacillus thuringiensi
<400> 30
   Met Asp Asn Asn Pro Asn Ile Asn Glu Cys Ile Pro Tyr Asn Cys Leu
   1 5 10 15
   Ser Asn Pro Glu Val Glu Val Leu Gly Gly Glu Arg Ile Glu Thr Gly
   20 25 30
   Tyr Thr Pro Ile Asp Ile Ser Leu Ser Leu Thr Gln Phe Leu Leu Ser
   35 40 45
   Glu Phe Val Pro Gly Ala Gly Phe Val Leu Gly Leu Val Asp Ile Ile
   50 55 60
   Trp Gly Ile Phe Gly Pro Ser Gln Trp Asp Ala Phe Pro Val Gln Ile
   65 70 75 80
   Glu Gln Leu Ile Asn Gln Arg Ile Glu Glu Phe Ala Arg Asn Gln Ala
   85 90 95
   Ile Ser Arg Leu Glu Gly Leu Ser Asn Leu Tyr Gln Ile Tyr Ala Glu
   100 105 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Leu Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp Tyr Ala Val
           195                 200                 205
   Arg Trp Tyr Asn Thr Gly Leu Glu Arg Val Trp Gly Pro Asp Ser Arg
       210                 215                 220
   Asp Trp Val Arg Tyr Asn Gln Phe Arg Arg Glu Leu Thr Leu Thr Val
   225                 230                 235                 240
   Leu Asp Ile Val Ala Leu Phe Ser Asn Tyr Asp Ser Arg Arg Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Met Ala Gln Arg Ile Glu
           275                 280                 285
   Gln Asn Ile Arg Gln Pro His Leu Met Asp Ile Leu Asn Ser Ile Thr
       290                 295                 300
   Ile Tyr Thr Asp Val His Arg Gly Phe Asn Tyr Trp Ser Gly His Gln
   305                 310                 315                 320
   Ile Thr Ala Ser Pro Val Gly Phe Ser Gly Pro Glu Phe Ala Phe Pro
                   325                 330                 335
   Leu Phe Gly Asn Ala Gly Asn Ala Ala Pro Pro Val Leu Val Ser Leu
               340                 345                 350
   Thr Gly Leu Gly Ile Phe Arg Thr Leu Ser Ser Pro Leu Tyr Arg Arg
           355                 360                 365
   Ile Ile Leu Gly Ser Gly Pro Asn Asn Gln Glu Leu Phe Val Leu Asp
       370                 375                 380
   Gly Thr Glu Phe Ser Phe Ala Ser Leu Thr Thr Asn Leu Pro Ser Thr
   385                 390                 395                 400
   Ile Tyr Arg Gln Arg Gly Thr Val Asp Ser Leu Asp Val Ile Pro Pro
                   405                 410                 415
   Gln Asp Asn Ser Val Pro Pro Arg Ala Gly Phe Ser His Arg Leu Ser
               420                 425                 430
   His Val Thr Met Leu Ser Gln Ala Ala Gly Ala Val Tyr Thr Leu Arg
           435                 440                 445
   Ala Pro Thr Phe Ser Trp Gln His Arg Ser Ala Glu Phe Asn Asn Ile
       450                 455                 460
   Ile Pro Ser Ser Gln Ile Thr Gln Ile Pro Leu Thr Lys Ser Thr Asn
   465                 470                 475                 480
   Leu Gly Ser Gly Thr Ser Val Val Lys Gly Pro Gly Phe Thr Gly Gly
                   485                 490                 495
   Asp Ile Leu Arg Arg Thr Ser Pro Gly Gln Ile Ser Thr Leu Arg Val
               500                 505                 510
   Asn Ile Thr Ala Pro Leu Ser Gln Arg Tyr Arg Val Arg Ile Arg Tyr
           515                 520                 525
   Ala Ser Thr Thr Asn Leu Gln Phe His Thr Ser Ile Asp Gly Arg Pro
       530                 535                 540
   Ile Asn Gln Gly Asn Phe Ser Ala Thr Met Ser Ser Gly Ser Asn Leu
   545                 550                 555                 560
   Gln Ser Gly Ser Phe Arg Thr Val Gly Phe Thr Thr Pro Phe Asn Phe
                   565                 570                 575
   Ser Asn Gly Ser Ser Val Phe Thr Leu Ser Ala His Val Phe Asn Ser
               580                 585                 590
   Gly Asn Glu Val Tyr Ile Asp Arg Ile Glu Phe Val Pro Ala Glu Val
           595                 600                 605
   Thr Phe Glu Ala Glu Tyr Asp Leu Glu Arg Ala Gln Lys Ala Val Asn
       610                 615                 620
   Glu Leu Phe Thr Ser Ser Asn Gln Ile Gly Leu Lys Thr Asp Val Thr
   625                 630                 635                 640
   Asp Tyr His Ile Asp Gln Val Ser Asn Leu Val Glu Cys Leu Ser Asp
                   645                 650                 655
   Glu Phe Cys Leu Asp Glu Lys Gln Glu Leu Ser Glu Lys Val Lys His
                       660                 665                 670
   Ala Lys Arg Leu Ser Asp Glu Arg Asn Leu Leu Gln Asp Pro Asn Phe
           675                 680                 685
   Arg Gly Ile Asn Arg Gln Leu Asp Arg Gly Trp Arg Gly Ser Thr Asp
       690                 695                 700
   Ile Thr Ile Gln Gly Gly Asp Asp Val Phe Lys Glu Asn Tyr Val Thr
   705                 710                 715                 720
   Leu Leu Gly Thr Phe Asp Glu Cys Tyr Pro Thr Tyr Leu Tyr Gln Lys
                   725                 730                 735
   Ile Asp Glu Ser Lys Leu Lys Ala Tyr Thr Arg Tyr Gln Leu Arg Gly
               740                 745                 750
   Tyr Ile Glu Asp Ser Gln Asp Leu Glu Ile Tyr Leu Ile Arg Tyr Asn
           755                 760                 765
   Ala Lys His Glu Thr Val Asn Val Pro Gly Thr Gly Ser Leu Trp Pro
       770                 775                 780
   Leu Ser Ala Gln Ser Pro Ile Gly Lys Cys Gly Glu Pro Asn Arg Cys
   785                 790                 795                 800
   Ala Pro His Leu Glu Trp Asn Pro Asp Leu Asp Cys Ser Cys Arg Asp
                   805                 810                 815
   Gly Glu Lys Cys Ala His His Ser His His Phe Ser Leu Asp Ile Asp
               820                 825                 830
   Val Gly Cys Thr Asp Leu Asn Glu Asp Leu Gly Val Trp Val Ile Phe
           835                 840                 845
   Lys Ile Lys Thr Gln Asp Gly His Ala Arg Leu Gly Asn Leu Glu Phe
       850                 855                 860
   Leu Glu Glu Lys Pro Leu Val Gly Glu Ala Leu Ala Arg Val Lys Arg
   865                 870                 875                 880
   Ala Glu Lys Lys Trp Arg Asp Lys Arg Glu Lys Leu Glu Trp Glu Thr
                   885                 890                 895
   Asn Ile Val Tyr Lys Glu Ala Lys Glu Ser Val Asp Ala Leu Phe Val
               900                 905                 910
   Asn Ser Gln Tyr Asp Gln Leu Gln Ala Asp Thr Asn Ile Ala Met Ile
           915                 920                 925
   His Ala Ala Asp Lys Arg Val His Ser Ile Arg Glu Ala Tyr Leu Pro
       930                 935                 940
   Glu Leu Ser Val Ile Pro Gly Val Asn Ala Ala Ile Phe Glu Glu Leu
   945                 950                 955                 960
   Glu Gly Arg Ile Phe Thr Ala Phe Ser Leu Tyr Asp Ala Arg Asn Val
                   965                 970                 975
   Ile Lys Asn Gly Asp Phe Asn Asn Gly Leu Ser Cys Trp Asn Val Lys
               980                 985                 990
   Gly His Val Asp Val Glu Glu Gln Asn Asn Gln Arg Ser Val Leu Val
           995                 1000                1005
   Val Pro Glu Trp Glu Ala Glu Val Ser Gln Glu Val Arg Val Cys Pro
       1010                1015                1020
   Gly Arg Gly Tyr Ile Leu Arg Val Thr Ala Tyr Lys Glu Gly Tyr Gly
   1025                1030                1035                1040
   Glu Gly Cys Val Thr Ile His Glu Ile Glu Asn Asn Thr Asp Glu Leu
                   1045                1050                1055
   Lys Phe Ser Asn Cys Val Glu Glu Glu Ile Tyr Pro Asn Asn Thr Val
               1060                1065                1070
   Thr Cys Asn Asp Tyr Thr Val Asn Gln Glu Glu Tyr Gly Gly Ala Tyr
           1075                1080                1085
   Thr Ser Arg Asn Arg Gly Tyr Asn Glu Ala Pro Ser Val Pro Ala Asp
       1090                1095                1100
   Tyr Ala Ser Val Tyr Glu Glu Lys Ser Tyr Thr Asp Gly Arg Arg Glu
   1105                1110                1115                1120
   Asn Pro Cys Glu Phe Asn Arg Gly Tyr Arg Asp Tyr Thr Pro Leu Pro
                   1125                1130                1135
   Val Gly Tyr Val Thr Lys Glu Leu Glu Tyr Phe Pro Glu Thr Asp Lys
               1140                1145                1150
   Val Trp Ile Glu Ile Gly Glu Thr Glu Gly Thr Phe Ile Val Asp Ser
           1155                1160                1165
   Val Glu Leu Leu Leu Met Glu Glu
       1170                1175
<210> 31
   <211> 1178
   <212> PRT
   <213> Bacillus thuringiensi
<400> 31
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp Tyr Ala Val
           195                 200                 205
   Arg Trp Tyr Asn Thr Gly Leu Glu Arg Val Trp Gly Pro Asp Ser Arg
       210                 215                 220
   Asp Trp Val Arg Tyr Asn Gln Phe Arg Arg Glu Leu Thr Leu Thr Val
   225                 230                 235                 240
   Leu Asp Ile Val Ala Leu Phe Pro Asn Tyr Asp Ser Arg Arg Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Arg Ser Ile Arg Ser Pro His Leu Met Asp Ile Leu Asn Ser Ile Thr
       290                 295                 300
   Ile Tyr Thr Asp Ala His Arg Gly Tyr Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385                 390                 395                 400
   Tyr Arg Lys Ser Gly Thr Val Asp Ser Leu Asp Glu Ile Pro Pro Gln
                   405                 410                 415
   Asn Asn Asn Val Pro Pro Arg Gln Gly Phe Ser His Arg Leu Ser His
               420                 425                 430
   Val Ser Met Phe Arg Ser Gly Phe Ser Asn Ser Ser Val Ser Ile Ile
           435                 440                 445
   Arg Ala Pro Met Phe Ser Trp Ile His Arg Ser Ala Glu Phe Asn Asn
       450                 455                 460
   Ile Ile Ala Ser Asp Ser Ile Thr Gln Ile Pro Ala Val Lys Gly Asn
   465                 470                 475                 480
   Phe Leu Phe Asn Gly Ser Val Ile Ser Gly Pro Gly Phe Thr Gly Gly
                   485                 490                 495
   Asp Leu Val Arg Leu Asn Ser Ser Gly Asn Asn Ile Gln Asn Arg Gly
                   500                 505                 510
   Tyr Ile Glu Val Pro Ile His Phe Pro Ser Thr Ser Thr Arg Tyr Arg
               515                 520                 525
   Val Arg Val Arg Tyr Ala Ser Val Thr Pro Ile His Leu Asn Val Asn
           530                 535                 540
   Trp Gly Asn Ser Ser Ile Phe Ser Asn Thr Val Pro Ala Thr Ala Thr
   545                 550                 555                 560
   Ser Leu Asp Asn Leu Gln Ser Ser Asp Phe Gly Tyr Phe Glu Ser Ala
                   565                 570                 575
   Asn Ala Phe Thr Ser Ser Leu Gly Asn Ile Val Gly Val Arg Asn Phe
               580                 585                 590
   Ser Gly Thr Ala Gly Val Ile Ile Asp Arg Phe Glu Phe Ile Pro Val
           595                 600                 605
   Thr Ala Thr Leu Glu Ala Glu Tyr Asn Leu Glu Arg Ala Gln Lys Ala
       610                 615                 620
   Val Asn Ala Leu Phe Thr Ser Thr Asn Gln Leu Gly Leu Lys Thr Asn
   625                 630                 635                 640
   Val Thr Asp Tyr His Ile Asp Gln Val Ser Asn Leu Val Thr Tyr Leu
                   645                 650                 655
   Ser Asp Glu Phe Cys Leu Asp Glu Lys Arg Glu Leu Ser Glu Lys Val
               660                 665                 670
   Lys His Ala Lys Arg Leu Ser Asp Glu Arg Asn Leu Leu Gln Asp Ser
           675                 680                 685
   Asn Phe Lys Asp Ile Asn Arg Gln Pro Glu Arg Gly Trp Gly Gly Ser
       690                 695                 700
   Thr Gly Ile Thr Ile Gln Gly Gly Asp Asp Val Phe Lys Glu Asn Tyr
   705                 710                 715                 720
   Val Thr Leu Ser Gly Thr Phe Asp Glu Cys Tyr Pro Thr Tyr Leu Tyr
                   725                 730                 735
   Gln Lys Ile Asp Glu Ser Lys Leu Lys Ala Phe Thr Arg Tyr Gln Leu
               740                 745                 750
   Arg Gly Tyr Ile Glu Asp Ser Gln Asp Leu Glu Ile Tyr Leu Ile Arg
           755                 760                 765
   Tyr Asn Ala Lys His Glu Thr Val Asn Val Pro Gly Thr Gly Ser Leu
       770                 775                 780
   Trp Pro Leu Ser Ala Gln Ser Pro Ile Gly Lys Cys Gly Glu Pro Asn
   785                 790                 795                 800
   Arg Cys Ala Pro His Leu Glu Trp Asn Pro Asp Leu Asp Cys Ser Cys
                   805                 810                 815
   Arg Asp Gly Glu Lys Cys Ala His His Ser His His Phe Ser Leu Asp
               820                 825                 830
   Ile Asp Val Gly Cys Thr Asp Leu Asn Glu Asp Leu Gly Val Trp Val
           835                 840                 845
   Ile Phe Lys Ile Lys Thr Gln Asp Gly His Ala Arg Leu Gly Asn Leu
       850                 855                 860
   Glu Phe Leu Glu Glu Lys Pro Leu Val Gly Glu Ala Leu Ala Arg Val
   865                 870                 875                 880
   Lys Arg Ala Glu Lys Lys Trp Arg Asp Lys Arg Glu Lys Leu Glu Trp
                   885                 890                 895
   Glu Thr Asn Ile Val Tyr Lys Glu Ala Lys Glu Ser Val Asp Ala Leu
               900                 905                 910
   Phe Val Asn Ser Gln Tyr Asp Gln Leu Gln Ala Asp Thr Asn Ile Ala
           915                 920                 925
   Met Ile His Ala Ala Asp Lys Arg Val His Ser Ile Arg Glu Ala Tyr
       930                 935                 940
   Leu Pro Glu Leu Ser Val Ile Pro Gly Val Asn Ala Ala Ile Phe Glu
   945                 950                 955                 960
   Glu Leu Glu Gly Arg Ile Phe Thr Ala Phe Ser Leu Tyr Asp Ala Arg
                   965                 970                 975
   Asn Val Ile Lys Asn Gly Asp Phe Asn Asn Gly Leu Ser Cys Trp Asn
               980                 985                 990
   Val Lys Gly His Val Asp Val Glu Glu Gln Asn Asn Gln Arg Ser Val
           995                 1000                1005
   Leu Val Val Pro Glu Trp Glu Ala Glu Val Ser Gln Glu Val Arg Val
       1010                1015                1020
   Cys Pro Gly Arg Gly Tyr Ile Leu Arg Val Thr Ala Tyr Lys Glu Gly
   1025                1030                1035                1040
   Tyr Gly Glu Gly Cys Val Thr Ile His Glu Ile Glu Asn Asn Thr Asp
                   1045                1050                1055
   Glu Leu Lys Phe Ser Asn Cys Val Glu Glu Glu Ile Tyr Pro Asn Asn
               1060                1065                1070
   Thr Val Thr Cys Asn Asp Tyr Thr Val Asn Gln Glu Glu Tyr Gly Gly
           1075                1080                1085
   Ala Tyr Thr Ser Arg Asn Arg Gly Tyr Asn Glu Ala Pro Ser Val Pro
       1090                1095                1100
   Ala Asp Tyr Ala Ser Val Tyr Glu Glu Lys Ser Tyr Thr Asp Gly Arg
   1105                1110                1115                1120
   Arg Glu Asn Pro Cys Glu Phe Asn Arg Gly Tyr Arg Asp Tyr Thr Pro
                   1125                1130                1135
   Leu Pro Val Gly Tyr Val Thr Lys Glu Leu Glu Tyr Phe Pro Glu Thr
               1140                1145                1150
   Asp Lys Val Trp Ile Glu Ile Gly Glu Thr Glu Gly Thr Phe Ile Val
           1155                1160                1165
   Asp Ser Val Glu Leu Leu Leu Met Glu Glu
       1170                1175
<210> 32
   <211> 1189
   <212> PRT
   <213> Bacillus thuringiensi
<400> 32
   Met Glu Glu Asn Asn Gln Asn Gln Cys Ile Pro Tyr Asn Cys Leu Ser
   1               5                   10                  15
   Asn Pro Glu Glu Val Leu Leu Asp Gly Glu Arg Ile Ser Thr Gly Asn
               20                  25                  30
   Ser Ser Ile Asp Ile Ser Leu Ser Leu Val Gln Phe Leu Val Ser Asn
           35                  40                  45
   Phe Val Pro Gly Gly Gly Phe Leu Val Gly Leu Ile Asp Phe Val Trp
       50                  55                  60
   Gly Ile Val Gly Pro Ser Gln Trp Asp Ala Phe Leu Val Gln Ile Glu
   65                  70                  75                  80
   Gln Leu Ile Asn Glu Arg Ile Ala Glu Phe Ala Arg Asn Ala Ala Ile
                   85                  90                  95
   Ala Asn Leu Glu Gly Leu Gly Asn Asn Phe Asn Ile Tyr Val Glu Ala
               100                 105                 110
   Phe Lys Glu Trp Glu Glu Asp Pro Asn Asn Pro Ala Thr Arg Thr Arg
           115                 120                 125
   Val Ile Asp Arg Phe Arg Ile Leu Asp Gly Leu Leu Glu Arg Asp Ile
       130                 135                 140
   Pro Ser Phe Arg Ile Ser Gly Phe Glu Val Pro Leu Leu Ser Val Tyr
   145                 150                 155                 160
   Ala Gln Ala Ala Asn Leu His Leu Ala Ile Leu Arg Asp Ser Val Ile
                   165                 170                 175
   Phe Gly Glu Arg Trp Gly Leu Thr Thr Ile Asn Val Asn Glu Asn Tyr
               180                 185                 190
   Asn Arg Leu Ile Arg His Ile Asp Glu Tyr Ala Asp His Cys Ala Asn
           195                 200                 205
   Thr Tyr Asn Arg Gly Leu Asn Asn Leu Pro Lys Ser Thr Tyr Gln Asp
       210                 215                 220
   Trp Ile Thr Tyr Asn Arg Leu Arg Arg Asp Leu Thr Leu Thr Val Leu
   225                 230                 235                 240
   Asp Ile Ala Ala Phe Phe Pro Asn Tyr Asp Asn Arg Arg Tyr Pro Ile
                   245                 250                 255
   Gln Pro Val Gly Gln Leu Thr Arg Glu Val Tyr Thr Asp Pro Leu Ile
               260                 265                 270
   Asn Phe Asn Pro Gln Leu Gln Ser Val Ala Gln Leu Pro Thr Phe Asn
           275                 280                 285
   Val Met Glu Ser Ser Ala Ile Arg Asn Pro His Leu Phe Asp Ile Leu
       290                 295                 300
   Asn Asn Leu Thr Ile Phe Thr Asp Trp Phe Ser Val Gly Arg Asn Phe
   305                 310                 315                 320
   Tyr Trp Gly Gly His Arg Val Ile Ser Ser Leu Ile Gly Gly Gly Asn
                   325                 330                 335
   Ile Thr Ser Pro Ile Tyr Gly Arg Glu Ala Asn Gln Glu Pro Pro Arg
   340 345 350
   Ser Phe Thr Phe Asn Gly Pro Val Phe Arg Thr Leu Ser Asn Pro Thr
   355 360 365
   Leu Arg Leu Leu Gln Gln Pro Trp Pro Ala Pro Pro Phe Asn Leu Arg
   370 375 380
   Gly Val Glu Gly Val Glu Phe Ser Thr Pro Thr Asn Ser Phe Thr Tyr
   385 390 395 400
   Arg Gly Arg Gly Thr Val Asp Ser Leu Thr Glu Leu Pro Pro Glu Asp
   405 410 415
   Asn Ser Val Pro Pro Arg Glu Gly Tyr Ser His Arg Leu Cys His Ala
   420 425 430
   Thr Phe Val Gln Arg Ser Gly Thr Pro Phe Leu Thr Thr Gly Val Val
   435 440 445
   Phe Ser Trp Thr His Arg Ser Ala Thr Leu Thr Asn Thr Ile Asp Pro
   450 455 460
   Glu Arg Ile Asn Gln Ile Pro Leu Val Lys Gly Phe Arg Val Trp Gly
   465 470 475 480
   Gly Thr Ser Val Ile Thr Gly Pro Gly Phe Thr Gly Gly Asp Ile Leu
   485 490 495
   Arg Arg Asn Thr Phe Gly Asp Phe Val Ser Leu Gln Val Asn Ile Asn
   500 505 510
   Ser Pro Ile Thr Gln Arg Tyr Arg Leu Arg Phe Arg Tyr Ala Ser Ser
   515 520 525
   Arg Asp Ala Arg Val Ile Val Leu Thr Gly Ala Ala Ser Thr Gly Val
   530 535 540
   Gly Gly Gln Val Ser Val Asn Met Pro Leu Gln Lys Thr Met Glu Ile
   545 550 555 560
   Gly Glu Asn Leu Thr Ser Arg Thr Phe Arg Tyr Thr Asp Phe Ser Asn
   565 570 575
   Pro Phe Ser Phe Arg Ala Asn Pro Asp Ile Ile Gly Ile Ser Glu Gln
   580 585 590
   Pro Leu Phe Gly Ala Gly Ser Ile Ser Ser Gly Glu Leu Tyr Ile Asp
   595 600 605
   Lys Ile Glu Ile Ile Leu Ala Asp Ala Thr Phe Glu Ala Glu Ser Asp
   610 615 620
   Leu Glu Arg Ala Gln Lys Ala Val Asn Ala Leu Phe Thr Ser Ser Asn
   625 630 635 640
   Gln Ile Gly Leu Lys Thr Asp Val Thr Asp Tyr His Ile Asp Gln Val
   645 650 655
   Ser Asn Leu Val Asp Cys Leu Ser Asp Glu Phe Cys Leu Asp Glu Lys
   660 665 670
   Arg Glu Leu Ser Glu Lys Val Lys His Ala Lys Arg Leu Ser Asp Glu
   675 680 685
   Arg Asn Leu Leu Gln Asp Pro Asn Phe Arg Gly Ile Asn Arg Gln Pro
   690 695 700
   Asp Arg Gly Trp Arg Gly Ser Thr Asp Ile Thr Ile Gln Gly Gly Asp
   705 710 715 720
   Asp Val Phe Lys Glu Asn Tyr Val Thr Leu Pro Gly Thr Val Asp Glu
   725 730 735
   Cys Tyr Pro Thr Tyr Leu Tyr Gln Lys Ile Asp Glu Ser Lys Leu Lys
   740 745 750
   Ala Tyr Thr Arg Tyr Glu Leu Arg Gly Tyr Ile Glu Asp Ser Gln Asp
   755 760 765
   Leu Glu Ile Tyr Leu Ile Arg Tyr Asn Ala Lys His Glu Ile Val Asn
   770 775 780
   Val Pro Gly Thr Gly Ser Leu Trp Pro Leu Ser Ala Gln Ser Pro Ile
   785 790 795 800
   Gly Lys Cys Gly Glu Pro Asn Arg Cys Ala Pro His Leu Glu Trp Asn
   805 810 815
   Pro Asp Leu Asp Cys Ser Cys Arg Asp Gly Glu Lys Cys Ala His His
   820 825 830
   Ser His His Phe Thr Leu Asp Ile Asp Val Gly Cys Thr Asp Leu Asn
   835 840 845
   Glu Asp Leu Gly Val Trp Val Ile Phe Lys Ile Lys Thr Gln Asp Gly
   850 855 860
   His Ala Arg Leu Gly Asn Leu Glu Phe Leu Glu Glu Lys Pro Leu Leu
   865 870 875 880
   Gly Glu Ala Leu Ala Arg Val Lys Arg Ala Glu Lys Lys Trp Arg Asp
   885 890 895
   Lys Arg Glu Lys Leu Gln Leu Glu Thr Asn Ile Val Tyr Lys Glu Ala
   900 905 910
   Lys Glu Ser Val Asp Ala Leu Phe Val Asn Ser Gln Tyr Asp Arg Leu
   915 920 925
   Gln Val Asp Thr Asn Ile Ala Met Ile His Ala Ala Asp Lys Arg Val
   930 935 940
   His Arg Ile Arg Glu Ala Tyr Leu Pro Glu Leu Ser Val Ile Pro Gly
   945 950 955 960
   Val Asn Ala Ala Ile Phe Glu Glu Leu Glu Gly Arg Ile Phe Thr Ala
   965 970 975
   Tyr Ser Leu Tyr Asp Ala Arg Asn Val Ile Lys Asn Gly Asp Phe Asn
   980 985 990
   Asn Gly Leu Leu Cys Trp Asn Val Lys Gly His Val Asp Val Glu Glu
   995 1000 1005
   Gln Asn Asn His Arg Ser Val Leu Val Ile Pro Glu Trp Glu Ala Glu
   1010 1015 1020
   Val Ser Gln Glu Val Arg Val Cys Pro Gly Arg Gly Tyr Ile Leu Arg
   1025 1030 1035 1040
   Val Thr Ala Tyr Lys Glu Gly Tyr Gly Glu Gly Cys Val Thr Ile His
   1045 1050 1055
   Glu Ile Glu Asp Asn Thr Asp Glu Leu Lys Phe Ser Asn Cys Val Glu
   1060 1065 1070
   Glu Glu Val Tyr Pro Asn Asn Thr Val Thr Cys Asn Asn Tyr Thr Gly
   1075 1080 1085
   Thr Gln Glu Glu Tyr Glu Gly Thr Tyr Thr Ser Arg Asn Gln Gly Tyr
   1090 1095 1100
   Asp Glu Ala Tyr Gly Asn Asn Pro Ser Val Pro Ala Asp Tyr Ala Ser
   1105 1110 1115 1120
   Val Tyr Glu Glu Lys Ser Tyr Thr Asp Gly Arg Arg Glu Asn Pro Cys
   1125 1130 1135
   Glu Ser Asn Arg Gly Tyr Gly Asp Tyr Thr Pro Leu Pro Ala Gly Tyr
   1140 1145 1150
   Val Thr Lys Asp Leu Glu Tyr Phe Pro Glu Thr Asp Lys Val Trp Ile
   1155 1160 1165
   Glu Ile Gly Glu Thr Glu Gly Thr Phe Ile Val Asp Ser Val Glu Leu
   1170 1175 1180
   Leu Leu Met Glu Glu
   1185
<210> 33
   <211> 719
   <212> PRT
   <213> Bacillus thuringiensi
<400> 33
   Met Lys Leu Lys Asn Gln Asp Lys His Gln Ser Phe Ser Ser Asn Ala
    1               5                  10                  15
   Lys Val Asp Lys Ile Ser Thr Asp Ser Leu Lys Asn Glu Thr Asp Ile
               20                  25                  30
   Glu Leu Gln Asn Ile Asn His Glu Asp Cys Leu Lys Met Ser Glu Tyr
           35                  40                  45
   Glu Asn Val Glu Pro Phe Val Ser Ala Ser Thr Ile Gln Thr Gly Ile
       50                  55                  60
   Gly Ile Ala Gly Lys Ile Leu Gly Thr Leu Gly Val Pro Phe Ala Gly
   65                  70                  75                  80
   Gln Val Ala Ser Leu Tyr Ser Phe Ile Leu Gly Glu Leu Trp Pro Lys
                   85                  90                  95
   Gly Lys Asn Gln Trp Glu Ile Phe Met Glu His Val Glu Glu Ile Ile
               100                 105                 110
   Asn Gln Lys Ile Ser Thr Tyr Ala Arg Asn Lys Ala Leu Thr Asp Leu
           115                 120                 125
   Lys Gly Leu Gly Asp Ala Leu Ala Val Tyr His Asp Ser Leu Glu Ser
       130                 135                 140
   Trp Val Gly Asn Arg Asn Asn Thr Arg Ala Arg Ser Val Val Lys Ser
   145                 150                 155                 160
   Gln Tyr Ile Ala Leu Glu Leu Met Phe Val Gln Lys Leu Pro Ser Phe
                           165                     170                     175
   Ala Val Ser Gly Glu Glu Val Pro Leu Leu Pro Ile Tyr Ala Gln Ala
                       180                 185                     190
   Ala Asn Leu His Leu Leu Leu Leu Arg Asp Ala Ser Ile Phe Gly Lys
           195                     200                     205
   Glu Trp Gly Leu Ser Ser Ser Glu Ile Ser Thr Phe Tyr Asn Arg Gln
       210                     215                     220
   Val Glu Arg Ala Gly Asp Tyr Ser Asp His Cys Val Lys Trp Tyr Ser
   225                     230                     235                     240
   Thr Gly Leu Asn Asn Leu Arg Gly Thr Asn Ala Glu Ser Trp Val Arg
                   245                     250                     255
   Tyr Asn Gln Phe Arg Arg Asp Met Thr Leu Met Val Leu Asp Leu Val
               260                     265                     270
   Ala Leu Phe Pro Ser Tyr Asp Thr Gln Met Tyr Pro Ile Lys Thr Thr
           275                     280                     285
   Ala Gln Leu Thr Arg Glu Val Tyr Thr Asp Ala Ile Gly Thr Val His
       290                     295                     300
   Pro His Pro Ser Phe Thr Ser Thr Thr Trp Tyr Asn Asn Asn Ala Pro
   305                     310                     315                     320
   Ser Phe Ser Ala Ile Glu Ala Ala Val Val Arg Asn Pro His Leu Leu
                   325                     330                     335
   Asp Phe Leu Glu Gln Val Thr Ile Tyr Ser Leu Leu Ser Arg Trp Ser
               340                     345                     350
   Asn Thr Gln Tyr Met Asn Met Trp Gly Gly His Lys Leu Glu Phe Arg
           355                     360                     365
   Thr Ile Gly Gly Thr Leu Asn Ile Ser Thr Gln Gly Ser Thr Asn Thr
       370                     375                     380
   Ser Ile Asn Pro Val Thr Leu Pro Phe Thr Ser Arg Asp Val Tyr Arg
   385                     390                     395                     400
   Thr Glu Ser Leu Ala Gly Leu Asn Leu Phe Leu Thr Gln Pro Val Asn
                   405                     410                     415
   Gly Val Pro Arg Val Asp Phe His Trp Lys Phe Val Thr His Pro Ile
               420                     425                     430
   Ala Ser Asp Asn Phe Tyr Tyr Pro Gly Tyr Ala Gly Ile Gly Thr Gln
           435                     440                     445
   Leu Gln Asp Ser Glu Asn Glu Leu Pro Pro Glu Ala Thr Gly Gln Pro
       450                     455                     460
   Asn Tyr Glu Ser Tyr Ser His Arg Leu Ser His Ile Gly Leu Ile Ser
   465                     470                     475                     480
   Ala Ser His Val Lys Ala Leu Val Tyr Ser Trp Thr His Arg Ser Ala
                   485                     490                     495
   Asp Arg Thr Asn Thr Ile Glu Pro Asn Ser Ile Thr Gln Ile Pro Leu
               500                     505                     510
   Val Lys Ala Phe Asn Leu Ser Ser Gly Ala Ala Val Val Arg Gly Pro
           515                     520                     525
   Gly Phe Thr Gly Gly Asp Ile Leu Arg Arg Thr Asn Thr Gly Thr Phe
       530                     535                     540
   Gly Asp Ile Arg Val Asn Ile Asn Pro Pro Phe Ala Gln Arg Tyr Arg
   545                     550                     555                     560
   Val Arg Ile Arg Tyr Ala Ser Thr Thr Asp Leu Gln Phe His Thr Ser
                   565                     570                     575
   Ile Asn Gly Lys Ala Ile Asn Gln Gly Asn Phe Ser Ala Thr Met Asn
               580                     585                     590
   Arg Gly Glu Asp Leu Asp Tyr Lys Thr Phe Arg Thr Val Gly Phe Thr
           595                     600                     605
   Thr Pro Phe Ser Phe Leu Asp Val Gln Ser Thr Phe Thr Ile Gly Ala
       610                     615                     620
   Trp Asn Phe Ser Ser Gly Asn Glu Val Tyr Ile Asp Arg Ile Glu Phe
   625                     630                     635                     640
   Val Pro Val Glu Val Thr Tyr Glu Ala Glu Tyr Asp Phe Glu Lys Ala
                   645                     650                     655
   Gln Glu Lys Val Thr Ala Leu Phe Thr Ser Thr Asn Pro Arg Gly Leu
               660                     665                     670
   Lys Thr Asp Val Lys Asp Tyr His Ile Asp Gln Val Ser Asn Leu Val
           675                     680                     685
   Glu Ser Leu Ser Asp Glu Phe Tyr Leu Asp Glu Lys Arg Glu Leu Phe
       690                     695                     700
   Glu Ile Val Lys Tyr Ala Lys Gln Leu His Ile Glu Arg Asn Met
   705 710 715
<210> 34
   <211> 633
   <212> PRT
   <213> Bacillus thuringiensi
<400> 34
   Met Asn Asn Val Leu Asn Ser Gly Arg Thr Thr Ile Cys Asp Ala Tyr
   1               5                   10                  15
   Asn Val Val Ala His Asp Pro Phe Ser Phe Glu His Lys Ser Leu Asp
               20                  25                  30
   Thr Ile Gln Lys Glu Trp Met Glu Trp Lys Arg Thr Asp His Ser Leu
           35                  40                  45
   Tyr Val Ala Pro Val Val Gly Thr Val Ser Ser Phe Leu Leu Lys Lys
       50                  55                  60
   Val Gly Ser Leu Ile Gly Lys Arg Ile Leu Ser Glu Leu Trp Gly Ile
   65                  70                  75                  80
   Ile Phe Pro Ser Gly Ser Thr Asn Leu Met Gln Asp Ile Leu Arg Glu
                   85                  90                  95
   Thr Glu Gln Phe Leu Asn Gln Arg Leu Asn Thr Asp Thr Leu Ala Arg
               100                 105                 110
   Val Asn Ala Glu Leu Ile Gly Leu Gln Ala Asn Ile Arg Glu Phe Asn
           115                 120                 125
   Gln Gln Val Asp Asn Phe Leu Asn Pro Thr Gln Asn Pro Val Pro Leu
       130                 135                 140
   Ser Ile Thr Ser Ser Val Asn Thr Met Gln Gln Leu Phe Leu Asn Arg
   145                 150                 155                 160
   Leu Pro Gln Phe Gln Ile Gln Gly Tyr Gln Leu Leu Leu Leu Pro Leu
                   165                 170                 175
   Phe Ala Gln Ala Ala Asn Met His Leu Ser Phe Ile Arg Asp Val Ile
               180                 185                 190
   Leu Asn Ala Asp Glu Trp Gly Ile Ser Ala Ala Thr Leu Arg Thr Tyr
           195                 200                 205
   Arg Asp Tyr Leu Arg Asn Tyr Thr Arg Asp Tyr Ser Asn Tyr Cys Ile
       210                 215                 220
   Asn Thr Tyr Gln Thr Ala Phe Arg Gly Leu Asn Thr Arg Leu His Asp
   225                 230                 235                 240
   Met Leu Glu Phe Arg Thr Tyr Met Phe Leu Asn Val Phe Glu Tyr Val
                   245                 250                 255
   Ser Ile Trp Ser Leu Phe Lys Tyr Gln Ser Leu Met Val Ser Ser Gly
               260                 265                 270
   Ala Asn Leu Tyr Ala Ser Gly Ser Gly Pro Gln Gln Thr Gln Ser Phe
           275                 280                 285
   Thr Ala Gln Asn Trp Pro Phe Leu Tyr Ser Leu Phe Gln Val Asn Ser
       290                 295                 300
   Asn Tyr Ile Leu Ser Gly Ile Ser Gly Thr Arg Leu Ser Ile Thr Phe
   305                 310                 315                 320
   Pro Asn Ile Gly Gly Leu Pro Gly Ser Thr Thr Thr His Ser Leu Asn
                   325                 330                 335
   Ser Ala Arg Val Asn Tyr Ser Gly Gly Val Ser Ser Gly Leu Ile Gly
               340                 345                 350
   Ala Thr Asn Leu Asn His Asn Phe Asn Cys Ser Thr Val Leu Pro Pro
           355                 360                 365
   Leu Ser Thr Pro Phe Val Arg Ser Trp Leu Asp Ser Gly Thr Asp Arg
       370                 375                 380
   Glu Gly Val Ala Thr Ser Thr Asn Trp Gln Thr Glu Ser Phe Gln Thr
   385                 390                 395                 400
   Thr Leu Ser Leu Arg Cys Gly Ala Phe Ser Ala Arg Gly Asn Ser Asn
                   405                 410                 415
   Tyr Phe Pro Asp Tyr Phe Ile Arg Asn Ile Ser Gly Val Pro Leu Val
               420                 425                 430
   Ile Arg Asn Glu Asp Leu Thr Arg Pro Leu His Tyr Asn Gln Ile Arg
           435                 440                 445
   Asn Ile Glu Ser Pro Ser Gly Thr Pro Gly Gly Ala Arg Ala Tyr Leu
       450                 455                 460
   Val Ser Val His Asn Arg Lys Asn Asn Ile Tyr Ala Ala Asn Glu Asn
   465                 470                 475                 480
   Gly Thr Met Ile His Leu Ala Pro Glu Asp Tyr Thr Gly Phe Thr Ile
                   485                 490                 495
   Ser Pro Ile His Ala Thr Gln Val Asn Asn Gln Thr Arg Thr Phe Ile
               500                 505                 510
   Ser Glu Lys Phe Gly Asn Gln Gly Asp Ser Leu Arg Phe Glu Gln Ser
           515                 520                 525
   Asn Thr Thr Ala Arg Tyr Thr Leu Arg Gly Asn Gly Asn Ser Tyr Asn
       530                 535                 540
   Leu Tyr Leu Arg Val Ser Ser Ile Gly Asn Ser Thr Ile Arg Val Thr
   545                 550                 555                 560
   Ile Asn Gly Arg Val Tyr Thr Val Ser Asn Val Asn Thr Thr Thr Asn
                   565                 570                 575
   Asn Asp Gly Val Asn Asp Asn Gly Ala Arg Phe Ser Asp Ile Asn Ile
               580                 585                 590
   Gly Asn Ile Val Ala Ser Asp Asn Thr Asn Val Thr Leu Asp Ile Asn
           595                 600                 605
   Val Thr Leu Asn Ser Gly Thr Pro Phe Asp Leu Met Asn Ile Met Phe
       610                 615                 620
   Val Pro Thr Asn Leu Pro Pro Leu Tyr
   625                 630
<210> 35
   <211> 652
   <212> PRT
   <213> Bacillus thuringiensi
<400> 35
   Met Ile Arg Lys Gly Gly Arg Lys Met Asn Pro Asn Asn Arg Ser Glu
   1               5                   10                  15
   His Asp Thr Ile Lys Thr Thr Glu Asn Asn Glu Val Pro Thr Asn His
               20                  25                  30
   Val Gln Tyr Pro Leu Ala Glu Thr Pro Asn Pro Thr Leu Glu Asp Leu
           35                  40                  45
   Asn Tyr Lys Glu Phe Leu Arg Met Thr Ala Asp Asn Asn Thr Glu Ala
       50                  55                  60
   Leu Asp Ser Ser Thr Thr Lys Asp Val Ile Gln Lys Gly Ile Ser Val
   65                  70                  75                  80
   Val Gly Asp Leu Leu Gly Val Val Gly Phe Pro Phe Gly Gly Ala Leu
                   85                  90                  95
   Val Ser Phe Tyr Thr Asn Phe Leu Asn Thr Ile Trp Pro Ser Glu Asp
               100                 105                 110
   Pro Trp Lys Ala Phe Met Glu Gln Val Glu Ala Leu Met Asp Gln Lys
           115                 120                 125
   Ile Ala Asp Tyr Ala Lys Asn Lys Ala Leu Ala Glu Leu Gln Gly Leu
       130                 135                 140
   Gln Asn Asn Val Glu Asp Tyr Val Ser Ala Leu Ser Ser Trp Gln Lys
   145                 150                 155                 160
   Asn Pro Val Ser Ser Arg Asn Pro His Ser Gln Gly Arg Ile Arg Glu
                   165                 170                 175
   Leu Phe Ser Gln Ala Glu Ser His Phe Arg Asn Ser Met Pro Ser Phe
               180                 185                 190
   Ala Ile Ser Gly Tyr Glu Val Leu Phe Leu Thr Thr Tyr Ala Gln Ala
           195                 200                 205
   Ala Asn Thr His Leu Phe Leu Leu Lys Asp Ala Gln Ile Tyr Gly Glu
       210                 215                 220
   Glu Trp Gly Tyr Glu Lys Glu Asp Ile Ala Glu Phe Tyr Lys Arg Gln
   225                 230                 235                 240
   Leu Lys Leu Thr Gln Glu Tyr Thr Asp His Cys Val Lys Trp Tyr Asn
                   245                 250                 255
   Val Gly Leu Asp Lys Leu Arg Gly Ser Ser Tyr Glu Ser Trp Val Asn
                   260                 265                 270
   Phe Asn Arg Tyr Arg Arg Glu Met Thr Leu Thr Val Leu Asp Leu Ile
           275                 280                 285
   Ala Leu Phe Pro Leu Tyr Asp Val Arg Leu Tyr Pro Lys Glu Val Lys
       290                 295                 300
   Thr Glu Leu Thr Arg Asp Val Leu Thr Asp Pro Ile Val Gly Val Asn
   305                 310                 315                 320
   Asn Leu Arg Gly Tyr Gly Thr Thr Phe Ser Asn Ile Glu Asn Tyr Ile
                   325                 330                 335
   Arg Lys Pro His Leu Phe Asp Tyr Leu His Arg Ile Gln Phe His Thr
               340                 345                 350
   Arg Phe Gln Pro Gly Tyr Tyr Gly Asn Asp Ser Phe Asn Tyr Trp Ser
           355                 360                 365
   Gly Asn Tyr Val Ser Thr Arg Pro Ser Ile Gly Ser Asn Asp Ile Ile
       370                 375                 380
   Thr Ser Pro Phe Tyr Gly Asn Lys Ser Ser Glu Pro Val Gln Asn Leu
   385                 390                 395                 400
   Glu Phe Asn Gly Glu Lys Val Tyr Arg Ala Val Ala Asn Thr Asn Leu
                   405                 410                 415
   Ala Val Trp Pro Ser Ala Val Tyr Ser Gly Val Thr Lys Val Glu Phe
               420                 425                 430
   Ser Gln Tyr Asn Asp Gln Thr Asp Glu Ala Ser Thr Gln Thr Tyr Asp
           435                 440                 445
   Ser Lys Arg Asn Val Gly Ala Val Ser Trp Asp Ser Ile Asp Gln Leu
       450                 455                 460
   Pro Pro Glu Thr Thr Asp Glu Pro Leu Glu Lys Gly Tyr Ser His Gln
   465                 470                 475                 480
   Leu Asn Tyr Val Met Cys Phe Leu Met Gln Gly Ser Arg Gly Thr Ile
                   485                 490                 495
   Pro Val Leu Thr Trp Thr His Lys Ser Val Asp Phe Phe Asn Met Ile
               500                 505                 510
   Asp Ser Lys Lys Ile Thr Gln Leu Pro Leu Val Lys Ala Tyr Lys Leu
           515                 520                 525
   Gln Ser Gly Ala Ser Val Val Ala Gly Pro Arg Phe Thr Gly Gly Asp
       530                 535                 540
   Ile Ile Gln Cys Thr Glu Asn Gly Ser Ala Ala Thr Ile Tyr Val Thr
   545                 550                 555                 560
   Pro Asp Val Ser Tyr Ser Gln Lys Tyr Arg Ala Arg Ile His Tyr Ala
                   565                 570                 575
   Ser Thr Ser Gln Ile Thr Phe Thr Leu Ser Leu Asp Gly Ala Pro Phe
               580                 585                 590
   Asn Gln Tyr Tyr Phe Asp Lys Thr Ile Asn Lys Gly Asp Thr Leu Thr
           595                 600                 605
   Tyr Asn Ser Phe Asn Leu Ala Ser Phe Ser Thr Pro Phe Glu Leu Ser
       610                 615                 620
   Gly Asn Asn Leu Gln Ile Gly Val Thr Gly Leu Ser Ala Gly Asp Lys
   625                 630                 635                 640
   Val Tyr Ile Asp Lys Ile Glu Phe Ile Pro Val Asn
                   645                 650
<210> 36 .
   <211> 659
   <212> PRT
   <213> Bacillus thuringiensi
<400> 36
   Met Ile Arg Met Gly Gly Arg Lys Met Asn Pro Asn Asn Arg Ser Glu
    1               5                  10                  15
   Tyr Asp Thr Ile Lys Val Thr Pro Asn Ser Glu Leu Pro Thr Asn His
               20                  25                  30
   Asn Gln Tyr Pro Leu Ala Asp Asn Pro Asn Ser Thr Leu Glu Glu Leu
           35                  40                  45
   Asn Tyr Lys Glu Phe Leu Arg Met Thr Ala Asp Asn Ser Thr Glu Val
       50                  55                  60
   Leu Asp Ser Ser Thr Val Lys Asp Ala Val Gly Thr Gly Ile Ser Val
   65                  70                  75                  80
   Val Gly Gln Ile Leu Gly Val Val Gly Val Pro Phe Ala Gly Ala Leu
                   85                  90                  95
   Thr Ser Phe Tyr Gln Ser Phe Leu Asn Ala Ile Trp Pro Ser Asp Ala
               100                 105                 110
   Asp Pro Trp Lys Ala Phe Met Ala Gln Val Glu Val Leu Ile Asp Lys
           115                 120                 125
   Lys Ile Glu Glu Tyr Ala Lys Ser Lys Ala Leu Ala Glu Leu Gln Gly
           130                 135                 140
   Leu Gln Asn Asn Phe Glu Asp Tyr Val Asn Ala Leu Asp Ser Trp Lys
   145                 150                 155                 160
   Lys Ala Pro Val Asn Leu Arg Ser Arg Arg Ser Gln Asp Arg Ile Arg
                   165                 170                 175
   Glu Leu Phe Ser Gln Ala Glu Ser His Phe Arg Asn Ser Met Pro Ser
               180                 185                 190
   Phe Ala Val Ser Lys Phe Glu Val Leu Phe Leu Pro Thr Tyr Ala Gln
           195                 200                 205
   Ala Ala Asn Thr His Leu Leu Leu Leu Lys Asp Ala Gln Val Phe Gly
       210                 215                 220
   Glu Glu Trp Gly Tyr Ser Ser Glu Asp Ile Ala Glu Phe Tyr Gln Arg
   225                 230                 235                 240
   Gln Leu Lys Leu Thr Gln Gln Tyr Thr Asp His Cys Val Asn Trp Tyr
                   245                 250                 255
   Asn Val Gly Leu Asn Ser Leu Arg Gly Ser Thr Tyr Asp Ala Trp Val
               260                 265                 270
   Lys Phe Asn Arg Phe Arg Arg Glu Met Thr Leu Thr Val Leu Asp Leu
           275                 280                 285
   Ile Val Leu Phe Pro Phe Tyr Asp Val Arg Leu Tyr Ser Lys Gly Val
       290                 295                 300
   Lys Thr Glu Leu Thr Arg Asp Ile Phe Thr Asp Pro Ile Phe Thr Leu
   305                 310                 315                 320
   Asn Ala Leu Gln Glu Tyr Gly Pro Thr Phe Ser Ser Ile Glu Asn Ser
                   325                 330                 335
   Ile Arg Lys Pro His Leu Phe Asp Tyr Leu Arg Gly Ile Glu Phe His
               340                 345                 350
   Thr Arg Leu Arg Pro Gly Tyr Ser Gly Lys Asp Ser Phe Asn Tyr Trp
           355                 360                 365
   Ser Gly Asn Tyr Val Glu Thr Arg Pro Ser Ile Gly Ser Asn Asp Thr
       370                 375                 380
   Ile Thr Ser Pro Phe Tyr Gly Asp Lys Ser Ile Glu Pro Ile Gln Lys
   385                 390                 395                 400
   Leu Ser Phe Asp Gly Gln Lys Val Tyr Arg Thr Ile Ala Asn Thr Asp
                   405                 410                 415
   Ile Ala Ala Phe Pro Asp Gly Lys Ile Tyr Phe Gly Val Thr Lys Val
               420                 425                 430
   Asp Phe Ser Gln Tyr Asp Asp Gln Lys Asn Glu Thr Ser Thr Gln Thr
           435                 440                 445
   Tyr Asp Ser Lys Arg Tyr Asn Gly Tyr Leu Gly Ala Gln Asp Ser Ile
       450                 455                 460
   Asp Gln Leu Pro Pro Glu Thr Thr Asp Glu Pro Leu Glu Lys Ala Tyr
   465                 470                 475                 480
   Ser His Gln Leu Asn Tyr Ala Glu Cys Phe Leu Met Gln Asp Arg Arg
                   485                 490                 495
   Gly Thr Ile Pro Phe Phe Thr Trp Thr His Arg Ser Val Asp Phe Phe
               500                 505                 510
   Asn Thr Ile Asp Ala Glu Lys Ile Thr Gln Leu Pro Val Val Lys Ala
           515                 520                 525
   Tyr Ala Leu Ser Ser Gly Ala Ser Ile Ile Glu Gly Pro Gly Phe Thr
       530                 535                 540
   Gly Gly Asn Leu Leu Phe Leu Lys Glu Ser Ser Asn Ser Ile Ala Lys
   545                 550                 555                 560
   Phe Lys Val Thr Leu Asn Ser Ala Ala Leu Leu Gln Arg Tyr Arg Val
                   565                 570                 575
   Arg Ile Arg Tyr Ala Ser Thr Thr Asn Leu Arg Leu Phe Val Gln Asn
               580                 585                 590
   Ser Asn Asn Asp Phe Leu Val Ile Tyr Ile Asn Lys Thr Met Asn Ile
           595                 600                 605
   Asp Gly Asp Leu Thr Tyr Gln Thr Phe Asp Phe Ala Thr Ser Asn Ser
       610                 615                 620
   Asn Met Gly Phe Ser Gly Asp Thr Asn Asp Phe Ile Ile Gly Ala Glu
   625                 630                 635                 640
   Ser Phe Val Ser Asn Glu Lys Ile Tyr Ile Asp Lys Ile Glu Phe Ile
                   645                 650                 655
   Pro Val Gln
<210> 37
   <211> 652
   <212> PRT
   <213> Bacillus thuringiensi
<400> 37
   Met Asn Pro Asn Asn Arg Ser Glu His Asp Thr Ile Lys Val Thr Pro
   1               5                   10                  15
   Asn Ser Glu Leu Gln Thr Asn His Asn Gln Tyr Pro Leu Ala Asp Asn
               20                  25                  30
   Pro Asn Ser Thr Leu Glu Glu Leu Asn Tyr Lys Glu Phe Leu Arg Met
           35                  40                  45
   Thr Glu Asp Ser Ser Thr Glu Val Leu Asp Asn Ser Thr Val Lys Asp
       50                  55                  60
   Ala Val Gly Thr Gly Ile Ser Val Val Gly Gln Ile Leu Gly Val Val
   65                  70                  75                  80
   Gly Val Pro Phe Ala Gly Ala Leu Thr Ser Phe Tyr Gln Ser Phe Leu
                   85                  90                  95
   Asn Thr Ile Trp Pro Ser Asp Ala Asp Pro Trp Lys Ala Phe Met Ala
               100                 105                 110
   Gln Val Glu Val Leu Ile Asp Lys Lys Ile Glu Glu Tyr Ala Lys Ser
           115                 120                 125
   Lys Ala Leu Ala Glu Leu Gln Gly Leu Gln Asn Asn Phe Glu Asp Tyr
       130                 135                 140
   Val Asn Ala Leu Asn Ser Trp Lys Lys Thr Pro Leu Ser Leu Arg Ser
   145                 150                 155                 160
   Lys Arg Ser Gln Asp Arg Ile Arg Glu Leu Phe Ser Gln Ala Glu Ser
                   165                 170                 175
   His Phe Arg Asn Ser Met Pro Ser Phe Ala Val Ser Lys Phe Glu Val
               180                 185                 190
   Leu Phe Leu Pro Thr Tyr Ala Gln Ala Ala Asn Thr His Leu Leu Leu
           195                 200                 205
   Leu Lys Asp Ala Gln Val Phe Gly Glu Glu Trp Gly Tyr Ser Ser Glu
       210                 215                 220
   Asp Val Ala Glu Phe Tyr His Arg Gln Leu Lys Leu Thr Gln Gln Tyr
   225                 230                 235                 240
   Thr Asp His Cys Val Asn Trp Tyr Asn Val Gly Leu Asn Gly Leu Arg
                   245                 250                 255
   Gly Ser Thr Tyr Asp Ala Trp Val Lys Phe Asn Arg Phe Arg Arg Glu
               260                 265                 270
   Met Thr Leu Thr Val Leu Asp Leu Ile Val Leu Phe Pro Phe Tyr Asp
           275                 280                 285
   Ile Arg Leu Tyr Ser Lys Gly Val Lys Thr Glu Leu Thr Arg Asp Ile
       290                 295                 300
   Phe Thr Asp Pro Ile Phe Ser Leu Asn Thr Leu Gln Glu Tyr Gly Pro
   305                 310                 315                 320
   Thr Phe Leu Ser Ile Glu Asn Ser Ile Arg Lys Pro His Leu Phe Asp
                   325                 330                 335
   Tyr Leu Gln Gly Ile Glu Phe His Thr Arg Leu Gln Pro Gly Tyr Phe
               340                 345                 350
   Gly Lys Asp Ser Phe Asn Tyr Trp Ser Gly Asn Tyr Val Glu Thr Arg
           355                 360                 365
   Pro Ser Ile Gly Ser Ser Lys Thr Ile Thr Ser Pro Phe Tyr Gly Asp
       370                 375                 380
   Lys Ser Thr Glu Pro Val Gln Lys Leu Ser Phe Asp Gly Gln Lys Val
   385                 390                 395                 400
   Tyr Arg Thr Ile Ala Asn Thr Asp Val Ala Ala Trp Pro Asn Gly Lys
                   405                 410                 415
   Val Tyr Leu Gly Val Thr Lys Val Asp Phe Ser Gln Tyr Asp Asp Gln
               420                 425                 430
   Lys Asn Glu Thr Ser Thr Gln Thr Tyr Asp Ser Lys Arg Asn Asn Gly
           435                 440                 445
   His Val Ser Ala Gln Asp Ser Ile Asp Gln Leu Pro Pro Glu Thr Thr
       450                 455                 460
   Asp Glu Pro Leu Glu Lys Ala Tyr Ser His Gln Leu Asn Tyr Ala Glu
   465                 470                 475                 480
   Cys Phe Leu Met Gln Asp Arg Arg Gly Thr Ile Pro Phe Phe Thr Trp
                           485                                     490                                     495
   Thr His Arg Ser Val Asp Phe Phe Asn Thr Ile Asp Ala Glu Lys Ile
                   500                                     505                                     510
   Thr Gln Leu Pro Val Val Lys Ala Tyr Ala Leu Ser Ser Gly Ala Ser
           515                                     520                                     525
   Ile Ile Glu Gly Pro Gly Phe Thr Gly Gly Asn Leu Leu Phe Leu Lys
       530                                     535                                     540
   Glu Ser Ser Asn Ser Ile Ala Lys Phe Lys Val Thr Leu Asn Ser Ala
   545                                     550                                     555                                     560
   Ala Leu Leu Gln Arg Tyr Arg Val Arg Ile Arg Tyr Ala Ser Thr Thr
                           565                                     570                                     575
   Asn Leu Arg Leu Phe Val Gln Asn Ser Asn Asn Asp Phe Leu Val Ile
                   580                                     585                                     590
   Tyr Ile Asn Lys Thr Met Asn Lys Asp Asp Asp Leu Thr Tyr Gln Thr
           595                                     600                                     605
   Phe Asp Leu Ala Thr Thr Asn Ser Asn Met Gly Phe Ser Gly Asp Lys
       610                                     615                                     620
   Asn Glu Leu Ile Ile Gly Ala Glu Ser Phe Val Ser Asn Glu Lys Ile
   625                                     630                                     635                                     640
   Tyr Ile Asp Lys Ile Glu Phe Ile Pro Val Gln Leu
                           645                                     650
<210> 38
   <211> 1180
   <212> PRT
   <213> Bacillus thuringiensi
<400> 38
   Met Asn Pro Tyr Gln Asn Lys Asn Glu Tyr Glu Thr Leu Asn Ala Ser
   1 5 10 15
   Gln Lys Lys Leu Asn Ile Ser Asn Asn Tyr Thr Arg Tyr Pro Ile Glu
   20 25 30
   Asn Ser Pro Lys Gln Leu Leu Gln Ser Thr Asn Tyr Lys Asp Trp Leu
   35 40 45
   Asn Met Cys Gln Gln Asn Gln Gln Tyr Gly Gly Asp Phe Glu Thr Phe
   50 55 60
   Ile Asp Ser Gly Glu Leu Ser Ala Tyr Thr Ile Val Val Gly Thr Val
   65 70 75 80
   Leu Thr Gly Phe Gly Phe Thr Thr Pro Leu Gly Leu Ala Leu Ile Gly
   85 90 95
   Phe Gly Thr Leu Ile Pro Val Leu Phe Pro Ala Gln Asp Gln Ser Asn
   100 105 110
   Thr Trp Ser Asp Phe Ile Thr Gln Thr Lys Asn Ile Ile Lys Lys Glu
   115 120 125
   Ile Ala Ser Thr Tyr Ile Ser Asn Ala Asn Lys Ile Leu Asn Arg Ser
   130 135 140
   Phe Asn Val Ile Ser Thr Tyr His Asn His Leu Lys Thr Trp Glu Asn
   145 150 155 160
   Asn Pro Asn Pro Gln Asn Thr Gln Asp Val Arg Thr Gln Ile Gln Leu
   165 170 175
   Val His Tyr His Phe Gln Asn Val Ile Pro Glu Leu Val Asn Ser Cys
   180 185 190
   Pro Pro Asn Pro Ser Asp Cys Asp Tyr Tyr Asn Ile Leu Val Leu Ser
   195 200 205
   Ser Tyr Ala Gln Ala Ala Asn Leu His Leu Thr Val Leu Asn Gln Ala
   210 215 220
   Val Lys Phe Glu Ala Tyr Leu Lys Asn Asn Arg Gln Phe Asp Tyr Leu
   225 230 235 240
   Glu Pro Leu Pro Thr Ala Ile Asp Tyr Tyr Pro Val Leu Thr Lys Ala
   245 250 255
   Ile Glu Asp Tyr Thr Asn Tyr Cys Val Thr Thr Tyr Lys Lys Gly Leu
   260 265 270
   Asn Leu Ile Lys Thr Thr Pro Asp Ser Asn Leu Asp Gly Asn Ile Asn
   275 280 285
   Trp Asn Thr Tyr Asn Thr Tyr Arg Thr Lys Met Thr Thr Ala Val Leu
   290 295 300
   Asp Leu Val Ala Leu Phe Pro Asn Tyr Asp Val Gly Lys Tyr Pro Ile
   305 310 315 320
   Gly Val Gln Ser Glu Leu Thr Arg Glu Ile Tyr Gln Val Leu Asn Phe
   325 330 335
   Glu Glu Ser Pro Tyr Lys Tyr Tyr Asp Phe Gln Tyr Gln Glu Asp Ser
   340 345 350
   Leu Thr Arg Arg Pro His Leu Phe Thr Trp Leu Asp Ser Leu Asn Phe
   355 360 365
   Tyr Glu Lys Ala Gln Thr Thr Pro Asn Asn Phe Phe Thr Ser His Tyr
   370 375 380
   Asn Met Phe His Tyr Thr Leu Asp Asn Ile Ser Gln Lys Ser Ser Val
   385 390 395 400
   Phe Gly Asn His Asn Val Thr Asp Lys Leu Lys Ser Leu Gly Leu Ala
   405 410 415
   Thr Asn Ile Tyr Ile Phe Leu Leu Asn Val Ile Ser Leu Asp Asn Lys
   420 425 430
   Tyr Leu Asn Asp Tyr Asn Asn Ile Ser Lys Met Asp Phe Phe Ile Thr
   435 440 445
   Asn Gly Thr Arg Leu Leu Glu Lys Glu Leu Thr Ala Gly Ser Gly Gln
   450 455 460
   Ile Thr Tyr Asp Val Asn Lys Asn Ile Phe Gly Leu Pro Ile Leu Lys
   465 470 475 480
   Arg Arg Glu Asn Gln Gly Asn Pro Thr Leu Phe Pro Thr Tyr Asp Asn
   485 490 495
   Tyr Ser His Ile Leu Ser Phe Ile Lys Ser Leu Ser Ile Pro Ala Thr
   500 505 510
   Tyr Lys Thr Gln Val Tyr Thr Phe Ala Trp Thr His Ser Ser Val Asp
   515 520 525
   Pro Lys Asn Thr Ile Tyr Thr His Leu Thr Thr Gln Ile Pro Ala Val
   530 535 540
   Lys Ala Asn Ser Leu Gly Thr Ala Ser Lys Val Val Gln Gly Pro Gly
   545 550 555 560
   His Thr Gly Gly Asp Leu Ile Asp Phe Lys Asp His Phe Lys Ile Thr
   565 570 575
   Cys Gln His Ser Asn Phe Gln Gln Ser Tyr Phe Ile Arg Ile Arg Tyr
   580 585 590
   Ala Ser Asn Gly Ser Ala Asn Thr Arg Ala Val Ile Asn Leu Ser Ile
   595 600 605
   Pro Gly Val Ala Glu Leu Gly Met Ala Leu Asn Pro Thr Phe Ser Gly
   610 615 620
   Thr Asp Tyr Thr Asn Leu Lys Tyr Lys Asp Phe Gln Tyr Leu Glu Phe
   625 630 635 640
   Ser Asn Glu Val Lys Phe Ala Pro Asn Gln Asn Ile Ser Leu Val Phe
   645 650 655
   Asn Arg Ser Asp Val Tyr Thr Asn Thr Thr Val Leu Ile Asp Lys Ile
   660 665 670
   Glu Phe Leu Pro Ile Thr Arg Ser Ile Arg Glu Asp Arg Glu Lys Gln
   675 680 685
   Lys Leu Glu Thr Val Gln Gln Ile Ile Asn Thr Phe Tyr Ala Asn Pro
   690 695 700
   Ile Lys Asn Thr Leu Gln Ser Glu Leu Thr Asp Tyr Asp Ile Asp Gln
   705 710 715 720
   Ala Ala Asn Leu Val Glu Cys Ile Ser Glu Glu Leu Tyr Pro Lys Glu
   725 730 735
   Lys Met Leu Leu Leu Asp Glu Val Lys Asn Ala Lys Gln Leu Ser Gln
   740 745 750
   Ser Arg Asn Val Leu Gln Asn Gly Asp Phe Glu Ser Ala Thr Leu Gly
   755 760 765
   Trp Thr Thr Ser Asp Asn Ile Thr Ile Gln Glu Asp Asp Pro Ile Phe
   770 775 780
   Lys Gly His Tyr Leu His Met Ser Gly Ala Arg Asp Ile Asp Gly Thr
   785 790 795 800
   Ile Phe Pro Thr Tyr Ile Phe Gln Lys Ile Asp Glu Ser Lys Leu Lys
   805 810 815
   Pro Tyr Thr Arg Tyr Leu Val Arg Gly Phe Val Gly Ser Ser Lys Asp
   820 825 830
   Val Glu Leu Val Val Ser Arg Tyr Gly Glu Glu Ile Asp Ala Ile Met
   835 840 845
   Asn Val Pro Ala Asp Leu Asn Tyr Leu Tyr Pro Ser Thr Phe Asp Cys
   850 855 860
   Glu Gly Ser Asn Arg Cys Glu Thr Ser Ala Val Pro Ala Asn Ile Gly
   865 870 875 880
   Asn Thr Ser Asp Met Leu Tyr Ser Cys Gln Tyr Asp Thr Gly Lys Lys
   885 890 895
   His Val Val Cys Gln Asp Ser His Gln Phe Ser Phe Thr Ile Asp Thr
   900 905 910
   Gly Ala Leu Asp Thr Asn Glu Asn Ile Gly Val Trp Val Met Phe Lys
   915 920 925
   Ile Ser Ser Pro Asp Gly Tyr Ala Ser Leu Asp Asn Leu Glu Val Ile
   930 935 940
   Glu Glu Gly Pro Ile Asp Gly Glu Ala Leu Ser Arg Val Lys His Met
   945 950 955 960
   Glu Lys Lys Trp Asn Asp Gln Met Glu Ala Lys Arg Ser Glu Thr Gln
   965 970 975
   Gln Ala Tyr Asp Val Ala Lys Gln Ala Ile Asp Ala Leu Phe Thr Asn
   980 985 990
   Val Gln Asp Glu Ala Leu Gln Phe Asp Thr Thr Leu Ala Gln Ile Gln
   995 1000 1005
   Tyr Ala Glu Tyr Leu Val Gln Ser Ile Pro Tyr Val Tyr Asn Asp Trp
   1010 1015 1020
   Leu Ser Asp Val Pro Gly Met Asn Tyr Asp Ile Tyr Val Glu Leu Asp
   1025 1030 1035 1040
   Ala Arg Val Ala Gln Ala Arg Tyr Leu Tyr Asp Thr Arg Asn Ile Ile
   1045 1050 1055
   Lys Asn Gly Asp Phe Thr Gln Gly Val Met Gly Trp His Val Thr Gly
   1060 1065 1070
   Asn Ala Asp Val Gln Gln Ile Asp Gly Val Ser Val Leu Val Leu Ser
   1075 1080 1085
   Asn Trp Ser Ala Gly Val Ser Gln Asn Val His Leu Gln His Asn His
   1090 1095 1100
   Gly Tyr Val Leu Arg Val Ile Ala Lys Lys Glu Gly Pro Gly Asn Gly
   1105 1110 1115 1120
   Tyr Val Thr Leu Met Asp Cys Glu Glu Asn Gln Glu Lys Leu Thr Phe
   1125 1130 1135
   Thr Ser Cys Glu Glu Gly Tyr Ile Thr Lys Thr Val Asp Val Phe Pro
   1140 1145 1150
   Asp Thr Asp Arg Val Arg Ile Glu Ile Gly Glu Thr Glu Gly Ser Phe
   1155 1160 1165
   Tyr Ile Glu Ser Ile Glu Leu Ile Cys Met Asn Glu
   1170 1175 1180
<210> 39
   <211> 1136
   <212> PRT
   <213> Bacillus thuringiensi
<400> 39
   Met Asn Ser Gly Tyr Pro Leu Ala Asn Asp Leu Gln Gly Ser Met Lys
    1               5                  10                  15
   Asn Thr Asn Tyr Lys Asp Trp Leu Ala Met Cys Glu Asn Asn Gln Gln
               20                  25                  30
   Tyr Gly Val Asn Pro Ala Ala Ile Asn Ser Ser Ser Val Ser Thr Ala
           35                  40                  45
   Leu Lys Val Ala Gly Ala Ile Leu Lys Phe Val Asn Pro Pro Ala Gly
       50                  55                  60
   Thr Val Leu Thr Val Leu Ser Ala Val Leu Pro Ile Leu Trp Pro Thr
   65                  70                  75                  80
   Asn Thr Pro Thr Pro Glu Arg Val Trp Asn Asp Phe Met Thr Asn Thr
                   85                  90                  95
   Gly Asn Leu Ile Asp Gln Thr Val Thr Ala Tyr Val Arg Thr Asp Ala
               100                 105                 110
   Asn Ala Lys Met Thr Val Val Lys Asp Tyr Leu Asp Gln Tyr Thr Thr
           115                 120                 125
   Lys Phe Asn Thr Trp Lys Arg Glu Pro Asn Asn Gln Ser Tyr Arg Thr
       130                 135                 140
   Ala Val Ile Thr Gln Phe Asn Leu Thr Ser Ala Lys Leu Arg Glu Thr
   145                 150                 155                 160
   Ala Val Tyr Phe Ser Asn Leu Val Gly Tyr Glu Leu Leu Leu Leu Pro
                   165                 170                 175
   Ile Tyr Ala Gln Val Ala Asn Phe Asn Leu Leu Leu Ile Arg Asp Gly
               180                 185                 190
   Leu Ile Asn Ala Gln Glu Trp Ser Leu Ala Arg Ser Ala Gly Asp Gln
           195                 200                 205
   Leu Tyr Asn Thr Met Val Gln Tyr Thr Lys Glu Tyr Ile Ala His Ser
       210                 215                 220
   Ile Thr Trp Tyr Asn Lys Gly Leu Asp Val Leu Arg Asn Lys Ser Asn
   225                 230                 235                 240
   Gly Gln Trp Ile Thr Phe Asn Asp Tyr Lys Arg Glu Met Thr Ile Gln
                   245                 250                 255
   Val Leu Asp Ile Leu Ala Leu Phe Ala Ser Tyr Asp Pro Arg Arg Tyr
               260                 265                 270
   Pro Ala Asp Lys Ile Asp Asn Thr Lys Leu Ser Lys Thr Glu Phe Thr
           275                 280                 285
   Arg Glu Ile Tyr Thr Ala Leu Val Glu Ser Pro Ser Ser Lys Ser Ile
       290                 295                 300
   Ala Ala Leu Glu Ala Ala Leu Thr Arg Asp Val His Leu Phe Thr Trp
   305                 310                 315                 320
   Leu Lys Arg Val Asp Phe Trp Thr Asn Thr Ile Tyr Gln Asp Leu Arg
                   325                 330                 335
   Phe Leu Ser Ala Asn Lys Ile Gly Phe Ser Tyr Thr Asn Ser Ser Ala
               340                 345                 350
   Met Gln Glu Ser Gly Ile Tyr Gly Ser Ser Gly Phe Gly Ser Asn Leu
           355                 360                 365
   Thr His Gln Ile Gln Leu Asn Ser Asn Val Tyr Lys Thr Ser Ile Thr
       370                 375                 380
   Asp Thr Ser Ser Pro Ser Asn Arg Val Thr Lys Met Asp Phe Tyr Lys
   385                 390                 395                 400
   Ile Asp Gly Thr Leu Ala Ser Tyr Asn Ser Asn Ile Thr Pro Thr Pro
                   405                 410                 415
   Glu Gly Leu Arg Thr Thr Phe Phe Gly Phe Ser Thr Asn Glu Asn Thr
               420                 425                 430
   Pro Asn Gln Pro Thr Val Asn Asp Tyr Thr His Ile Leu Ser Tyr Ile
           435                 440                 445
   Lys Thr Asp Val Ile Asp Tyr Asn Ser Asn Arg Val Ser Phe Ala Trp
       450                 455                 460
   Thr His Lys Ile Val Asp Pro Asn Asn Gln Ile Tyr Thr Asp Ala Ile
   465                 470                 475                 480
   Thr Gln Val Pro Ala Val Lys Ser Asn Phe Leu Asn Ala Thr Ala Lys
                   485                 490                 495
   Val Ile Lys Gly Pro Gly His Thr Gly Gly Asp Leu Val Ala Leu Thr
               500                 505                 510
   Ser Asn Gly Thr Leu Ser Gly Arg Met Glu Ile Gln Cys Lys Thr Ser
           515                 520                 525
   Ile Phe Asn Asp Pro Thr Arg Ser Tyr Gly Leu Arg Ile Arg Tyr Ala
       530                 535                 540
   Ala Asn Ser Pro Ile Val Leu Asn Val Ser Tyr Val Leu Gln Gly Val
   545                 550                 555                 560
   Ser Arg Gly Thr Thr Ile Ser Thr Glu Ser Thr Phe Ser Arg Pro Asn
                   565                 570                 575
   Asn Ile Ile Pro Thr Asp Leu Lys Tyr Glu Glu Phe Arg Tyr Lys Asp
               580                 585                 590
   Pro Phe Asp Ala Ile Val Pro Met Arg Leu Ser Ser Asn Gln Leu Ile
           595                 600                 605
   Thr Ile Ala Ile Gln Pro Leu Asn Met Thr Ser Asn Asn Gln Val Ile
       610                 615                 620
   Ile Asp Arg Ile Glu Ile Ile Pro Ile Thr Gln Ser Val Leu Asp Glu
   625                 630                 635                 640
   Thr Glu Asn Gln Asn Leu Glu Ser Glu Arg Glu Val Val Asn Ala Leu
                   645                 650                 655
   Phe Thr Asn Asp Ala Lys Asp Ala Leu Asn Ile Gly Thr Thr Asp Tyr
               660                 665                 670
   Asp Ile Asp Gln Ala Ala Asn Leu Val Glu Cys Ile Ser Glu Glu Leu
           675                 680                 685
   Tyr Pro Lys Glu Lys Met Leu Leu Leu Asp Glu Val Lys Asn Ala Lys
           690                 695                 700
   Gln Leu Ser Gln Ser Arg Asn Val Leu Gln Asn Gly Asp Phe Glu Ser
   705                 710                 715                 720
   Ala Thr Leu Gly Trp Thr Thr Ser Asp Asn Ile Thr Ile Gln Glu Asp
                   725                 730                 735
   Asp Pro Ile Phe Lys Gly His Tyr Leu His Met Ser Gly Ala Arg Asp
               740                 745                 750
   Ile Asp Gly Thr Ile Phe Pro Thr Tyr Ile Phe Gln Lys Ile Asp Glu
           755                 760                 765
   Ser Lys Leu Lys Pro Tyr Thr Arg Tyr Leu Val Arg Gly Phe Val Gly
       770                 775                 780
   Ser Ser Lys Asp Val Glu Leu Val Val Ser Arg Tyr Gly Glu Glu Ile
   785                 790                 795                 800
   Asp Ala Ile Met Asn Val Pro Ala Asp Leu Asn Tyr Leu Tyr Pro Ser
                   805                 810                 815
   Thr Phe Asp Cys Glu Gly Ser Asn Arg Cys Glu Thr Ser Ala Val Pro
               820                 825                 830
   Ala Asn Ile Gly Asn Thr Ser Asp Met Leu Tyr Ser Cys Gln Tyr Asp
           835                 840                 845
   Thr Gly Lys Lys His Val Val Cys Gln Asp Ser His Gln Phe Ser Phe
       850                 855                 860
   Thr Ile Asp Thr Gly Ala Leu Asp Thr Asn Glu Asn Ile Gly Val Trp
   865                 870                 875                 880
   Val Met Phe Lys Ile Ser Ser Pro Asp Gly Tyr Ala Ser Leu Asp Asn
                   885                 890                 895
   Leu Glu Val Ile Glu Glu Gly Pro Ile Asp Gly Glu Ala Leu Ser Arg
               900                 905                 910
   Val Lys His Met Glu Lys Lys Trp Asn Asp Gln Met Glu Ala Lys Arg
           915                 920                 925
   Ser Glu Thr Gln Gln Ala Tyr Asp Val Ala Lys Gln Ala Ile Asp Ala
       930                 935                 940
   Leu Phe Thr Asn Val Gln Asp Glu Ala Leu Gln Phe Asp Thr Thr Leu
   945                 950                 955                 960
   Ala Gln Ile Gln Tyr Ala Glu Tyr Leu Val Gln Ser Ile Pro Tyr Val
                   965                 970                 975
   Tyr Asn Asp Trp Leu Ser Asp Val Pro Gly Met Asn Tyr Asp Ile Tyr
               980                 985                 990
   Val Glu Leu Asp Ala Arg Val Ala Gln Ala Arg Tyr Leu Tyr Asp Thr
           995                 1000                1005
   Arg Asn Ile Ile Lys Asn Gly Asp Phe Thr Gln Gly Val Met Gly Trp
       1010                1015                1020
   His Val Thr Gly Asn Ala Asp Val Gln Gln Ile Asp Gly Val Ser Val
   1025                1030                1035                1040
   Leu Val Leu Ser Asn Trp Ser Ala Gly Val Ser Gln Asn Val His Leu
                   1045                1050                1055
   Gln His Asn His Gly Tyr Val Leu Arg Val Ile Ala Lys Lys Glu Gly
               1060                1065                1070
   Pro Gly Asn Gly Tyr Val Thr Leu Met Asp Cys Glu Glu Asn Gln Glu
           1075                1080                1085
   Lys Leu Thr Phe Thr Ser Cys Glu Glu Gly Tyr Ile Thr Lys Thr Val
       1090                1095                1100
   Asp Val Phe Pro Asp Thr Asp Arg Val Arg Ile Glu Ile Gly Glu Thr
   1105                1110                1115                1120
   Glu Gly Ser Phe Tyr Ile Glu Ser Ile Glu Leu Ile Cys Met Asn Glu
                   1125                1130                1135
<210> 40
   <211> 475
   <212> PRT
   <213> Bacillus thuringiensi
<400> 40
   Met Ile Ile Asp Ser Lys Thr Thr Leu Pro Arg His Ser Leu Ile His
   1               5                   10                  15
   Thr Ile Lys Leu Asn Ser Asn Lys Lys Tyr Gly Pro Gly Asp Met Thr
               20                  25                  30
   Asn Gly Asn Gln Phe Ile Ile Ser Lys Gln Glu Trp Ala Thr Ile Gly
           35                  40                  45
   Ala Tyr Ile Gln Thr Gly Leu Gly Leu Pro Val Asn Glu Gln Gln Leu
       50                  55                  60
   Arg Thr His Val Asn Leu Ser Gln Asp Ile Ser Ile Pro Ser Asp Phe
   65                  70                  75                  80
   Ser Gln Leu Tyr Asp Val Tyr Cys Ser Asp Lys Thr Ser Ala Glu Trp
                   85                  90                  95
   Trp Asn Lys Asn Leu Tyr Pro Leu Ile Ile Lys Ser Ala Asn Asp Ile
               100                 105                 110
   Ala Ser Tyr Gly Phe Lys Val Ala Gly Asp Pro Ser Ile Lys Lys Asp
           115                 120                 125
   Gly Tyr Phe Lys Lys Leu Gln Asp Glu Leu Asp Asn Ile Val Asp Asn
       130                 135                 140
   Asn Ser Asp Asp Asp Ala Ile Ala Lys Ala Ile Lys Asp Phe Lys Ala
   145                 150                 155                 160
   Arg Cys Gly Ile Leu Ile Lys Glu Ala Lys Gln Tyr Glu Glu Ala Ala
                   165                 170                 175
   Lys Asn Ile Val Thr Ser Leu Asp Gln Phe Leu His Gly Asp Gln Lys
               180                 185                 190
   Lys Leu Glu Gly Val Ile Asn Ile Gln Lys Arg Leu Lys Glu Val Gln
           195                 200                 205
   Thr Ala Leu Asn Gln Ala His Gly Glu Ser Ser Pro Ala His Lys Glu
       210                 215                 220
   Leu Leu Glu Lys Val Lys Asn Leu Lys Thr Thr Leu Glu Arg Thr Ile
   225                 230                 235                 240
   Lys Ala Glu Gln Asp Leu Glu Lys Lys Val Glu Tyr Ser Phe Leu Leu
                   245                 250                 255
   Gly Pro Leu Leu Gly Phe Val Val Tyr Glu Ile Leu Glu Asn Thr Ala
               260                 265                 270
   Val Gln His Ile Lys Asn Gln Ile Asp Glu Ile Lys Lys Gln Leu Asp
           275                 280                 285
   Ser Ala Gln His Asp Leu Asp Arg Asp Val Lys Ile Ile Gly Met Leu
       290                 295                 300
   Asn Ser Ile Asn Thr Asp Ile Asp Asn Leu Tyr Ser Gln Gly Gln Glu
   305                 310                 315                 320
   Ala Ile Lys Val Phe Gln Lys Leu Gln Gly Ile Trp Ala Thr Ile Gly
                   325                 330                 335
   Ala Gln Ile Glu Asn Leu Arg Thr Thr Ser Leu Gln Glu Val Gln Asp
               340                 345                 350
   Ser Asp Asp Ala Asp Glu Ile Gln Ile Glu Leu Glu Asp Ala Ser Asp
           355                 360                 365
   Ala Trp Leu Val Val Ala Gln Glu Ala Arg Asp Phe Thr Leu Asn Ala
       370                 375                 380
   Tyr Ser Thr Asn Ser Arg Gln Asn Leu Pro Ile Asn Val Ile Ser Asp
   385                 390                 395                 400
   Ser Cys Asn Cys Ser Thr Thr Asn Met Thr Ser Asn Gln Tyr Ser Asn
                   405                 410                 415
   Pro Thr Thr Asn Met Thr Ser Asn Gln Tyr Met Ile Ser His Glu Tyr
               420                 425                 430
   Thr Ser Leu Pro Asn Asn Phe Met Leu Ser Arg Asn Ser Asn Leu Glu
           435                 440                 445
   Tyr Lys Cys Pro Glu Asn Asn Phe Met Ile Tyr Trp Tyr Asn Asn Ser
       450                 455                 460
   Asp Trp Tyr Asn Asn Ser Asp Trp Tyr Asn Asn
   465                 470                 475
<210> 41
   <211> 1138
   <212> PRT
   <213> Bacillus thuringiensi
<400> 41
   Met Asn Leu Asn Asn Leu Asp Gly Tyr Glu Asp Ser Asn Arg Thr Leu
   1               5                   10                  15
   Asn Asn Ser Leu Asn Tyr Pro Thr Gln Lys Ala Leu Ser Pro Ser Leu
               20                  25                  30
   Lys Asn Met Asn Tyr Gln Asp Phe Leu Ser Ile Thr Glu Arg Glu Gln
           35                  40                  45
   Pro Glu Ala Leu Ala Ser Gly Asn Thr Ala Ile Asn Thr Val Val Ser
       50                  55                  60
   Val Thr Gly Ala Thr Leu Ser Ala Leu Gly Val Pro Gly Ala Ser Phe
   65                  70                  75                  80
   Ile Thr Asn Phe Tyr Leu Lys Ile Ala Gly Leu Leu Trp Pro Glu Asn
                   85                  90                  95
   Gly Lys Ile Trp Asp Glu Phe Met Thr Glu Val Glu Ala Leu Ile Asp
               100                 105                 110
   Gln Lys Ile Glu Glu Tyr Val Arg Asn Lys Ala Ile Ala Glu Leu Asp
           115                 120                 125
   Gly Leu Gly Ser Ala Leu Asp Lys Tyr Gln Lys Ala Leu Ala Asp Trp
       130                 135                 140
   Leu Gly Lys Gln Asp Asp Pro Glu Ala Ile Leu Ser Val Ala Thr Glu
   145                 150                 155                 160
   Phe Arg Ile Ile Asp Ser Leu Phe Glu Phe Ser Met Pro Ser Phe Lys
                   165                 170                 175
   Val Thr Gly Tyr Glu Ile Pro Leu Leu Thr Val Tyr Ala Gln Ala Ala
               180                 185                 190
   Asn Leu His Leu Ala Leu Leu Arg Asp Ser Thr Leu Tyr Gly Asp Lys
           195                 200                 205
   Trp Gly Phe Thr Gln Asn Asn Ile Glu Glu Asn Tyr Asn Arg Gln Lys
       210                 215                 220
   Lys Arg Ile Ser Glu Tyr Ser Asp His Cys Thr Lys Trp Tyr Asn Ser
   225                 230                 235                 240
   Gly Leu Ser Arg Leu Asn Gly Ser Thr Tyr Glu Gln Trp Ile Asn Tyr
                   245                 250                 255
   Asn Arg Phe Arg Arg Glu Met Ile Leu Met Ala Leu Asp Leu Val Ala
               260                 265                 270
   Val Phe Pro Phe His Asp Pro Arg Arg Tyr Ser Met Glu Thr Ser Thr
           275                 280                 285
   Gln Leu Thr Arg Glu Val Tyr Thr Asp Pro Val Ser Leu Ser Ile Ser
       290                 295                 300
   Asn Pro Asp Ile Gly Pro Ser Phe Ser Gln Met Glu Asn Thr Ala Ile
   305                 310                 315                 320
   Arg Thr Pro His Leu Val Asp Tyr Leu Asp Glu Leu Tyr Ile Tyr Thr
                   325                 330                 335
   Ser Lys Tyr Lys Ala Phe Ser His Glu Ile Gln Pro Asp Leu Phe Tyr
               340                 345                 350
   Trp Ser Ala His Lys Val Ser Phe Lys Lys Ser Glu Gln Ser Asn Leu
           355                 360                 365
   Tyr Thr Thr Gly Ile Tyr Gly Lys Thr Ser Gly Tyr Ile Ser Ser Gly
       370                 375                 380
   Ala Tyr Ser Phe His Gly Asn Asp Ile Tyr Arg Thr Leu Ala Ala Pro
   385                 390                 395                 400
   Ser Val Val Val Tyr Pro Tyr Thr Gln Asn Tyr Gly Val Glu Gln Val
                   405                 410                 415
   Glu Phe Tyr Gly Val Lys Gly His Val His Tyr Arg Gly Asp Asn Lys
               420                 425                 430
   Tyr Asp Leu Thr Tyr Asp Ser Ile Asp Gln Leu Pro Pro Asp Gly Glu
           435                 440                 445
   Pro Ile His Glu Lys Tyr Thr His Arg Leu Cys His Ala Thr Ala Ile
       450                 455                 460
   Phe Lys Ser Thr Pro Asp Tyr Asp Asn Ala Thr Ile Pro Ile Phe Ser
   465                 470                 475                 480
   Trp Thr His Arg Ser Ala Glu Tyr Tyr Asn Arg Ile Tyr Pro Asn Lys
                   485                 490                 495
   Ile Thr Lys Ile Pro Ala Val Lys Met Tyr Lys Leu Asp Asp Pro Ser
               500                 505                 510
   Thr Val Val Lys Gly Pro Gly Phe Thr Gly Gly Asp Leu Val Lys Arg
           515                 520                 525
   Gly Ser Thr Gly Tyr Ile Gly Asp Ile Lys Ala Thr Val Asn Ser Pro
       530                 535                 540
   Leu Ser Gln Lys Tyr Arg Val Arg Val Arg Tyr Ala Thr Asn Val Ser
   545                 550                 555                 560
   Gly Gln Phe Asn Val Tyr Ile Asn Asp Lys Ile Thr Leu Gln Thr Lys
                   565                 570                 575
   Phe Gln Asn Thr Val Glu Thr Ile Gly Glu Gly Lys Asp Leu Thr Tyr
                           580                           585                           590
   Gly Ser Phe Gly Tyr Ile Glu Tyr Ser Thr Thr Ile Gln Phe Pro Asp
               595                     600                     605
   Glu His Pro Lys Ile Thr Leu His Leu Ser Asp Leu Ser Asn Asn Ser
       610                     615                     620
   Ser Phe Tyr Val Asp Ser Ile Glu Phe Ile Pro Val Asp Val Asn Tyr
   625                     630                     635                     640
   Ala Glu Lys Glu Lys Leu Glu Lys Ala Gln Lys Ala Val Asn Thr Leu
                   645                     650                     655
   Phe Thr Glu Gly Arg Asn Ala Leu Gln Lys Asp Val Thr Asp Tyr Lys
               660                     665                     670
   Val Asp Gln Val Ser Ile Leu Val Asp Cys Ile Ser Gly Asp Leu Tyr
           675                     680                     685
   Pro Asn Glu Lys Arg Glu Leu Gln Asn Leu Val Lys Tyr Ala Lys Arg
       690                     695                     700
   Leu Ser Tyr Ser Arg Asn Leu Leu Leu Asp Pro Thr Phe Asp Ser Ile
   705                     710                     715                     720
   Asn Ser Ser Glu Glu Asn Gly Trp Tyr Gly Ser Asn Gly Ile Val Ile
                   725                     730                     735
   Gly Asn Gly Asp Phe Val Phe Lys Gly Asn Tyr Leu Ile Phe Ser Gly
               740                     745                     750
   Thr Asn Asp Thr Gln Tyr Pro Thr Tyr Leu Tyr Gln Lys Ile Asp Glu
           755                     760                     765
   Ser Lys Leu Lys Glu Tyr Thr Arg Tyr Lys Leu Lys Gly Phe Ile Glu
       770                     775                     780
   Ser Ser Gln Asp Leu Glu Ala Tyr Val Ile Arg Tyr Asp Ala Lys His
   785                     790                     795                     800
   Arg Thr Leu Asp Val Ser Asp Asn Leu Leu Pro Asp Ile Leu Pro Glu
                   805                     810                     815
   Asn Thr Cys Gly Glu Pro Asn Arg Cys Ala Ala Gln Gln Tyr Leu Asp
               820                     825                     830
   Glu Asn Pro Ser Pro Glu Cys Ser Ser Met Gln Asp Gly Ile Leu Ser
           835                     840                     845
   Asp Ser His Ser Phe Ser Leu Asn Ile Asp Thr Gly Ser Ile Asn His
       850                     855                     860
   Asn Glu Asn Leu Gly Ile Trp Val Leu Phe Lys Ile Ser Thr Leu Glu
   865                     870                     875                     880
   Gly Tyr Ala Lys Phe Gly Asn Leu Glu Val Ile Glu Asp Gly Pro Val
                   885                     890                     895
   Ile Gly Glu Ala Leu Ala Arg Val Lys Arg Gln Glu Thr Lys Trp Arg
               900                     905                     910
   Asn Lys Leu Ala Gln Leu Thr Thr Glu Thr Gln Ala Ile Tyr Thr Arg
           915                     920                     925
   Ala Lys Gln Ala Leu Asp Asn Leu Phe Ala Asn Ala Gln Asp Ser His
       930                     935                     940
   Leu Lys Arg Asp Val Thr Phe Ala Glu Ile Ala Ala Ala Arg Lys Ile
   945                     950                     955                     960
   Val Gln Ser Ile Arg Glu Ala Tyr Met Ser Trp Leu Ser Val Val Pro
                   965                     970                     975
   Gly Val Asn His Pro Ile Phe Thr Glu Leu Ser Gly Arg Val Gln Arg
               980                     985                     990
   Ala Phe Gln Leu Tyr Asp Val Arg Asn Val Val Arg Asn Gly Arg Phe
           995                     1000                    1005
   Leu Asn Gly Leu Ser Asp Trp Ile Val Thr Ser Asp Val Lys Val Gln
       1010                    1015                    1020
   Glu Glu Asn Gly Asn Asn Val Leu Val Leu Asn Asn Trp Asp Ala Gln
   1025                    1030                    1035                    1040
   Val Leu Gln Asn Val Lys Leu Tyr Gln Asp Arg Gly Tyr Ile Leu His
                   1045                    1050                    1055
   Val Thr Ala Arg Lys Ile Gly Ile Gly Glu Gly Tyr Ile Thr Ile Thr
               1060                    1065                    1070
   Asp Glu Glu Gly His Thr Asp Gln Leu Arg Phe Thr Ala Cys Glu Glu
           1075                    1080                    1085
   Ile Asp Ala Ser Asn Ala Phe Ile Ser Gly Tyr Ile Thr Lys Glu Leu
       1090                    1095                    1100
   Glu Phe Phe Pro Asp Thr Glu Lys Val His Ile Glu Ile Gly Glu Thr
   1105                    1110                    1115                    1120
   Glu Gly Ile Phe Leu Val Glu Ser Ile Glu Leu Phe Leu Met Glu Glu
   1125 1130 1135
   Leu Cys
<210> 42
   <211> 1157
   <212> PRT
   <213> Bacillus thuringiensi
<400> 42
   Met Ser Pro Asn Asn Gln Asn Glu Tyr Glu Ile Ile Asp Ala Thr Pro
   1 5 10 15
   Ser Thr Ser Val Ser Ser Asp Ser Asn Arg Tyr Pro Phe Ala Asn Glu
   20 25 30
   Pro Thr Asp Ala Leu Gln Asn Met Asn Tyr Lys Asp Tyr Leu Lys Met
   35 40 45
   Ser Gly Gly Glu Asn Pro Glu Leu Phe Gly Asn Pro Glu Thr Phe Ile
   50 55 60
   Ser Ser Ser Thr Ile Gln Thr Gly Ile Gly Ile Val Gly Arg Ile Leu
   65 70 75 80
   Gly Ala Leu Gly Val Pro Phe Ala Ser Gln Ile Ala Ser Phe Tyr Ser
   85 90 95
   Phe Ile Val Gly Gln Leu Trp Pro Ser Lys Ser Val Asp Ile Trp Gly
   100 105 110
   Glu Ile Met Glu Arg Val Glu Glu Leu Val Asp Gln Lys Ile Glu Lys
   115 120 125
   Tyr Val Lys Asp Lys Ala Leu Ala Glu Leu Lys Gly Leu Gly Asn Ala
   130 135 140
   Leu Asp Val Tyr Gln Gln Ser Leu Glu Asp Trp Leu Glu Asn Arg Asn
   145 150 155 160
   Asp Ala Arg Thr Arg Ser Val Val Ser Asn Gln Phe Ile Ala Leu Asp
   165 170 175
   Leu Asn Phe Val Ser Ser Ile Pro Ser Phe Ala Val Ser Gly His Glu
   180 185 190
   Val Leu Leu Leu Ala Val Tyr Ala Gln Ala Val Asn Leu His Leu Leu
   195 200 205
   Leu Leu Arg Asp Ala Ser Ile Phe Gly Glu Glu Trp Gly Phe Thr Pro
   210 215 220
   Gly Glu Ile Ser Arg Phe Tyr Asn Arg Gln Val Gln Leu Thr Ala Glu
   225 230 235 240
   Tyr Ser Asp Tyr Cys Val Lys Trp Tyr Lys Ile Gly Leu Asp Lys Leu
   245 250 255
   Lys Gly Thr Thr Ser Lys Ser Trp Leu Asn Tyr His Gln Phe Arg Arg
   260 265 270
   Glu Met Thr Leu Leu Val Leu Asp Leu Val Ala Leu Phe Pro Asn Tyr
   275 280 285
   Asp Thr His Met Tyr Pro Ile Glu Thr Thr Ala Gln Leu Thr Arg Asp
   290 295 300
   Val Tyr Thr Asp Pro Ile Ala Phe Asn Ile Val Thr Ser Thr Gly Phe
   305 310 315 320
   Cys Asn Pro Trp Ser Thr His Ser Gly Ile Leu Phe Tyr Glu Val Glu
   325 330 335
   Asn Asn Val Ile Arg Pro Pro His Leu Phe Asp Ile Leu Ser Ser Val
   340 345 350
   Glu Ile Asn Thr Ser Arg Gly Gly Ile Thr Leu Asn Asn Asp Ala Tyr
   355 360 365
   Ile Asn Tyr Trp Ser Gly His Thr Leu Lys Tyr Arg Arg Thr Ala Asp
   370 375 380
   Ser Thr Val Thr Tyr Thr Ala Asn Tyr Gly Arg Ile Thr Ser Glu Lys
   385 390 395 400
   Asn Ser Phe Ala Leu Glu Asp Arg Asp Ile Phe Glu Ile Asn Ser Thr
   405 410 415
   Val Ala Asn Leu Ala Asn Tyr Tyr Gln Lys Ala Tyr Gly Val Pro Gly
   420 425 430
   Ser Trp Phe His Met Val Lys Arg Gly Thr Ser Ser Thr Thr Ala Tyr
   435 440 445
   Leu Tyr Ser Lys Thr His Thr Ala Leu Gln Gly Cys Thr Gln Val Tyr
   450 455 460
   Glu Ser Ser Asp Glu Ile Pro Leu Asp Arg Thr Val Pro Val Ala Glu
   465 470 475 480
   Ser Tyr Ser His Arg Leu Ser His Ile Thr Ser His Ser Phe Ser Lys
   485 490 495
   Asn Gly Ser Ala Tyr Tyr Gly Ser Phe Pro Val Phe Val Trp Thr His
   500 505 510
   Thr Ser Ala Asp Leu Asn Asn Thr Ile Tyr Ser Asp Lys Ile Thr Gln
   515 520 525
   Ile Pro Ala Val Lys Gly Asp Met Leu Tyr Leu Gly Gly Ser Val Val
   530 535 540
   Gln Gly Pro Gly Phe Thr Gly Gly Asp Ile Leu Lys Arg Thr Asn Pro
   545 550 555 560
   Ser Ile Leu Gly Thr Phe Ala Val Thr Val Asn Gly Ser Leu Ser Gln
   565 570 575
   Arg Tyr Arg Val Arg Ile Arg Tyr Ala Ser Thr Thr Asp Phe Glu Phe
   580 585 590
   Thr Leu Tyr Leu Gly Asp Thr Ile Glu Lys Asn Arg Phe Asn Lys Thr
   595 600 605
   Met Asp Asn Gly Ala Ser Leu Thr Tyr Glu Thr Phe Lys Phe Ala Ser
   610 615 620
   Phe Ile Thr Asp Phe Gln Phe Arg Glu Thr Gln Asp Lys Ile Leu Leu
   625 630 635 640
   Ser Met Gly Asp Phe Ser Ser Gly Gln Glu Val Tyr Ile Asp Arg Ile
   645 650 655
   Glu Phe Ile Pro Val Asp Glu Thr Tyr Glu Ala Glu Gln Asp Leu Glu
   660 665 670
   Ala Ala Lys Lys Ala Val Asn Ala Leu Phe Thr Asn Thr Lys Asp Gly
   675 680 685
   Leu Arg Pro Gly Val Thr Asp Tyr Glu Val Asn Gln Ala Ala Asn Leu
   690 695 700
   Val Glu Cys Leu Ser Asp Asp Leu Tyr Pro Asn Glu Lys Arg Leu Leu
   705 710 715 720
   Phe Asp Ala Val Arg Glu Ala Lys Arg Leu Ser Gly Ala Arg Asn Leu
   725 730 735
   Leu Gln Asp Pro Asp Phe Gln Glu Ile Asn Gly Glu Asn Gly Trp Ala
   740 745 750
   Ala Ser Thr Gly Ile Glu Ile Val Glu Gly Asp Ala Val Phe Lys Gly
   755 760 765
   Arg Tyr Leu Arg Leu Pro Gly Ala Arg Glu Ile Asp Thr Glu Thr Tyr
   770 775 780
   Pro Thr Tyr Leu Tyr Gln Lys Val Glu Glu Gly Val Leu Lys Pro Tyr
   785 790 795 800
   Thr Arg Tyr Arg Leu Arg Gly Phe Val Gly Ser Ser Gln Gly Leu Glu
   805 810 815
   Ile Tyr Thr Ile Arg His Gln Thr Asn Arg Ile Val Lys Asn Val Pro
   820 825 830
   Asp Asp Leu Leu Pro Asp Val Ser Pro Val Asn Ser Asp Gly Ser Ile
   835 840 845
   Asn Arg Cys Ser Glu Gln Lys Tyr Val Asn Ser Arg Leu Glu Gly Glu
   850 855 860
   Asn Arg Ser Gly Asp Ala His Glu Phe Ser Leu Pro Ile Asp Ile Gly
   865 870 875 880
   Glu Leu Asp Tyr Asn Glu Asn Ala Gly Ile Trp Val Gly Phe Lys Ile
   885 890 895
   Thr Asp Pro Glu Gly Tyr Ala Thr Leu Gly Asn Leu Glu Leu Val Glu
   900 905 910
   Glu Gly Pro Leu Ser Gly Asp Ala Leu Glu Arg Leu Gln Arg Glu Glu
   915 920 925
   Gln Gln Trp Lys Ile Gln Met Thr Arg Arg Arg Glu Glu Thr Asp Arg
   930 935 940
   Arg Tyr Met Ala Ser Lys Gln Ala Val Asp Arg Leu Tyr Ala Asp Tyr
   945 950 955 960
   Gln Asp Gln Gln Leu Asn Pro Asp Val Glu Ile Thr Asp Leu Thr Ala
   965 970 975
   Ala Gln Asp Leu Ile Gln Ser Ile Pro Tyr Val Tyr Asn Glu Met Phe
   980 985 990
   Pro Glu Ile Pro Gly Met Asn Tyr Thr Lys Phe Thr Glu Leu Thr Asp
           995                 1000                1005
   Arg Leu Gln Gln Ala Trp Asn Leu Tyr Asp Gln Arg Asn Ala Ile Pro
       1010                1015                1020
   Asn Gly Asp Phe Arg Asn Gly Leu Ser Asn Trp Asn Ala Thr Pro Gly
   1025                1030                1035                1040
   Val Glu Val Gln Gln Ile Asn His Thr Ser Val Leu Val Ile Pro Asn
                   1045                1050                1055
   Trp Asp Glu Gln Val Ser Gln Gln Phe Thr Val Gln Pro Asn Gln Arg
               1060                1065                1070
   Tyr Val Leu Arg Val Thr Ala Arg Lys Glu Gly Val Gly Asn Gly Tyr
           1075                1080                1085
   Val Ser Ile Arg Asp Gly Gly Asn Gln Ser Glu Thr Leu Thr Phe Ser
       1090                1095                1100
   Ala Ser Asp Tyr Asp Thr Asn Gly Val Tyr Asn Asp Gln Thr Gly Tyr
   1105                1110                1115                1120
   Ile Thr Lys Thr Val Thr Phe Ile Pro Tyr Thr Asp Gln Met Trp Ile
                   1125                1130                1135
   Glu Ile Ser Glu Thr Glu Gly Thr Phe Tyr Ile Glu Ser Val Glu Leu
               1140                1145                1150
   Ile Val Asp Val Glu
           1155
<210> 43
   <211> 675
   <212> PRT
   <213> Bacillus thuringiensi
<400> 43
   Met Asn Pro Tyr Gln Asn Lys Asn Glu Tyr Glu Ile Phe Asn Ala Pro
    1               5                  10                  15
   Ser Asn Gly Phe Ser Lys Ser Asn Asn Tyr Ser Arg Tyr Pro Leu Ala
               20                  25                  30
   Asn Lys Pro Asn Gln Pro Leu Lys Asn Thr Asn Tyr Lys Asp Trp Leu
           35                  40                  45
   Asn Val Cys Gln Asp Asn Gln Gln Tyr Gly Asn Asn Ala Gly Asn Phe
       50                  55                  60
   Ala Ser Ser Glu Thr Ile Val Gly Val Ser Ala Gly Ile Ile Val Val
   65                  70                  75                  80
   Gly Thr Met Leu Gly Ala Phe Ala Ala Pro Val Leu Ala Ala Gly Ile
                   85                  90                  95
   Ile Ser Phe Gly Thr Leu Leu Pro Ile Phe Trp Gln Gly Ser Asp Pro
               100                 105                 110
   Ala Asn Val Trp Gln Asp Leu Leu Asn Ile Gly Gly Arg Pro Ile Gln
           115                 120                 125
   Glu Ile Asp Lys Asn Ile Ile Asn Val Leu Thr Ser Ile Val Thr Pro
       130                 135                 140
   Ile Lys Asn Gln Leu Asp Lys Tyr Gln Glu Phe Phe Asp Lys Trp Glu
   145                 150                 155                 160
   Pro Ala Arg Thr His Ala Asn Ala Lys Ala Val His Asp Leu Phe Thr
                   165                 170                 175
   Thr Leu Glu Pro Ile Ile Asp Lys Asp Leu Asp Met Leu Lys Asn Asn
               180                 185                 190
   Ala Ser Tyr Arg Ile Pro Thr Leu Pro Ala Tyr Ala Gln Ile Ala Thr
           195                 200                 205
   Trp His Leu Asn Leu Leu Lys His Ala Ala Thr Tyr Tyr Asn Ile Trp
       210                 215                 220
   Leu Gln Asn Gln Gly Ile Asn Pro Ser Thr Phe Asn Ser Ser Asn Tyr
   225                 230                 235                 240
   Tyr Gln Gly Tyr Leu Lys Arg Lys Ile Gln Glu Tyr Thr Asp Tyr Cys
                   245                 250                 255
   Ile Gln Thr Tyr Asn Ala Gly Leu Thr Met Ile Arg Thr Asn Thr Asn
               260                 265                 270
   Ala Thr Trp Asn Met Tyr Asn Thr Tyr Arg Leu Glu Met Thr Leu Thr
           275                 280                 285
   Val Leu Asp Leu Ile Ala Ile Phe Pro Asn Tyr Asp Pro Glu Lys Tyr
       290                 295                 300
   Pro Ile Gly Val Lys Ser Glu Leu Ile Arg Glu Val Tyr Thr Asn Val
   305                 310                 315                 320
   Asn Ser Asp Thr Phe Arg Thr Ile Thr Glu Leu Glu Asn Gly Leu Thr
                   325                 330                 335
   Arg Asn Pro Thr Leu Phe Thr Trp Ile Asn Gln Gly Arg Phe Tyr Thr
               340                 345                 350
   Arg Asn Ser Arg Asp Ile Leu Asp Pro Tyr Asp Ile Phe Ser Phe Thr
           355                 360                 365
   Gly Asn Gln Met Ala Phe Thr His Thr Asn Asp Asp Arg Asn Ile Ile
       370                 375                 380
   Trp Gly Ala Val His Gly Asn Ile Ile Ser Gln Asp Thr Ser Lys Val
   385                 390                 395                 400
   Phe Pro Phe Tyr Arg Asn Lys Pro Ile Asp Lys Val Glu Ile Val Arg
                   405                 410                 415
   His Arg Glu Tyr Ser Asp Ile Ile Tyr Glu Met Ile Phe Phe Ser Asn
               420                 425                 430
   Ser Ser Glu Val Phe Arg Tyr Ser Ser Asn Ser Thr Ile Glu Asn Asn
           435                 440                 445
   Tyr Lys Arg Thr Asp Ser Tyr Met Ile Pro Lys Gln Thr Trp Lys Asn
       450                 455                 460
   Glu Glu Tyr Gly His Thr Leu Ser Tyr Ile Lys Thr Asp Asn Tyr Ile
   465                 470                 475                 480
   Phe Ser Val Val Arg Glu Arg Arg Arg Val Ala Phe Ser Trp Thr His
                   485                 490                 495
   Thr Ser Val Asp Phe Gln Asn Thr Ile Asp Leu Asp Asn Ile Thr Gln
               500                 505                 510
   Ile His Ala Leu Lys Ala Leu Lys Val Ser Ser Asp Ser Lys Ile Val
           515                 520                 525
   Lys Gly Pro Gly His Thr Gly Gly Asp Leu Val Ile Leu Lys Asp Ser
       530                 535                 540
   Met Asp Phe Arg Val Arg Phe Leu Lys Asn Val Ser Arg Gln Tyr Gln
   545                 550                 555                 560
   Val Arg Ile Arg Tyr Ala Thr Asn Ala Pro Lys Thr Thr Val Phe Leu
                   565                 570                 575
   Thr Gly Ile Asp Thr Ile Ser Val Glu Leu Pro Ser Thr Thr Ser Arg
               580                 585                 590
   Gln Asn Pro Asn Ala Thr Asp Leu Thr Tyr Ala Asp Phe Gly Tyr Val
           595                 600                 605
   Thr Phe Pro Arg Thr Val Pro Asn Lys Thr Phe Glu Gly Glu Asp Thr
       610                 615                 620
   Leu Leu Met Thr Leu Tyr Gly Thr Pro Asn His Ser Tyr Asn Ile Tyr
   625                 630                 635                 640
   Ile Asp Lys Ile Glu Phe Ile Pro Ile Thr Gln Ser Val Leu Asp Tyr
                   645                 650                 655
   Thr Glu Lys Gln Asn Ile Glu Lys Thr Gln Lys Ile Val Asn Asp Leu
               660                 665                 670
   Phe Val Asn
           675
<210> 44
   <211> 648
   <212> PRT
   <213> Bacillus thuringiensi
<400> 44
   Met His Tyr Tyr Gly Asn Arg Asn Glu Tyr Asp Ile Leu Asn Ala Ser
    1               5                  10                  15
   Ser Asn Asp Ser Asn Met Ser Asn Thr Tyr Pro Arg Tyr Pro Leu Ala
               20                  25                  30
   Asn Pro Gln Gln Asp Leu Met Gln Asn Thr Asn Tyr Lys Asp Trp Leu
           35                  40                  45
   Asn Val Cys Glu Gly Tyr His Ile Glu Asn Pro Arg Glu Ala Ser Val
       50                  55                  60
   Arg Ala Gly Leu Gly Lys Gly Leu Gly Ile Val Ser Thr Ile Val Gly
   65                  70                  75                  80
   Phe Phe Gly Gly Ser Ile Ile Leu Asp Thr Ile Gly Leu Phe Tyr Gln
                   85                  90                  95
   Ile Ser Glu Leu Leu Trp Pro Glu Asp Asp Thr Gln Gln Tyr Thr Trp
                   100                 105                 110
   Gln Asp Ile Met Asn His Val Glu Asp Leu Ile Asp Lys Arg Ile Thr
               115                 120                 125
   Glu Val Ile Arg Gly Asn Ala Ile Arg Thr Leu Ala Asp Leu Gln Gly
       130                 135                 140
   Lys Val Asp Asp Tyr Asn Asn Trp Leu Lys Lys Trp Lys Asp Asp Pro
   145                 150                 155                 160
   Lys Ser Thr Gly Asn Leu Ser Thr Leu Val Thr Lys Phe Thr Ala Leu
                   165                 170                 175
   Asp Ser Asp Phe Asn Gly Ala Ile Arg Thr Val Asn Asn Gln Gly Ser
               180                 185                 190
   Pro Gly Tyr Glu Leu Leu Leu Leu Pro Val Tyr Ala Gln Ile Ala Asn
           195                 200                 205
   Leu His Leu Leu Leu Leu Arg Asp Ala Gln Ile Tyr Gly Asp Lys Trp
       210                 215                 220
   Trp Ser Ala Arg Ala Asn Ala Arg Asp Asn Tyr Tyr Gln Ile Gln Leu
   225                 230                 235                 240
   Glu Lys Thr Lys Glu Tyr Thr Glu Tyr Cys Ile Asn Trp Tyr Asn Lys
                   245                 250                 255
   Gly Leu Asn Asp Phe Arg Thr Ala Gly Gln Trp Val Asn Phe Asn Arg
               260                 265                 270
   Tyr Arg Arg Glu Met Thr Leu Thr Val Leu Asp Ile Ile Ser Met Phe
           275                 280                 285
   Pro Ile Tyr Asp Ala Arg Leu Tyr Pro Thr Glu Val Lys Thr Glu Leu
       290                 295                 300
   Thr Arg Glu Ile Tyr Ser Asp Val Ile Asn Gly Glu Ile Tyr Gly Leu
   305                 310                 315                 320
   Met Thr Pro Tyr Phe Ser Phe Glu Lys Ala Glu Ser Leu Tyr Thr Arg
                   325                 330                 335
   Ala Pro His Leu Phe Thr Trp Leu Lys Gly Phe Arg Phe Val Thr Asn
               340                 345                 350
   Ser Ile Ser Tyr Trp Thr Phe Leu Ser Gly Gly Gln Asn Lys Tyr Ser
           355                 360                 365
   Tyr Thr Asn Asn Ser Ser Ile Asn Glu Gly Ser Phe Arg Gly Gln Asp
       370                 375                 380
   Thr Asp Tyr Gly Gly Thr Ser Ser Thr Ile Asn Ile Pro Ser Asn Ser
   385                 390                 395                 400
   Tyr Val Tyr Asn Leu Trp Thr Glu Asn Tyr Glu Tyr Ile Tyr Pro Trp
                   405                 410                 415
   Gly Asp Pro Val Asn Ile Thr Lys Met Asn Phe Ser Val Thr Asp Asn
               420                 425                 430
   Asn Ser Ser Lys Glu Leu Ile Tyr Gly Ala His Arg Thr Asn Lys Pro
           435                 440                 445
   Val Val Arg Thr Asp Phe Asp Phe Leu Thr Asn Lys Glu Gly Thr Glu
       450                 455                 460
   Leu Ala Lys Tyr Asn Asp Tyr Asn His Ile Leu Ser Tyr Met Leu Ile
   465                 470                 475                 480
   Asn Gly Glu Thr Phe Gly Gln Lys Arg His Gly Tyr Ser Phe Ala Phe
                   485                 490                 495
   Thr His Ser Ser Val Asp Pro Asn Asn Thr Ile Ala Ala Asn Lys Ile
               500                 505                 510
   Thr Gln Ile Pro Val Val Lys Ala Ser Ser Ile Asn Gly Ser Ile Ser
           515                 520                 525
   Ile Glu Lys Gly Pro Gly Phe Thr Gly Gly Asp Leu Val Lys Met Arg
       530                 535                 540
   Ala Asp Ser Gly Leu Thr Met Arg Phe Lys Ala Glu Leu Leu Asp Lys
   545                 550                 555                 560
   Lys Tyr Arg Val Arg Ile Arg Tyr Lys Cys Asn Tyr Ser Ser Lys Leu
                   565                 570                 575
   Ile Leu Arg Lys Trp Lys Gly Glu Gly Tyr Ile Gln Gln Gln Ile His
               580                 585                 590
   Asn Ile Ser Pro Thr Tyr Gly Ala Phe Ser Tyr Leu Glu Ser Phe Thr
           595                 600                 605
   Ile Thr Thr Thr Glu Asn Ile Phe Asp Leu Thr Met Glu Val Thr Tyr
       610                 615                 620
   Pro Tyr Gly Arg Gln Phe Val Glu Asp Ile Pro Ser Leu Ile Leu Asp
   625                 630                 635                 640
   Lys Ile Glu Phe Leu Pro Thr Asn
   645
<210> 45
   <211> 682
   <212> PRT
   <213> Bacillus thuringiensi
<400> 45
   Met Asn Ser Tyr Gln Asn Lys Asn Glu Tyr Glu Ile Leu Asp Ala Lys
   1 5 10 15
   Arg Asn Thr Cys His Met Ser Asn Cys Tyr Pro Lys Tyr Pro Leu Ala
   20 25 30
   Asn Asp Pro Gln Met Tyr Leu Arg Asn Thr His Tyr Lys Asp Trp Ile
   35 40 45
   Asn Met Cys Glu Glu Ala Ser Tyr Ala Ser Ser Gly Pro Ser Gln Leu
   50 55 60
   Phe Lys Val Gly Gly Ser Ile Val Ala Lys Ile Leu Gly Met Ile Pro
   65 70 75 80
   Glu Val Gly Pro Leu Leu Ser Trp Met Val Ser Leu Phe Trp Pro Thr
   85 90 95
   Ile Glu Glu Lys Asn Thr Val Trp Glu Asp Met Ile Lys Tyr Val Ala
   100 105 110
   Asn Leu Leu Lys Gln Glu Leu Thr Asn Asp Thr Leu Asn Arg Ala Thr
   115 120 125
   Ser Asn Leu Ser Gly Leu Asn Glu Ser Leu Asn Ile Tyr Asn Arg Ala
   130 135 140
   Leu Ala Ala Trp Lys Gln Asn Lys Asn Asn Phe Ala Ser Gly Glu Leu
   145 150 155 160
   Ile Arg Ser Tyr Ile Asn Asp Leu His Ile Leu Phe Thr Arg Asp Ile
   165 170 175
   Gln Ser Asp Phe Ser Leu Gly Gly Tyr Glu Thr Val Leu Leu Pro Ser
   180 185 190
   Tyr Ala Ser Ala Ala Asn Leu His Leu Leu Leu Leu Arg Asp Val Ala
   195 200 205
   Ile Tyr Gly Lys Glu Leu Gly Tyr Pro Ser Thr Asp Val Glu Phe Tyr
   210 215 220
   Tyr Asn Glu Gln Lys Tyr Tyr Thr Glu Lys Tyr Ser Asn Tyr Cys Val
   225 230 235 240
   Asn Thr Tyr Lys Ser Gly Leu Glu Ser Lys Lys Gln Ile Gly Trp Ser
   245 250 255
   Asp Phe Asn Arg Tyr Arg Arg Glu Met Thr Leu Ser Val Leu Asp Ile
   260 265 270
   Val Ala Leu Phe Pro Leu Tyr Asp Thr Gly Leu Tyr Pro Ser Lys Asp
   275 280 285
   Gly Lys Ile His Val Lys Ala Glu Leu Thr Arg Glu Ile Tyr Ser Asp
   290 295 300
   Val Ile Asn Asp His Val Tyr Gly Leu Met Val Pro Tyr Ile Ser Phe
   305 310 315 320
   Glu His Ala Glu Ser Leu Tyr Thr Arg Arg Pro His Ala Phe Thr Trp
   325 330 335
   Leu Lys Gly Phe Arg Phe Val Thr Asn Ser Ile Asn Ser Trp Thr Phe
   340 345 350
   Leu Ser Gly Gly Glu Asn Arg Tyr Phe Leu Thr His Gly Glu Gly Thr
   355 360 365
   Ile Tyr Asn Gly Pro Phe Leu Gly Gln Asp Thr Glu Tyr Gly Gly Thr
   370 375 380
   Ser Ser Tyr Ile Asp Ile Ser Asn Asn Ser Ser Ile Tyr Asn Leu Trp
   385 390 395 400
   Thr Lys Asn Tyr Glu Trp Ile Tyr Pro Trp Thr Asp Pro Val Asn Ile
   405 410 415
   Thr Lys Ile Asn Phe Ser Ile Thr Asp Asn Ser Asn Ser Ser Glu Ser
   420 425 430
   Ile Tyr Gly Ala Glu Arg Met Asn Lys Pro Thr Val Arg Thr Asp Phe
   435 440 445
   Asn Phe Leu Leu Asn Arg Ala Gly Asn Gly Pro Thr Thr Tyr Asn Asp
   450 455 460
   Tyr Asn His Ile Leu Ser Tyr Met Leu Ile Asn Gly Glu Thr Phe Gly
   465                     470                     475                     480
   Gln Lys Arg His Gly Tyr Ser Phe Ala Phe Thr His Ser Ser Val Asp
                   485                     490                     495
   Arg Tyr Asn Thr Ile Val Pro Asp Lys Ile Val Gln Ile Pro Ala Val
               500                     505                     510
   Lys Thr Asn Leu Val Gly Ala Asn Ile Ile Lys Gly Pro Gly His Thr
           515                     520                     525
   Gly Gly Asp Leu Leu Lys Leu Glu Tyr Glu Arg Phe Leu Ser Leu Arg
       530                     535                     540
   Ile Lys Leu Ile Ala Ser Met Thr Phe Arg Ile Arg Ile Arg Tyr Ala
   545                     550                     555                     560
   Ser Asn Ile Ser Gly Gln Met Met Ile Asn Ile Gly Tyr Gln Asn Pro
                   565                     570                     575
   Thr Tyr Phe Asn Ile Ile Pro Thr Thr Ser Arg Asp Tyr Thr Glu Leu
               580                     585                     590
   Lys Phe Glu Asp Phe Gln Leu Val Asp Thr Ser Tyr Ile Tyr Ser Gly
           595                     600                     605
   Gly Pro Ser Ile Ser Ser Asn Thr Leu Trp Leu Asp Asn Phe Ser Asn
       610                     615                     620
   Gly Pro Val Ile Ile Asp Lys Ile Glu Phe Ile Pro Leu Gly Ile Thr
   625                     630                     635                     640
   Leu Asn Gln Ala Gln Gly Tyr Asp Thr Tyr Asp Gln Asn Ala Asn Gly
                   645                     650                     655
   Met Tyr His Gln Asn Tyr Ser Asn Ser Gly Tyr Asn Tyr Asn Gln Glu
               660                     665                     670
   Tyr Asn Thr Tyr Tyr Gln Ser Tyr Asn Asn
           675                     680
<210> 46
   <211> 529
   <212> PRT
   <213> Bacillus thuringiensi
<400> 46
   Val Asn Phe Met Leu Thr Ser Gly Ala Lys Asn Met Leu Lys Leu Glu
    1               5                  10                  15
   Thr Thr Asp Tyr Glu Ile Asp Gln Met Ala Asn Ala Ile Glu Asn Met
               20                  25                  30
   Ser Gly Glu Gln Tyr Ser Gln Glu Lys Met Met Gln Trp His Asp Ile
           35                  40                  45
   Lys Tyr Ala Lys Gln Leu Ser Gln Ala Arg Asn Leu Leu Gln Asn Gly
       50                  55                  60
   Asp Phe Glu Asp Leu Phe Ser Gly Trp Thr Thr Ser Asn Gln Met Ser
   65                  70                  75                  80
   Ile Gln Ala Asp Asn Ala Thr Phe Lys Gly Asn Tyr Leu His Met Ser
                   85                  90                  95
   Gly Ala Arg Asp Ile Tyr Gly Thr Ile Phe Pro Thr Tyr Ile Tyr Gln
               100                 105                 110
   Lys Ile Asp Glu Ser Lys Leu Lys Pro Tyr Thr Arg Tyr Leu Val Arg
           115                 120                 125
   Gly Phe Val Gly Ser Ser Lys Asp Leu Glu Leu Met Val Met Arg Tyr
       130                 135                 140
   Gly Lys Glu Ile Asp Thr Val Met Asn Val Pro Asn Asp Ile Pro Tyr
   145                 150                 155                 160
   Val Pro Ser Met Pro Val Cys Asn Glu Leu Tyr Asp Gly Gln Gln Pro
                   165                 170                 175
   Tyr Pro Asn Arg His Val Gly Tyr Tyr Asn Pro Met Pro Val Ser Gln
               180                 185                 190
   Pro Ser Tyr Thr Ser Asp Thr Cys Gln Cys Thr Pro Gly Lys Lys His
           195                 200                 205
   Val Val Cys His Asp Ser His Gln Phe Lys Phe His Ile Asp Thr Gly
       210                 215                 220
   Glu Val Asp Tyr Asn Thr Asn Leu Gly Ile Trp Val Leu Phe Lys Ile
   225                 230                 235                 240
   Ser Ser Pro Asp Gly Tyr Ala Thr Leu Asp Asn Leu Glu Val Ile Glu
                   245                 250                 255
   Glu Gly Pro Val Arg Gly Glu Ala Val Thr His Val Lys Gln Lys Glu
               260                 265                 270
   Lys Lys Trp Asn Gln Gln Met Glu Lys Lys Arg Met Glu Thr Lys Arg
           275                 280                 285
   Val Tyr Asp Arg Ala Lys Gln Ala Val Asp Ala Leu Phe Thr Gly Glu
       290                 295                 300
   Glu Leu Asn Tyr Asp Val Thr Leu Ser His Ile Lys Asn Ala Asp Asp
   305                 310                 315                 320
   Leu Val Gln Ser Ile Pro Tyr Val His Asn Glu Trp Leu Pro Asp Phe
                   325                 330                 335
   Pro Gly Met Asn Tyr Asp Ile Tyr Gln Glu Leu Asn Ala Arg Ile Met
               340                 345                 350
   Gln Ala Arg Tyr Leu Tyr Asp Ala Arg Asn Val Ile Thr Asn Gly Asp
           355                 360                 365
   Phe Ala Gln Gly Leu Gln Gly Trp His Ala Glu Gly Lys Val Glu Val
       370                 375                 380
   Gln Gln Met Asn Gly Thr Ser Val Leu Val Leu Ser Asn Trp Ser Ser
   385                 390                 395                 400
   Gly Val Ser Gln Asn Leu His Val Gln His Pro His Gly Tyr Leu Leu
                   405                 410                 415
   Arg Val Ser Ala Lys Lys Glu Gly Ser Gly Lys Gly Tyr Val Thr Arg
               420                 425                 430
   Met Ser Cys Asn Gly Lys Gln Glu Thr Leu Thr Phe Thr Ser Cys Asp
           435                 440                 445
   Gly Gly Tyr Met Thr Lys Thr Val Glu Val Phe Pro Glu Ser Asp Arg
       450                 455                 460
   Val Arg Ile Glu Ile Gly Glu Thr Glu Gly Ser Phe Tyr Ile Glu Ser
   465                 470                 475                 480
   Ile Glu Leu Ile Cys Met Asn Gly Tyr Thr Ser Asn Asn Asn Gln Asn
                   485                 490                 495
   Met Ser Asn Met Tyr Asp Gln Ser Tyr Ser Gly Asn Tyr Ser Gln Asn
               500                 505                 510
   Thr Ser Asp Met Tyr Asp Gln Gly Gly Ser Val Ala Lys Phe Glu Lys
           515                 520                 525
   Glu
<210> 47
   <211> 674
   <212> PRT
   <213> Bacillus thuringiensi
<400> 47
   Met Asn Gln Tyr Gln Asn Lys Asn Glu Tyr Glu Ile Leu Glu Ser Ser
    1               5                  10                  15
   Gln Asn Asn Met Asn Met Pro Asn Arg Tyr Pro Phe Ala Asp Asp Pro
               20                  25                  30
   Asn Ala Val Met Lys Asn Gly Asn Tyr Lys Asp Trp Val Asn Glu Cys
           35                  40                  45
   Glu Gly Ser Asn Ile Ser Pro Ser Pro Ala Ala Ala Ile Thr Ser Lys
       50                  55                  60
   Ile Val Ser Ile Val Leu Lys Thr Leu Ala Lys Ala Val Ala Ser Ser
   65                  70                  75                  80
   Leu Ala Asp Ser Ile Lys Ser Ser Leu Gly Ile Ser Lys Thr Ile Thr
                   85                  90                  95
   Glu Asn Asn Val Ser Gln Val Ser Met Val Gln Val His Gln Ile Ile
               100                 105                 110
   Asn Arg Arg Ile Gln Glu Thr Ile Leu Asp Leu Gly Glu Ser Ser Leu
           115                 120                 125
   Asn Gly Leu Val Ala Ile Tyr Asn Arg Asp Tyr Leu Gly Ala Leu Glu
       130                 135                 140
   Ala Trp Asn Asn Asn Lys Ser Asn Ile Asn Tyr Gln Thr Asn Val Ala
   145                 150                 155                 160
   Glu Ala Phe Lys Thr Val Glu Arg Glu Phe Phe Thr Lys Leu Lys Gly
                   165                 170                 175
   Ile Tyr Arg Thr Ser Ser Ser Gln Ile Thr Leu Leu Pro Thr Phe Thr
               180                 185                 190
   Gln Ala Ala Asn Leu His Leu Ser Met Leu Arg Asp Ala Val Met Tyr
           195                 200                 205
   Gln Glu Gly Trp Asn Leu Gln Ser His Ile Asn Tyr Ser Lys Glu Leu
       210                 215                 220
   Asp Asp Ala Leu Glu Asp Tyr Thr Asn Tyr Cys Val Glu Val Tyr Thr
   225                 230                 235                 240
   Lys Gly Leu Asn Ala Leu Arg Gly Ser Thr Ala Ile Asp Trp Leu Glu
                   245                 250                 255
   Phe Asn Ser Phe Arg Arg Asp Met Thr Leu Met Val Leu Asp Leu Val
               260                 265                 270
   Ala Ile Phe Pro Asn Tyr Asn Pro Val Arg Tyr Pro Leu Ser Thr Lys
           275                 280                 285
   Ile Ser Leu Ser Arg Lys Ile Tyr Thr Asp Pro Val Gly Arg Thr Asp
       290                 295                 300
   Ser Pro Ser Phe Gly Asp Trp Thr Asn Thr Gly Arg Thr Leu Ala Asn
   305                 310                 315                 320
   Phe Asn Asp Leu Glu Arg Glu Val Thr Asp Ser Pro Ser Leu Val Lys
                   325                 330                 335
   Trp Leu Gly Asp Met Thr Ile Tyr Thr Gly Ala Ile Asp Ser Tyr Arg
               340                 345                 350
   Pro Thr Ser Pro Gly Asp Arg Ile Gly Val Trp Tyr Gly Asn Ile Asn
           355                 360                 365
   Ala Phe Tyr His Thr Gly Arg Thr Asp Val Val Met Phe Arg Gln Thr
       370                 375                 380
   Gly Asp Thr Ala Tyr Glu Asp Pro Ser Thr Phe Ile Ser Asn Ile Leu
   385                 390                 395                 400
   Tyr Asp Asp Ile Tyr Lys Leu Asp Leu Arg Ala Ala Ala Val Ser Thr
                   405                 410                 415
   Ile Gln Gly Ala Met Asp Thr Thr Phe Gly Val Ser Ser Ser Arg Phe
               420                 425                 430
   Phe Asp Ile Arg Gly Arg Asn Gln Leu Tyr Gln Ser Asn Lys Pro Tyr
           435                 440                 445
   Pro Ser Leu Pro Ile Thr Ile Thr Phe Pro Gly Glu Glu Ser Ser Glu
       450                 455                 460
   Gly Asn Ala Asn Asp Tyr Ser His Leu Leu Cys Asp Val Lys Ile Leu
   465                 470                 475                 480
   Gln Glu Asp Ser Ser Asn Ile Cys Glu Gly Arg Ser Ser Leu Leu Ser
                   485                 490                 495
   His Ala Trp Thr His Ala Ser Leu Asp Arg Asn Asn Thr Ile Leu Pro
               500                 505                 510
   Asp Glu Ile Thr Gln Ile Pro Ala Val Thr Ala Tyr Glu Leu Arg Gly
           515                 520                 525
   Asn Ser Ser Val Val Ala Gly Pro Gly Ser Thr Gly Gly Asp Leu Val
       530                 535                 540
   Lys Met Ser Tyr His Ser Val Trp Ser Phe Lys Val Tyr Cys Ser Glu
   545                 550                 555                 560
   Leu Lys Asn Tyr Arg Val Arg Ile Arg Tyr Ala Ser His Gly Asn Cys
                   565                 570                 575
   Gln Phe Leu Met Lys Arg Trp Pro Ser Thr Gly Val Ala Pro Arg Gln
               580                 585                 590
   Trp Ala Arg His Asn Val Gln Gly Thr Phe Ser Asn Ser Met Arg Tyr
           595                 600                 605
   Glu Ala Phe Lys Tyr Leu Asp Ile Phe Thr Ile Thr Pro Glu Glu Asn
       610                 615                 620
   Asn Phe Ala Phe Thr Ile Asp Leu Glu Ser Gly Gly Asp Leu Phe Ile
   625                 630                 635                 640
   Asp Lys Ile Glu Phe Ile Pro Val Ser Gly Ser Ala Phe Glu Tyr Glu
                   645                 650                 655
   Gly Lys Gln Asn Ile Glu Lys Thr Gln Lys Ala Val Asn Asp Leu Phe
               660                 665                 670
   Ile Asn
<210> 48
   <211> 675
   <212> PRT
   <213> Bacillus thuringiensi
<400> 48
   Met Asn Pro Tyr Gln Asn Lys Ser Glu Cys Glu Ile Leu Asn Ala Pro
    1              5                  10                  15
   Leu Asn Asn Ile Asn Met Pro Asn Arg Tyr Pro Phe Ala Asn Asp Pro
               20                  25                  30
   Asn Ala Val Met Lys Asn Gly Asn Tyr Lys Asp Trp Leu Asn Glu Cys
           35                  40                  45
   Asp Gly Ile Thr Pro Ser Ile Phe Gly Thr Leu Gly Val Leu Ala Ser
       50                  55                  60
   Ile Val Ile Ser Thr Ile Asn Leu Ala Thr Ser Pro Ser Ile Gly Asp
   65                  70                  75                  80
   Ala Phe Ala Leu Val Ser Ser Ile Gly Glu Tyr Trp Pro Glu Thr Lys
                   85                  90                  95
   Thr Ser Phe Pro Leu Ser Val Ala Asp Val Asn Arg Leu Ile Arg Glu
               100                 105                 110
   Ala Leu Asp Gln Asn Ala Ile Asn Arg Ala Thr Gly Lys Phe Asn Gly
           115                 120                 125
   Leu Met Asp Thr Tyr Asn Thr Val Tyr Leu Lys Asn Leu Gln Asp Trp
       130                 135                 140
   Tyr Asp Thr Arg Ile Pro Ala Asn Pro Gln Gly Asp Ser Gln Leu Arg
   145                 150                 155                 160
   Glu Ala Ala Arg Arg Ser Leu Glu Glu Ile Glu Arg Asp Phe Arg Lys
                   165                 170                 175
   Ala Leu Ala Gly Glu Phe Ala Glu Ala Gly Ser Gln Ile Val Leu Leu
               180                 185                 190
   Pro Ile Tyr Ala Gln Ala Ala Asn Ile His Leu Leu Ile Leu Lys Asp
           195                 200                 205
   Ala Met Gln Phe Arg Thr Asp Leu Gly Leu Ile Arg Pro Val Gly Val
       210                 215                 220
   Pro Ile Thr Thr Ser Ala Glu Asp Pro Phe Glu Ser Glu Phe Leu Leu
   225                 230                 235                 240
   Arg Ile Lys Lys Tyr Thr Asp His Cys Ile Ser Tyr Tyr Asp Asp Gly
                   245                 250                 255
   Leu Ala Lys Ile Arg Ser Arg Gly Ser Asp Gly Glu Thr Trp Trp Glu
               260                 265                 270
   Phe Asn Lys Phe Arg Arg Glu Met Thr Leu Thr Val Leu Asp Leu Val
           275                 280                 285
   Ala Leu Tyr Pro Thr His Asn Ile Lys Leu Tyr Pro Ile Pro Thr Gln
       290                 295                 300
   Thr Glu Leu Ser Arg Val Val Tyr Thr Asp Pro Val Gly Cys Phe Gly
   305                 310                 315                 320
   Asn Arg Lys Ser Asp Ile Phe Ser Arg Leu Asn Phe Asp Tyr Leu Glu
                   325                 330                 335
   Asn Arg Leu Thr Arg Pro Arg Glu Pro Phe Asn Tyr Leu Asn Ser Val
               340                 345                 350
   Gln Leu Phe Ala Ser Thr Val Ser Asn Ser Asn Asn Gly Glu Val Leu
           355                 360                 365
   Arg Gly Asn Leu Asn Lys Ile Met Phe Glu Gly Gly Trp Thr Ala Ser
       370                 375                 380
   Arg Ser Gly Asp Gly Val Thr Thr Gly Thr Pro Phe Ser Thr Met Asp
   385                 390                 395                 400
   Trp Ser Tyr Gly Trp Gly Tyr Pro Arg Lys His Tyr Ala Glu Ile Thr
                   405                 410                 415
   Ser Arg Ser Gln Ala Leu Pro Gly Leu Asn Asn Ser Ile His Val Ile
               420                 425                 430
   Val Gly Ile Asp Ser Phe Arg Ala Ile Gly Pro Gly Gly Gln Gly Asp
           435                 440                 445
   His Thr Phe Ser Leu Pro Gly Gly Asp Met Tyr Asp Cys Gly Lys Val
       450                 455                 460
   Gln Ile Asn Pro Leu Glu Asp Tyr Arg Asn Ser Asp His Trp Ile Ser
   465                 470                 475                 480
   Asp Met Met Thr Ile Asn Gln Ser Val Gln Leu Ala Ser Asn Pro Thr
                   485                 490                 495
   Gln Thr Phe Ala Phe Ser Ala Leu Ser Leu Gly Trp His His Ser Ser
               500                 505                 510
   Ala Gly Asn Arg Asn Val Tyr Val Tyr Asp Lys Ile Thr Gln Ile Pro
           515                 520                 525
   Ala Thr Lys Thr Val Arg Glu His Pro Met Ile Lys Gly Pro Gly Phe
   530 535 540
   Thr Gly Gly Asp Leu Ala Asp Leu Ser Ser Asn Ser Asp Ile Leu Gln
   545 550 555 560
   Tyr Asp Leu Arg Ser Asp Tyr Asp Asp Arg Leu Thr Glu Asp Val Pro
   565 570 575
   Phe Arg Ile Arg Ile Arg Cys Ala Ser Ile Gly Val Ser Thr Ile Ser
   580 585 590
   Val Asp Asn Trp Gly Ser Ser Ser Pro Gln Val Thr Val Ala Ser Thr
   595 600 605
   Ala Ala Ser Leu Asp Thr Leu Lys Tyr Glu Ser Phe Gln Tyr Val Ser
   610 615 620
   Ile Pro Gly Asn Tyr Tyr Phe Asp Ser Ala Pro Arg Ile Arg Leu Leu
   625 630 635 640
   Arg Gln Pro Gly Arg Leu Leu Val Asp Arg Ile Glu Ile Ile Pro Val
   645 650 655
   Asn Phe Phe Pro Leu Ser Glu Gln Glu Asn Lys Ser Val Asp Ser Leu
   660 665 670
   Phe Ile Asn
   675
<210> 49
   <211> 659
   <212> PRT
   <213> Bacillus thuringiensi
<400> 49
   Asn Ser Tyr Glu Asn Lys Asn Glu Tyr Glu Ile Leu Asn Asp Ser Lys
    1               5                  10                  15
   Lys Ser Asn Met Ser Asn Pro Tyr Leu Arg Tyr Pro Leu Ala Asn Asp
               20                  25                  30
   Ser Leu Ala Ser Met Gln Asn Thr Asn Tyr Lys Asp Trp Leu Thr Met
           35                  40                  45
   Cys Asp Arg Thr Asp Thr Asp Val Leu Ser Ser Arg Gly Ala Val Ser
       50                  55                  60
   Thr Gly Val Gly Met Leu Ser Thr Ile Leu Ser Leu Phe Gly Ile Pro
   65                  70                  75                  80
   Leu Ile Gly Glu Gly Ile Asp Leu Leu Leu Gly Ala Ala Asp Phe Leu
                   85                  90                  95
   Trp Pro Glu Ser Asp Gly Gly His Gln Tyr Thr Trp Glu Asp Leu Met
               100                 105                 110
   Asn His Ile Glu Glu Leu Met Asp Glu Arg Leu Glu Thr Glu Lys Arg
           115                 120                 125
   Thr Thr Ala Leu Asp Asp Leu Arg Gly Leu Lys Ala Leu Leu Gly Leu
       130                 135                 140
   Phe Arg Asp Ala Phe Asp Ser Trp Glu Lys Asn Gln Asn Asp Pro Ile
   145                 150                 155                 160
   Ala Lys Asn Arg Val Gly Gly Tyr Phe Glu Asp Val His Thr His Phe
                   165                 170                 175
   Val Lys Asp Met Ala Ser Ile Phe Ser Ala Thr Asn Tyr Glu Val Leu
               180                 185                 190
   Leu Leu Pro Val Tyr Ala Gln Ala Ala Asn Leu His Leu Leu Leu Leu
           195                 200                 205
   Arg Glu Gly Val Ile Tyr Gly Ser Arg Trp Gly Ile Ala Pro Ala Ala
       210                 215                 220
   Asp Phe Tyr His Asp Gln Leu Leu Lys Tyr Thr Ala Ile Tyr Ala Asn
   225                 230                 235                 240
   His Cys Val Thr Trp Tyr Asn Asn Gly Leu Ala Gln Gln Lys Glu Leu
                   245                 250                 255
   Phe Ala Lys Ser Pro Asn Trp Asn Arg Phe Asn Ala Tyr Arg Arg Asp
               260                 265                 270
   Met Thr Ile Thr Val Leu Asp Ile Ile Ala Leu Phe Pro Thr Tyr Asp
           275                 280                 285
   Ala Arg Leu Tyr Thr Lys Pro Ile Lys Thr Glu Leu Thr Arg Glu Ile
       290                 295                 300
   Tyr Ser Asp Val Leu Asn Leu Asp Val Tyr Gly Val Gln Gln Thr Asp
   305                 310                 315                 320
   Leu Asn Lys Asn Glu Ala Ala Phe Thr Arg Ser Pro His Leu Val Thr
   325 330 335
   Arg Leu Arg Gly Phe Asp Phe Tyr Thr Arg Thr Lys Tyr Ala Tyr Trp
   340 345 350
   Arg Tyr Leu Ala Gly His Thr Asn Tyr Phe Ser Phe Thr Gly Asn Gly
   355 360 365
   Thr Ile Tyr Ser Ser Ser Phe Asn Asn Trp Tyr Asp Thr Asp Met Thr
   370 375 380
   Lys Ser Thr Ile Asn Ile Pro Asp Tyr Ala Asn Ile Tyr Lys Leu Trp
   385 390 395 400
   Thr Lys Ser Tyr Thr Asn Ile Ser Pro Tyr Thr Asp Pro Val Gly Ile
   405 410 415
   Ser Gln Met Gln Phe Ser Leu Thr Asn Asn Gln Gln Leu Thr Tyr Thr
   420 425 430
   Gly Thr Ser Ala Pro Lys Tyr Pro Val Arg Glu Thr Phe Phe Glu Ile
   435 440 445
   Pro Pro Thr Asp Glu Lys Pro Leu Thr Tyr Glu Asn Tyr Ser His Ile
   450 455 460
   Leu Ser Tyr Met Thr Ser Ala Gln His Phe Gly Asp Lys Lys Ile Gly
   465 470 475 480
   Tyr Thr Phe Ala Trp Met His Glu Ser Val Asp Phe Asp Asn Arg Val
   485 490 495
   Asp Pro Asp Lys Ile Thr Gln Ile Pro Ala Val Lys Gly Asp Tyr Leu
   500 505 510
   Gln Tyr Gly Tyr Val Lys Gln Gly Pro Gly His Thr Gly Gly Asp Leu
   515 520 525
   Val Ser Met Ile Arg Thr Asp Arg Leu Gly Ile Asn Val Tyr Phe Pro
   530 535 540
   Gln Pro Leu Asp Tyr Arg Ile Arg Ile Arg Tyr Ser Thr Ser Ser Asn
   545 550 555 560
   Gly Tyr Leu Tyr Ile Tyr Ser Pro Asn Thr Lys Ile Val Tyr Leu Pro
   565 570 575
   Pro Thr Thr Leu Val Asp Gly Gln Pro Thr Phe Asp Pro Met Asp Phe
   580 585 590
   Ser Ala Phe Arg Val Val Glu Val Pro Ala Ser Phe Arg Ala Ser Val
   595 600 605
   Ala Gly Tyr Thr Asn Phe Thr Ile Glu Ala Gly Phe Gly Pro Val Tyr
   610 615 620
   Ile Asp Lys Ile Glu Phe Ile Pro Asp Asn Thr Thr Thr Leu Glu Tyr
   625 630 635 640
   Glu Gly Gly Arg Asp Leu Glu Lys Thr Lys Asn Ala Val Asn Asp Leu
   645 650 655
   Phe Thr Asn
<210> 50
   <211> 558
   <212> PRT
   <213> Bacillus thuringiensi
<400> 50
   Met Phe Ile Ser Asn Ile Lys Asn Thr Leu Lys Ile Glu Thr Thr Asp
   1 5 10 15
   Tyr Glu Ile Asp Gln Ala Ala Ile Ser Ile Glu Cys Met Ser Asn Glu
   20 25 30
   His Ser Ser Lys Glu Glu Met Met Leu Trp Asp Glu Val Lys Gln Ala
   35 40 45
   Lys Gln Leu Ser Trp Ser Arg Asn Leu Leu Tyr Asn Gly Asp Phe Glu
   50 55 60
   Asp Val Ser Asn Gly Trp Lys Thr Ser Asn Thr Ile Glu Ile Arg Glu
   65 70 75 80
   Asn Ser Pro Val Phe Lys Gly His Tyr Leu His Met Phe Gly Ala Arg
   85 90 95
   Asp Ile Asp Gly Thr Leu Phe Pro Thr Tyr Ile Tyr Gln Lys Ile Glu
   100 105 110
   Glu Ser Lys Leu Lys Pro Tyr Thr Arg Tyr Arg Val Arg Gly Phe Val
   115 120 125
   Gly Ser Ser Lys Asp Leu Lys Leu Met Val Thr Arg Tyr Gly Lys Glu
           130                 135                 140
   Ile Asp Ala Met Met Asn Val Pro Asn Asp Leu Ala Tyr Met Gln Pro
   145                 150                 155                 160
   Thr Pro Ser Cys Gly Asp Ser Arg Cys Glu Ser Ser Ser Arg Tyr Val
                   165                 170                 175
   Ser Gln Gly Tyr Pro Thr Pro Val Thr Asp Gly Tyr Ala Ser Gly Arg
               180                 185                 190
   Tyr Ala Cys Gln Ser Asn Arg Gly Thr Lys His Val Lys Cys His Asp
           195                 200                 205
   Arg His Pro Phe Asp Phe His Ile Asp Thr Gly Glu Leu Asp Thr Asn
       210                 215                 220
   Thr Asn Val Gly Ile Asp Val Leu Phe Lys Ile Ser Asn Pro Asp Gly
   225                 230                 235                 240
   Tyr Ala Thr Leu Gly Asn Leu Glu Val Ile Glu Glu Gly Pro Leu Thr
                   245                 250                 255
   Gly Glu Ala Leu Thr His Val Lys Gln Lys Glu Lys Lys Trp Lys Gln
               260                 265                 270
   His Met Glu Lys Lys Arg Trp Glu Thr Gln Gln Ala Tyr Asp Pro Ala
           275                 280                 285
   Lys Gln Ala Val Asp Ala Leu Phe Thr Asn Glu Gln Glu Leu His Tyr
       290                 295                 300
   His Ile Thr Leu Asp His Ile Gln Asn Ala Asp Arg Leu Ile Gln Ala
   305                 310                 315                 320
   Ile Pro Tyr Val Tyr His Ala Trp Leu Pro Asp Ala Pro Gly Met Asn
                   325                 330                 335
   Tyr Asp Gly Tyr Gln Gly Leu Asn Ala Arg Ile Met Gln Ala Tyr Asn
               340                 345                 350
   Leu Tyr Asp Ala Arg Asn Val Ile Thr Asn Gly Asp Phe Thr Gln Gly
           355                 360                 365
   Leu Thr Gly Trp His Ala Ala Gly Lys Ala Met Val Gln Gln Met Asp
       370                 375                 380
   Gly Ala Ser Val Leu Val Leu Ser Asn Trp Ser Ala Gly Val Ser Gln
   385                 390                 395                 400
   Asn Leu His Val Gln Glu His His Gly Tyr Met Leu Arg Val Ile Ala
                   405                 410                 415
   Lys Lys Glu Gly Pro Gly Lys Gly Tyr Val Thr Met Met Asp Cys Asn
               420                 425                 430
   Gly Asn Arg Glu Thr Leu Lys Phe Thr Ser Cys Glu Glu Gly Tyr Met
           435                 440                 445
   Thr Lys Thr Val Glu Val Phe Pro Glu Ser Asp Arg Val Arg Ile Glu
       450                 455                 460
   Ile Gly Glu Thr Glu Gly Thr Phe Tyr Val Asp Ser Ile Glu Leu Leu
   465                 470                 475                 480
   Cys Met Gln Gly Tyr Ala Ser Asn Asn Asn Pro His Thr Gly Asn Met
                   485                 490                 495
   Tyr Gly Gln Ser Tyr Asn Gly Asn Tyr Asn Gln Asn Thr Ser Asp Val
               500                 505                 510
   Tyr His Gln Gly Tyr Thr Asn Asn Tyr Asn Gln Asn Ser Ser Asn Met
           515                 520                 525
   Tyr Asn Gln Asn Tyr Thr His Asn Asp Asp Leu His Ser Gly Cys Thr
       530                 535                 540
   Cys Asn Gln Gly His Asn Ser Gly Cys Thr Cys Ser Gln Gly
   545                 550                 555
<210> 51
   <211> 666
   <212> PRT
   <213> Bacillus thuringiensi
<400> 51
   Asn Ser Tyr Glu Asn Lys Asn Glu Tyr Glu Ile Leu Glu Ser Ser Ser
    1              5                   10                  15
   Asn Asn Thr Asn Met Pro Asn Arg Tyr Pro Phe Ala Asn Asp Arg Asp
               20                  25                  30
   Met Ser Thr Met Ser Phe Asn Asp Cys Gln Gly Ile Ser Trp Asp Glu
           35                  40                  45
   Ile Trp Glu Ser Ala Glu Thr Ile Thr Ser Ile Gly Ile Asp Leu Ile
           50                  55                  60
   Glu Phe Leu Met Glu Pro Ser Leu Gly Gly Ile Asn Thr Leu Phe Ser
   65                  70                  75                  80
   Ile Ile Gly Lys Leu Ile Pro Thr Asn His Gln Ser Val Ser Ala Leu
                   85                  90                  95
   Ser Ile Cys Asp Leu Leu Ser Ile Ile Arg Lys Glu Val Ala Asp Ser
               100                 105                 110
   Val Leu Ser Asp Ala Ile Cys Arg Phe Leu Asp Gly Lys Leu Lys Asn
           115                 120                 125
   Tyr Arg Glu Tyr Tyr Leu Pro Tyr Leu Glu Ala Trp Leu Lys Asp Gly
       130                 135                 140
   Lys Pro Leu Gln Lys Thr Asn Asn Ser Asp Ile Gly Gln Leu Val Lys
   145                 150                 155                 160
   Tyr Phe Glu Leu Ser Glu Arg Asp Phe Asn Glu Ile Leu Gly Gly Ser
                   165                 170                 175
   Leu Ala Arg Asn Asn Ala Gln Ile Leu Leu Leu Pro Tyr Phe Cys Ala
               180                 185                 190
   Ser Cys Lys Cys Gln Leu Leu Leu Leu Arg Asp Ala Val Gln Tyr Glu
           195                 200                 205
   Glu Gln Trp Phe Pro Phe Leu Ser Ala Glu Asn Val Arg Ser Glu Leu
       210                 215                 220
   Ile Ser Pro Asn Ser Gly Cys Asp Phe Thr Gly Asp Tyr Tyr Glu Arg
   225                 230                 235                 240
   Leu Lys Cys Lys Ile Ala Glu Tyr Thr Asp Tyr Cys Glu Tyr Trp Tyr
                   245                 250                 255
   Gln Ala Gly Leu Asn Gln Ile Lys Gln Ala Gly Thr Gly Ala Asp Thr
               260                 265                 270
   Trp Ala Lys Phe Asn Lys Phe Arg Arg Glu Met Thr Leu Thr Val Leu
           275                 280                 285
   Asp Ile Ile Ala Ile Phe Gln Thr Tyr Asp Phe Lys Lys Tyr Pro Leu
       290                 295                 300
   Pro Thr His Val Glu Leu Thr Arg Glu Ile Tyr Thr Asp Pro Val Gly
   305                 310                 315                 320
   Tyr Ser Ser Gly Thr Tyr Ser Trp Leu Lys Tyr Trp Thr Gly Ala Phe
                   325                 330                 335
   Asn Thr Leu Glu Ala Asn Gly Thr Arg Gly Pro Gly Leu Val Thr Trp
               340                 345                 350
   Leu Arg Ser Ile Gly Ile Tyr Asn Glu Tyr Val Ser Arg Tyr Phe Ser
           355                 360                 365
   Gly Trp Val Gly Thr Arg His Tyr Glu Asp Tyr Thr Thr Gly Asn Gly
       370                 375                 380
   Asn Phe Gln Arg Met Ser Gly Thr Thr Ser Asn Asp Leu Arg Asp Ile
   385                 390                 395                 400
   Ser Phe Pro Asn Ser Asp Ile Phe Lys Ile Glu Ser Lys Ala Ile Met
                   405                 410                 415
   Asn Leu Val Gly Glu Ile Asn Ala Arg Pro Glu Tyr Arg Val Ser Arg
               420                 425                 430
   Ala Glu Phe Ser Glu Ser Thr Ala Phe Ile Tyr Leu Tyr Asp Ala Gly
           435                 440                 445
   Asn Ser Gly Leu Ser Ser Met Thr Ile Thr Ser Lys Leu Pro Gly Ile
       450                 455                 460
   Lys Asn Pro Glu Pro Ser Tyr Arg Asp Tyr Ser His Arg Leu Ser Asn
   465                 470                 475                 480
   Ala Ala Cys Val Gly Ala Gly Asn Ser Arg Ile Asn Val Tyr Gly Trp
                   485                 490                 495
   Thr His Thr Ser Met Ser Lys Tyr Asn Leu Ile Tyr Pro Asp Lys Ile
               500                 505                 510
   Thr Gln Ile Pro Ala Val Lys Ala Phe Asp Ile Ser Asp Thr Gly Pro
           515                 520                 525
   Gly Gln Val Ile Ala Gly Pro Gly His Thr Gly Gly Asn Val Val Ser
       530                 535                 540
   Leu Pro Tyr Tyr Ser Arg Leu Lys Ile Arg Leu Ile Pro Ala Ser Thr
   545                 550                 555                 560
   Asn Lys Asn Tyr Leu Val Arg Val Arg Tyr Thr Ser Thr Ser Asn Gly
                   565                 570                 575
   Arg Leu Leu Val Glu Arg Trp Ser Pro Ser Ser Ile Ile Asn Ser Tyr
               580                 585                 590
   Phe Phe Leu Pro Ser Thr Gly Pro Gly Asp Ser Phe Gly Tyr Val Asp
                       595                     600                     605
   Thr Leu Val Thr Thr Phe Asn Gln Pro Gly Val Glu Ile Ile Ile Gln
           610                     615                     620
   Asn Leu Asp Thr Pro Ile Asn Val Asp Lys Val Glu Phe Ile Pro Val
   625                     630                     635                     640
   Asn Ser Thr Ala Leu Glu Tyr Glu Gly Lys Gln Ser Leu Glu Lys Ala
                   645                     650                     655
   Gln Asp Val Val Asn Asp Leu Phe Val Lys
               660                     665
<210> 52
   <211> 558
   <212> PRT
   <213> Bacillus thuringiensi
<400> 52
   Met Phe Thr Asn Gly Thr Lys Asn Thr Leu Lys Ile Glu Thr Thr Asp
   1               5                   10                  15
   Tyr Glu Ile Asp Gln Ala Ala Ile Ser Ile Glu Cys Met Ser Asp Glu
               20                  25                  30
   His Ser Pro Lys Glu Lys Met Met Leu Trp Asp Glu Val Lys Arg Ala
           35                  40                  45
   Lys Leu Leu Ser Gln Ser Arg Asn Leu Leu Gln Asn Gly Asp Phe Gly
       50                  55                  60
   Asp Phe Tyr Gly Asn Asp Trp Lys Phe Gly Asn Asn Ile Ile Ile Gly
   65                  70                  75                  80
   Ser Asn Asn Ser Ile Phe Lys Gly Asn Phe Leu Gln Met Ser Gly Ala
                   85                  90                  95
   Arg Asp Ile Tyr Gly Thr Ile Phe Pro Thr Tyr Ile Tyr Gln Lys Ile
               100                 105                 110
   Asp Glu Ser Lys Leu Lys Pro Tyr Thr Arg Tyr Arg Val Arg Gly Phe
           115                 120                 125
   Val Gly Ser Ser Lys Asp Leu Arg Leu Met Val Thr Arg Tyr Gly Lys
       130                 135                 140
   Glu Ile Asp Ala Met Met Asn Val Pro Asn Asp Leu Ala Tyr Met Gln
   145                 150                 155                 160
   Pro Asn Pro Ser Cys Gly Asp Ser Arg Cys Glu Ser Ser Ser Gln Tyr
                   165                 170                 175
   Val Ser Gln Gly Tyr Pro Thr Pro Thr Asp Gly Tyr Ala Pro Asp Arg
               180                 185                 190
   Tyr Ala Cys Pro Ser Ser Ser Asp Lys Lys His Val Met Cys His Asp
           195                 200                 205
   Arg His Pro Phe Asp Phe His Ile Asp Thr Gly Glu Leu Asp Thr Asn
       210                 215                 220
   Thr Asn Val Gly Ile Asp Val Leu Phe Lys Ile Ser Asn Pro Asp Gly
   225                 230                 235                 240
   Tyr Ala Thr Leu Gly Asn Leu Glu Val Ile Glu Glu Gly Pro Leu Thr
                   245                 250                 255
   Gly Glu Ala Leu Thr His Val Lys Gln Lys Glu Lys Lys Trp Lys Gln
               260                 265                 270
   His Met Glu Lys Lys Arg Trp Glu Thr Gln Gln Ala Tyr Asp Pro Ala
           275                 280                 285
   Lys Gln Ala Val Asp Thr Leu Phe Thr Asn Glu Gln Glu Leu His Tyr
       290                 295                 300
   His Ile Thr Leu Asp Tyr Ile Gln Thr Leu Ile Asp Trp Tyr Ser Arg
   305                 310                 315                 320
   Phe Pro Ile Tyr Thr Met Thr Gly Tyr Arg Asp Ala Pro Gly Met Asn
                   325                 330                 335
   Tyr Asp Gly Tyr Gln Gly Leu Asn Ala Arg Ile Met Gln Ala Tyr Asn
               340                 345                 350
   Leu Tyr Asp Ala Arg Asn Val Ile Thr Asn Gly Asp Phe Thr Lys Gly
           355                 360                 365
   Leu Gln Gly Trp His Ala Ala Gly Lys Ala Ala Val Gln Gln Ile Asp
       370                 375                 380
   Gly Ala Ser Val Leu Val Leu Ser Asn Trp Ser Ala Gly Val Ser Gln
   385                 390                 395                 400
   Asn Leu His Ala Gln Asp His His Gly Tyr Met Leu Arg Val Ile Ala
                           405                         410                         415
   Lys Lys Glu Gly Pro Gly Lys Gly Tyr Val Thr Met Met Asp Cys Asn
                   420                         425                         430
   Gly Asn Gln Glu Thr Leu Lys Phe Thr Ser Cys Glu Glu Gly Tyr Met
               435                         440                         445
   Thr Lys Thr Val Glu Val Phe Pro Glu Ser Asp Arg Val Arg Ile Glu
       450                         455                         460
   Ile Gly Glu Thr Glu Gly Thr Phe Tyr Val Asp Ser Ile Glu Leu Leu
   465                         470                         475                         480
   Cys Met Gln Gly Tyr Ala Ser Asn Asn Asn Pro His Thr Gly Asn Met
                           485                         490                         495
   Tyr Gly Gln Ser Tyr Asn Gly Asn Tyr Asn Gln Asn Thr Ser Asp Val
                   500                         505                         510
   Tyr His Gln Gly Tyr Thr Asn Asn Tyr Asn Gln Asn Ser Ser Asn Met
               515                         520                         525
   Tyr Asn Gln Asn Tyr Thr His Asn Asp Asp Leu His Ser Gly Cys Thr
       530                         535                         540
   Cys Asn Gln Gly His Asn Ser Gly Cys Thr Cys Ser Gln Gly
   545                         550                         555